# EUROPEAN PATENT SPECIFICATION

(11) **EP 3 458 448 B1**
(45) Date of publication and mention of the grant of the patent: **23.06.2021**
(21) Application number: 17790287.1
(22) Date of filing: 25.04.2017
(51) Int. Cl.: A61K 31/496, A61K 31/495, A61P 1/16, C07D 401/04, C07D 403/04, C07D 471/04

(54) **FASN INHIBITORS FOR USE IN TREATING NON-ALCOHOLIC STEATOHEPATITIS**
FASN-HEMMER ZUR BEHANDLUNG DER NICHT-ALKOHOLISCHEN STEATOHEPATITIS
INHIBITEURS DE FASN POUR UTILISATION DANS LE TRAITEMENT DE LA STÉATOHÉPATITE NON ALCOOLIQUE

(30) Priority: 25.04.2016 US 201662327167 P
(43) Date of publication of application: 27.03.2019
(73) Proprietor: Forma Therapeutics, Inc., Watertown, MA 02472 (US)
(72) Inventor: MILLAN, David S., Watertown, MA 02472 (US); LU, Wei, Watertown, MA 02472 (US); BATTULA, Sailaja, Watertown, MA 02472 (US); RADFAR, Stephane Soroosh, Watertown, MA 02472 (US); TAN, Seng-Lai, Watertown, MA 02472 (US)
(74) Representative: Oates, Edward Christopher
(86) International application number: PCT/US2017/029469
(87) International publication number: WO 2017/189613

(56) References cited:
- WO-A1-2014/146747
- WO-A1-2014/164749
- WO-A2-2008/106563
- US-A1- 2015 166 529
- US-A1- 2016 002 188
- US-A1- 2016 002 188
- US-A1- 2016 009 688
- HARRISON, SA ET AL.: 'A pilot study of orlistat treatment in obese, non-alcoholic steatohepatitis patients' ALIMENTARY PHARMACOLOGY & THERAPEUTICS vol. 20, no. 6, 01 September 2004, pages 623 - 628, XP055437073

## Description

The present invention relates to a FASN inhibitor for use in a method of treating non-alcoholic steatohepatitis (NASH), whereby the FASN inhibitor reduces steatosis, TH17-dependent inflammation, and fibrosis, as defined in claim 1.

### Background

It has been reported that NAFL to NASH progression in human patients is correlated with a significant increase in frequency of T_{H}17 cells in the liver, Rau, M., et al., J. Immunol. 2016; 196(1):97-105. In a mouse model of NAFL, obesity-driven production of IL-17, which is produced by T_{H}17 cells, has been found to be central to the development and progression of NAFL to steatohepatitis.

It is known that IL-17 receptor activation induces secretion of pro-inflammatory cytokines and IL-17 induces type I collagen production in hepatic stellate cells (Tan, Z., et al., J Immunol. 2013; 191(4):1835-44). Thus, inhibition or neutralization of IL-17 is expected to impact IL-17-mediated inflammation and fibrosis (Tan, Z., J Immunol. 1835-44).

M2-like macrophages differentiated with CSF-1 can acquire pro-fibrogenic and immunosuppressive properties. M2-like macrophages and CSF-1 signal transduction are implicated in fibrosis and in evasion of anti-tumor response in the tumor microenvironment.

Thus, there is a need for new inhibitors of T_{H}17 cells and M2 macrophages for the treatment of T_{H}17- and CSF1-driven disorders.
Imidazolin-5-one derivatives, pharmaceutical compositions containing them, and their uses as FASN inhibitors are described in US 2015/166529 A1.
Benzaminde derivatives, pharmaceutical compositions containing them, and their use as FASN inhibitors are described in US 2016/009688 A1.

### Summary

It has been surprisingly and unexpectedly observed that when human CD4+ T cells are cultured under T_{H}17 polarizing conditions in the presence of FASN inhibitors, production of IL-17 is significantly inhibited.

The invention is defined by the claims. Any subject matter falling outside the scope of the claims is provided for information purposes only. Any references in the description to methods of treatment refer to the compounds, pharmaceutical compositions and medicaments of the invention for use in a method for treatment of the human or animal body by therapy Aspects of the disclosure are not part of the claimed invention. The present invention provides a FASN inhibitor for use in a method of treating non-alcoholic steatohepatitis (NASH), whereby the FASN inhibitor reduces steatosis, TH17-dependent inflammation, and fibrosis, wherein said method comprises administering said FASN inhibitor to said patient, and wherein the FASN inhibitor is: 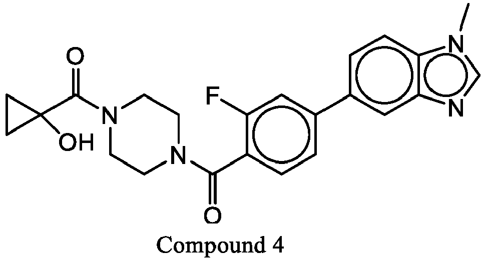 or a pharmaceutically acceptable salt thereof.
A first aspect of the disclosure relates to a method of treating a condition or disease associated with T_{H}17 mediated inflammation. The method comprises administering to a patient in need of a treatment for condition or a disease associated with T_{H}17 mediated inflammation an effective amount of a FASN inhibitor.

Another aspect of the present disclosure relates to a method of treating a condition or disease associated with IL-17 receptor activation. The method comprises administering to a patient in need of a treatment for condition or a disease associated with IL-17 receptor activation an effective amount of a FASN inhibitor.

Another aspect of the present disclosure relates to a method of treating a patient of organ transplantation to reduce organ rejection. The method comprises administering to a patient in need of a treatment for reducing organ rejection an effective amount of a FASN inhibitor.

Another aspect of the present disclosure relates to a method of inhibiting CSF-1 dependent macrophage differentiation. The method comprises contacting a cell with an effective amount of a FASN inhibitor.

Another aspect of the present disclosure relates to a method of treating a condition or disease associated with CSF-1 dependent macrophage differentiation. The method comprises administering to a patient in need a treatment for condition or a disease associated with CSF-1 dependent macrophage differentiation an effective amount of a FASN inhibitor.

### Brief Description of the Drawings

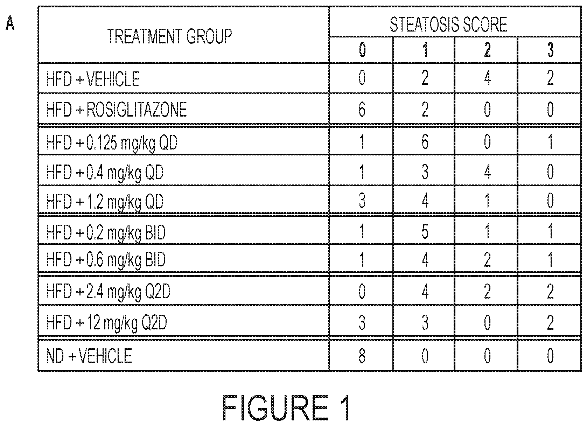

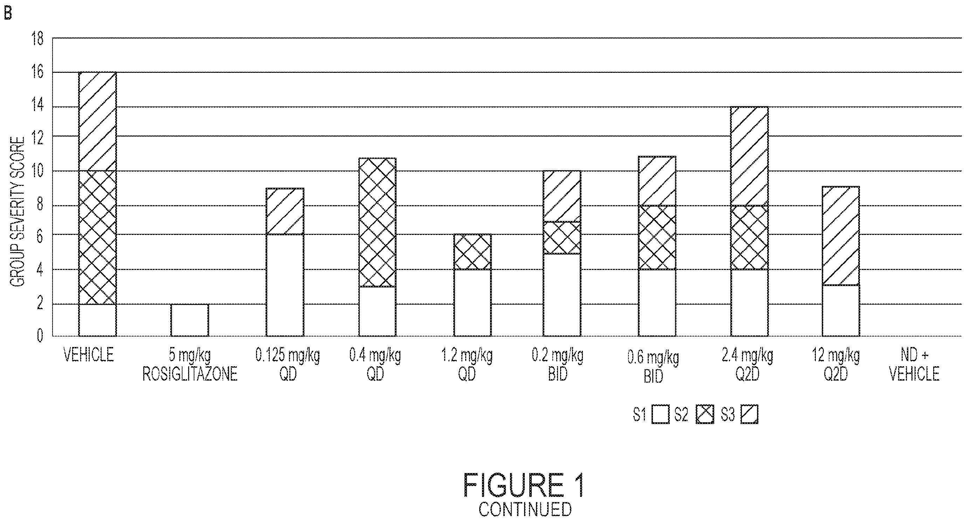

**Figure 1****:** Compound A reduced steatosis score of liver from HFD rat model. **(A):** The hematoxylin and eosin stained liver slides were evaluated for steatosis store by Kleiner scoring system (Kleiner, D.E., Brunt, E.M., Van Natta, M., Behling, C., Contos, M.J., Cummings, O.W., Ferrell, L.D., Liu, Y.-C., Torbenson, M.S., Unalp-Arida, A., et al. (2005). Design and validation of a histological scoring system for nonalcoholic fatty liver disease. Hepatol. Baltim. Md 41, 1313-1321). Steatosis scores were assessed by medium-power evaluation of parenchymal involvement by steatosis. Severity of steatosis was defined as follows: S0, no steatosis, <5% parenchymal involvement; S1, mild steatosis, 5-33% (bottom section of bar); S2, moderate steatosis, >33-66% (middle section of bar); S3, severe steatosis, >66% (top section of bar). **(B):** The group severity score was calculated to assist in the evaluation of the steatosis severity in each group. This was calculated by multiplying steatosis score by number of animals with that score and then adding them for the total score. For example, there are 2 of score 1, 4 of score 2 and 2 of score 3 in the vehicle treated group. The group severity score from the vehicle group is 2X1+4X2+2X3=16. The group severity score contribution from steatosis score 1, 2, 3 is marked in the bottom, middle, and top sections of each bar, respectively.
**Figure 2****:** Effect of compound A on the improvement of disease severity in EAE mouse model from BID **(A)** or QD **(B)** administration. The data were presented as Mean ± SEM. The clinical score criteria are listed in Table 6.1.
**Figure 3****:** Compound A reduced gene expression level of mRORc in splenic cells from EAE model. The data are presented as Mean ± SD. Compound A inhibited splenic mRORc by 45%, 62% and 81% for 1, 3, and 10 mg/kg BID groups as compared to the vehicle treated group.
**Figure 4****:** Compound A reduced the population of IL17+ CD4+ cells in lymph node **(A)** and spinal cord and brain tissue samples in the second EAE study **(B).** The data were presented as Mean ± SD. * P<0.05 ** P<0.01 as compared to vehicle control.

### Definitions

Compounds of this disclosure include those described generally above, and are further illustrated by the classes, subclasses, and species disclosed herein. As used herein, the following definitions shall apply unless otherwise indicated. For purposes of this invention, the chemical elements are identified in accordance with the Periodic Table of the Elements, CAS version, Handbook of Chemistry and Physics, 75th Ed. Additionally, general principles of organic chemistry are described in "Organic Chemistry", Thomas Sorrell, University Science Books, Sausalito: 1999, and "March's Advanced Organic Chemistry", 5th Ed., Ed.: Smith, M.B. and March, J., John Wiley & Sons, New York: 2001.

The abbreviations used herein have their conventional meaning within the chemical and biological arts. The chemical structures set forth herein are constructed according to the standard rules of chemical valency known in the chemical arts.

As used above, and throughout this disclosure, the following terms, unless otherwise indicated, shall be understood to have the following meanings. If a definition is missing, convention definition as known to one skilled in the art controls.

The term "about", when used herein in reference to a value, refers to a value that is similar, in context to the referenced value. In general, those skilled in the art, familiar with the context, will appreciate the relevant degree of variance encompassed by "about" in that context. For example, in some embodiments, the term "about" may encompass a range of values that within 25%, 20%, 19%, 18%, 17%, 16%, 15%, 14%, 13%, 12%, 11%, 10%, 9%, 8%, 7%, 6%, 5%, 4%, 3%, 2%, 1%, or less of the referred value.

As used herein, the term "administration" typically refers to the administration of a composition or compound to a subject or system. Those of ordinary skill in the art will be aware of a variety of routes that may, in appropriate circumstances, be utilized for administration to a subject, for example a human. For example, in some embodiments, administration may be ocular, oral, parenteral, topical, etc.. In some particular embodiments, administration may be bronchial (e.g., by bronchial instillation), buccal, dermal (which may be or comprise, for example, one or more of topical to the dermis, intradermal, interdermal, transdermal, etc.), enteral, intra-arterial, intradermal, intragastric, intramedullary, intramuscular, intranasal, intraperitoneal, intrathecal, intravenous, intraventricular, within a specific organ (e. g. intrahepatic), mucosal, nasal, oral, rectal, subcutaneous, sublingual, topical, tracheal (e.g., by intratracheal instillation), vaginal, vitreal, etc. In some embodiments, administration may involve dosing that is intermittent (e.g., a plurality of doses separated in time) and/or periodic (e.g., individual doses separated by a common period of time) dosing. In some embodiments, administration may involve continuous dosing (e.g., perfusion) for at least a selected period of time. In some embodiments, administration of a particular compound may be achieved by administration of a composition that includes or otherwise delivers the compound to the subject or system (or to a relevant part thereof or site therein). Thus, in some embodiments, administration of a compound may be achieved by administration of a composition comprising the compound.

As used herein, the term "patient" or "subject" refers to any organism to which provided compound or compounds described herein are administered in accordance with the present disclosure e.g., for experimental, diagnostic, prophylactic, and/or therapeutic purposes, and includes mammals and non-mammals. Examples of mammals include any member of the mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs. Examples of non-mammals include birds, and fish. In one embodiment of the present invention, the mammal is a human. In some aspects of the disclosure, a subject may be suffering from, and/or susceptible to a disease, disorder, and/or condition.

The term "FASN" refers all classes, types, subtypes, isotypes, segments, variants, and mutant forms of fatty acid synthase.

As used herein, the term "inhibitor" refers to an agent, condition, or event whose presence, level, degree, type, or form correlates with decreased level or activity of another agent (i.e., the inhibited agent, or target). In general, an inhibitor may be or include an agent of any chemical class including, for example, small molecules, polypeptides, nucleic acids, carbohydrates, lipids, metals, and/or any other entity, condition or event that shows the relevant inhibitory activity. In some aspects of the disclosure an inhibitor may be direct (in which case it exerts its influence directly upon its target, for example by binding to the target); in some aspects of the disclosure an inhibitor may be indirect (in which case it exerts its influence by interacting with and/or otherwise altering a regulator of the target, so that level and/or activity of the target is reduced).

The terms "effective amount" or "therapeutically effective amount" refer to a sufficient amount of the agent to provide the desired biological result. That result can be reduction and/or alleviation of the signs, symptoms, or causes of a disease, or any other desired alteration of a biological system. For example, an "effective amount" for therapeutic use is the amount of the composition comprising a compound as disclosed herein required to provide a clinically significant decrease in a disease, disorder, and/or condition. An appropriate "effective" amount in any individual case may be determined by one of ordinary skill in the art using routine experimentation. Thus, the expression "effective amount" generally refers to the quantity for which the active substance has therapeutic effects.

"Mammal" means humans and other mammalian animals.

As used herein, the term "prevent" or "prevention," when used in connection with the occurrence of a disease, disorder, and/or condition, refers to reducing the risk of developing the disease, disorder, and/or condition and/or to delaying onset of one or more characteristics or symptoms of the disease, disorder, or condition. Prevention may be considered complete when onset of a disease, disorder, or condition has been delayed for a predefined period of time.

As used herein, the terms "treat" or "treatment" are synonymous with the term "prevent" and are meant to indicate a postponement of development of diseases, preventing the development of diseases, and/or reducing severity of such symptoms that will or are expected to develop. Thus, these terms include ameliorating existing disease symptoms, preventing additional symptoms, ameliorating or preventing the underlying metabolic causes of symptoms, inhibiting the disorder or disease, e.g., arresting the development of the disorder or disease, relieving the disorder or disease, causing regression of the disorder or disease, relieving a condition caused by the disease or disorder, or stopping or alleviating the symptoms of the disease or disorder.

By "pharmaceutically acceptable is meant a material which is not biologically or otherwise undesirable-the material may be administered to an individual without causing any undesirable biological effects or interacting in a deleterious manner with any of the components of the composition in which it is contained.

"Carrier materials" or what are also referred to as "excipients" include any commonly used excipients in pharmaceutics and should be selected on the basis of compatibility and the release profile properties of the desired dosage form. Exemplary carrier materials include, e.g., binders, suspending agents, disintegration agents, filling agents, surfactants, solubilizers, stabilizers, lubricants, wetting agents, and diluents. "Pharmaceutically compatible carrier materials" may comprise, e.g., acacia, gelatin, colloidal silicon dioxide, calcium glycerophosphate, calcium lactate, maltodextrin, glycerine, magnesium silicate, sodium caseinate, soy lecithin, sodium chloride, tricalcium phosphate, dipotassium phosphate, sodium stearoyl lactylate, carrageenan, monoglyceride, diglyceride, or pregelatinized starch. See, e.g., Hoover, John E., Remington's Pharmaceutical Sciences, Mack Publishing Co., Easton, Pa. 1975.

The term "aliphatic" or "aliphatic group", as used herein, means a straight-chain (i.e., unbranched) or branched, substituted or unsubstituted hydrocarbon chain that is completely saturated or that contains one or more units of unsaturation, or a monocyclic hydrocarbon or bicyclic hydrocarbon that is completely saturated or that contains one or more units of unsaturation, but which is not aromatic (also referred to herein as "carbocyclyl," "cycloaliphatic" or "cycloalkyl"), that has a single point of attachment to the rest of the molecule. Unless otherwise specified, aliphatic groups contain 1-6 aliphatic carbon atoms. In some embodiments, aliphatic groups contain 1-5 aliphatic carbon atoms. In some embodiments, aliphatic groups contain 1-4 aliphatic carbon atoms. In some embodiments, aliphatic groups contain 1-3 aliphatic carbon atoms, and in yet other embodiments, aliphatic groups contain 1-2 aliphatic carbon atoms.

The term "heteroatom" means one or more of oxygen, sulfur, nitrogen, phosphorus, or silicon (including, any oxidized form of nitrogen, sulfur, phosphorus, or silicon; the quaternized form of any basic nitrogen or; a substitutable nitrogen of a heterocyclic ring, for example N (as in 3,4-dihydro-2*H*-pyrrolyl), NH (as in pyrrolidinyl) or NR⁺ (as in N-substituted pyrrolidinyl)).

The term "unsaturated," as used herein, means that a moiety has one or more units of unsaturation.

It should also be noted that any carbon as well as heteroatom with unsatisfied valences in the text, schemes, examples and Tables herein is assumed to have the sufficient number of hydrogen atom(s) to satisfy the valences.

As used herein, the term "composition" is intended to encompass a product comprising the specified ingredients in the specified amounts, as well as any product which results, directly or indirectly, from combination of the specified ingredients in the specified amounts.

The term "deuterium" as used herein means a stable isotope of hydrogen having odd numbers of protons and neutrons.

As used herein, the term "partially unsaturated" refers to a ring moiety that includes at least one double or triple bond. The term "partially unsaturated" is intended to encompass rings having multiple sites of unsaturation, but is not intended to include aryl or heteroaryl moieties, as herein defined.

As used herein, the term "pharmaceutically acceptable salt" refers to those salts which are, within the scope of sound medical judgment, suitable for use in contact with the tissues of humans and lower animals without undue toxicity, irritation, or allergic response, and are commensurate with a reasonable benefit/risk ratio. Pharmaceutically acceptable salts are well known in the art. For example, S. M. Berge et al., describe pharmaceutically acceptable salts in detail in J. Pharmaceutical Sciences, 1977, 66, 1-19.

Numerical ranges, as used herein, are intended to include sequential integers. For example, a range expressed as "from 0 to 4" would include 0, 1, 2, 3 and 4.

It must be noted that, as used in the specification and the appended claims, the singular forms "a," "an" and "the" include plural referents unless the context clearly dictates otherwise.

When used as a therapeutic agent the inhibitors of the FASN (FASN) described herein may be administered with one or more physiologically acceptable excipients. A physiologically acceptable carrier or excipient is a formulation to which the compound can be added to dissolve it or otherwise facilitate its administration.

The dosage forms of the present invention, may contain a mixture of one or more compounds of this invention, and may include additional materials known to those skilled in the art as pharmaceutical excipients. Stabilizing additives may be incorporated into the delivery agent solution. With some drugs, the presence of such additives promotes the stability and dispersibility of the agent in solution. The stabilizing additives may be employed at a concentration ranging from about 0.1 and 5% (W/V), preferably about 0.5% (W/V). Suitable, but non-limiting, examples of stabilizing additives include gum acacia, gelatin, methyl cellulose, polyethylene glycol, carboxylic acids and salts thereof, and polylysine. The preferred stabilizing additives are gum acacia, gelatin and methyl cellulose.

Acidifying agents (acetic acid, glacial acetic acid, citric acid, fumaric acid, hydrochloric acid, diluted hydrochloric acid, malic acid, nitric acid, phosphoric acid, diluted phosphoric acid, sulfuric acid, tartaric acid); Aerosol propellants (butane, dichlorodifluoro-methane, dichlorotetrafluoroethane, isobutane, propane, trichloromonofluoromethane); Air displacements (carbon dioxide, nitrogen); Alcohol denaturants (denatonium benzoate, methyl isobutyl ketone, sucrose octacetate); Alkalizing agents (strong ammonia solution, ammonium carbonate, diethanolamine, diisopropanolamine, potassium hydroxide, sodium bicarbonate, sodium borate, sodium carbonate, sodium hydroxide, trolamine); Anticaking agents (see *glidant*); Antifoaming agents (dimethicone, simethicone); Antimicrobial preservatives (benzalkonium chloride, benzalkonium chloride solution, benzelthonium chloride, benzoic acid, benzyl alcohol, butylparaben, cetylpyridinium chloride, chlorobutanol, chlorocresol, cresol, dehydroacetic acid, ethylparaben, methylparaben, methylparaben sodium, phenol, phenylethyl alcohol, phenylmercuric acetate, phenylmercuric nitrate, potassium benzoate, potassium sorbate, propylparaben, propylparaben sodium, sodium benzoate, sodium dehydroacetate, sodium propionate, sorbic acid, thimerosal, thymol); Antioxidants (ascorbic acid, acorbyl palmitate, butylated hydroxyanisole, butylated hydroxytoluene, hypophosphorous acid, monothioglycerol, propyl gallate, sodium formaldehyde sulfoxylate, sodium metabisulfite, sodium thiosulfate, sulfur dioxide, tocopherol, tocopherols excipient); Buffering agents (acetic acid, ammonium carbonate, ammonium phosphate, boric acid, citric acid, lactic acid, phosphoric acid, potassium citrate, potassium metaphosphate, potassium phosphate monobasic, sodium acetate, sodium citrate, sodium lactate solution, dibasic sodium phosphate, monobasic sodium phosphate); Capsule lubricants (see *tablet and capsule lubricant*); Chelating agents (edetate disodium, ethylenediaminetetraacetic acid and salts, edetic acid); Coating agents (sodium carboxymethylcellulose, cellulose acetate, cellulose acetate phthalate, ethylcellulose, gelatin, pharmaceutical glaze, hydroxypropyl cellulose, hydroxypropyl methylcellulose, hydroxypropyl methylcellulose phthalate, methacrylic acid copolymer, methylcellulose, polyethylene glycol, polyvinyl acetate phthalate, shellac, sucrose, titanium dioxide, carnauba wax, microcystalline wax, zein); Colorants (caramel, red, yellow, black or blends, ferric oxide); Complexing agents (ethylenediaminetetraacetic acid and salts (EDTA), edetic acid, gentisic acid ethanolmaide, oxyquinoline sulfate); Desiccants (calcium chloride, calcium sulfate, silicon dioxide); Emulsifying and/or solubilizing agents (acacia, cholesterol, diethanolamine (adjunct), glyceryl monostearate, lanolin alcohols, lecithin, mono- and di-glycerides, monoethanolamine (adjunct), oleic acid (adjunct), oleyl alcohol (stabilizer), poloxamer, polyoxyethylene 50 stearate, polyoxyl 35 caster oil, polyoxyl 40 hydrogenated castor oil, polyoxyl 10 oleyl ether, polyoxyl 20 cetostearyl ether, polyoxyl 40 stearate, polysorbate 20, polysorbate 40, polysorbate 60, polysorbate 80, propylene glycol diacetate, propylene glycol monostearate, sodium lauryl sulfate, sodium stearate, sorbitan monolaurate, soritan monooleate, sorbitan monopalmitate, sorbitan monostearate, stearic acid, trolamine, emulsifying wax); Filtering aids (powdered cellulose, purified siliceous earth); Flavors and perfumes (anethole, benzaldehyde, ethyl vanillin, menthol, methyl salicylate, monosodium glutamate, orange flower oil, peppermint, peppermint oil, peppermint spirit, rose oil, stronger rose water, thymol, tolu balsam tincture, vanilla, vanilla tincture, vanillin); Glidants and/or anticaking agents (calcium silicate, magnesium silicate, colloidal silicon dioxide, talc); Humectants (glycerin, hexylene glycol, propylene glycol, sorbitol); Plasticizers (castor oil, diacetylated monoglycerides, diethyl phthalate, glycerin, mono- and di-acetylated monoglycerides, polyethylene glycol, propylene glycol, triacetin, triethyl citrate); Polymers (e.g., cellulose acetate, alkyl celloloses, hydroxyalkylcelloloses, acrylic polymers and copolymers); Solvents (acetone, alcohol, diluted alcohol, amylene hydrate, benzyl benzoate, butyl alcohol, carbon tetrachloride, chloroform, corn oil, cottonseed oil, ethyl acetate, glycerin, hexylene glycol, isopropyl alcohol, methyl alcohol, methylene chloride, methyl isobutyl ketone, mineral oil, peanut oil, polyethylene glycol, propylene carbonate, propylene glycol, sesame oil, water for injection, sterile water for injection, sterile water for irrigation, purified water); Sorbents (powdered cellulose, charcoal, purified siliceous earth); Carbon dioxide sorbents (barium hydroxide lime, soda lime); Stiffening agents (hydrogenated castor oil, cetostearyl alcohol, cetyl alcohol, cetyl esters wax, hard fat, paraffin, polyethylene excipient, stearyl alcohol, emulsifying wax, white wax, yellow wax); Suspending and/or viscosity-increasing agents (acacia, agar, alginic acid, aluminum monostearate, bentonite, purified bentonite, magma bentonite, carbomer 934p, carboxymethylcellulose calcium, carboxymethylcellulose sodium, carboxymethycellulose sodium 12, carrageenan, microcrystalline and carboxymethylcellulose sodium cellulose, dextrin, gelatin, guar gum, hydroxyethyl cellulose, hydroxypropyl cellulose, hydroxypropyl methylcellulose, magnesium aluminum silicate, methylcellulose, pectin, polyethylene oxide, polyvinyl alcohol, povidone, propylene glycol alginate, silicon dioxide, colloidal silicon dioxide, sodium alginate, tragacanth, xanthan gum); Sweetening agents (aspartame, dextrates, dextrose, excipient dextrose, fructose, mannitol, saccharin, calcium saccharin, sodium saccharin, sorbitol, solution sorbitol, sucrose, compressible sugar, confectioner's sugar, syrup); Tablet binders (acacia, alginic acid, sodium carboxymethylcellulose, microcrystalline cellulose, dextrin, ethylcellulose, gelatin, liquid glucose, guar gum, hydroxypropyl methylcellulose, methycellulose, polyethylene oxide, povidone, pregelatinized starch, syrup); Tablet and/or capsule diluents (calcium carbonate, dibasic calcium phosphate, tribasic calcium phosphate, calcium sulfate, microcrystalline cellulose, powdered cellulose, dextrates, dextrin, dextrose excipient, fructose, kaolin, lactose, mannitol, sorbitol, starch, pregelatinized starch, sucrose, compressible sugar, confectioner's sugar); Tablet disintegrants (alginic acid, microcrystalline cellulose, croscarmellose sodium, corspovidone, polacrilin potassium, sodium starch glycolate, starch, pregelatinized starch); Tablet and/or capsule lubricants (calcium stearate, glyceryl behenate, magnesium stearate, light mineral oil, polyethylene glycol, sodium stearyl fumarate, stearic acid, purified stearic acid, talc, hydrogenated vegetable oil, zinc stearate); Tonicity agent (dextrose, glycerin, mannitol, potassium chloride, sodium chloride); Vehicle: flavored and/or sweetened (aromatic elixir, compound benzaldehyde elixir, iso-alcoholic elixir, peppermint water, sorbitol solution, syrup, tolu balsam syrup); Vehicle: oleaginous (almond oil, corn oil, cottonseed oil, ethyl oleate, isopropyl myristate, isopropyl palmitate, mineral oil, light mineral oil, myristyl alcohol, octyldodecanol, olive oil, peanut oil, persic oil, seame oil, soybean oil, squalane); Vehicle: solid carrier (sugar spheres); Vehicle: sterile (bacteriostatic water for injection, bacteriostatic sodium chloride injection); Viscosity-increasing (see *suspending agent*); Water repelling agent (cyclomethicone, dimethicone, simethicone); and Wetting and/or solubilizing agent (benzalkonium chloride, benzethonium chloride, cetylpyridinium chloride, docusate sodium, nonoxynol 9, nonoxynol 10, octoxynol 9, poloxamer, polyoxyl 35 castor oil, polyoxyl 40, hydrogenated castor oil, polyoxyl 50 stearate, polyoxyl 10 oleyl ether, polyoxyl 20, cetostearyl ether, polyoxyl 40 stearate, polysorbate 20, polysorbate 40, polysorbate 60, polysorbate 80, sodium lauryl sulfate, sorbitan monolaureate, sorbitan monooleate, sorbitan monopalmitate, sorbitan monostearate, tyloxapol) may be used as excipients. This list is not meant to be exclusive, but instead merely representative of the classes of excipients and the particular excipients which may be used in dosage forms of the present invention.

The compounds can form salts which are also within the scope of this disclosure. Reference to a compound of the formulae herein is understood to include reference to salts thereof, unless otherwise indicated. The term "salt(s)", as employed herein, denotes acidic salts formed with inorganic and/or organic acids, as well as basic salts formed with inorganic and/or organic bases. In addition, when a compound of the formulae contains both a basic moiety, such as, but not limited to a pyridine or imidazole, and an acidic moiety, such as, but not limited to a carboxylic acid, zwitterions ("inner salts") may be formed and are included within the term "salt(s)" as used herein. Pharmaceutically acceptable (i.e., non-toxic, physiologically acceptable) salts are preferred, although other salts are also useful. Salts of the compounds of the a formula may be formed, for example, by reacting a compound of a formula with an amount of acid or base, such as an equivalent amount, in a medium such as one in which the salt precipitates or in an aqueous medium followed by lyophilization.

Exemplary acid addition salts include acetates, ascorbates, benzoates, benzenesulfonates, bisulfates, borates, butyrates, citrates, camphorates, camphorsulfonates, fumarates, hydrochlorides, hydrobromides, hydroiodides, lactates, maleates, methanesulfonates, naphthalenesulfonates, nitrates, oxalates, phosphates, propionates, salicylates, succinates, sulfates, tartarates, thiocyanates, and toluenesulfonates (also known as tosylates,) . Additionally, acids which are generally considered suitable for the formation of pharmaceutically useful salts from basic pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1-19; P. Gould, International J. of Pharmaceutics (1986) 33 201-217; Anderson et al, The Practice of Medicinal Chemistry (1996), Academic Press, New York; and in The Orange Book (Food & Drug Administration, Washington, D.C. on their website).

Exemplary basic salts include ammonium salts, alkali metal salts such as sodium, lithium, and potassium salts, alkaline earth metal salts such as calcium and magnesium salts, salts with organic bases (for example, organic amines) such as dicyclohexylamines, t-butyl amines, and salts with amino acids such as arginine, and lysine. Basic nitrogen-containing groups may be quarternized with agents such as lower alkyl halides (e.g. methyl, ethyl, and butyl chlorides, bromides and iodides), dialkyl sulfates (e.g. dimethyl, diethyl, and dibutyl sulfates), long chain halides (e.g. decyl, lauryl, and stearyl chlorides, bromides and iodides), aralkyl halides (e.g. benzyl and phenethyl bromides), and others.

All such acid salts and base salts are intended to be pharmaceutically acceptable salts within the scope of the invention and all acid and base salts are considered equivalent to the free forms of the corresponding compounds for purposes of the invention.

Compounds, and salts, solvates, esters and prodrugs thereof, may exist in their tautomeric form (for example, as an amide or imino ether). All such tautomeric forms are contemplated herein as part of the present disclosure.

Unless otherwise stated, structures depicted herein are also meant to include all isomeric (e.g., enantiomeric, diastereomeric, and geometric (or conformational)) forms of the structure; for example, the R and S configurations for each asymmetric center, Z and E double bond isomers, and Z and E conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention. Additionally, unless otherwise stated, structures depicted herein are also meant to include compounds that differ only in the presence of one or more isotopically enriched atoms. For example, compounds having the present structures including the replacement of hydrogen by deuterium or tritium, or the replacement of a carbon by a ¹³C- or ¹⁴C-enriched carbon are within the scope of this invention. Such compounds are useful, for example, as analytical tools, as probes in biological assays, or as therapeutic agents in accordance with the present invention.

The symbol " 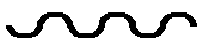 ", except when used as a bond to depict unknown or mixed stereochemistry, denotes the point of attachment of a chemical moiety to the remainder of a molecule or chemical formula.

### Detailed Description of Certain Embodiments

The present disclosure relates to, inter alia, methods of treating, preventing or ameliorating a T_{H}17- or CSF1-mediated disease or disorder by administering to a patient in need thereof a therapeutically effective amount of a FASN inhibitor, or a pharmaceutically acceptable salt, hydrate, solvate, prodrug, stereoisomer, or tautomer thereof. In some aspects, methods of the present disclosure can be used in the treatment of a variety of T_{H}17 or CSF1 dependent diseases and disorders. Inhibition of FASN for the treatment of T_{H}17- or CSF1-mediated diseases or disorders provides a novel approach to the treatment, prevention, or amelioration of diseases including, but not limited to, cancer, immunological disorders, and obesity.

In some aspects the present disclosure provides methods of inhibiting the secretion of IL-17, comprising contacting cells capable of secreting IL-17 with a FASN inhibitor. While not wishing to be bound by any particular theory, the present disclosure encompasses the recognition that FASN inhibitors may act on IL-17 production through blockage of T_{H}17 differentiation. The present disclosure also encompasses the recognition that FASN inhibitors that act to block T_{H}17 differentiation and/or secretion of IL-17 are particularly useful in the treatment of diseases, disorder, or conditions associated with such pathways, for example, NASH.

The present disclosure also encompasses the recognition that FASN inhibition is useful for reducing the rate of de novo lipogenesis (DNL), which, without wishing to be bound by any particular theory, affects lipid accumulation in the liver causing cellular damage, inflammation, and fibrosis associated with NASH. In certain aspects, the present disclosure provides a method for treating a condition or disease associated with reducing the rate of DNL. In certain aspects, the method comprises administering to a patient in need of a treatment for a condition or disease associated with reducing the rate of DNL an effective amount of a FASN inhibitor.

In some aspects, the present disclosure provides methods of treating conditions and diseases associated with IL-17 comprising administering to a patient in need thereof a FASN inhibitor. In some aspects, FASN inhibitors useful in such methods are those which, when incubated with activated CD4+ T cells, cultured under T_{H}17 polarizing conditions, inhibit the production of IL-17 in the presence of cytokines.

In one aspect, the disclosure relates to a method of treating a condition or disease associated with T_{H}17 mediated inflammation. In some aspects, the method comprises administering to a patient in need of a treatment for a condition or a disease associated with T_{H}17 mediated inflammation an effective amount of a FASN inhibitor.

Another aspect of the present disclosure relates to a method of treating a condition or disease associated with IL-17 receptor activation. In some aspects, the method comprises administering to a patient in need of a treatment for a condition or a disease associated with IL-17 receptor activation an effective amount of a FASN inhibitor.

Another aspect of the present disclosure relates to a method of treating a condition or disease associated with T_{H}17 differentiation. In some aspects, the method comprises administering to a patient in need of a treatment for a condition or a disease associated with IL-17 receptor activation an effective amount of a FASN inhibitor.

In some aspects, IL-17 secretion is inhibited by at least 1%. In some embodiments, IL-17 secretion is inhibited by at least 5%. In some aspects, IL-17 secretion is inhibited by at least 10%. In some aspects IL-17 secretion is inhibited by at least 25%. In some aspects IL-17 secretion is inhibited by at least 30%. In some embodiments, IL-17 secretion is inhibited by at least 35%. In some aspects IL-17 secretion is inhibited by at least 40%. In some aspects, IL-17 secretion is inhibited by at least 45%. In some aspects, IL-17 secretion is inhibited by at least 50%. In some aspects, IL-17 secretion is inhibited by at least 55%. In some embodiments, IL-17 secretion is inhibited by at least 60%. In some aspects, IL-17 secretion is inhibited by at least 65%. In some aspects IL-17 secretion is inhibited by at least 70%. In some aspects IL-17 secretion is inhibited by at least 75%. In some aspects, IL-17 secretion is inhibited by at least 80%. In some aspects, IL-17 secretion is inhibited by at least 85%. In some aspects, IL-17 secretion is inhibited by at least 90%. In some aspects, IL-17 secretion is inhibited by at least 95%. In some aspects, IL-17 secretion is inhibited by at least 99%.

In some aspects, FASN inhibitors are those disclosed in WO 2014/164749.

The compounds disclosed herein can be prepared in a manner similar to the procedures described in US 2016/0002188.

In some aspects of the disclosure, FASN inhibitors useful in provided methods are selected from the group consisting of 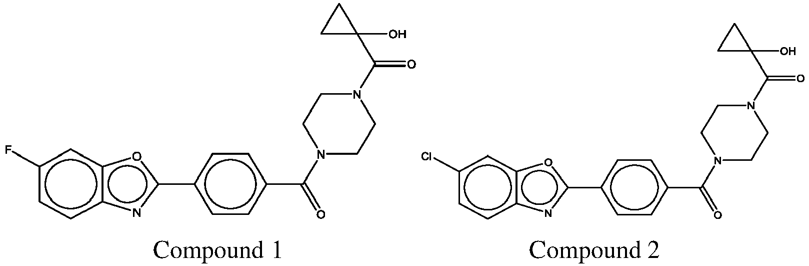 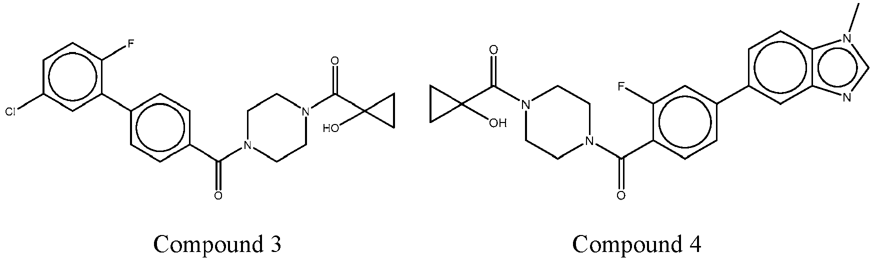 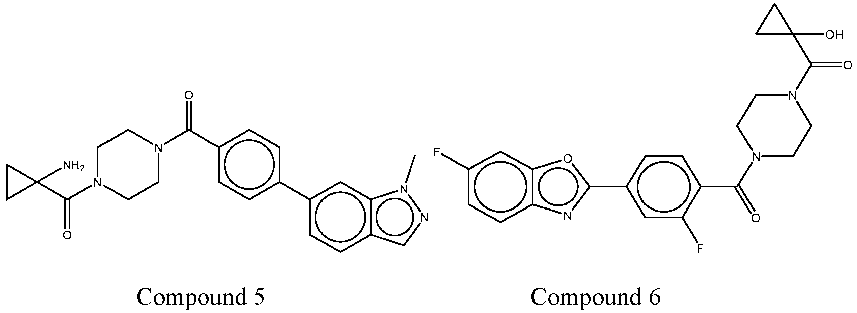 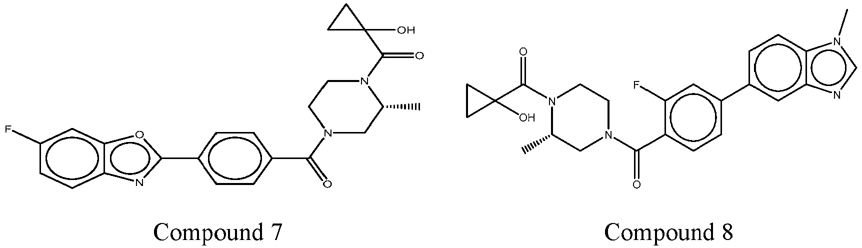 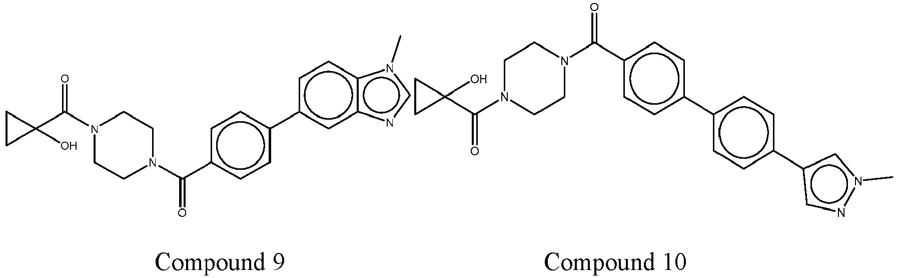 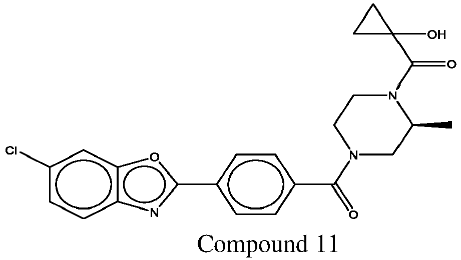 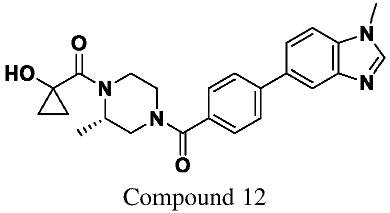 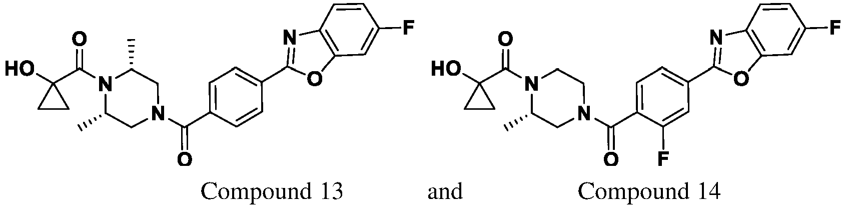 or pharmaceutically acceptable salts thereof According to the invention, the FASN inhibitor is compound 4.

In some aspects of the disclosure, Compound A is selected from one of Compounds 1-14 shown above.

In other aspects, FASN inhibitors include, but are not limited to TVB-2640; GSK2194069; GSK1995010; GSK837149A; JNJ54302833; BI00179; IPI-9119; Orlistat; Cerulenin and C75 (4-Methylene-2-octyl-5-oxotetrahydrofuran-3-carboxylic acid); as well as FASN inhibitors disclosed in WO 2014/146747.

In some aspects of the disclosure, the FASN inhibitors are those disclosed in WO 2014/164749.

In some aspects, the conditions and diseases associated with IL-17 are conditions and diseases associated with T_{H}17 mediated inflammation.

In some aspects, the conditions and diseases associated with IL-17 are conditions and diseases associated with IL-17 receptor activation.

In some aspects, a disease treated by the provided methods is cancer. In some aspects, the cancer is selected from the group consisting of prostate, ovary, pancreas, esophagus, thyroid, bladder, bone, bile duct, testicle, uterus, head, neck, salivary gland, cervix, colon, breast, lung, stomach cancers, hematologic cancer, such as but not limited to leukaemia, lymphoma and multiple myeloma; midline carcinomas, mesenchymal, hepatic, renal and neurological tumors; and melanoma, squamous cell carcinoma and cutaneous T-cell lymphoma; and small and non-small cell lung cancer, castration-resistant prostate cancer, glioblastoma, astrocytoma, mebulloblastoma, neuroblastoma, neurofibromatosis, merkel cell carcinoma, soft tissue sarcoma, osteosarcoma, Ewing's sarcoma, and cholangiocarcinoma.

### Uses for the treatment of NASH

NASH is a chronic liver disease with varying degrees of inflammation and fibrosis that can progress to cirrhosis and end-stage liver complications. The disease affects greater than 16 million people in the US and is the second leading cause of liver transplants in the US. Moreover, NASH is a strong predictor for type 2 diabetes, cardiovascular disease and end-stage kidney disease. There are no pharmacological agents currently approved for the treatment of NASH, which represents a substantial healthcare burden. The pathogenesis of NASH has been associated with obesity or aberrant metabolic syndrome. Strategies that target all three pathological processes (steatosis, inflammation and fibrosis) may most effectively manage this disease.

FASN is an important enzyme that catalyzes the final step of DNL by utilizing malonyl-CoA as a substrate to synthesize palmitate. Liver-specific ablation of the gene encoding FASN in mice (FASNLKO mice) results in malonyl-CoA increases and palmitate decreases in liver. Also, in leptin-deficient obese mice (ob/ob mice), both hepatic FASN gene expression and hepatic FASN activity increase compared to lean mice controls. Further reductions of hepatic lipid accumulation and hepatic DNL occurs following liver-specific inhibition of the lipogenic transcription factor carbohydrate response element binding protein (ChREBP) in ob/ob mice. Surprisingly, FASNLKO mice show normal liver triglyceride content, suggesting DNL may be dispensable in healthy liver and FASN inhibition may not negatively impact non-diseased liver function.

FASN controls the last step of DNL, and its gene expression level is aberrantly elevated in NASH patients as compared to healthy volunteers. Uncontrolled DNL is a key contributor for liver steatosis. Chronic activation of hepatic DNL and increase in the traffic of free fatty acids within hepatocytes can lead to generation of toxic lipid metabolites. These lipotoxic metabolites act as reactive oxygen species (ROS), triggering endoplasmic reticulum (ER) stress, apoptosis and necrosis in the liver resident cells, hepatocytes, Kupffer cells and hepatic stellate cells (HSC). Therefore, elevated FASN activity may be a major contributor to NASH through lipotoxicity mediated tissue injury and cell death.

In addition to FASN's role in steatosis and lipotoxicity, it may contribute to inflammation and the processes of fibrosis. Palmitate, the end product of the fatty acid synthesis pathway, upregulates TLR4, pro-inflammatory cytokines and tumor necrosis factor-a (TNF-α) production in primary hepatocytes and Kupffer cells. Hence, DNL generates fatty acids necessary for TH17 cell differentiation and function. Increased levels of TH17 cells in human liver are associated with the progression from non-alcoholic fatty liver (NAFL) to NASH. IL-17 also promotes liver fibrosis in a mouse model of liver disease (carbon tetrachloride (CC14)-induced liver injury model) through hepatic stellate cell activation and subsequent collagen production. Stimulation of HSC activation occurs both by elevation of IL-17 and by palmitate in mice.

The importance of the DNL pathway in NASH is validated by inhibitors of other enzymes in the pathway, such as acetyl-CoA carboxylase 1/2 (ACC). ACC1 is an enzyme directly upstream from FASN that catalyzes the production of malonyl-CoA from acetyl-CoA. Small molecule inhibitors of ACC1/2, such as NDI-010976 (Nimbus Therapeutics/Gilead), reduced steatosis and fibrosis in preclinical NASH models, although they may carry a mechanistic risk of undesired fatty acid β-oxidation.

Without wishing to be bound by any particular theory, it is believed that targeting FASN represents a potentially powerful multi-modal approach to treat NASH, by reducing steatosis, and TH17-dependent inflammation and fibrosis.

In certain aspects, the present disclosure provides a method for treating a disease, disorder, or condition selected from a fibrotic disease or fibrosis, an inflammatory diease, disorder, or condition, or an autoimmune disease, disorder, or condition, comprising administering to a patient in need thereof a FASN inhibitor.

In certain aspects, the present disclosure provides a method of treating NASH comprising administering to a patient in need thereof a FASN inhibitor .

In some aspects, the methods disclosed herein further comprises a step of measuring one or more biomarkers for the purpose of selecting a patient for therapy, monitoring patient therapy, and/or adjusting or terminating patient therapy. In some embodiments, a biomarker is a ¹³C-labelled lipid. In some aspects, a biomarker is ¹³C palmitate. In some aspects, a provided method further comprises the step of administering to a patient ¹³C acetate (e.g., ¹³C sodium acetate) prior to or simultaneously with administration of a FASN inhibitor.

In some embodiments, patients may be selected for treatments described herein based upon one or more characteristics. In some embodiments, a patient selected for treatment (e.g., for treatment of NASH) has one or more of the following characteristics:
i) overweight (e.g., BMI ≥ 25 kg/m²,
ii) male,
iii) a total cholesterol level of 400 mg/dL or less,
iv) a triglycerides level of 500 mg/dL or less,
v) no history of skin or eye sensitivities,
vi) no evidence of clinically significant hepatic or renal impairment,
vii) no history of severe allergic reaction (including anaphylaxis) to any substance, or previous status asthmaticus,
viii) no history of psoriasis or any dermatitis requiring oral or topical corticosteroids within the past 12 months,
ix) no history of Sjögren's syndrome or any history of dry eyes or allergic conjunctivitis, requiring artificial tears or medicated eye drops within the past 12 months,
x) has not used non-prescription medication within seven days prior to first administration and prescription medications and natural health products (except vitamin or mineral supplements, and hormone replacement) within 14 days prior to first administration,
xi) no history of intolerance to or malabsorption of fructose,
xii) is not positive for hepatitis B, hepatitis C, or human immunodeficiency virus.

In some embodiments, a patient selected for treatment (e.g., for NASH) has, or during treatment is placed on, a eucaloric diet or a high fructose diet.

### Additional Uses

In some aspects , the present disclosure provides a method of treating a disease, disorder, or condition associated with T_{H}17 mediated inflammation the method comprising administering to a patient in need thereof a FASN inhibitor.

In some aspects, the present disclosure provides a method of treating a disease, disorder, or condition associated with IL-17 receptor activation, the method comprising administering to a patient in need thereof a FASN inhibitor.

According to the invention, the disease is NASH.

In some aspects of the disclosure, the condition or disease is obesity (Berndt, et al., Diabetologia, 2007, 50: 1472-1480; Lodhi, et al., Cell Metabolism, 2012, 16, 189-201.). In other embodiments, the condition or disease is Type II diabetes.

In some aspects, the disease is selected from the group consisting of Inflammatory Bowel Disease and Chronic Obstructive Pulmonary Disease.

In some aspects, the disease is selected from the group consisting of allergic contact dermatitis; and autoimmune myositis.

In another aspect, the disease is selected from the group consisting of systemic or tissue inflammation, inflammatory responses to infection or hypoxia, cellular activation and proliferation, lipid metabolism, fibrosis and in the prevention and treatment of viral infections.

In another aspect, the disease is selected from the group consisting of chronic and inflammatory conditions, including but not limited to rheumatoid arthritis, osteoarthritis, periodontitis, acute gout, psoriasis, psoriatic arthritis, ankylosing spondylitis, HIV-associated nephropathy, amyotrophic lateral sclerosis, pulmonary arterial hypertension, acute allograft rejection, chronic organ transplant rejection, systemic lupus erythematosus, lupus nephritis, multiple sclerosis, Crohn's disease, ulcerative colitis, severe celiac/coeliac disease, asthma, chronic obstructive airways disease, pneumonitis, myocarditis, pericarditis, myositis, eczema, dermatitis, alopecia, vitiligo, bullous skin disease, nephrititis, vasculitis, atherosclerosis, Alzheimer's disease, depression, Sjogren's syndrome, siloadenitis, central retinal vein occlusion, branched retinal vein occlusion, Irvine-Gass syndrome, parafoveal telangiectasis, retinitis pigmentosa, pars planitis, birdshot retinochoroidopathy, epiretinal membrane, cystic macular edema, maculopathies, vitreomacular traction syndromes, retinal detachment, neuroretinitis, idiopathic macular edema, retinitis, dry eye, vernal keratoconjuctivitis, atopic keratoconjuctivitis, anterior uveitis, pan uveitis, posterior uveitis, uveitis-associated macular edema, scleritis, diabetic retinopathy, diabetic macular edema, age-related macular dystrophy, hepatitis, pancreatitis, primary biliary cirrhosis, sclerosing cholangitis, Addison's disease, hypophysitis, thyroiditis, type I diabetes and acute rejection of transplanted organisms.

In another aspect of the disclosure, the disease is selected from the group consisting of acute inflammatory conditions such as acute gout, giant cell arteritis, nephritis including lupus nephritis, vasculitis with organ involvement such as glomerulonephritis, vasculitis including giant cell arteritis, Wegner's granulomatosis, Polyarteritis nodosa, Becet's disease, Kawasaki disease, Takayasu's arteritis, pyoderma gangrenosum, vasculitis with organ involvement and acute rejection of transplanted organs.

In another aspect, the disease is selected from the group consisting of inflammatory responses to infections with bacteria, viruses, fungi, parasites or their toxins, such as but not limited to sepsis, sepsis syndrome, septic shock, endotoxaemia, systemic inflammatory response syndrome, multi organ dysfunction syndrome, toxic shock syndrome, acute lung injury, acute respiratory distress syndrome, acute renal failure, fulmiant hepatitis, burns, acute pancreatitis, post-surgical syndromes, sarcoidosis, Herxheimer reactions, encephalitis, myelitis, meningitis, malaria, systemic inflammatory responses associated with viral infections, such as but not limited to influenza, herpes zoster, herpes simplex and coronavirus.

Another aspect of the present disclosure relates to a method of treating a disease associated with systemic inflammatory response syndrome, such as but not limited to sepsis, burns, pancreatitis, major trauma, hemorrhage and ischaemia, the method comprising administering a FASN inhibitor.

Also provided are methods of treating a patient of organ transplantation to reduce organ rejection, the method comprising administering to a patent in need thereof a FASN inhibitor.

Another aspect of the present disclosure relates to a method of treating a patient of organ transplantation to reduce organ rejection. The method comprises administering to a patient in need of a treatment for reducing organ rejection an effective amount of a FASN inhibitor.

Another aspect of the present disclosure relates to a method of inhibiting CSF-1 dependent macrophage differentiation. The method comprises contacting a cell with an effective amount of a FASN inhibitor.

Another aspect of the present disclosure relates to a method of treating a condition or disease associated with CSF-1 dependent macrophage differentiation. The method comprises administering to a patient in need a treatment for condition or a disease associated with CSF-1 dependent macrophage differentiation an effective amount of a FASN inhibitor.

In some aspects, the conditions and diseases are selected from the group consisting of osteoarthritis and pain.

In some aspects, the condition or disease is malignant melanoma.

In some aspects, the condition or disease is an inflammatory disease.

In some aspects, the condition or disease is inflammatory bowel disease. In one aspect, the inflammatory bowel disease is Crohn's disease. In another aspect, the inflammatory bowel disease is ulcerative colitis. In another aspect, the inflammatory disease is rheumatoid arthritis.

In another aspect of the disclosure, a FASN inhibitor is selected from compounds shown in **Table A,** which lists the IUPAC names and the structures of the compounds The third compound on p. 188 corresponds to compound 4, which is the claimed compound. The other compounds are for reference.

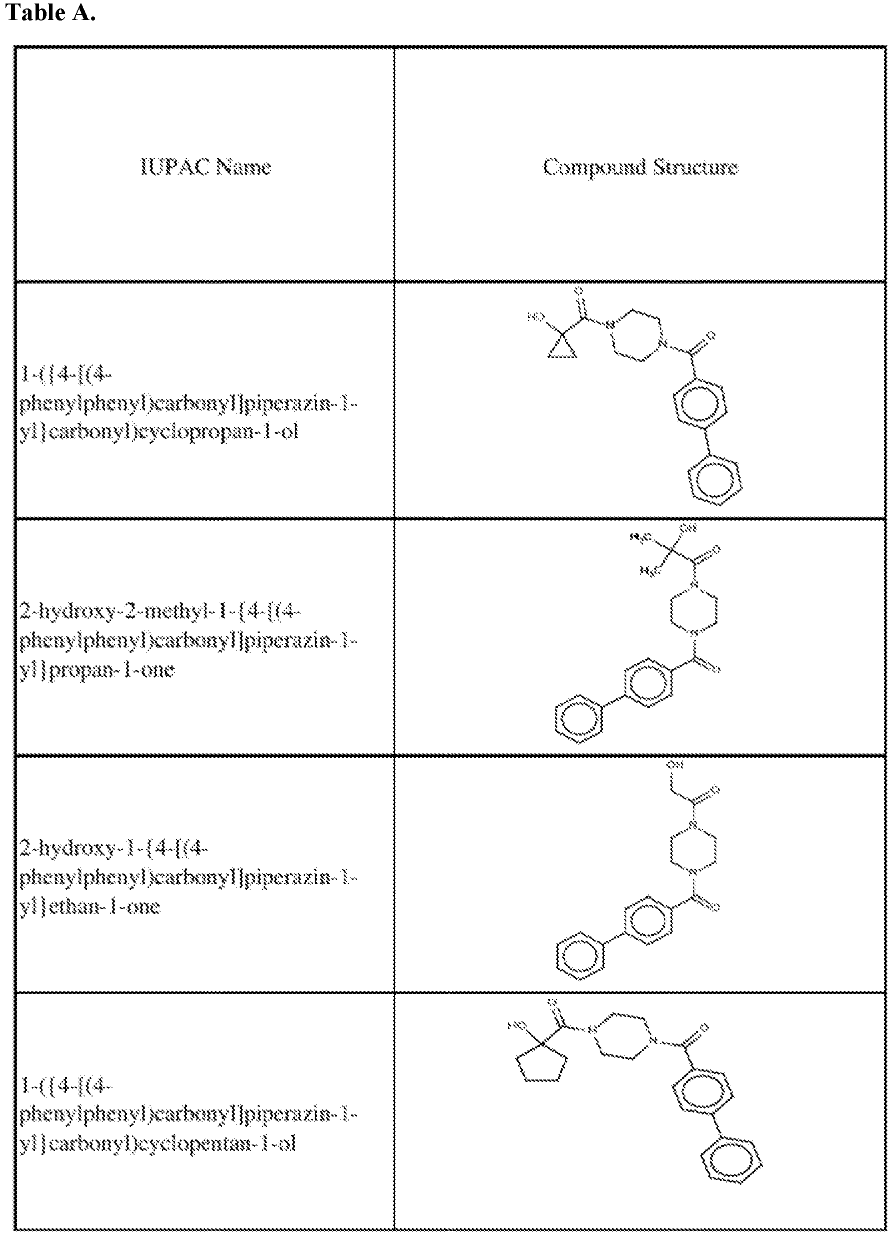

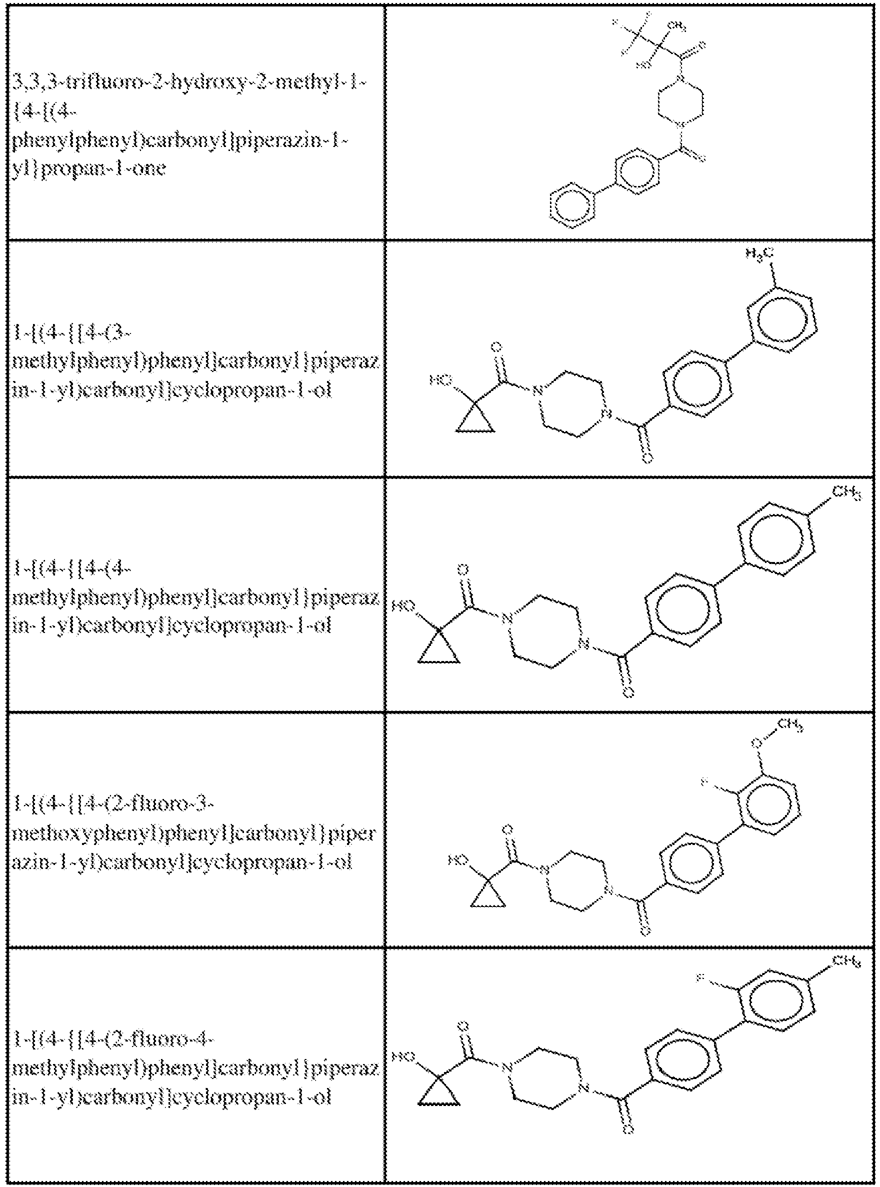

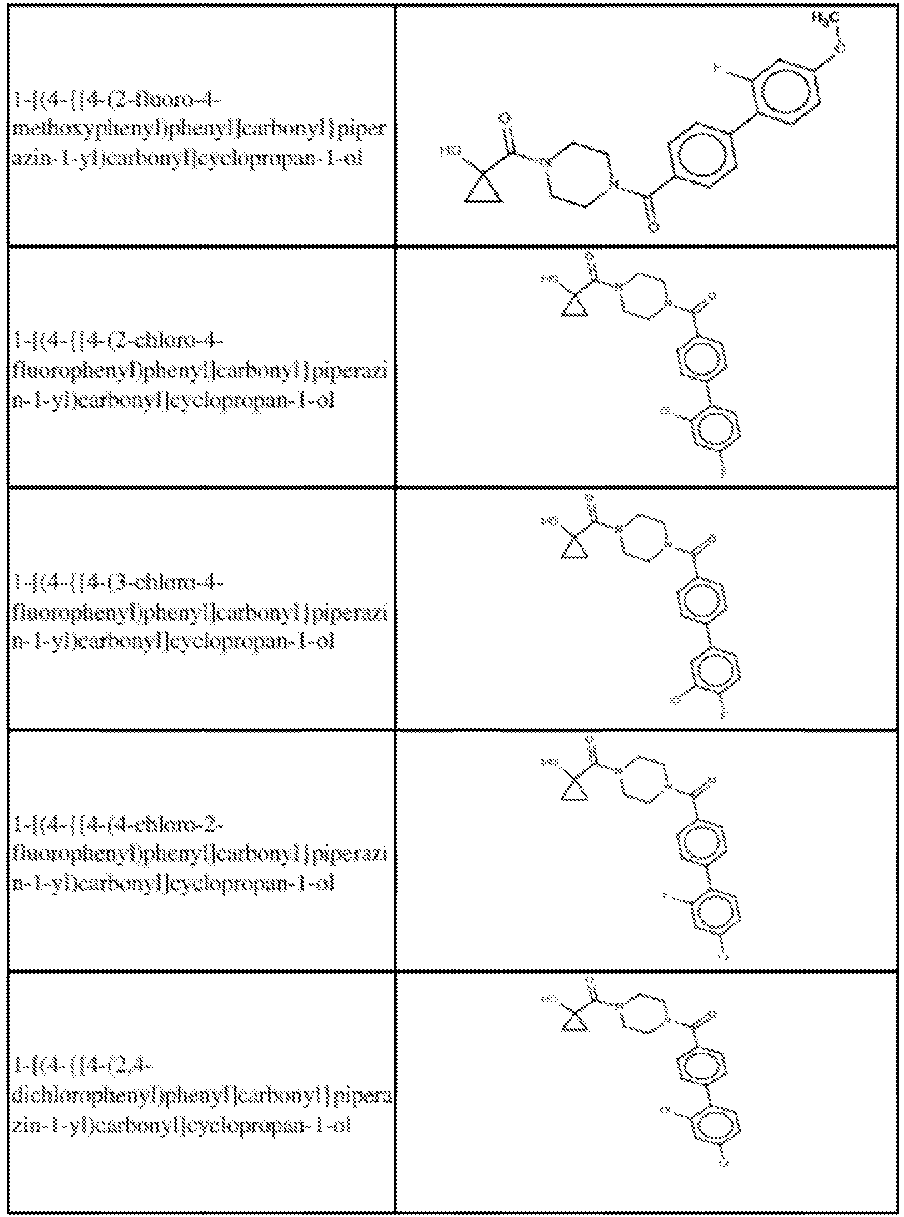

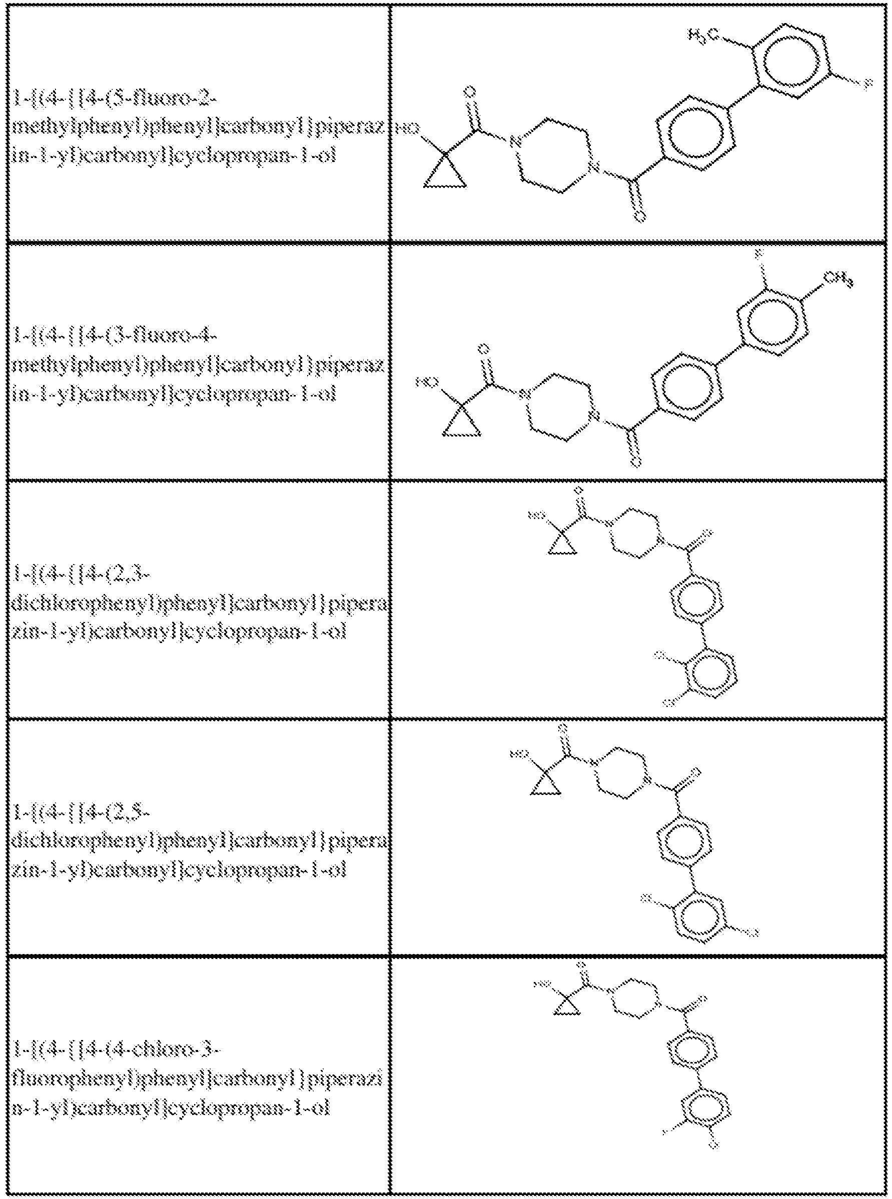

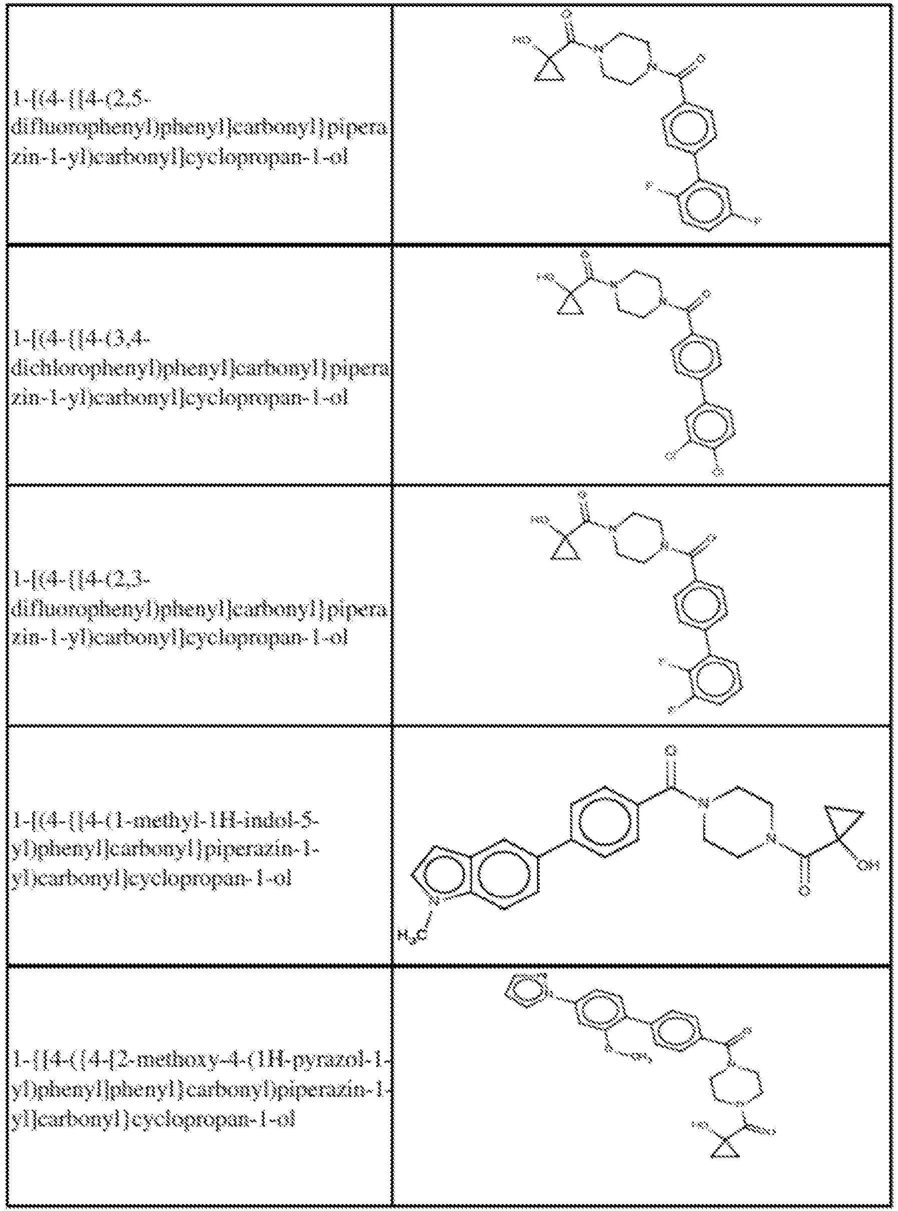

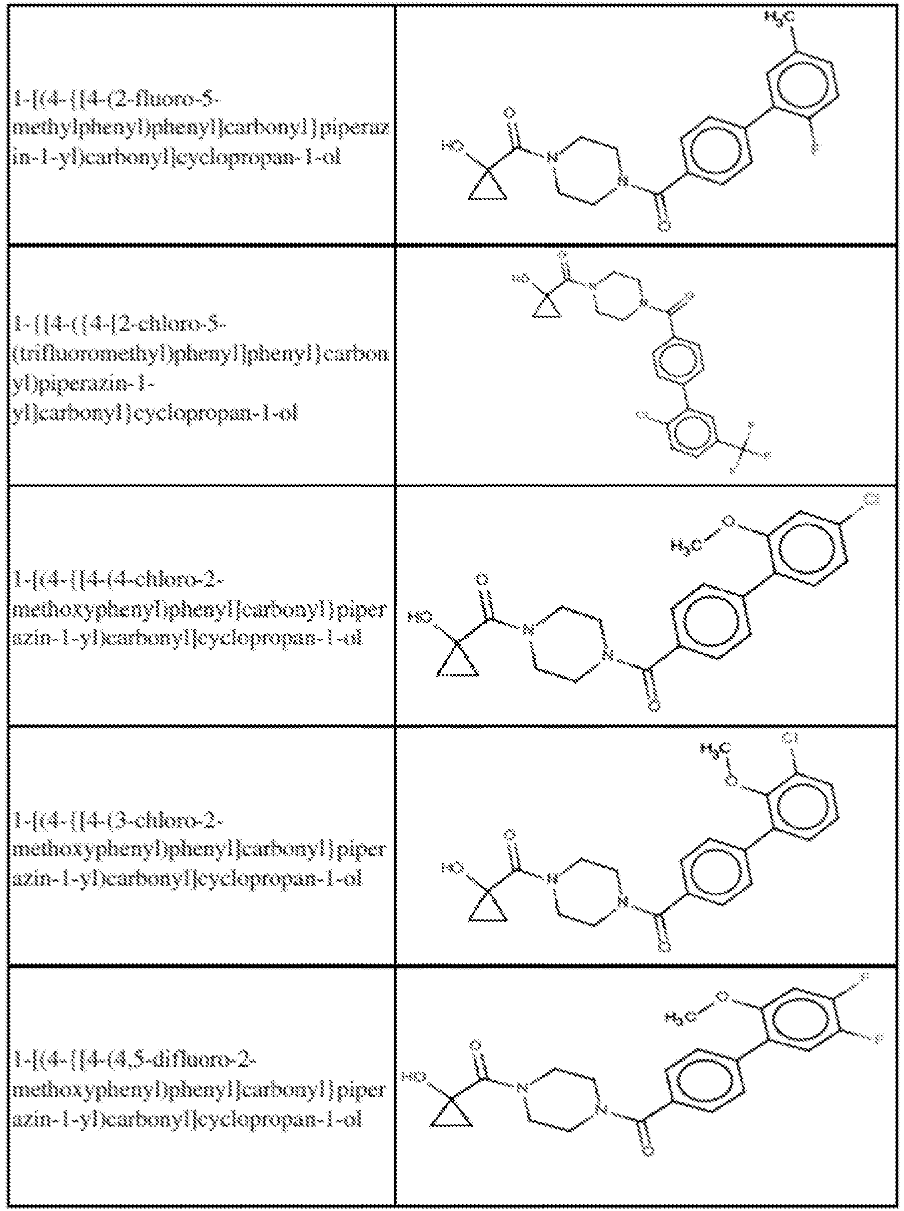

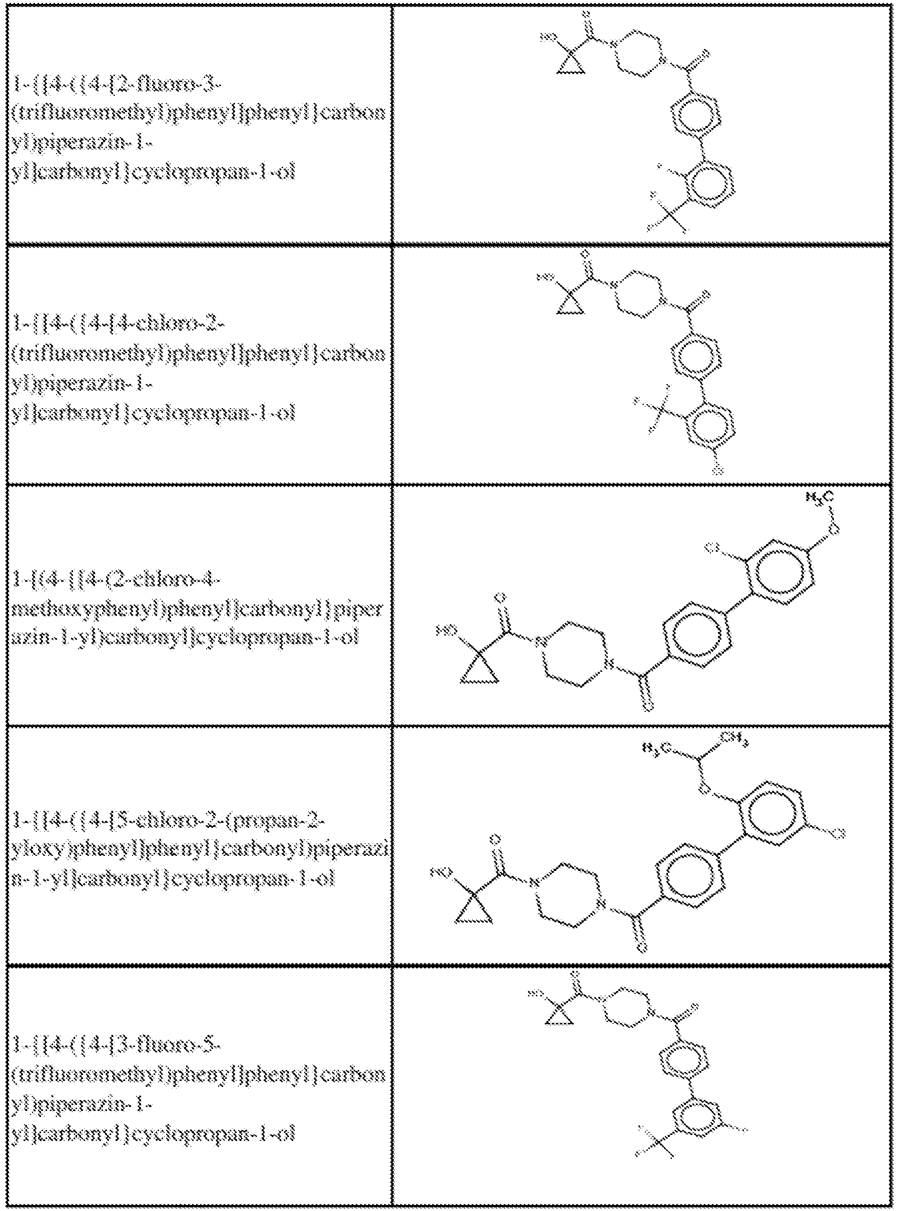

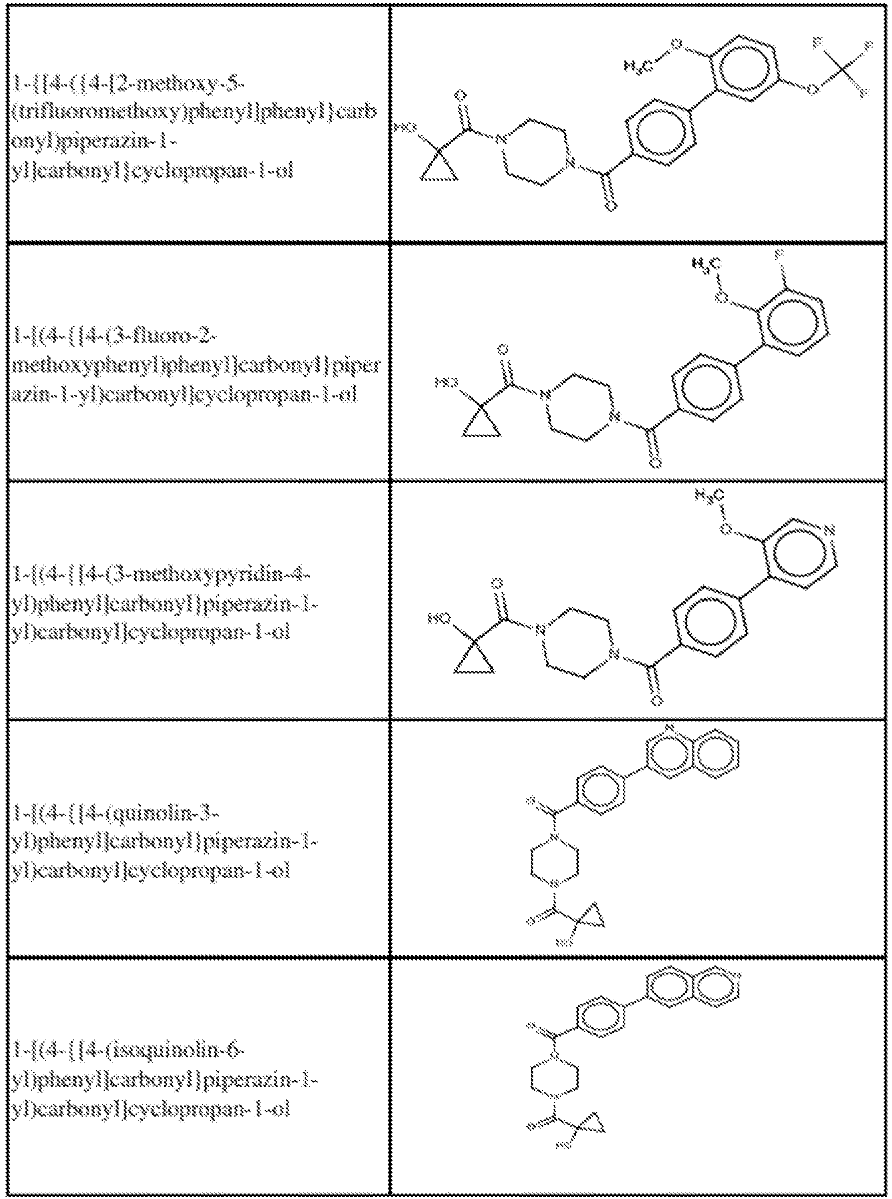

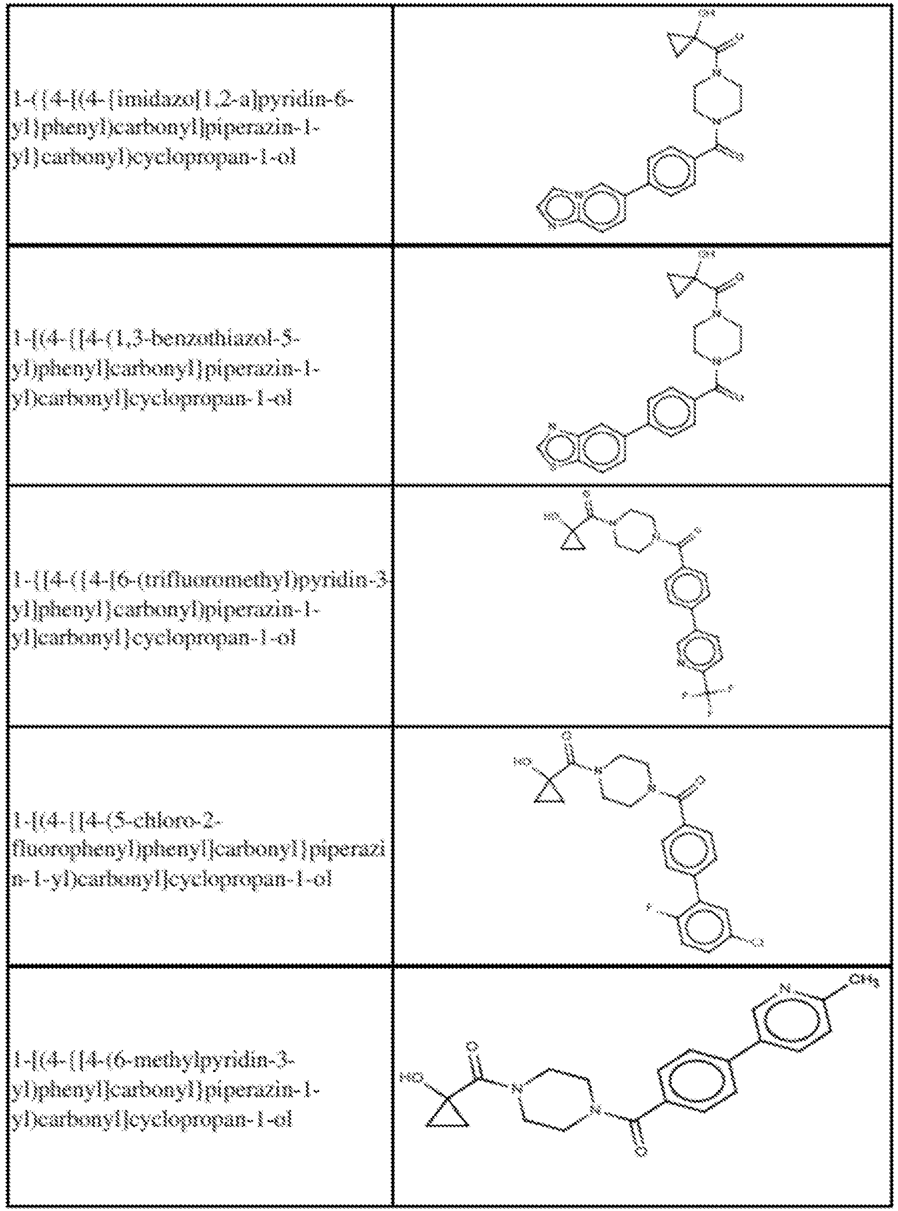

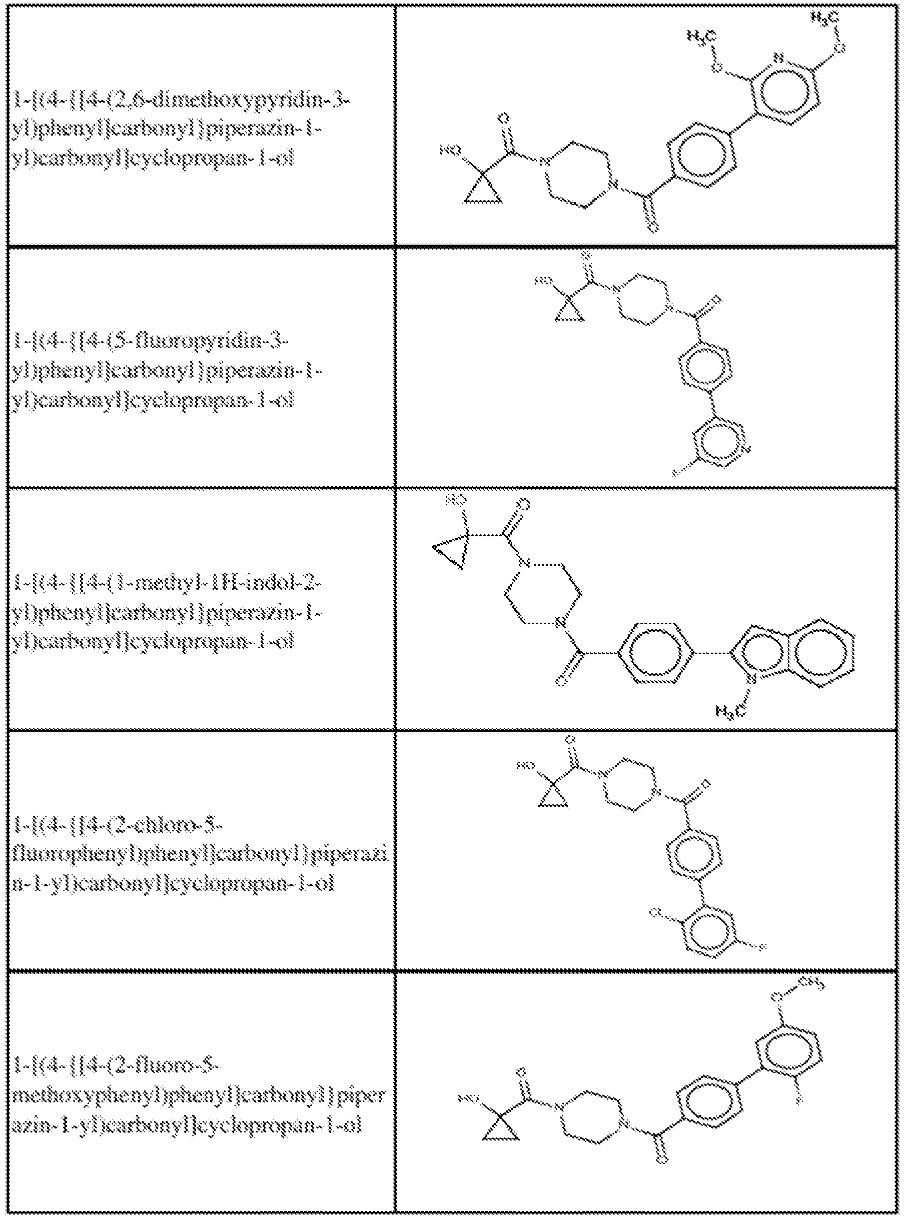

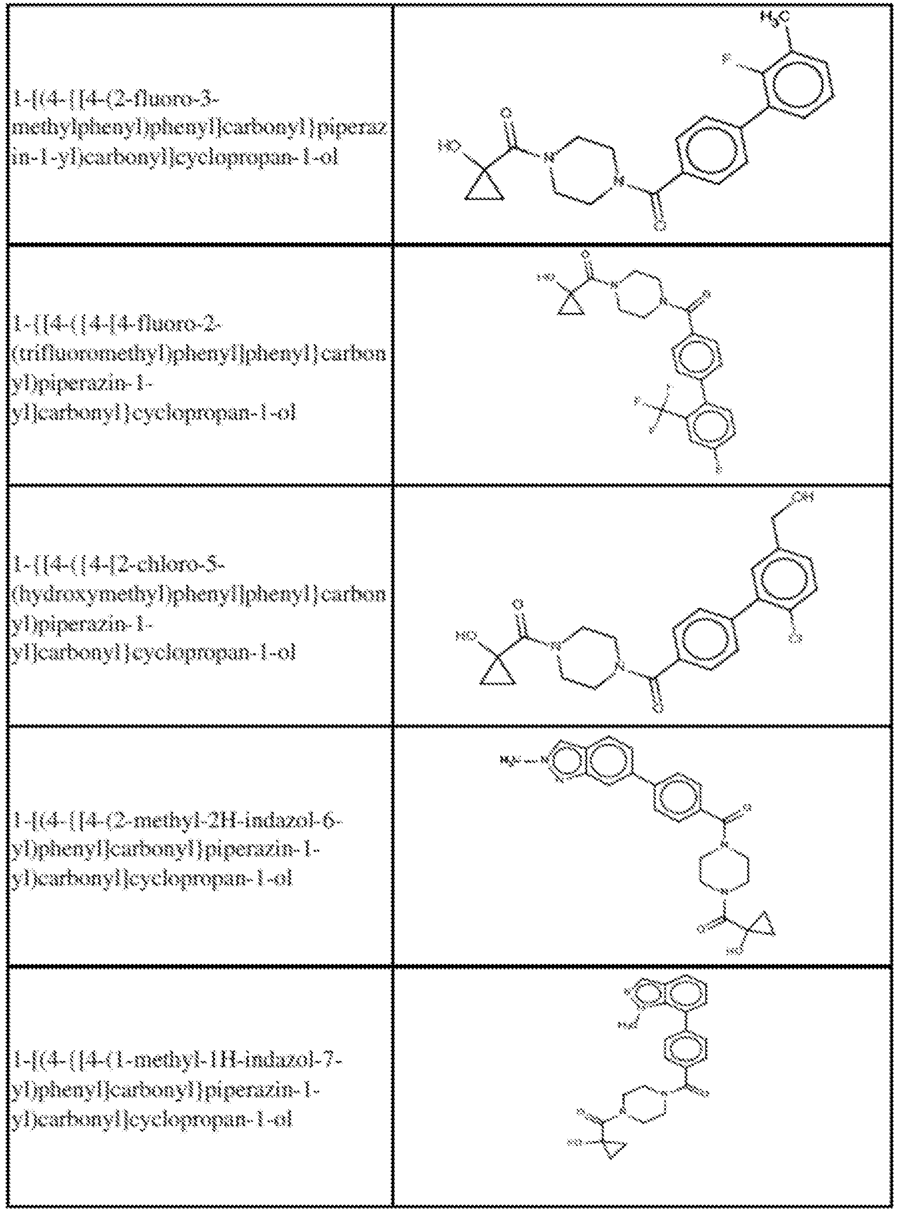

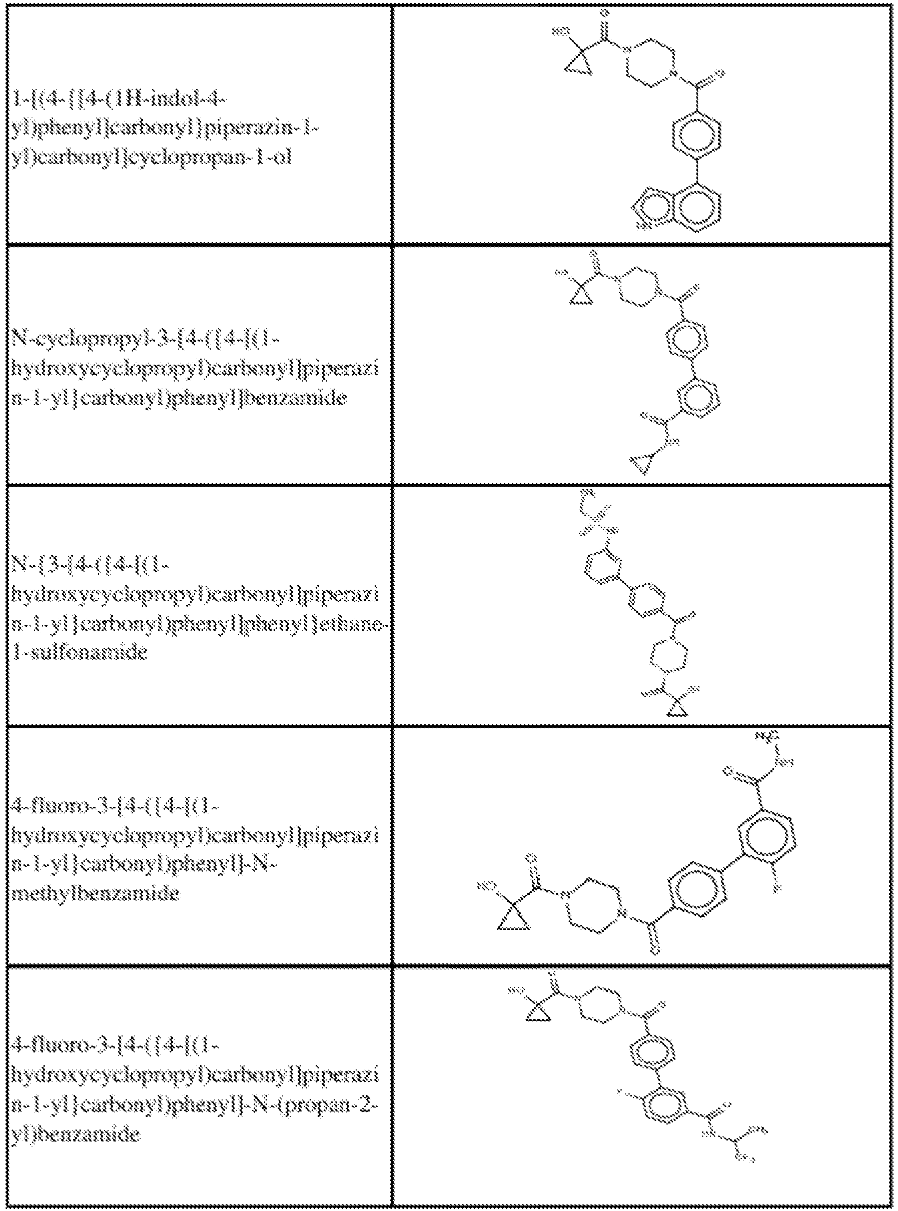

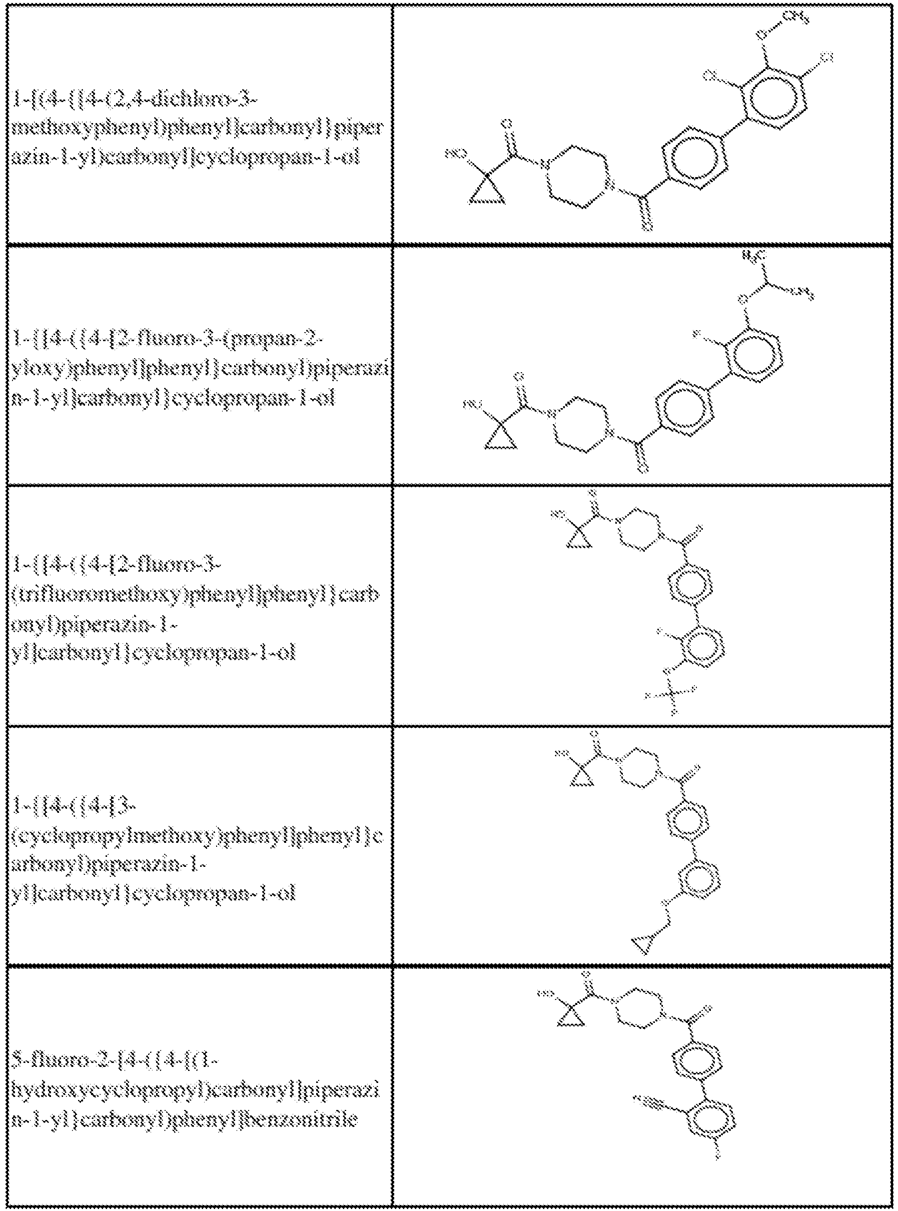

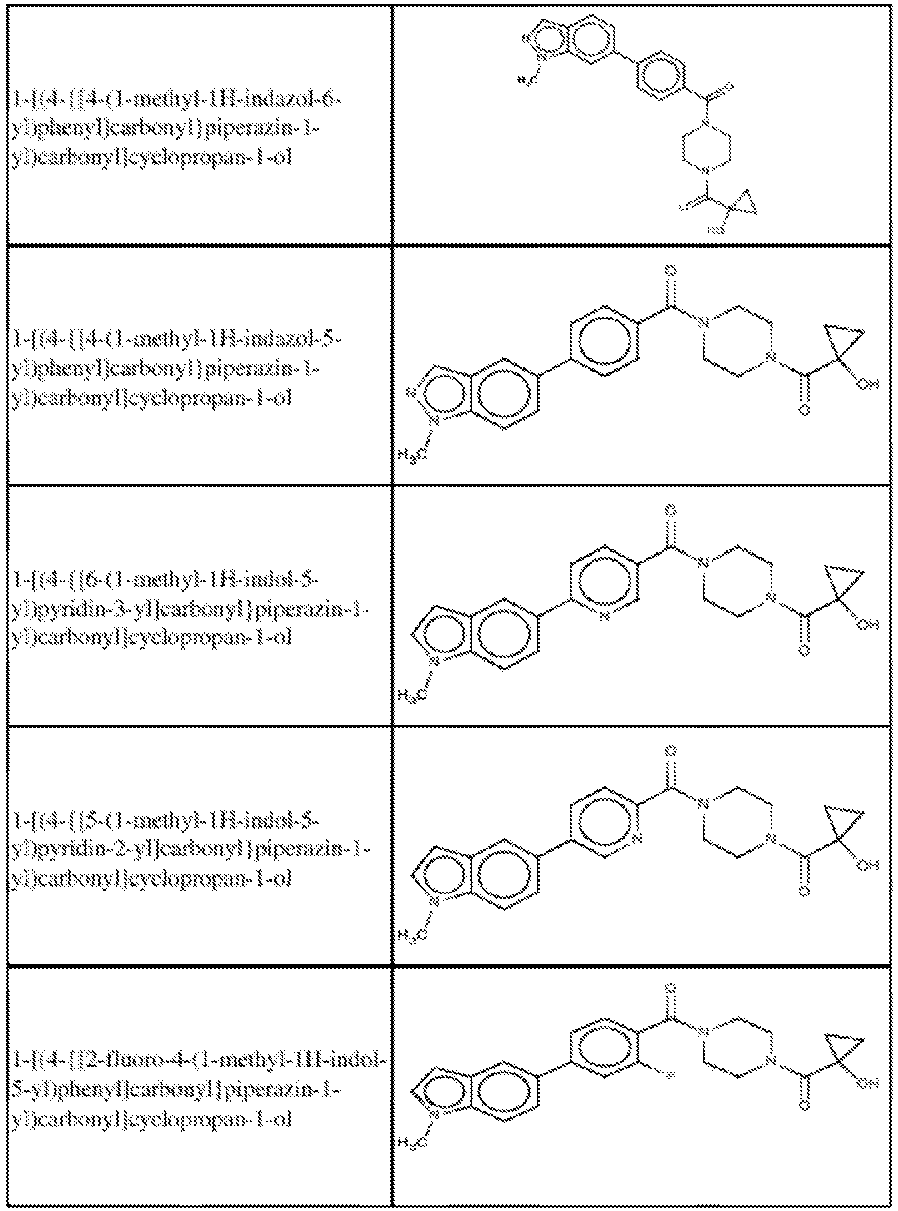

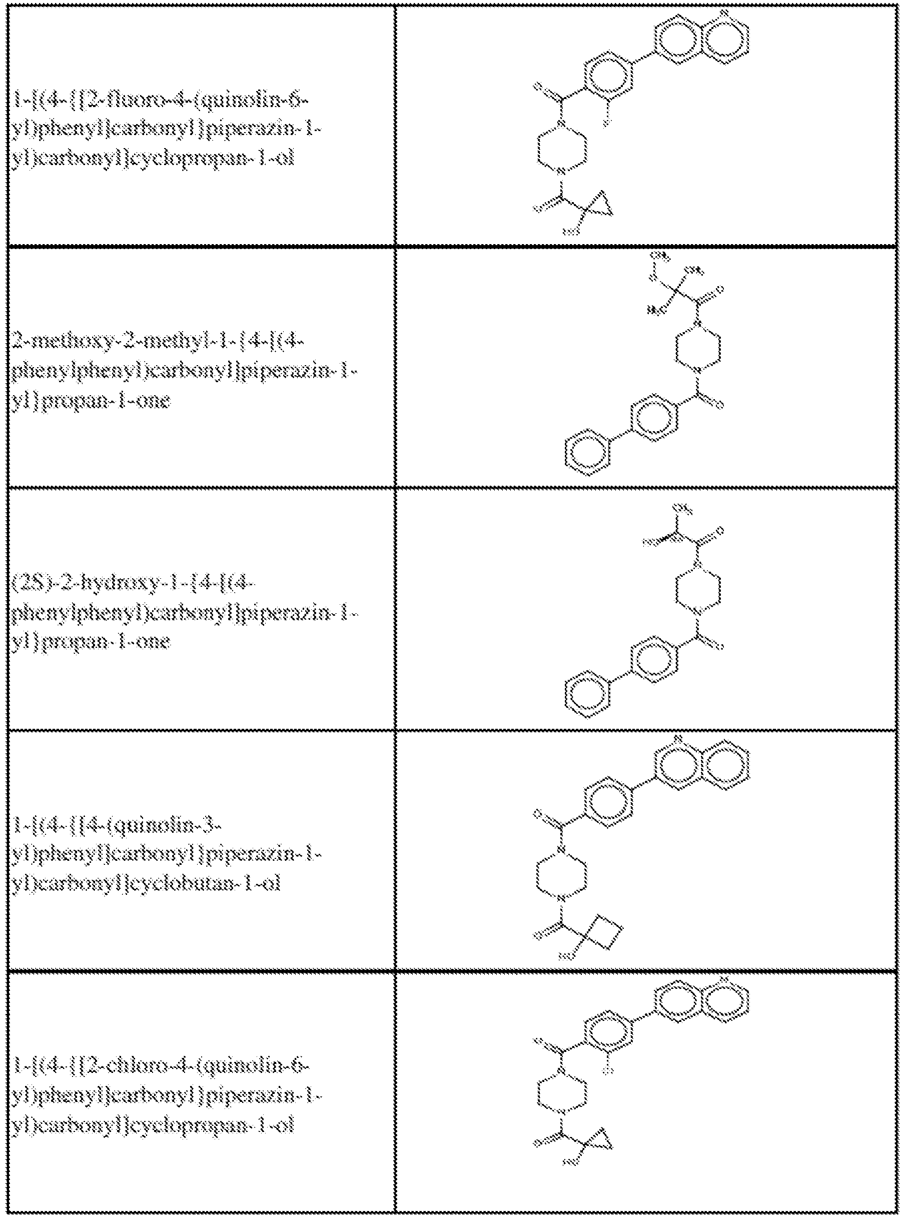

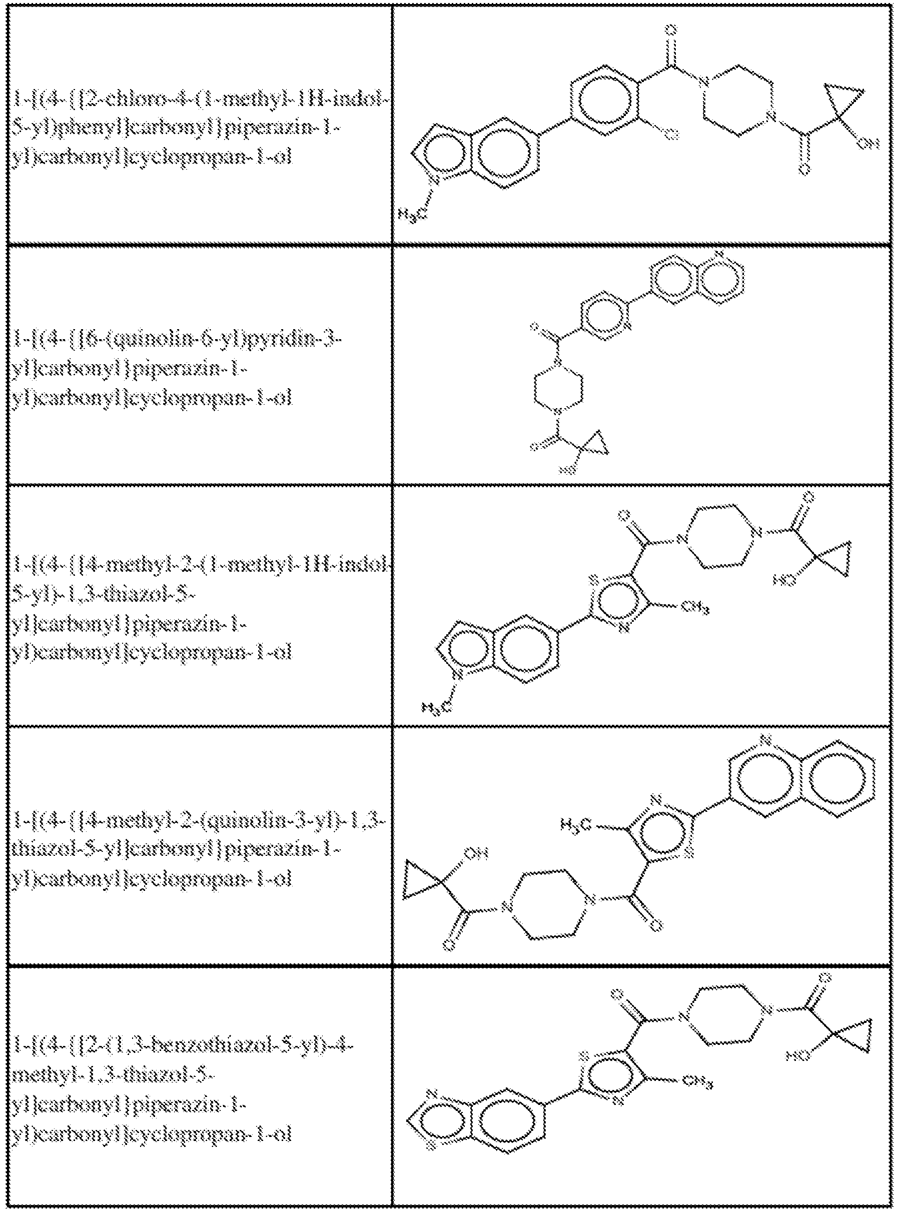

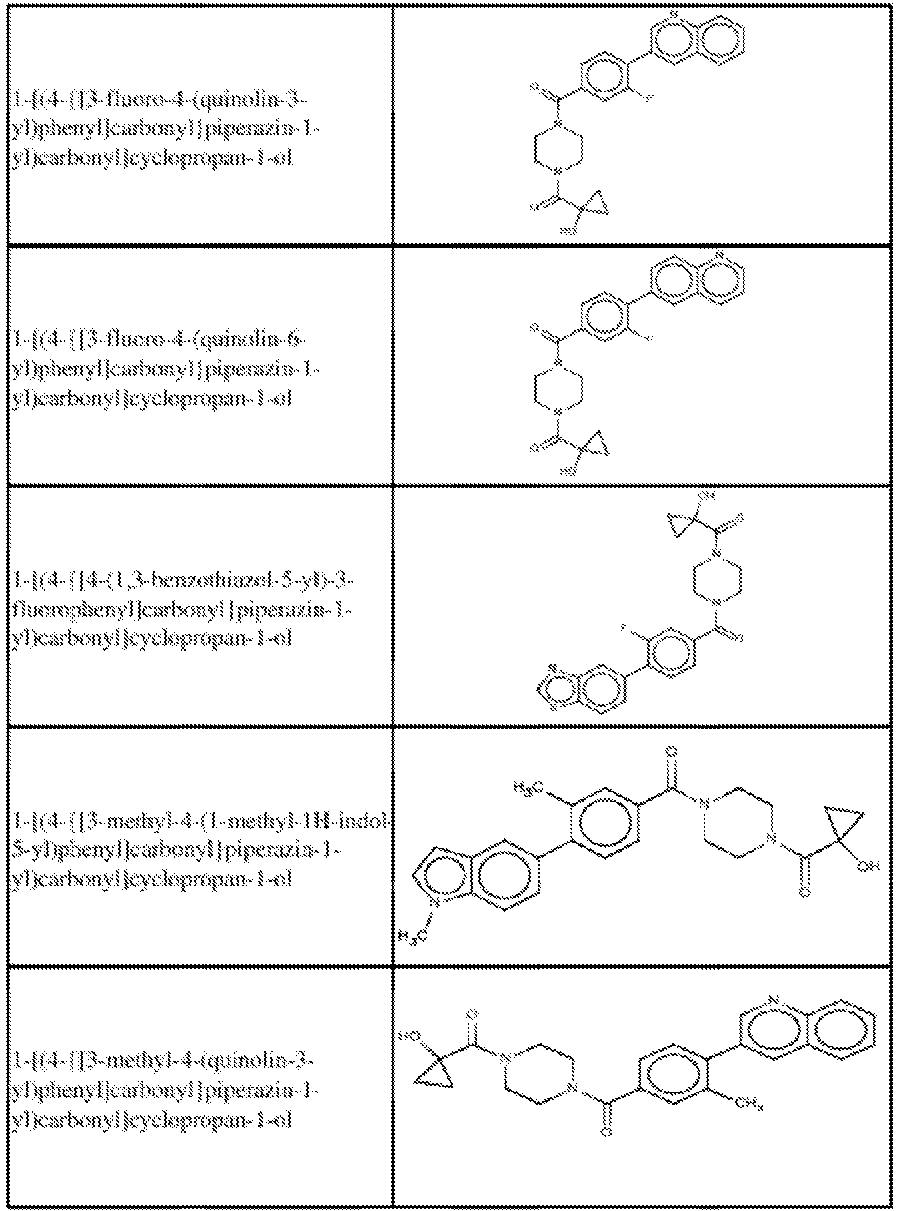

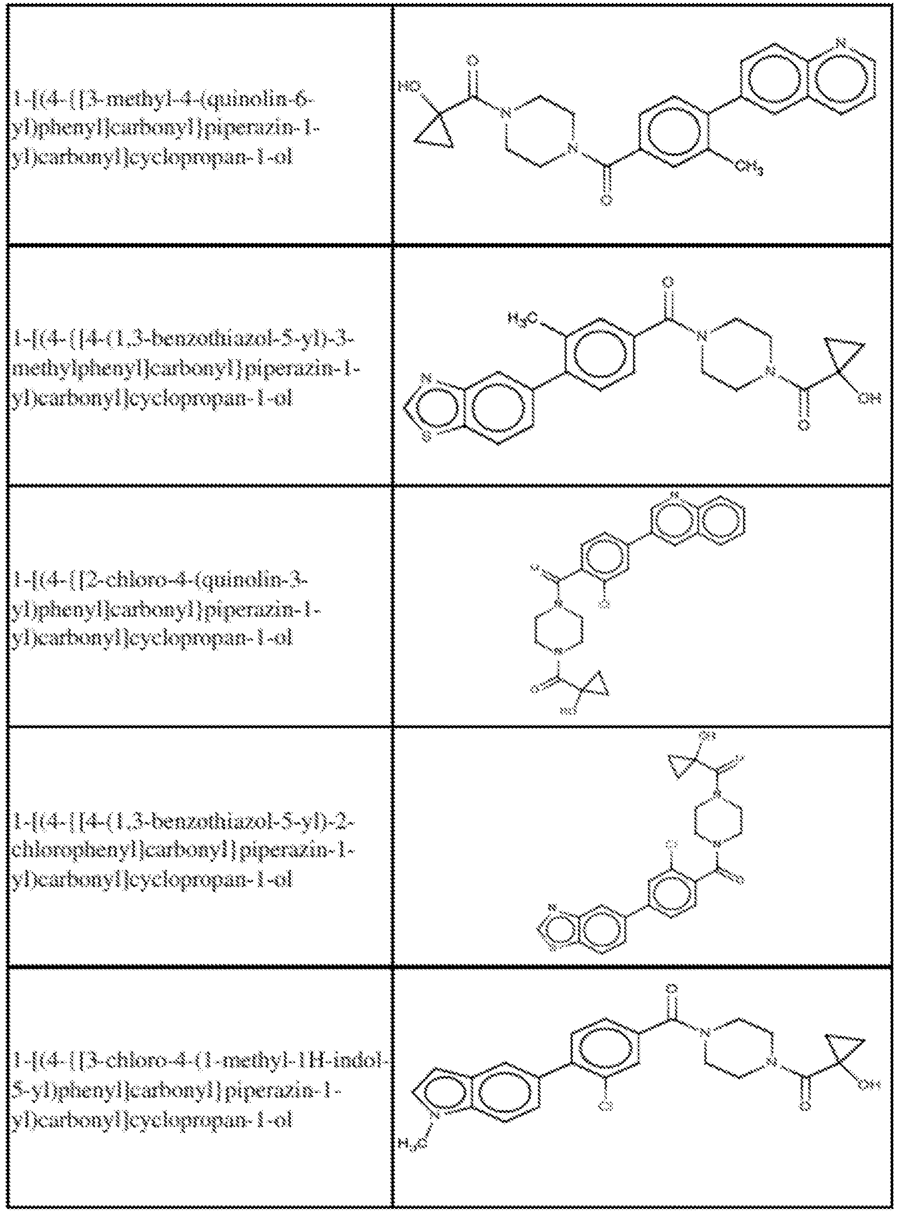

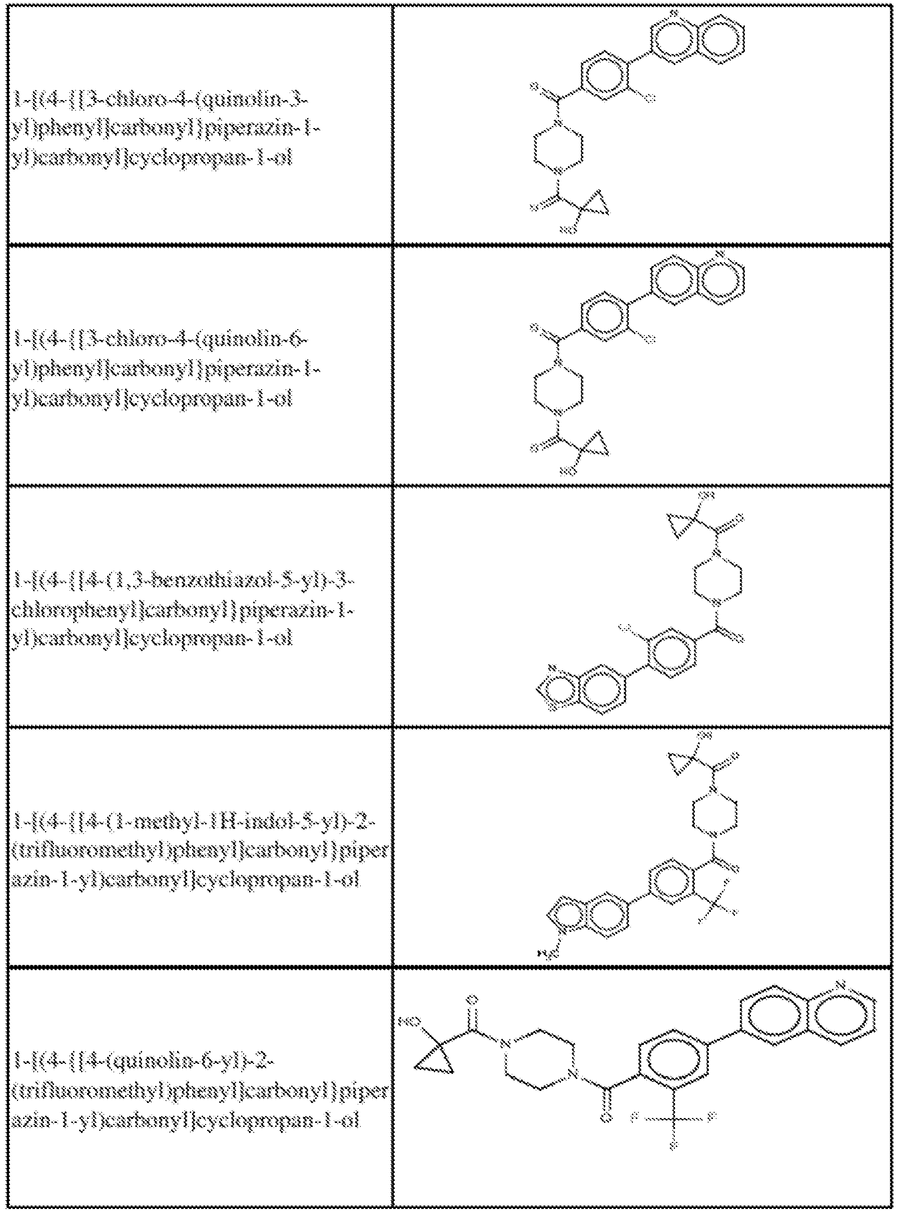

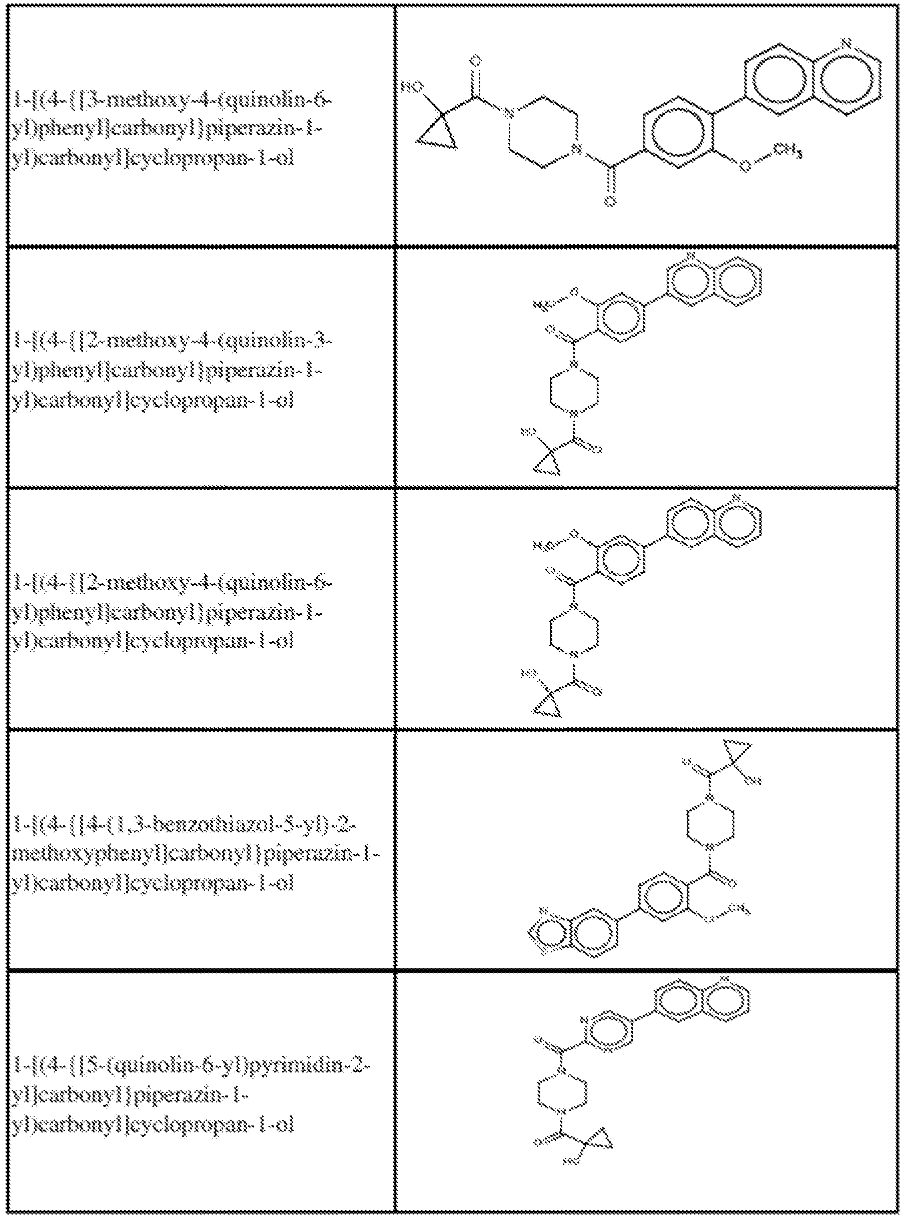

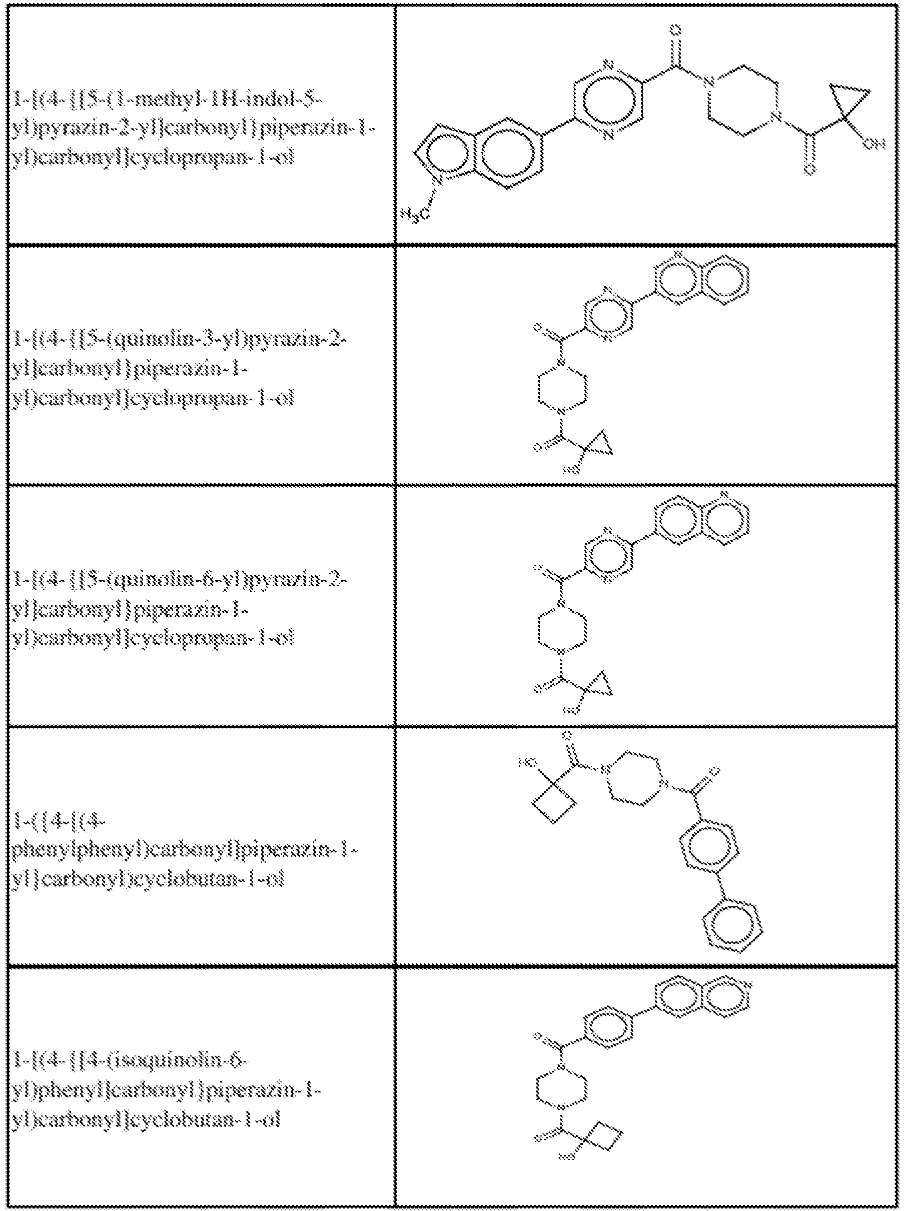

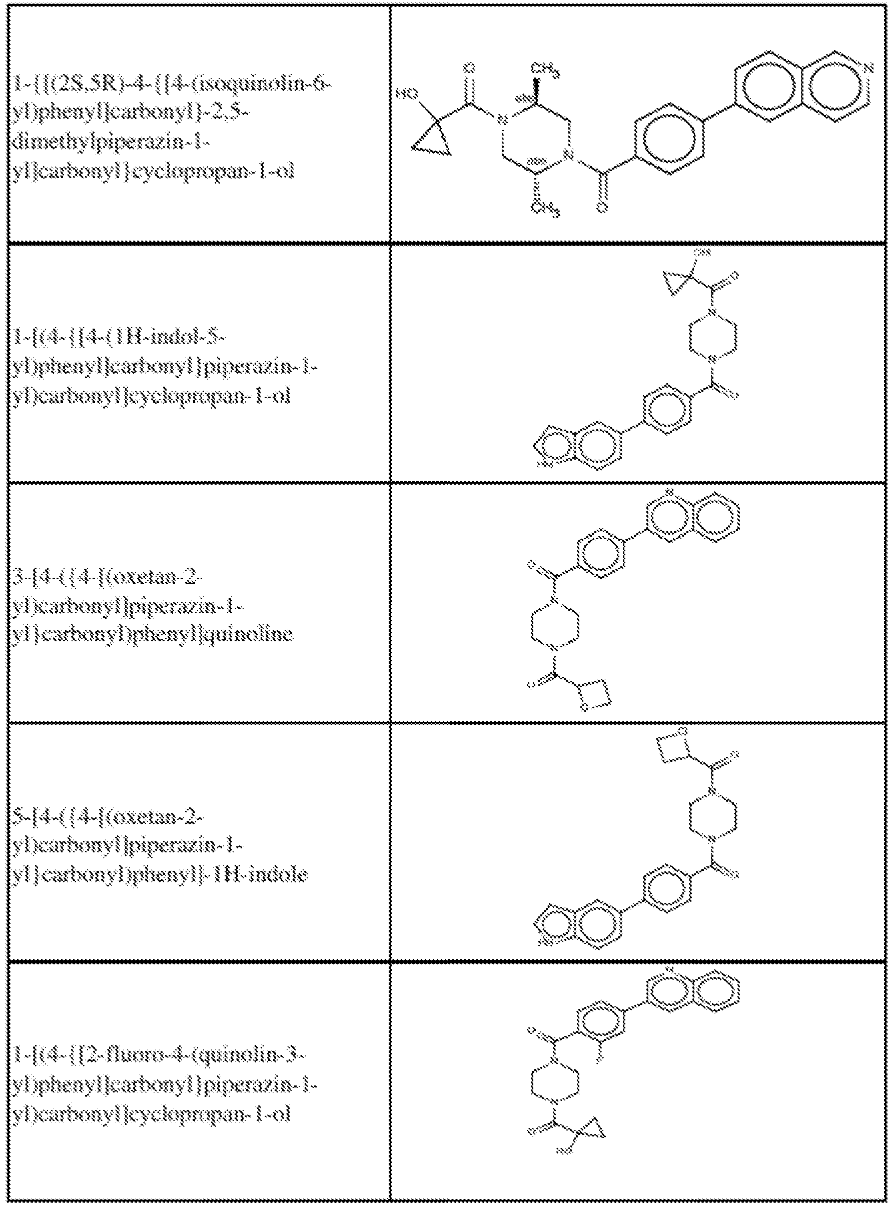

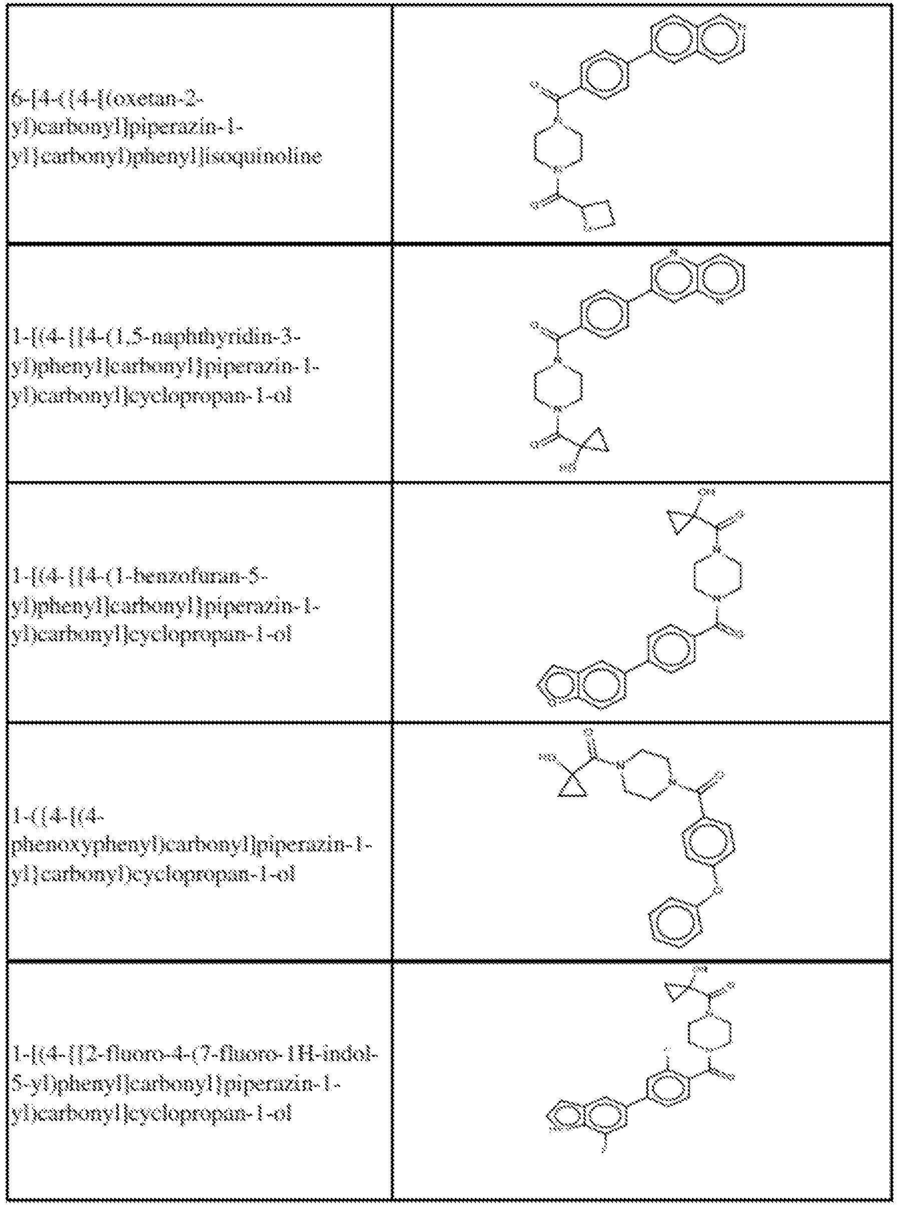

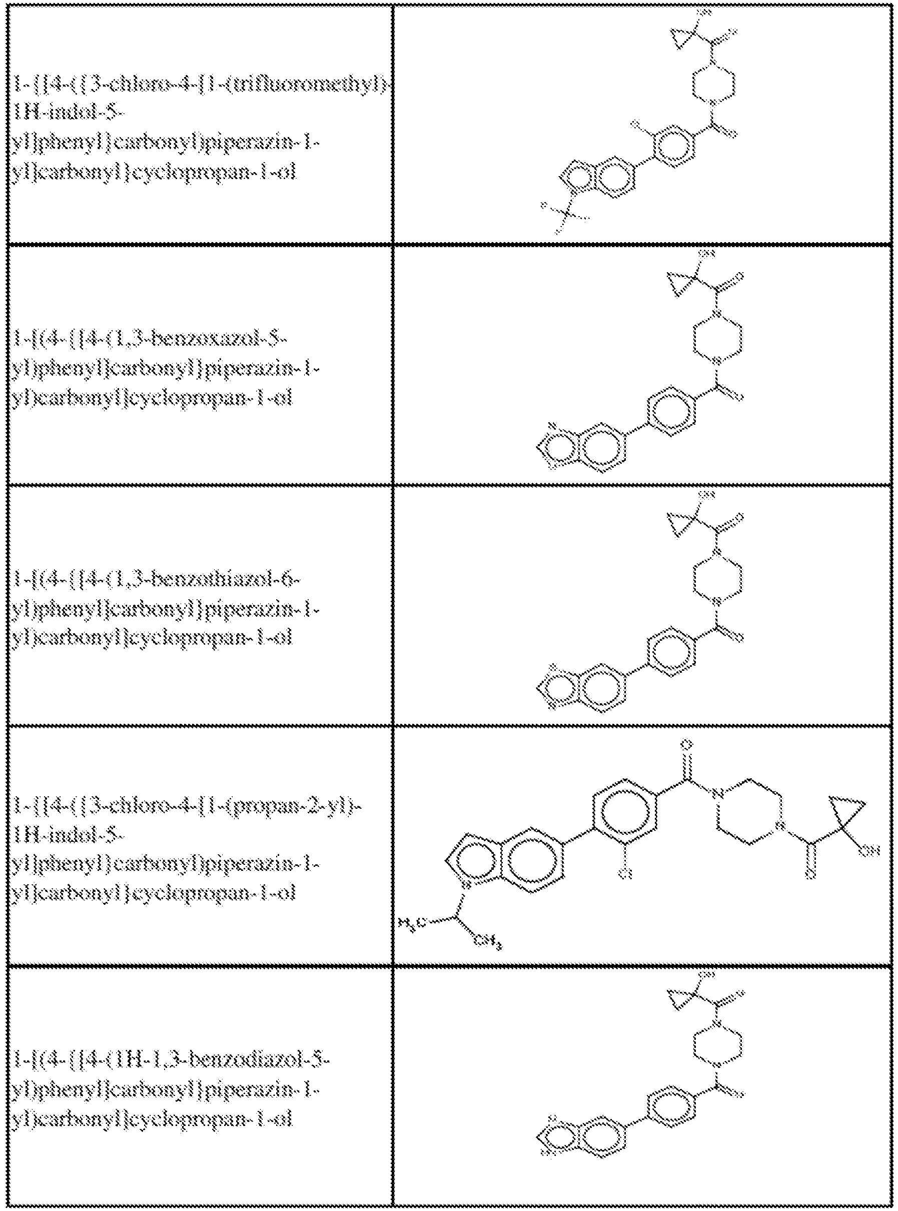

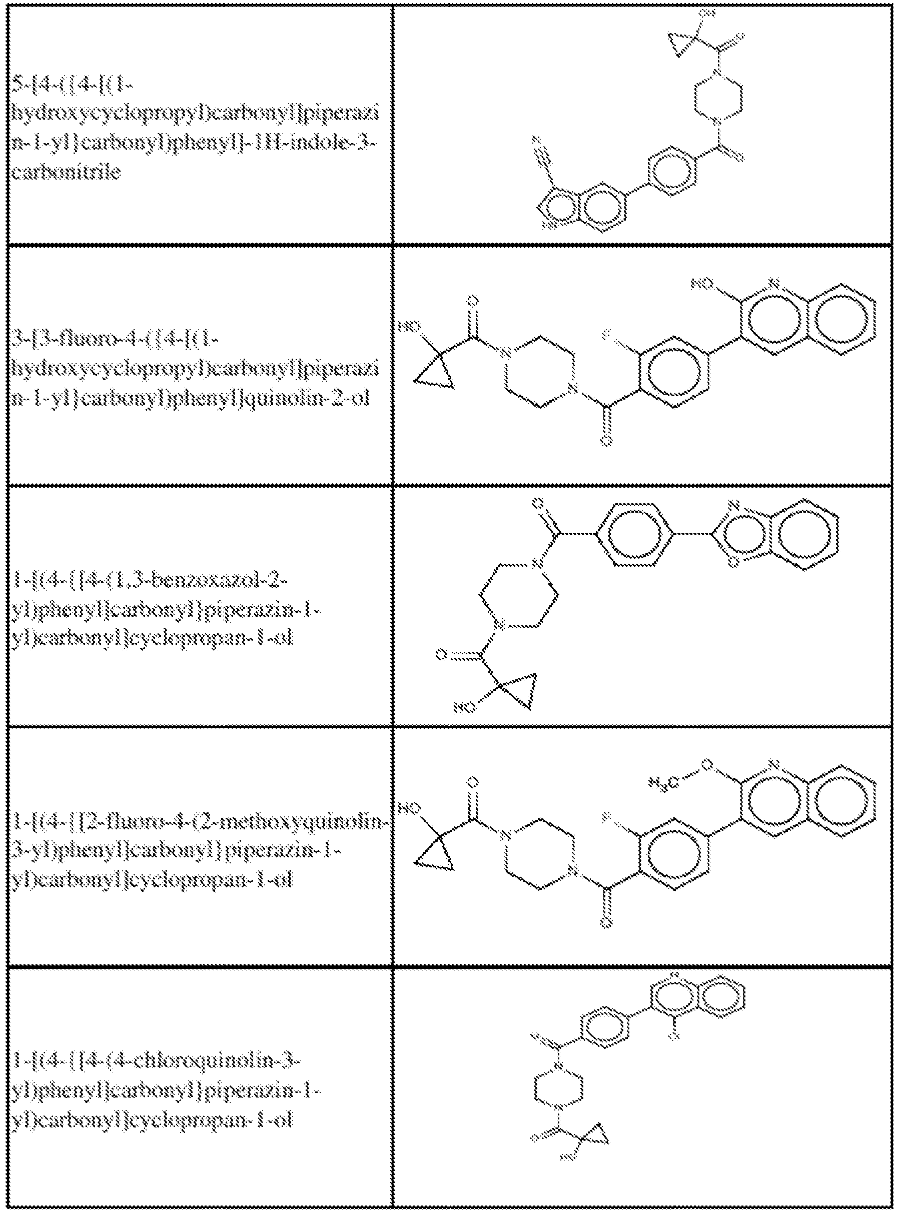

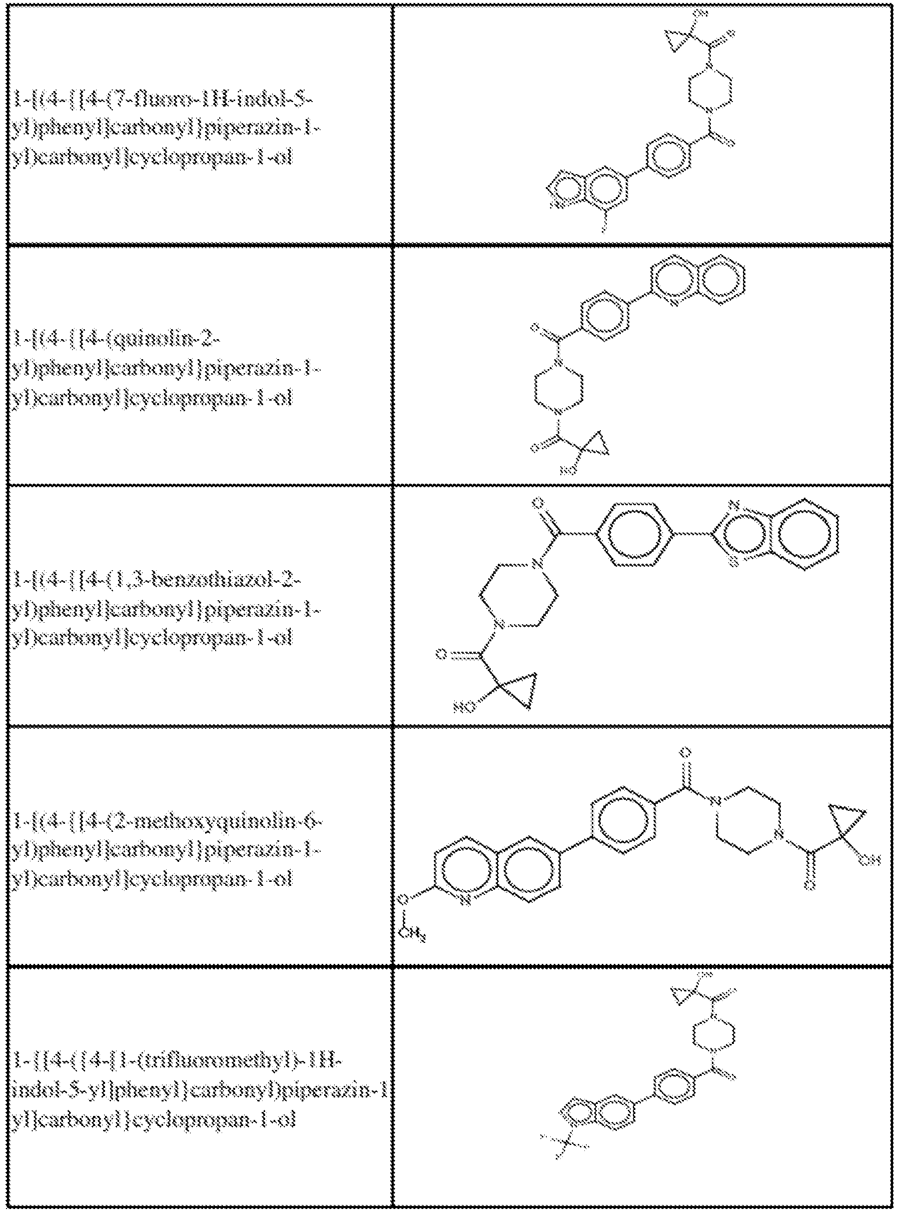

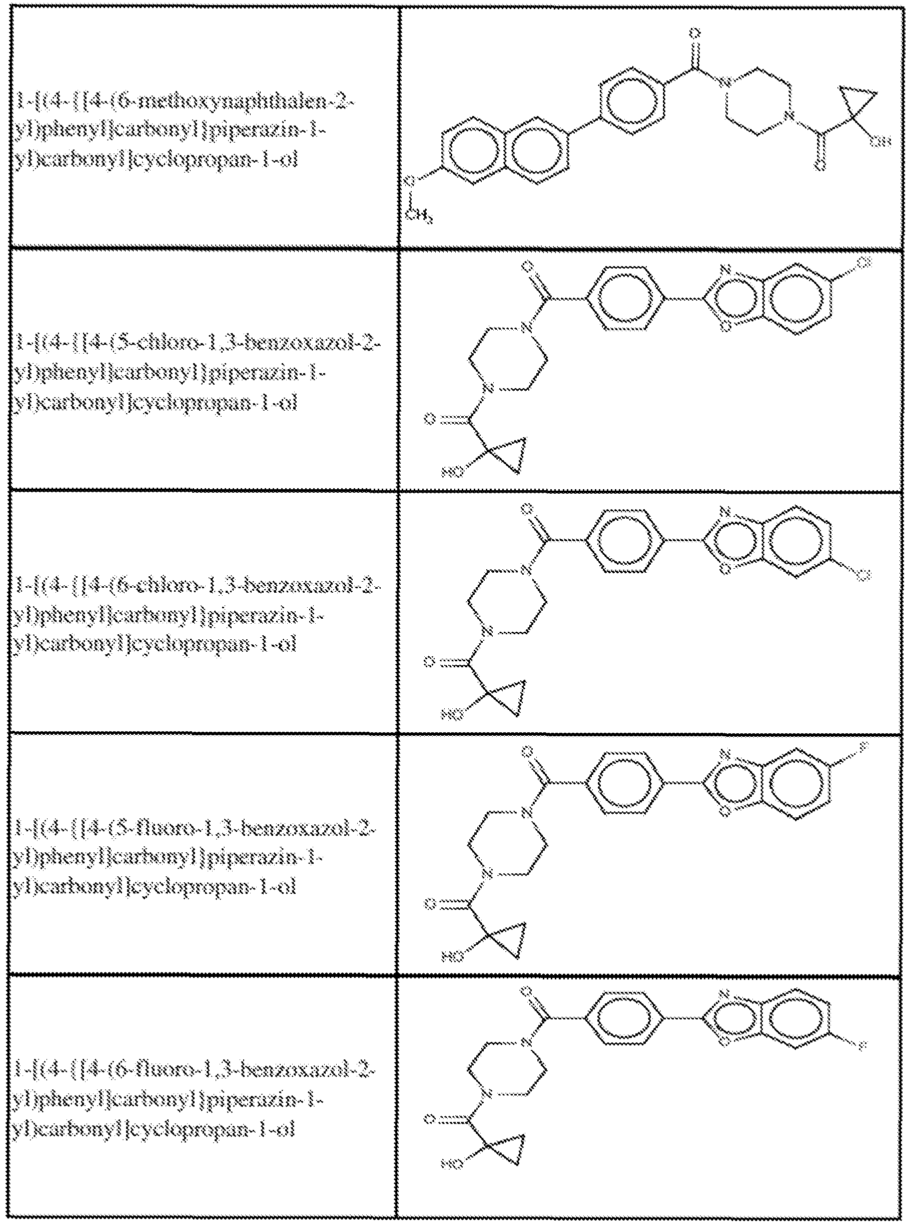

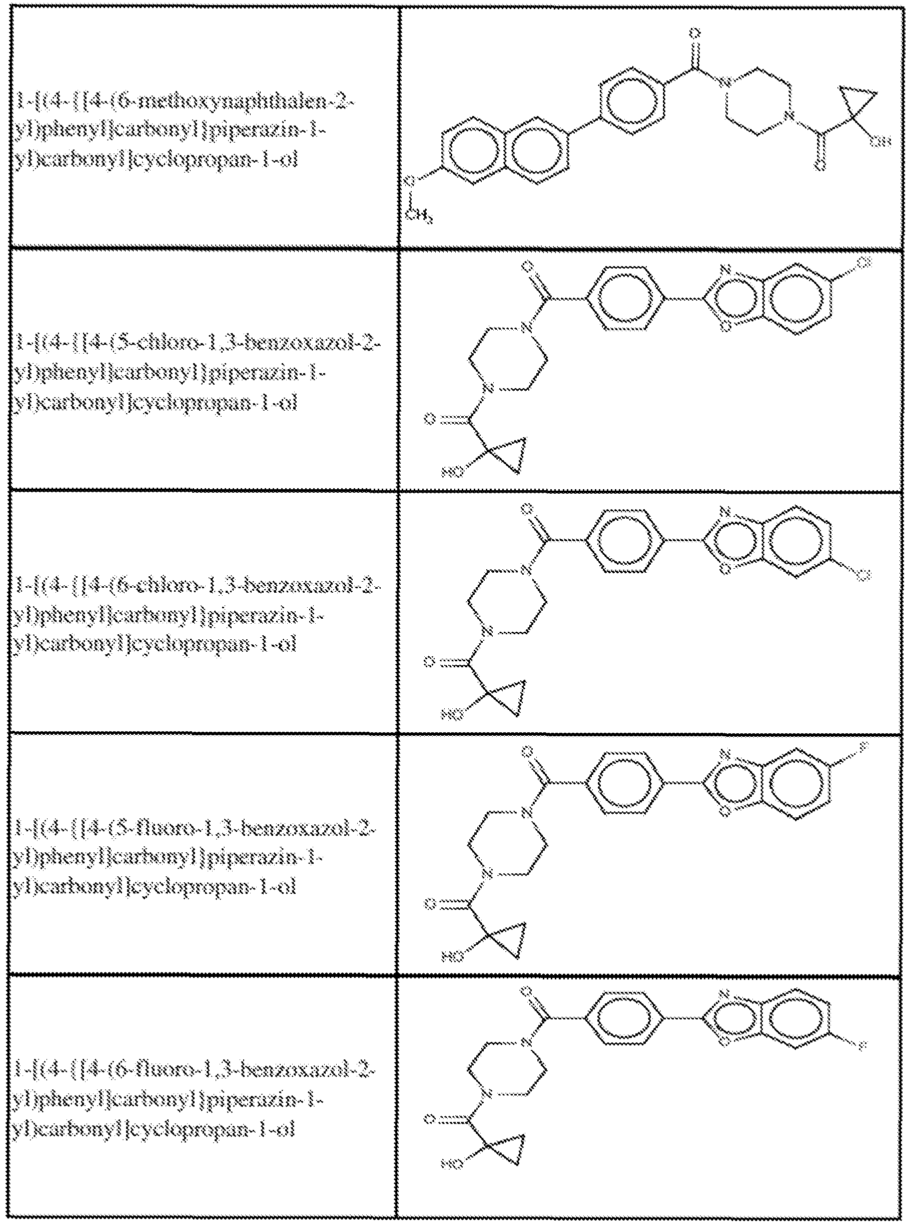

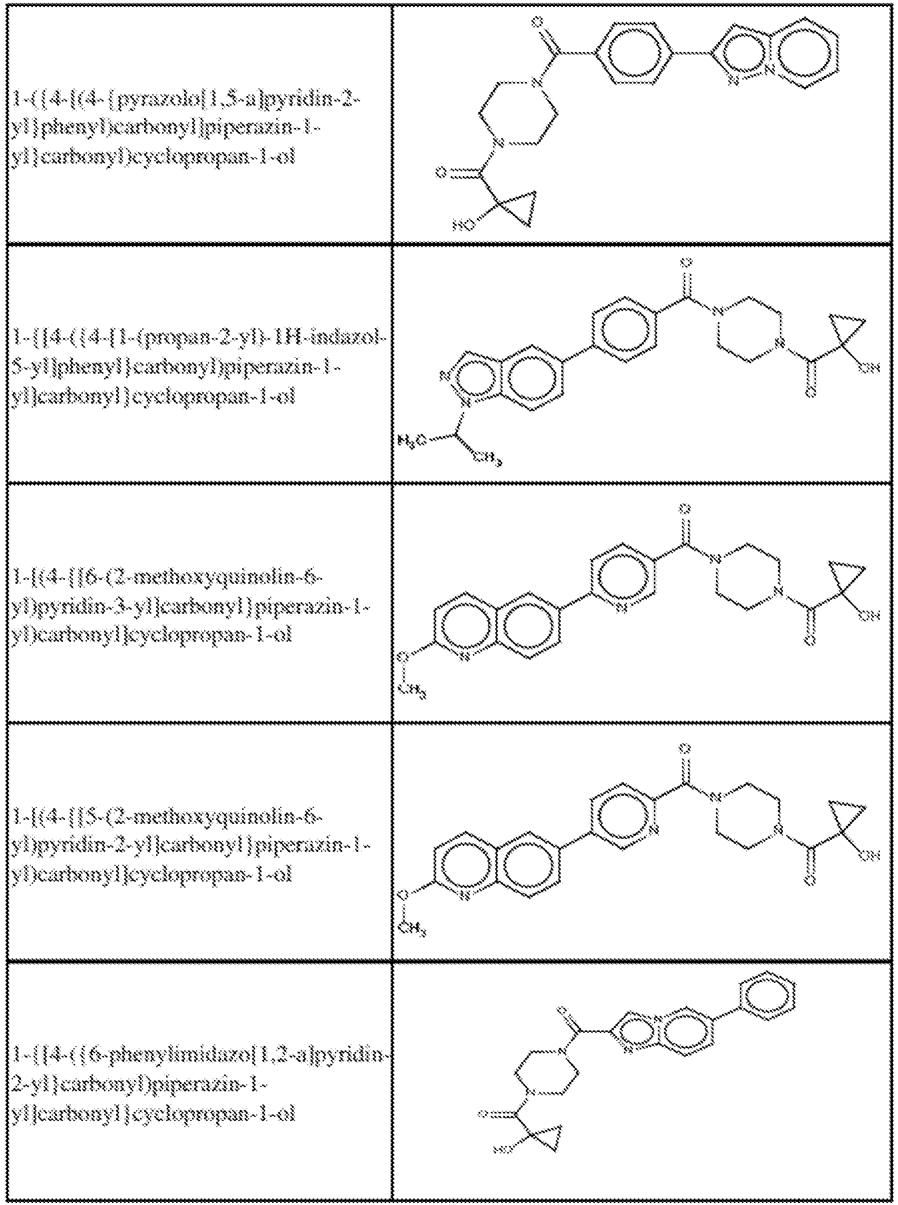

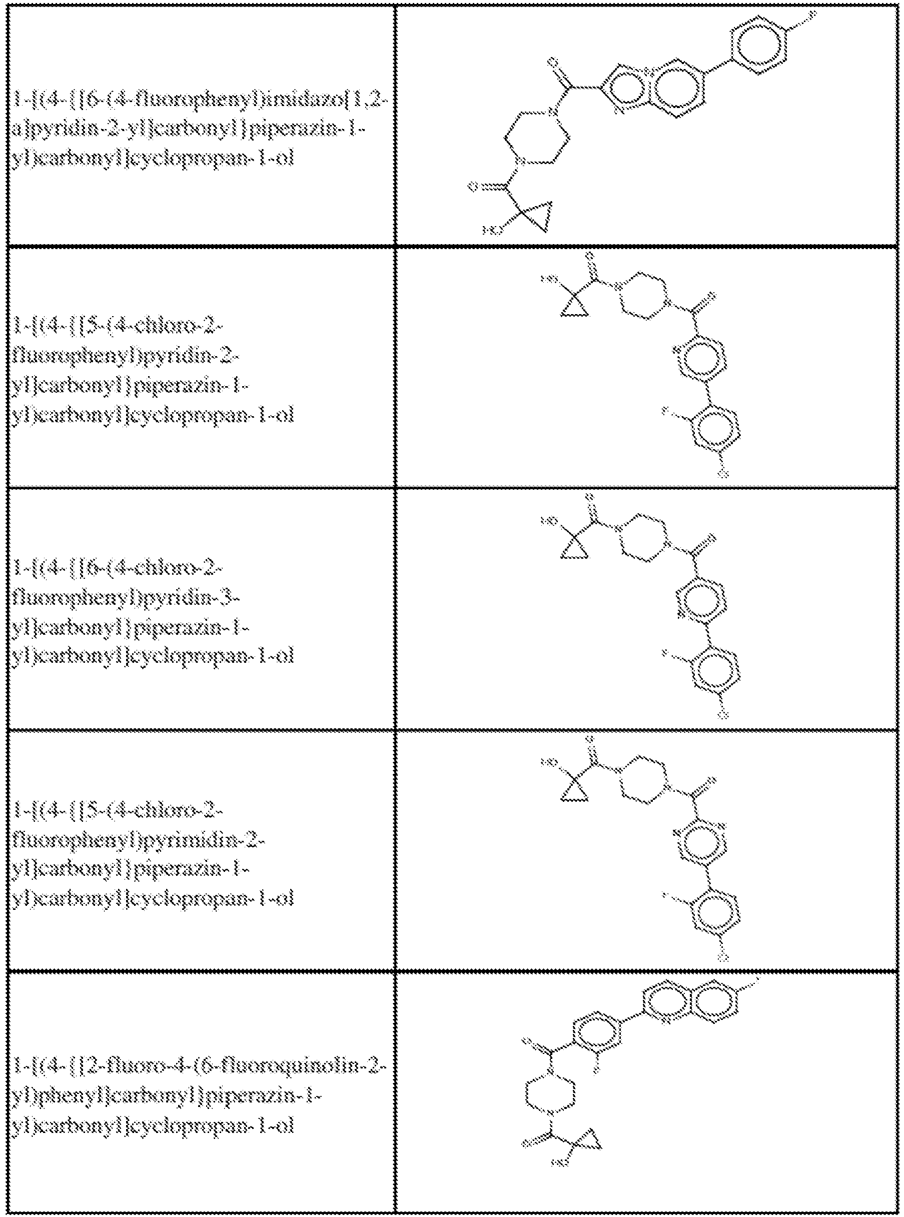

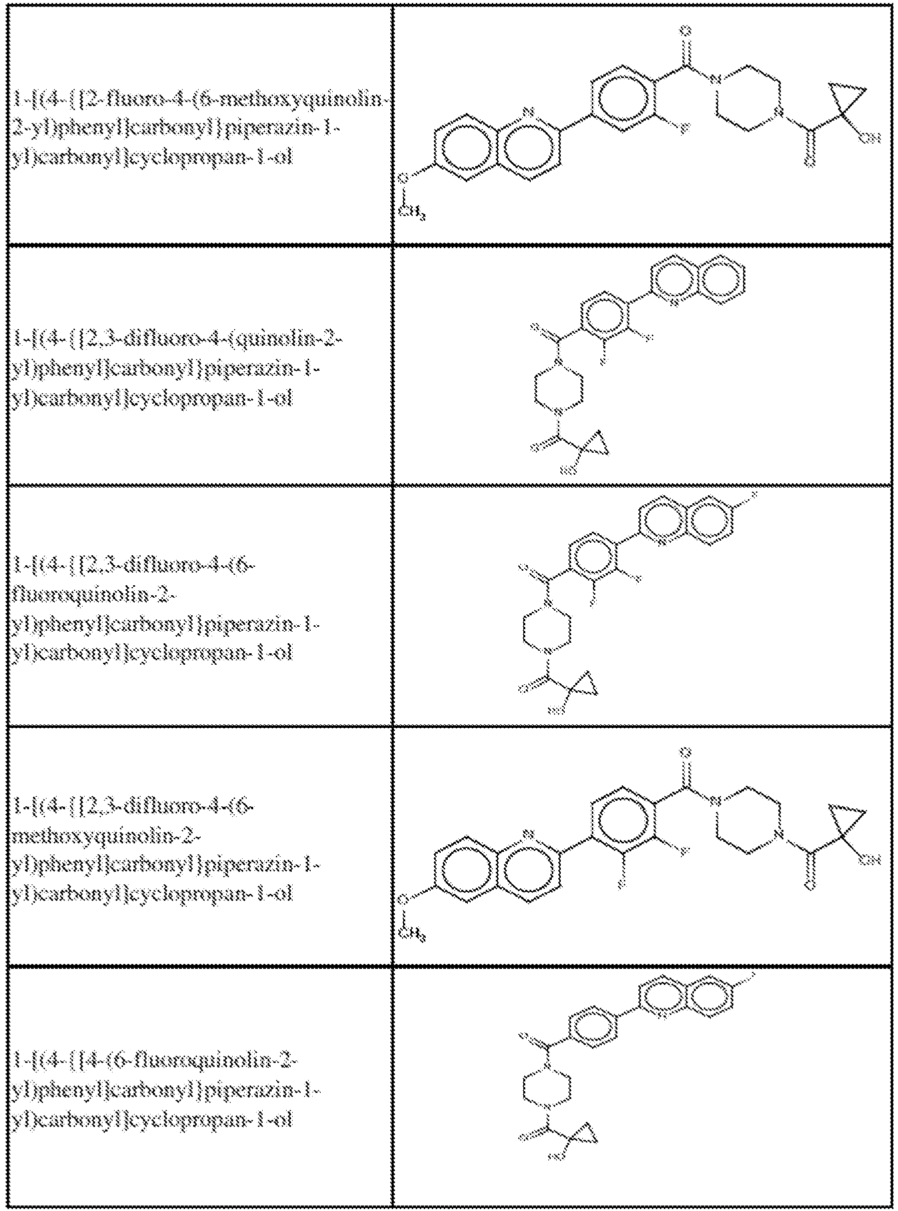

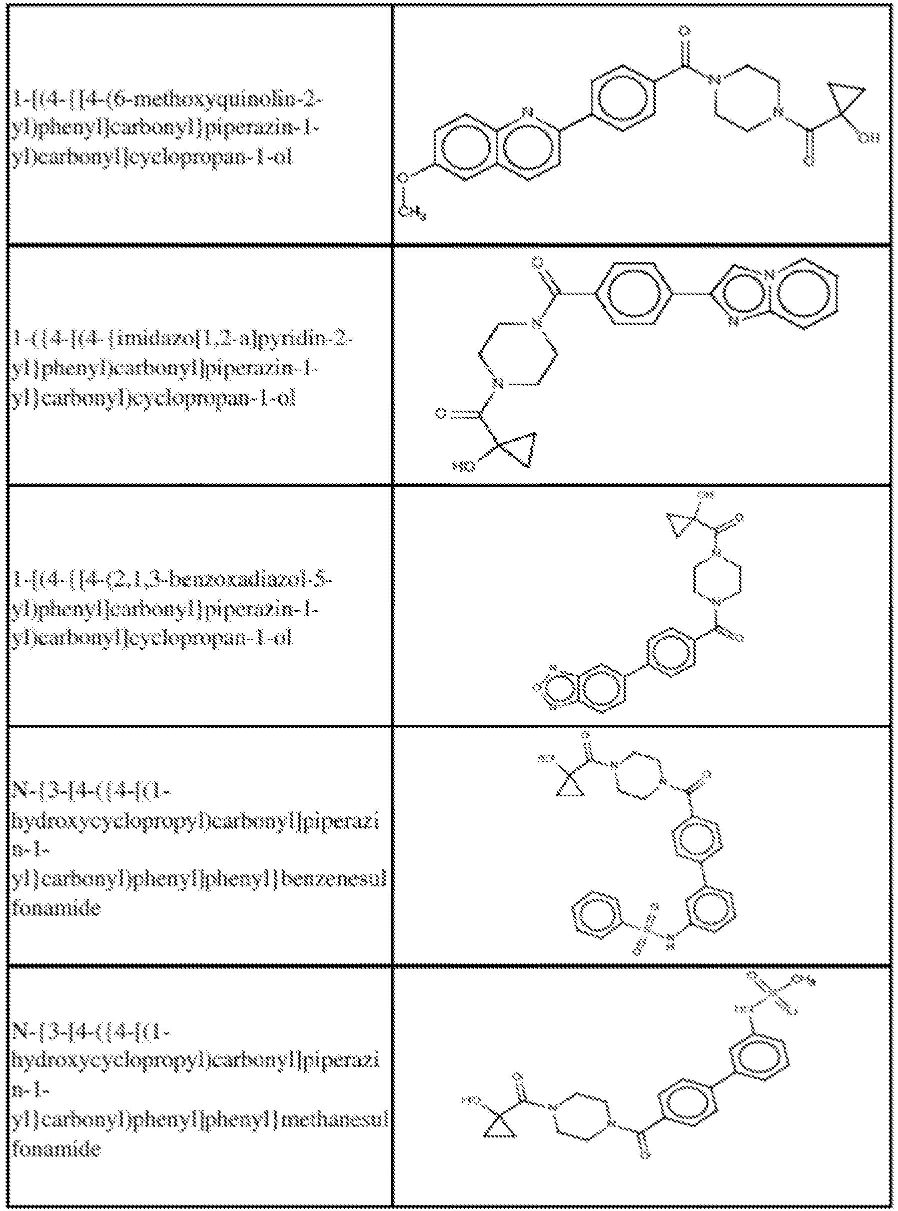

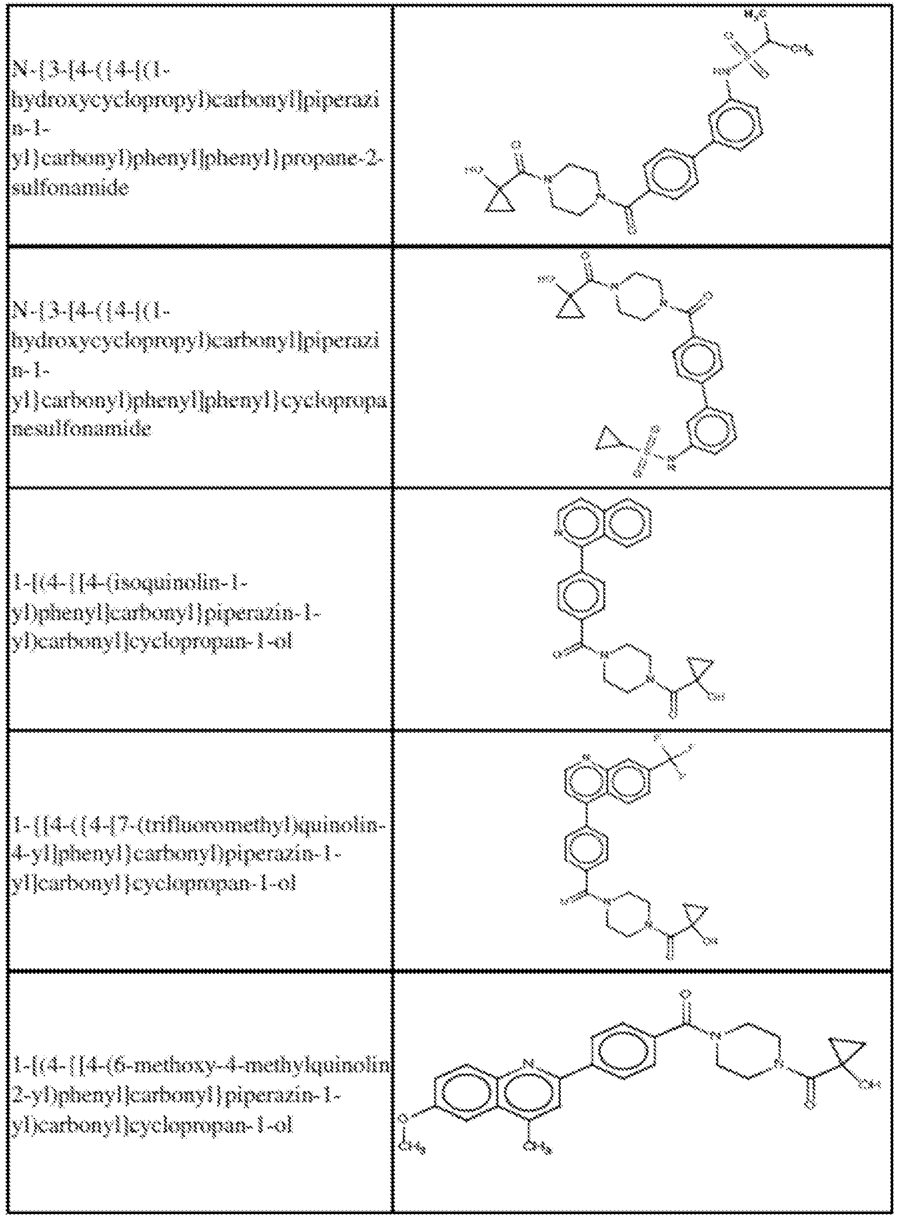

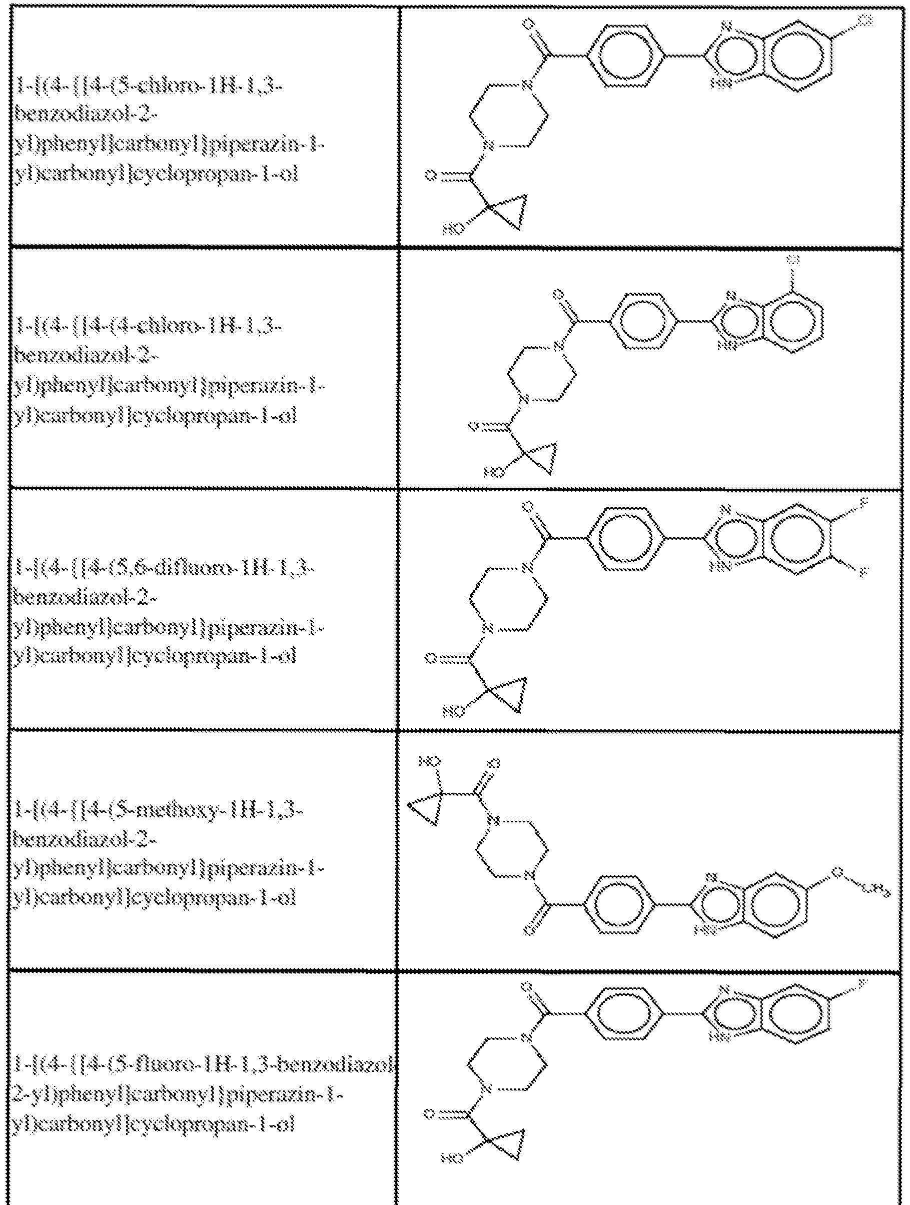

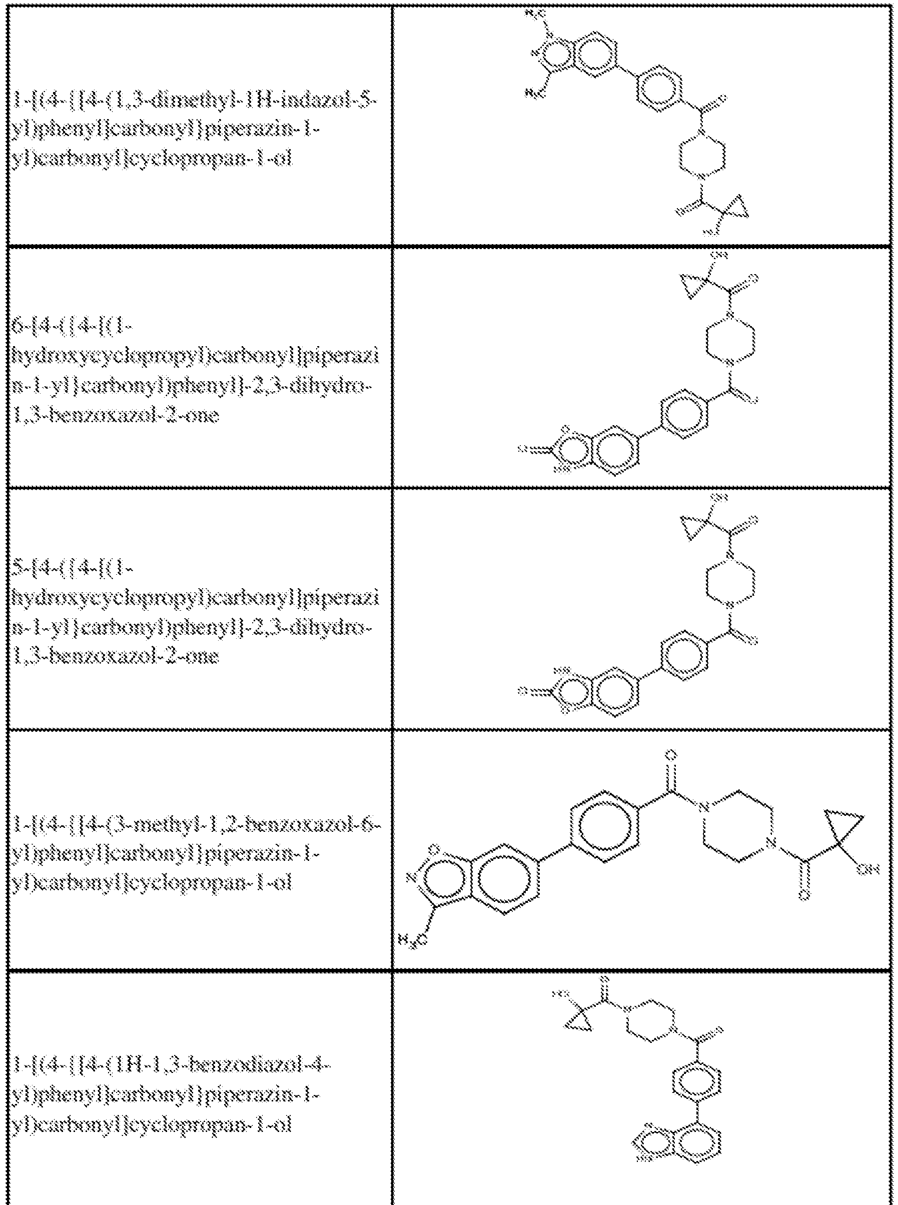

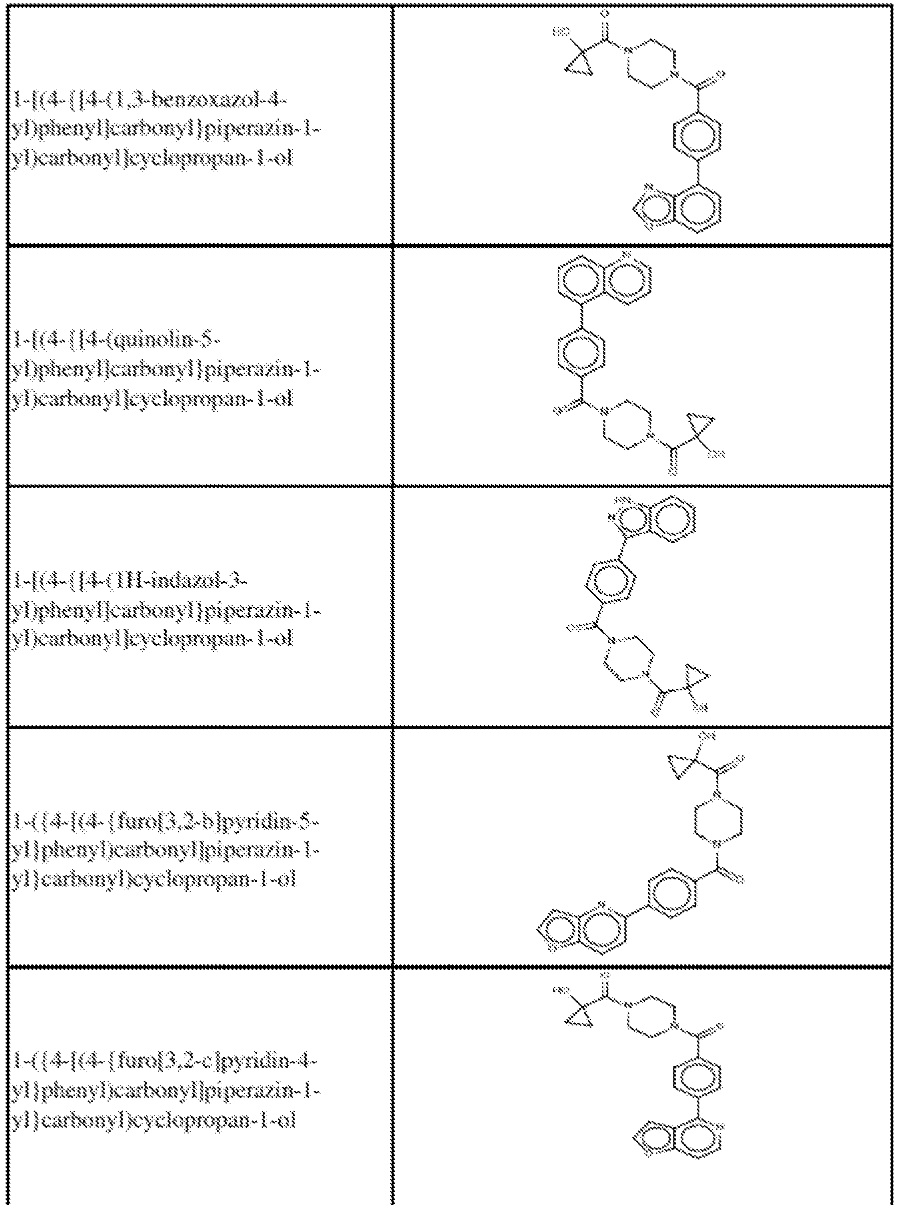

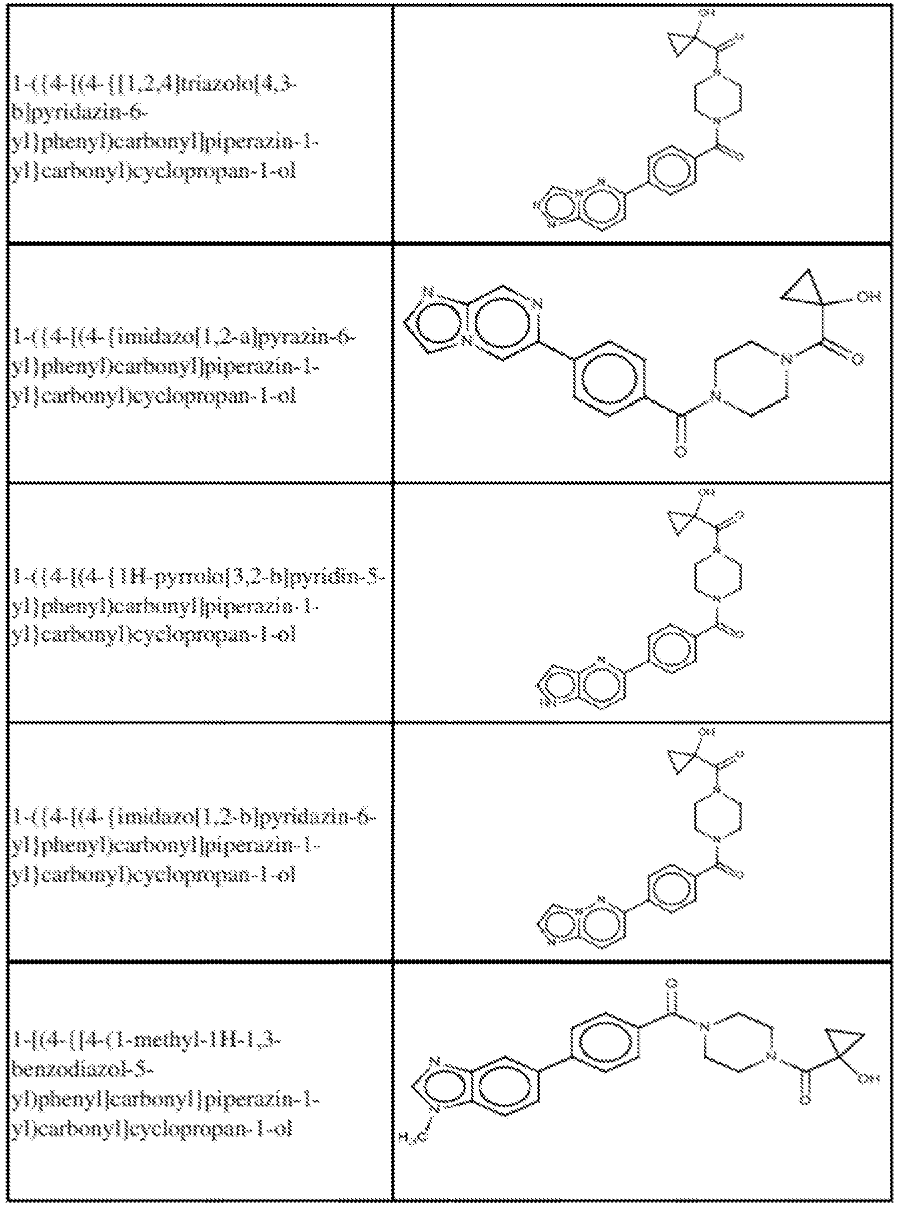

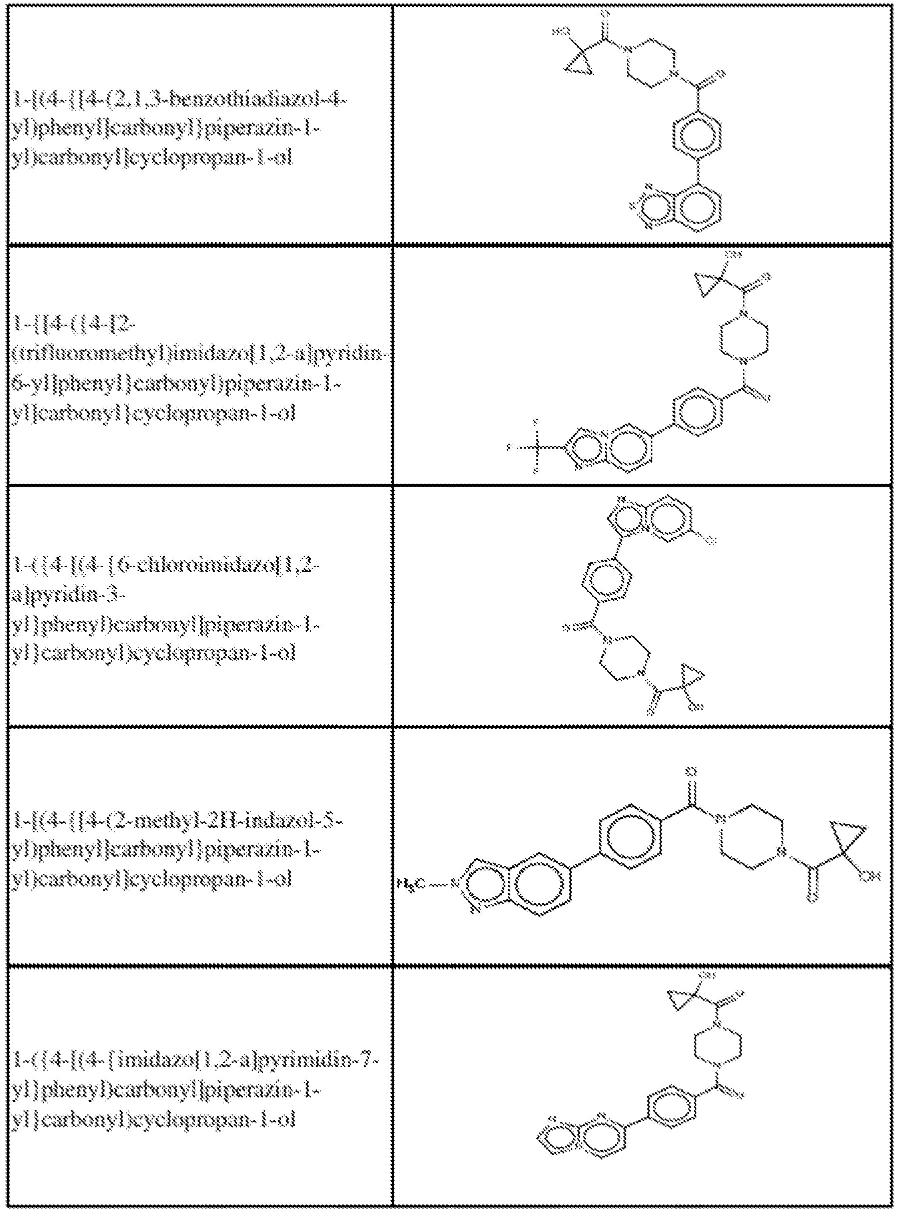

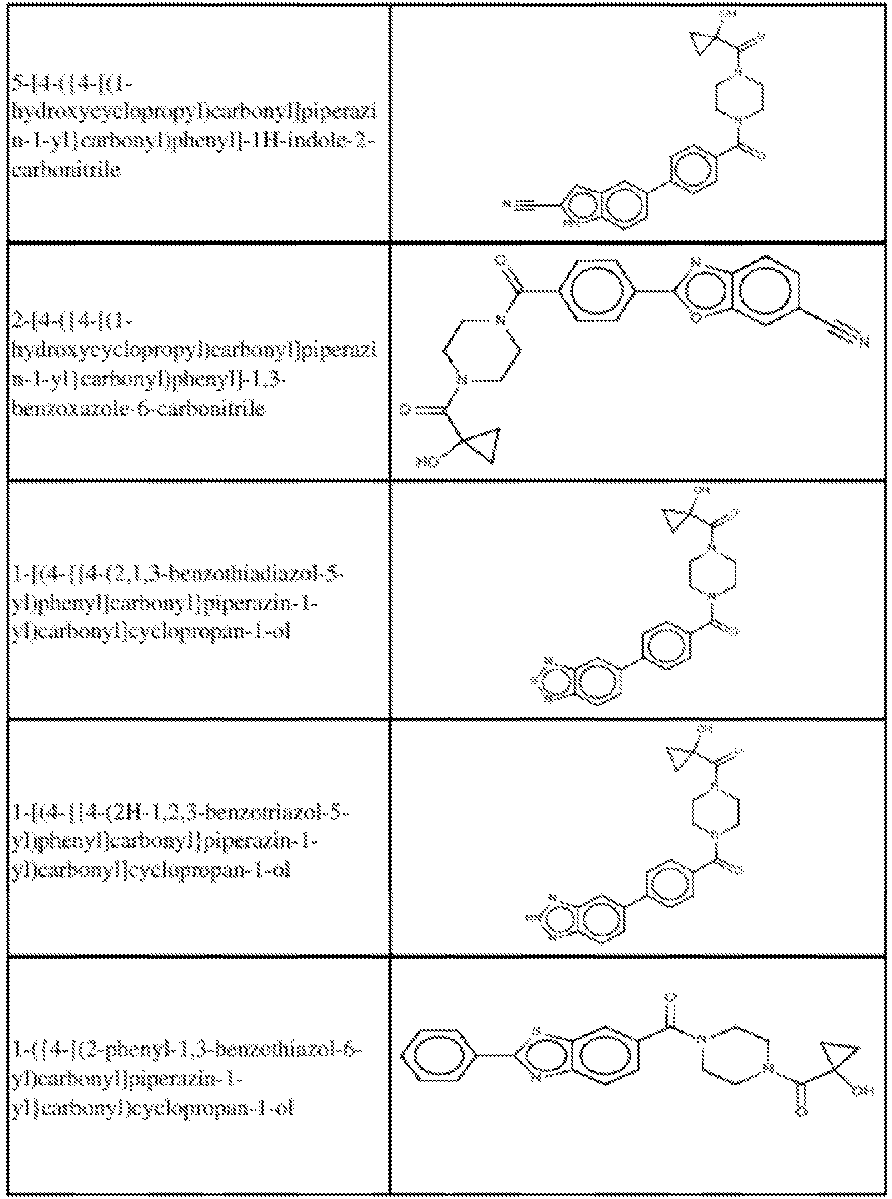

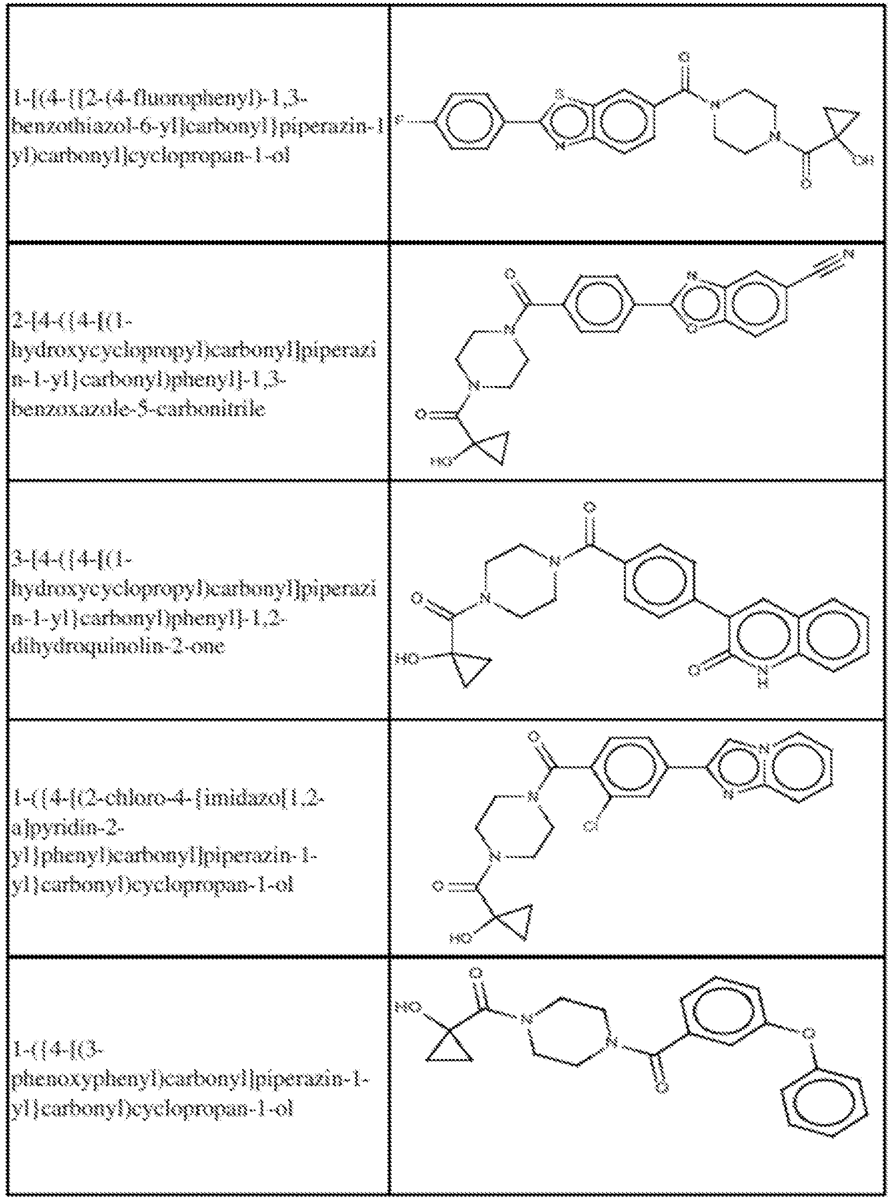

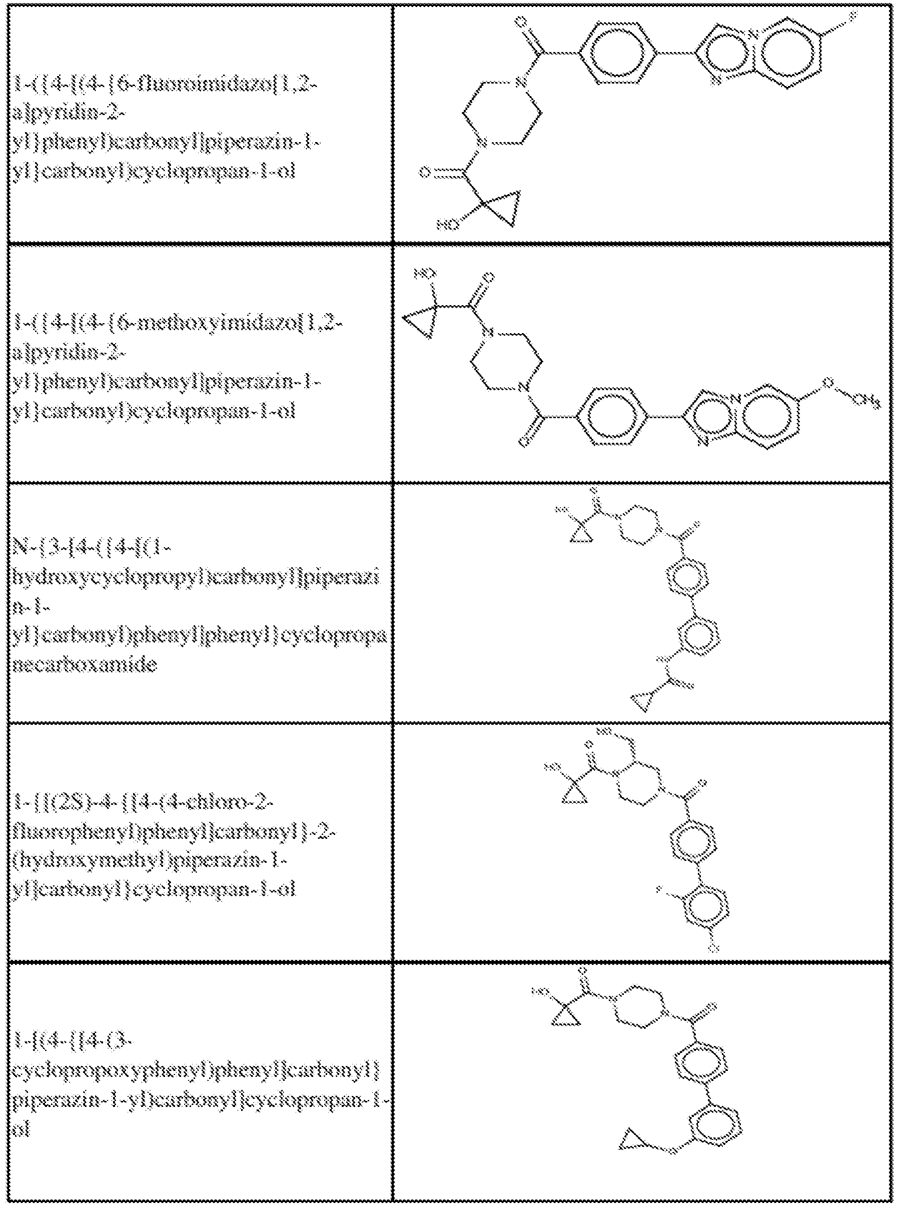

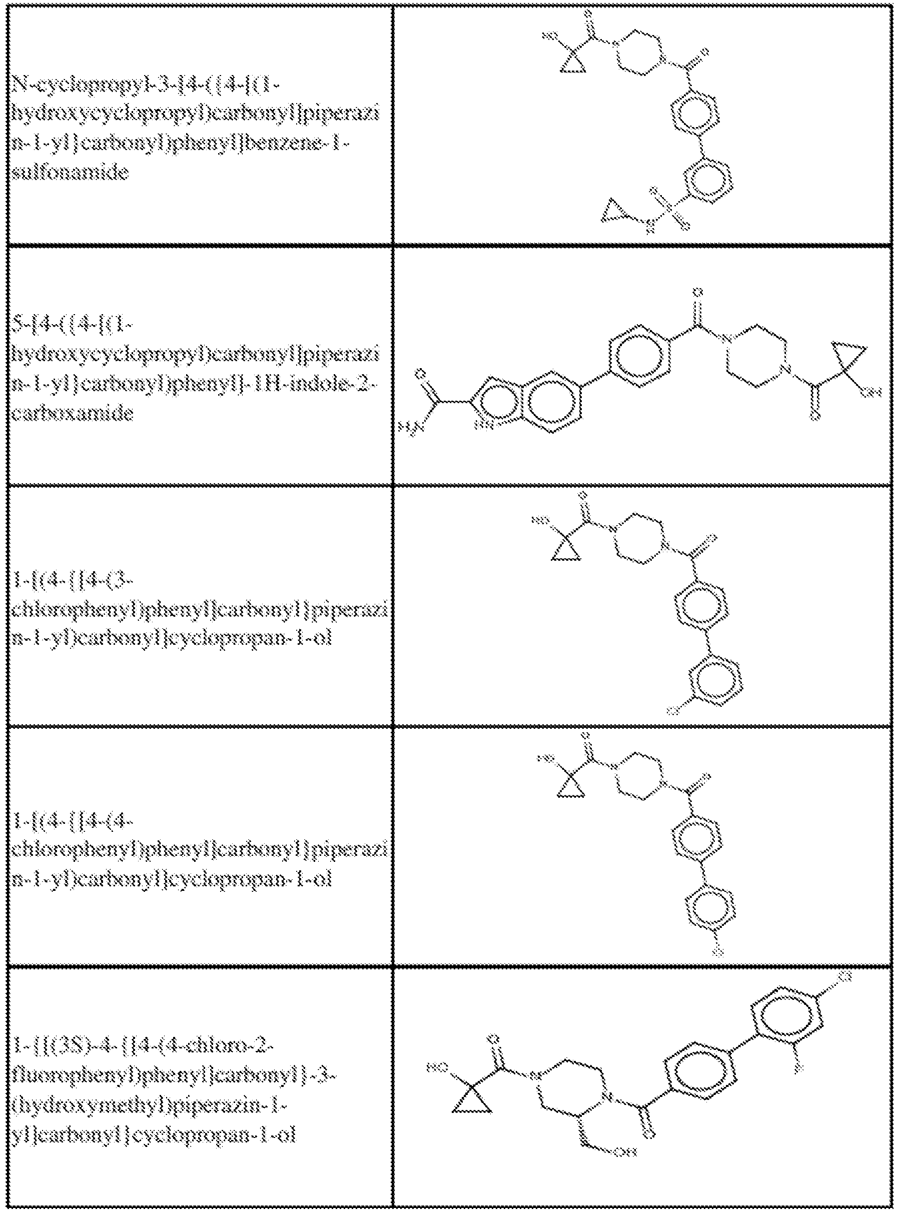

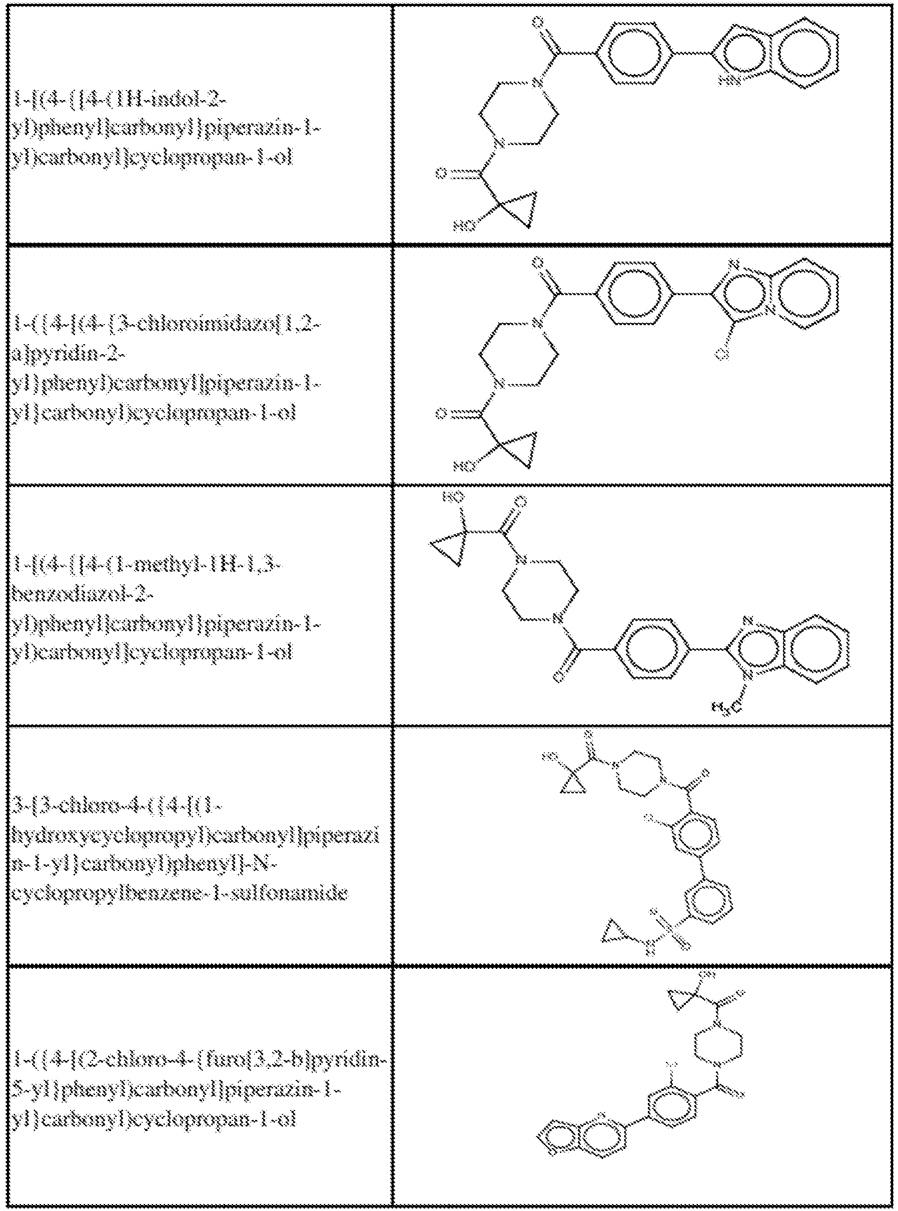

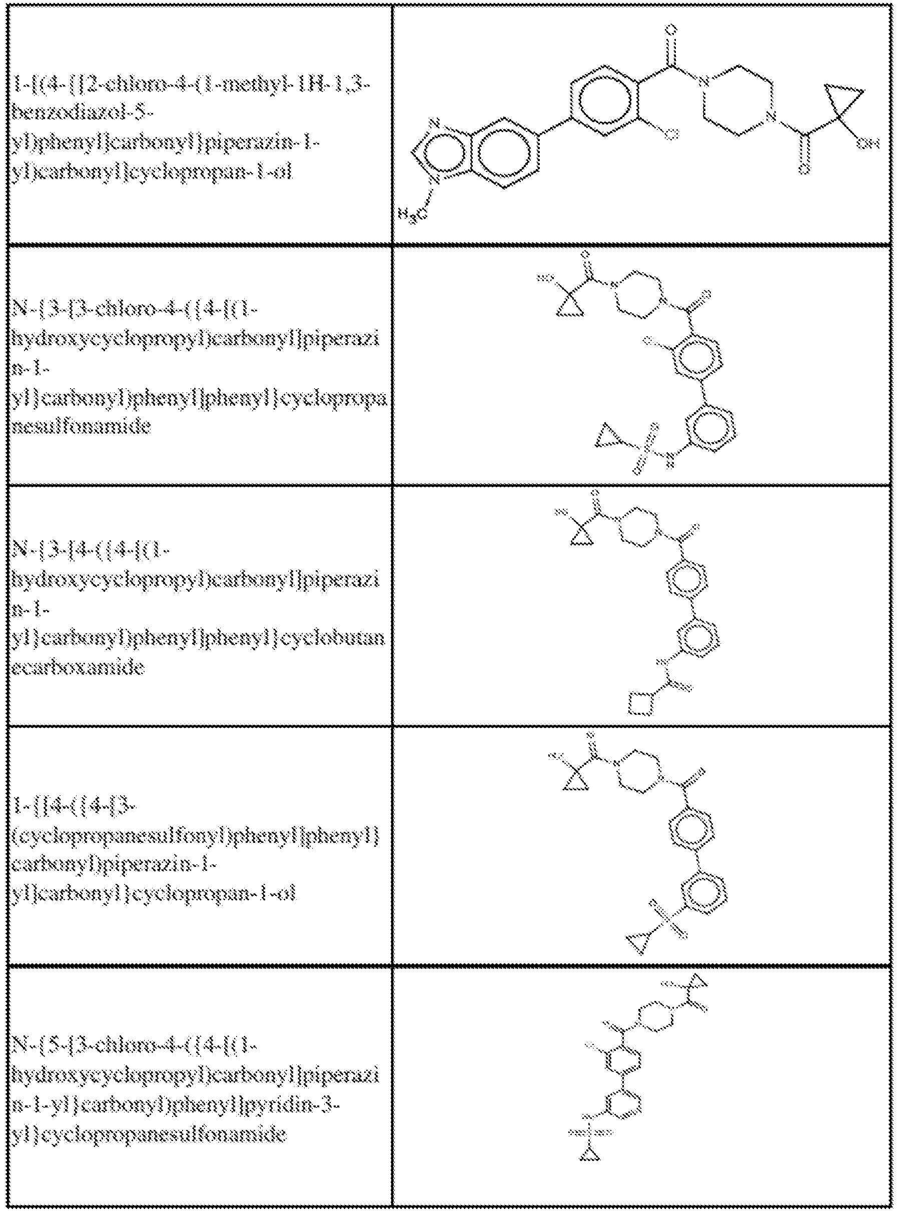

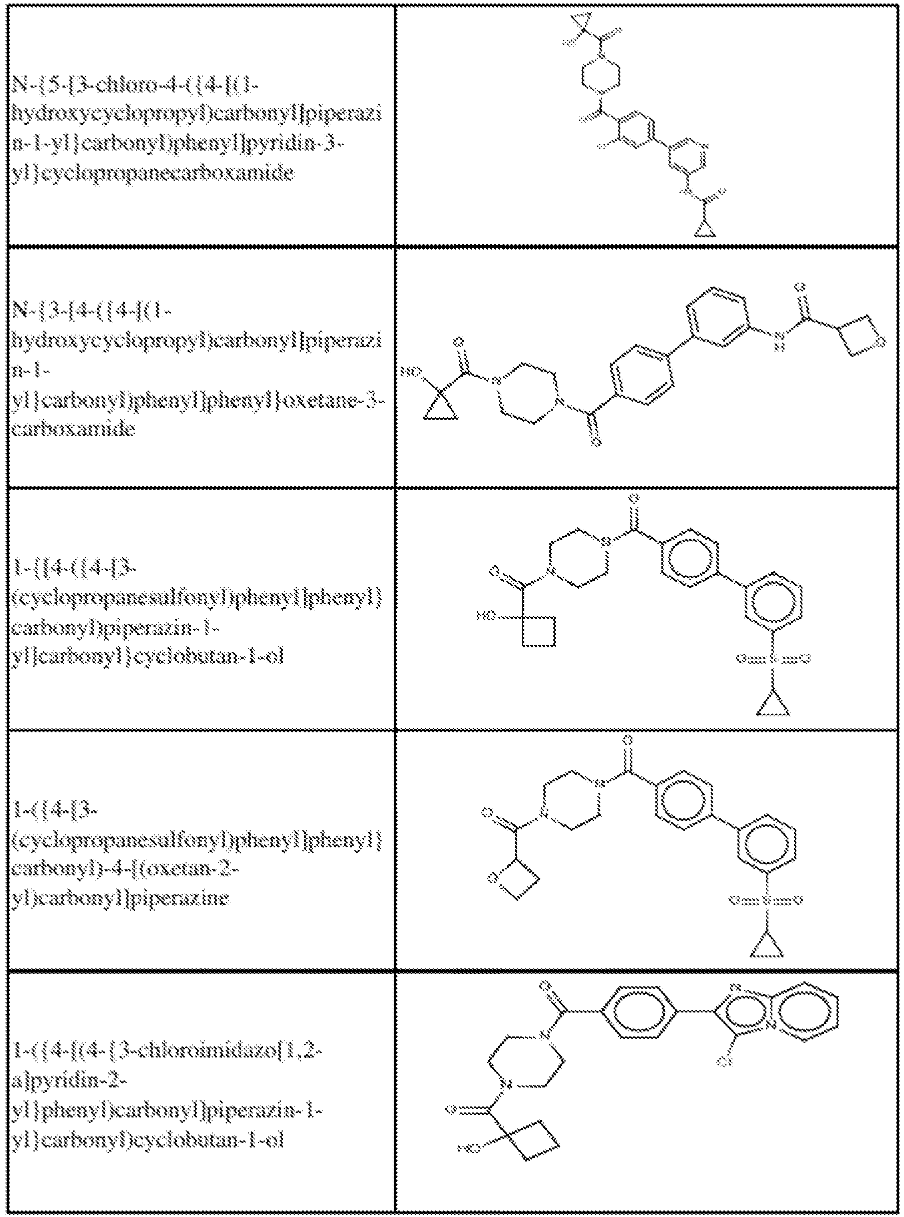

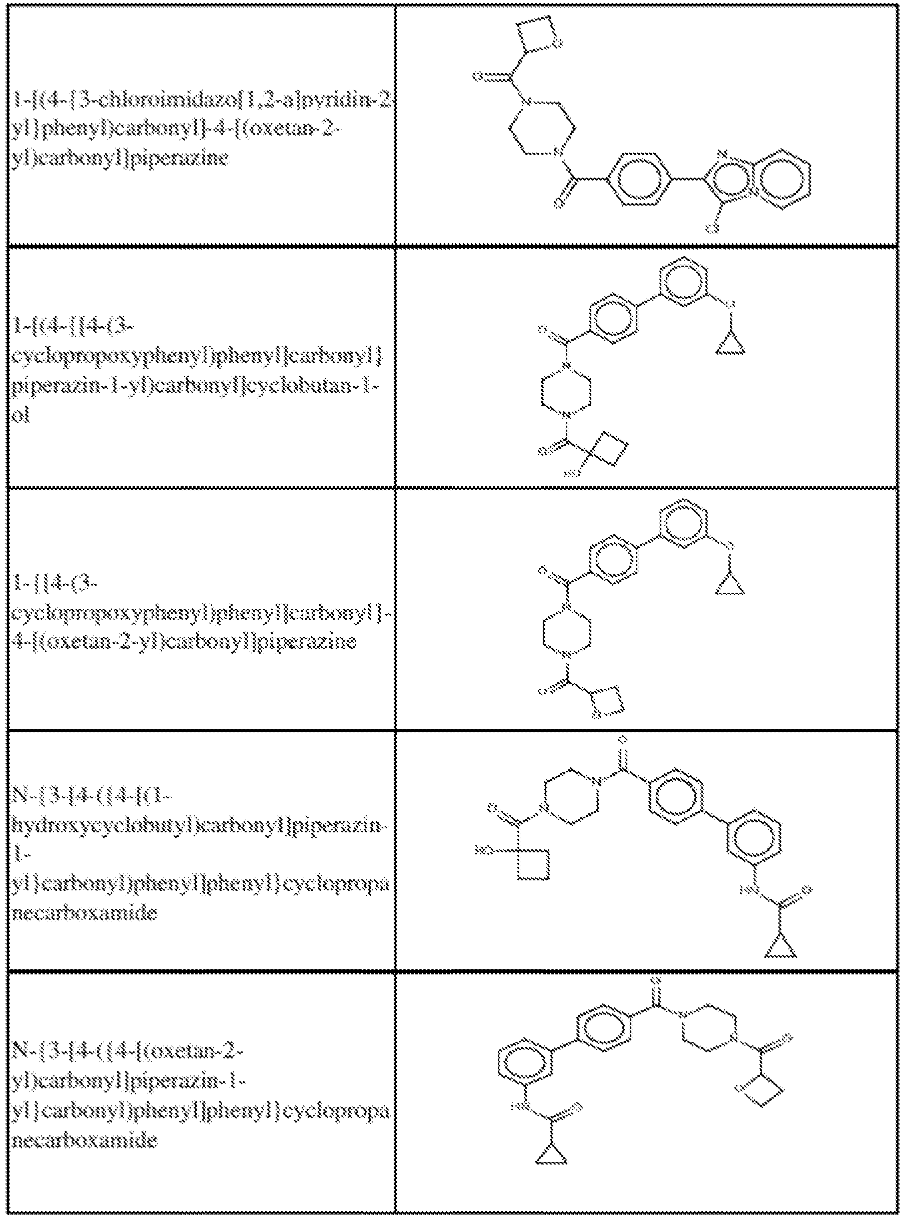

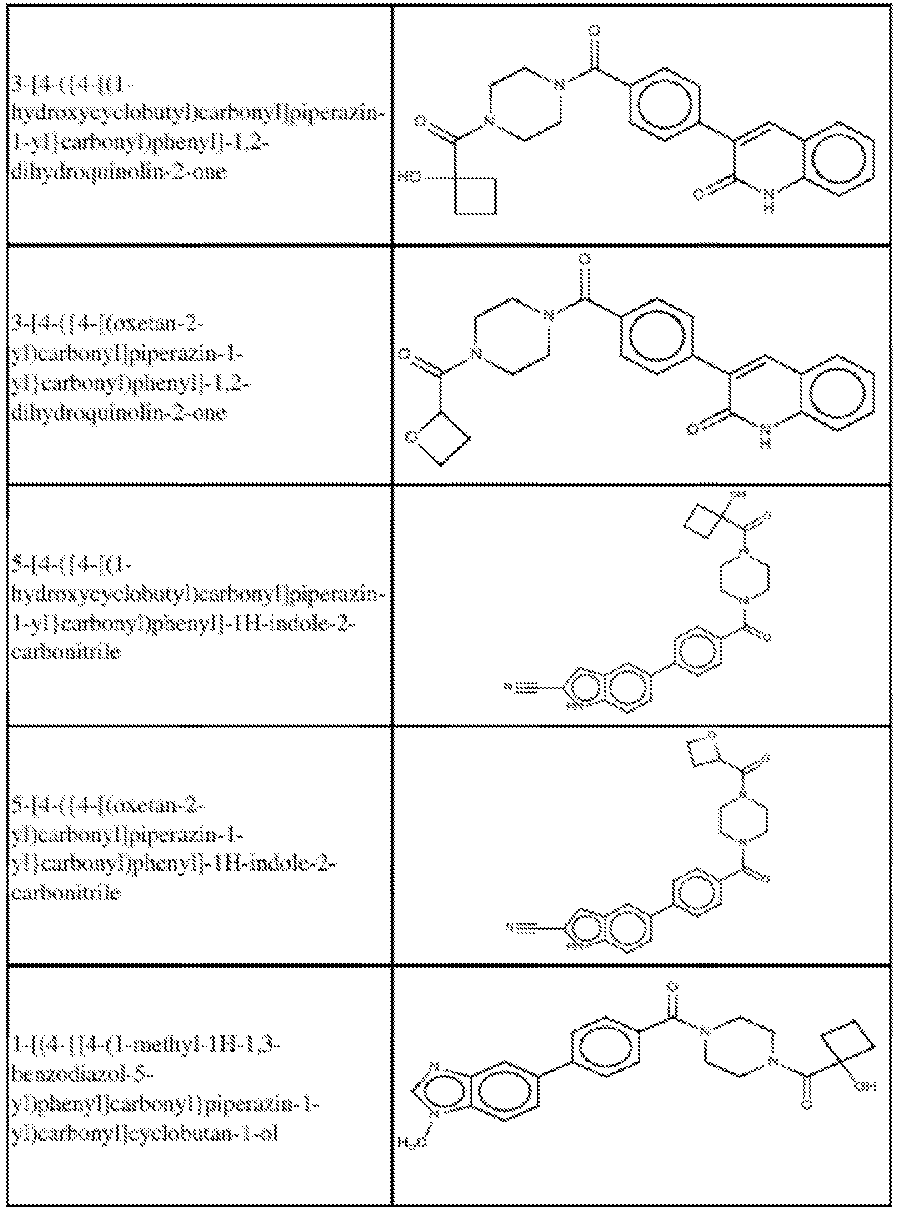

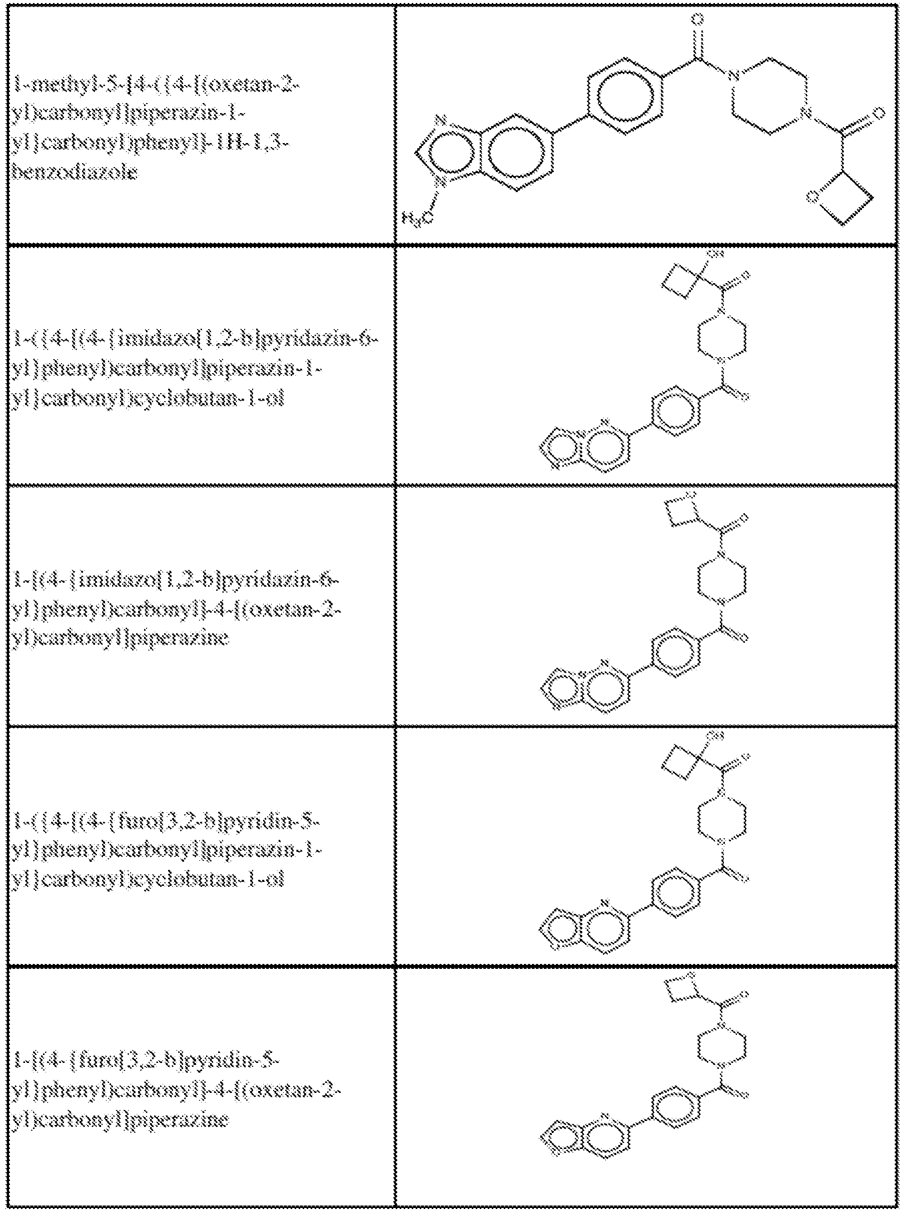

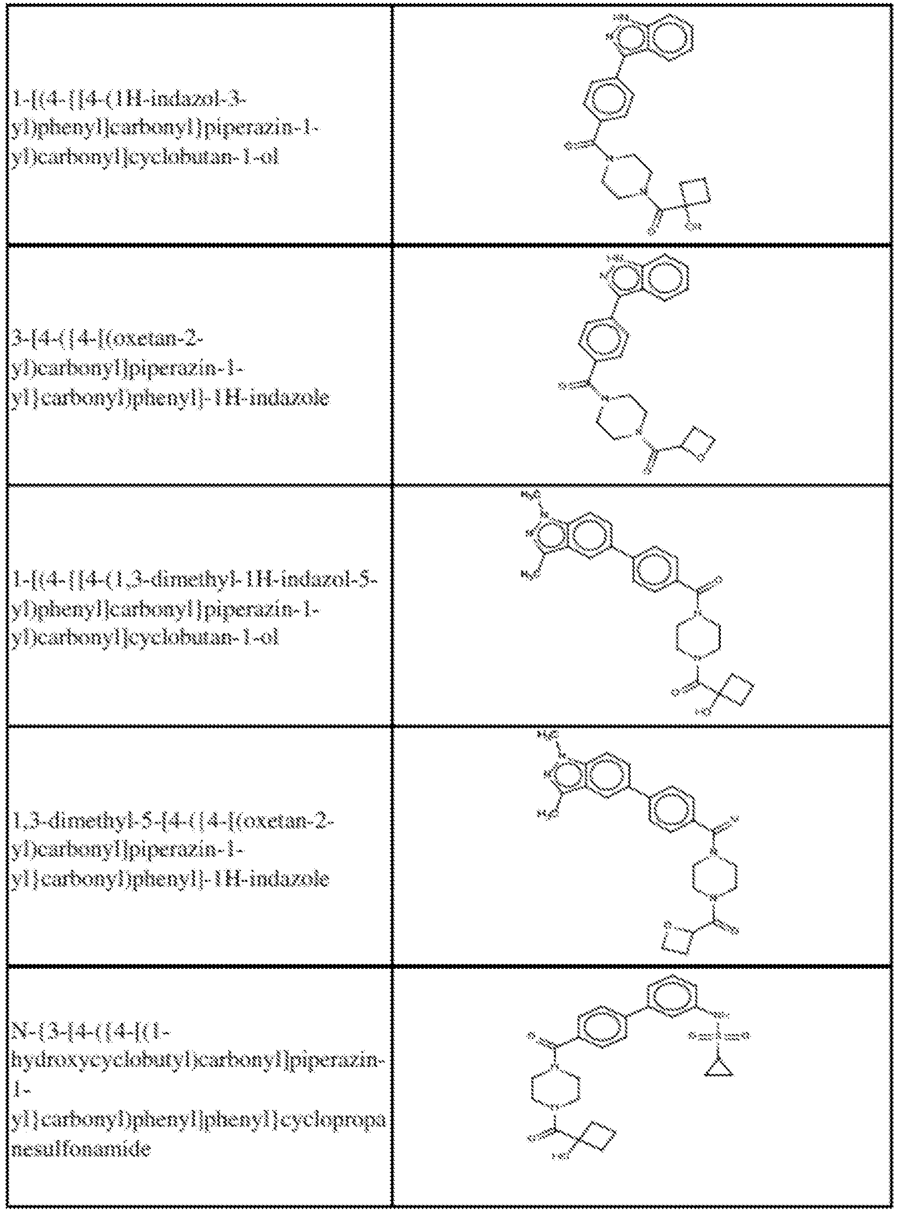

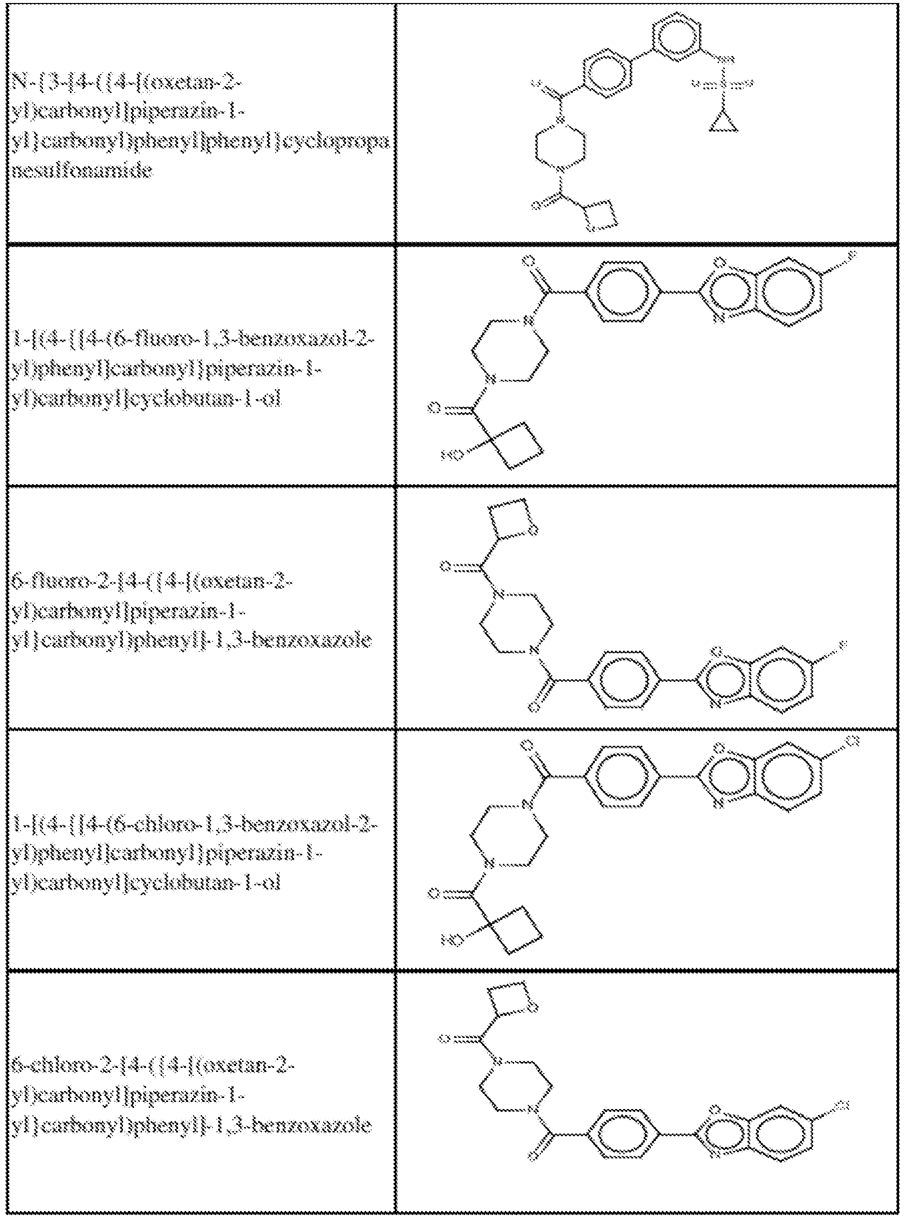

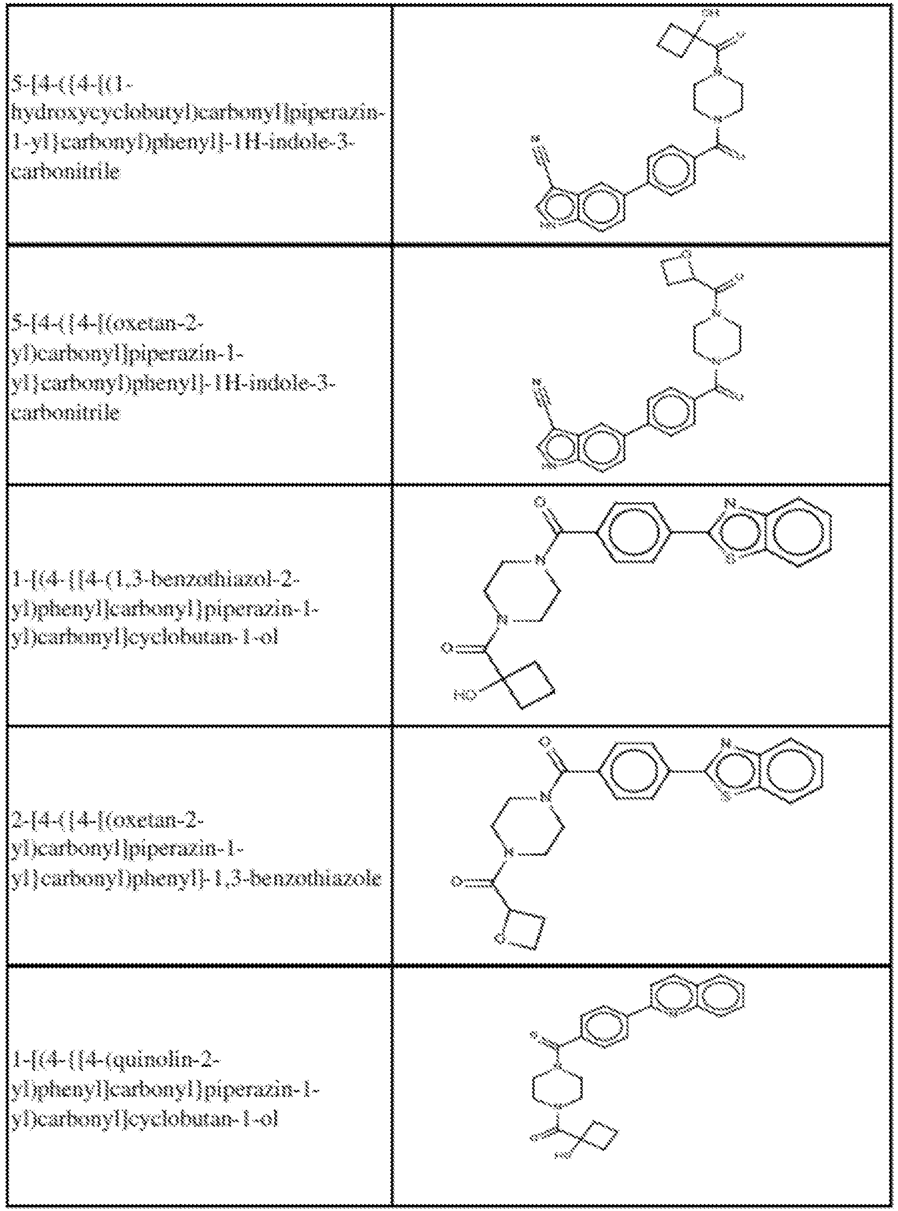

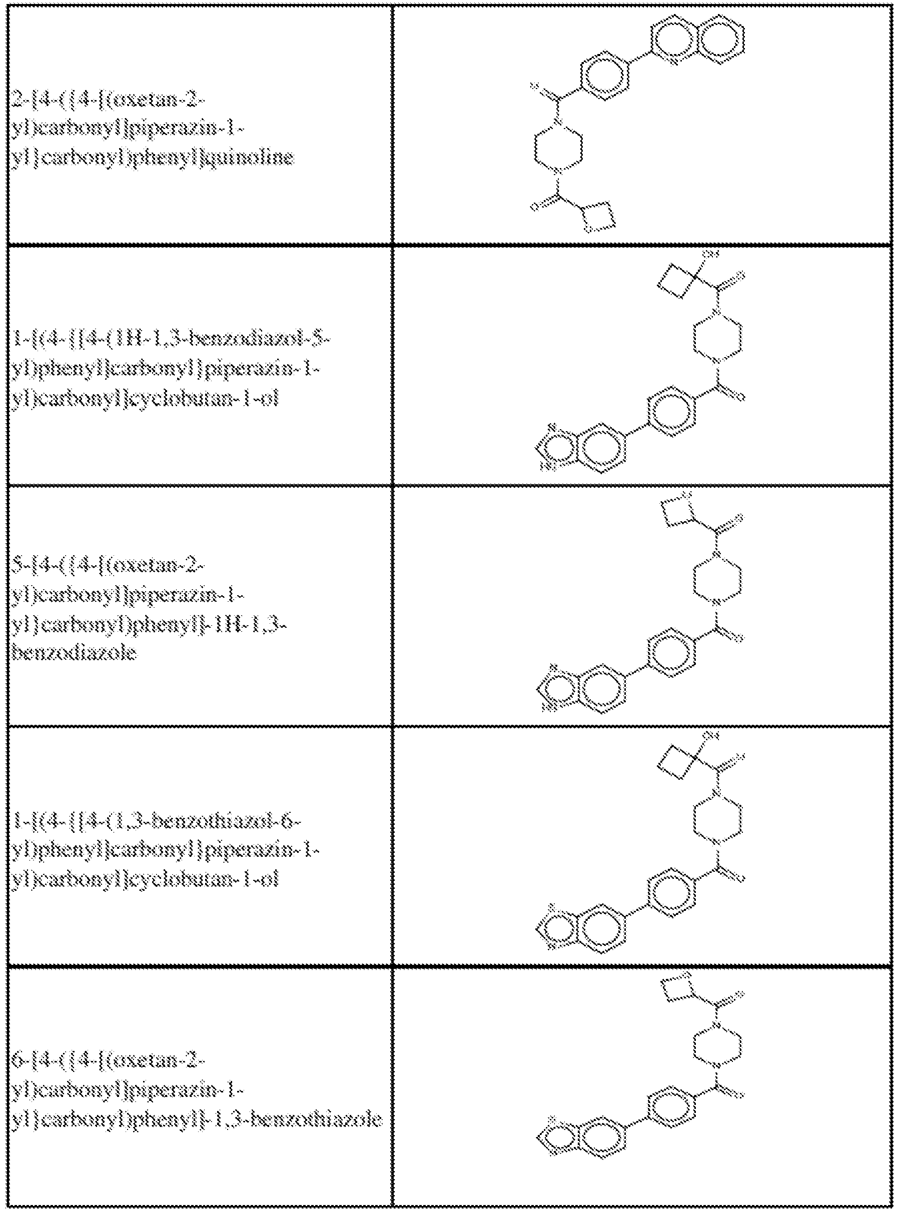

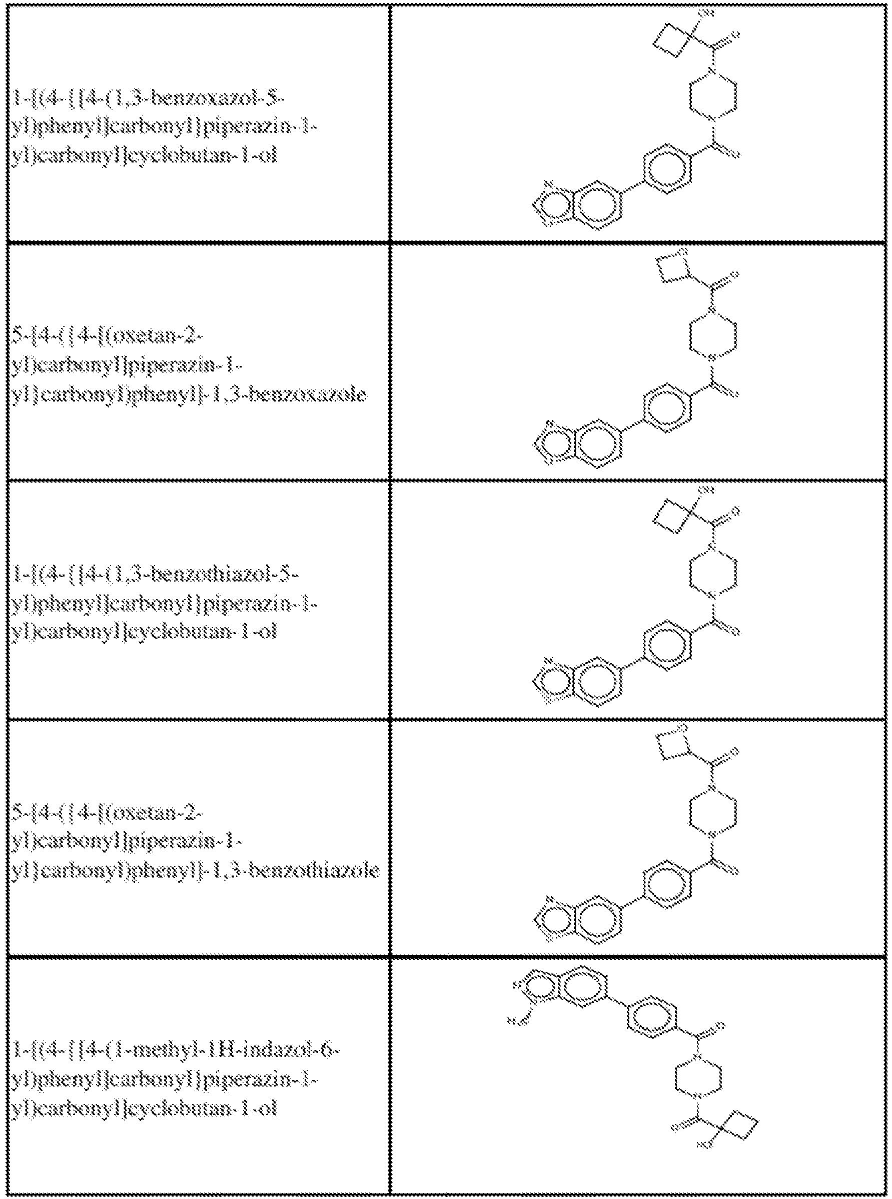

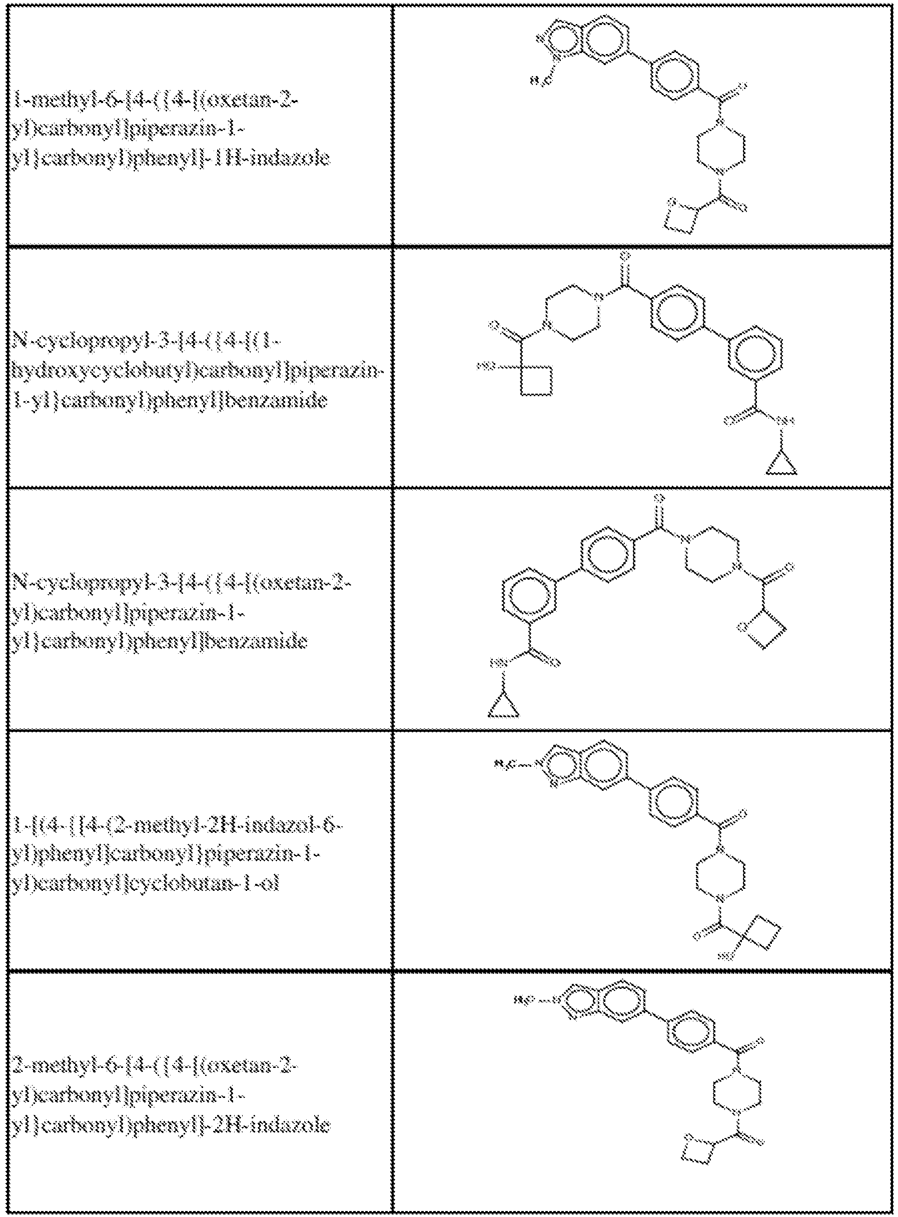

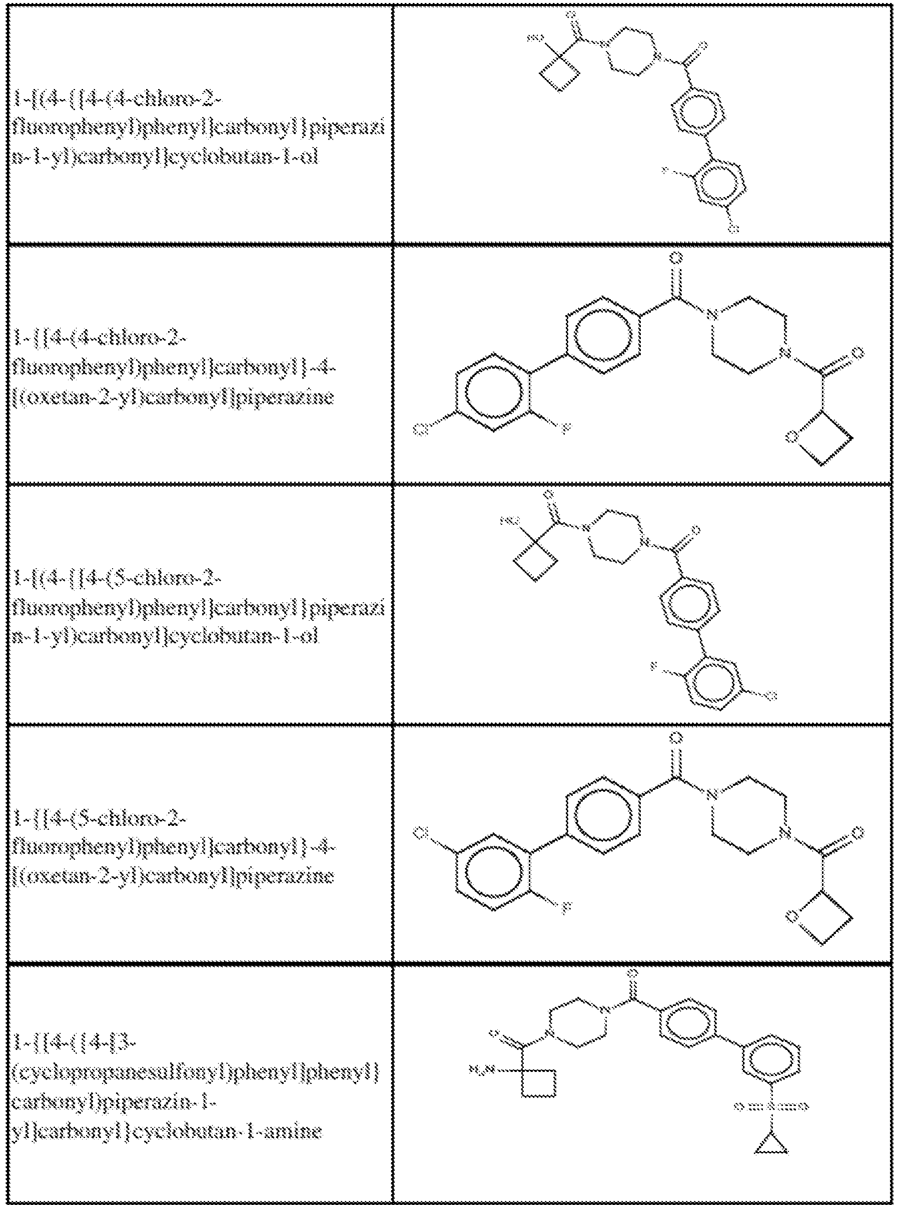

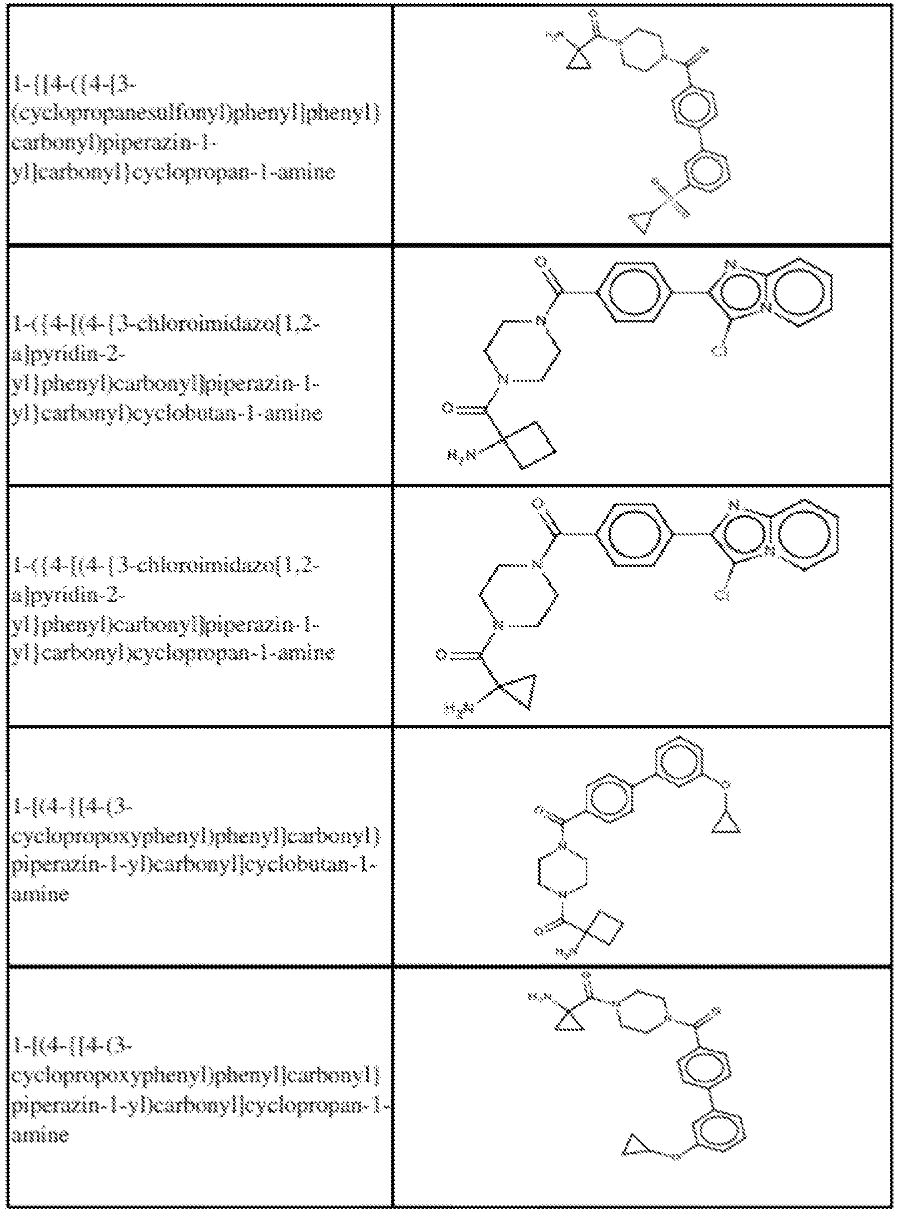

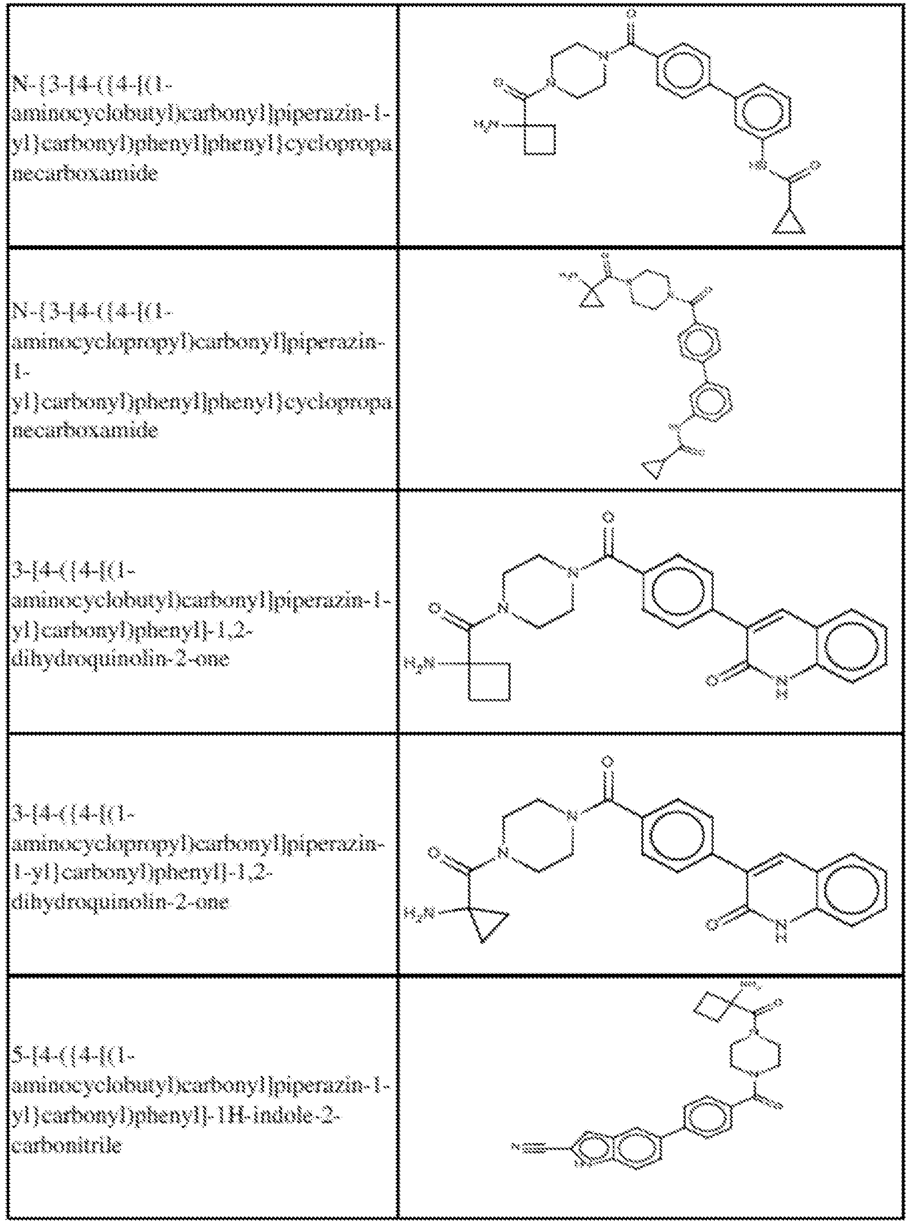

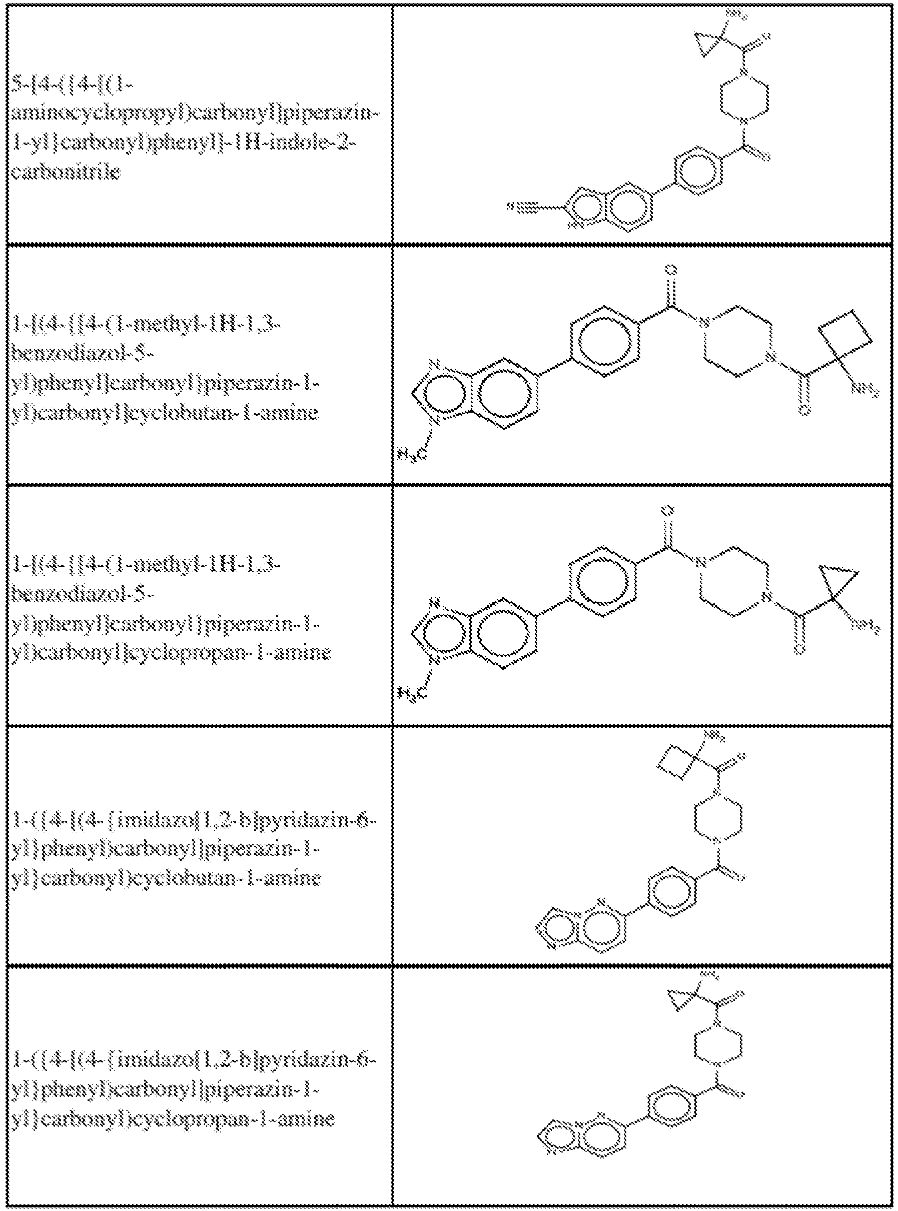

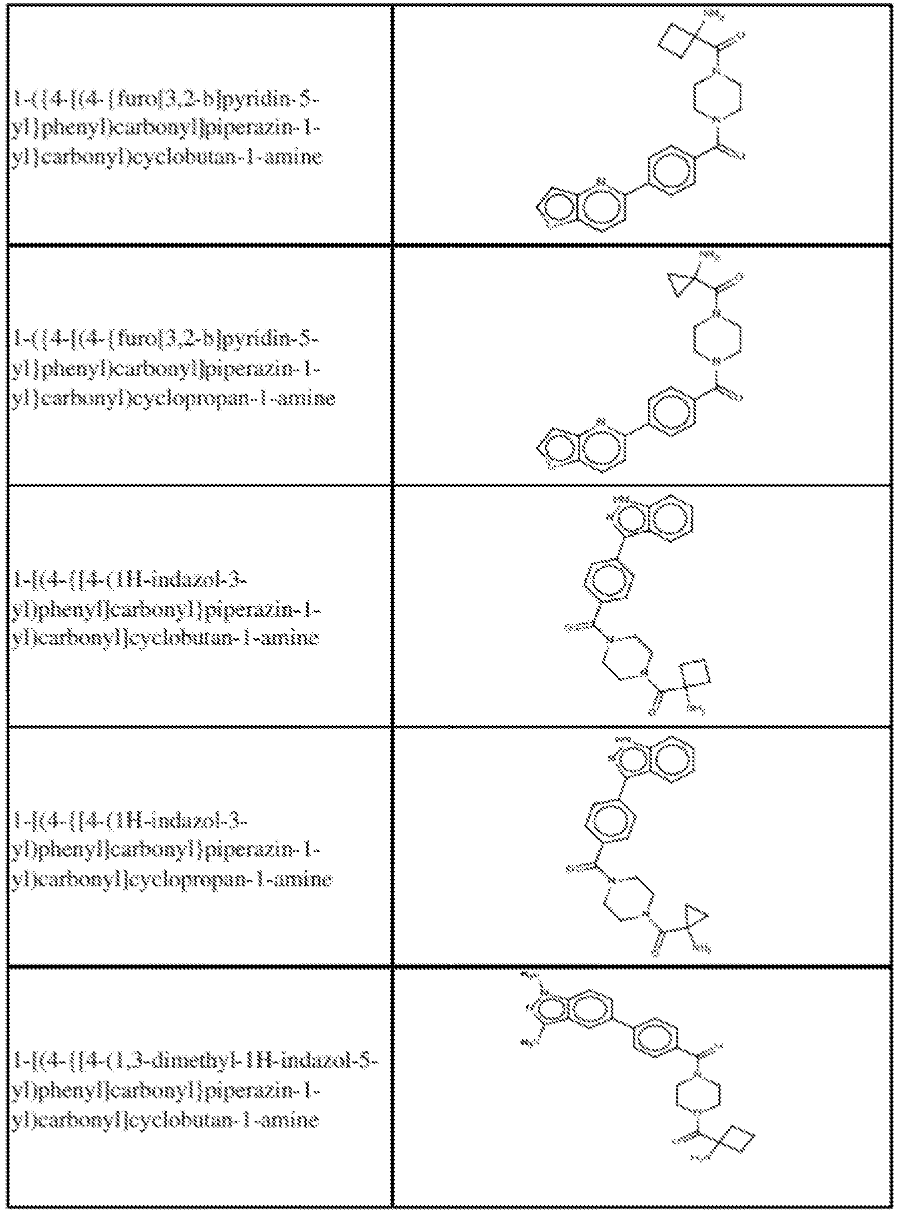

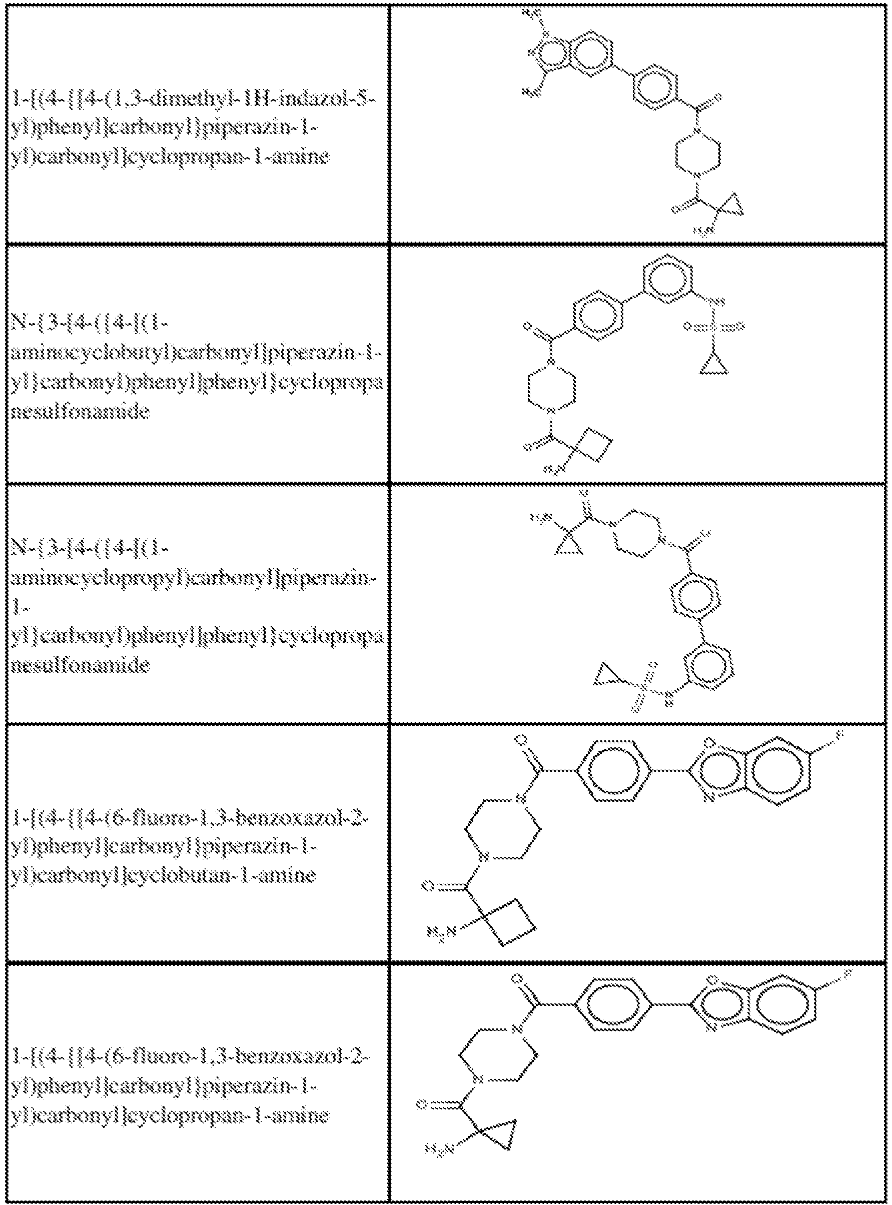

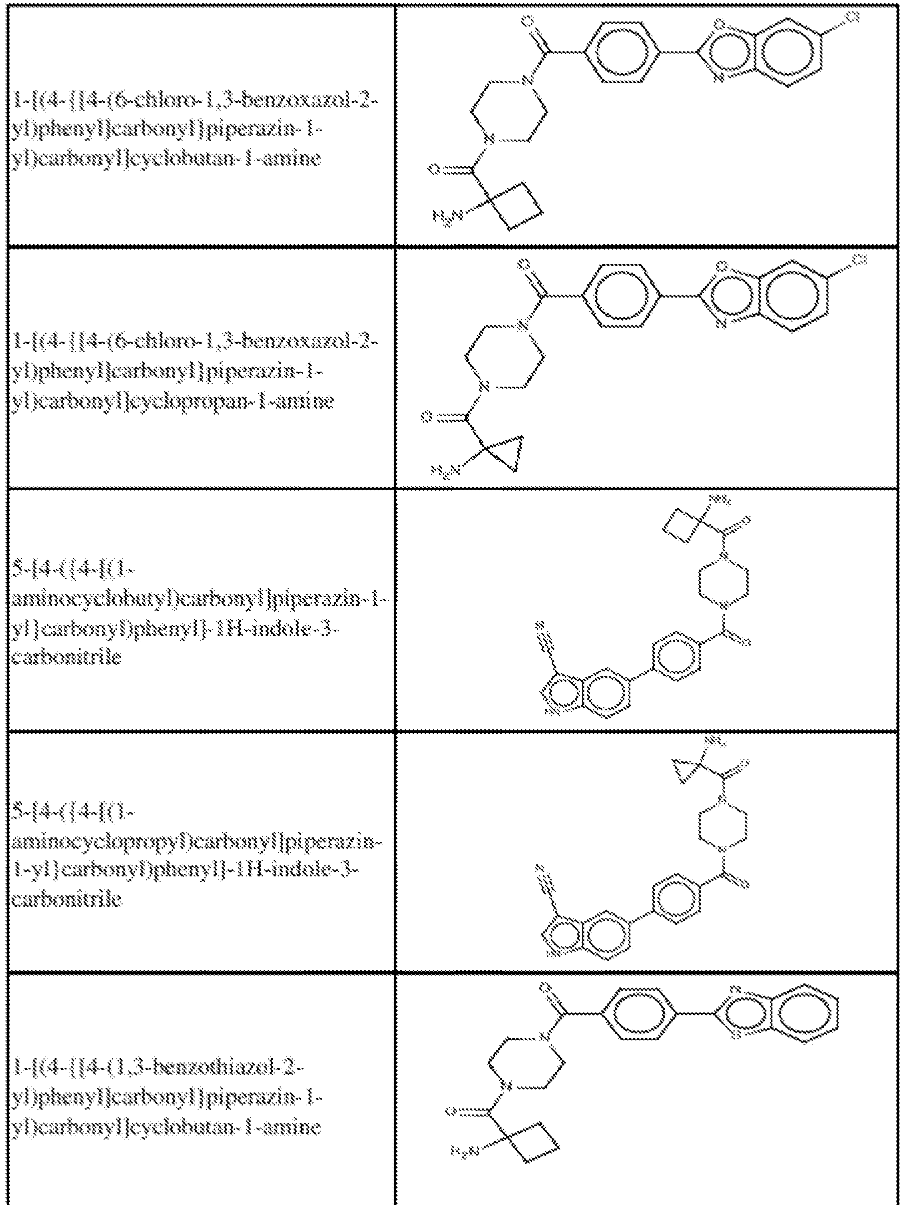

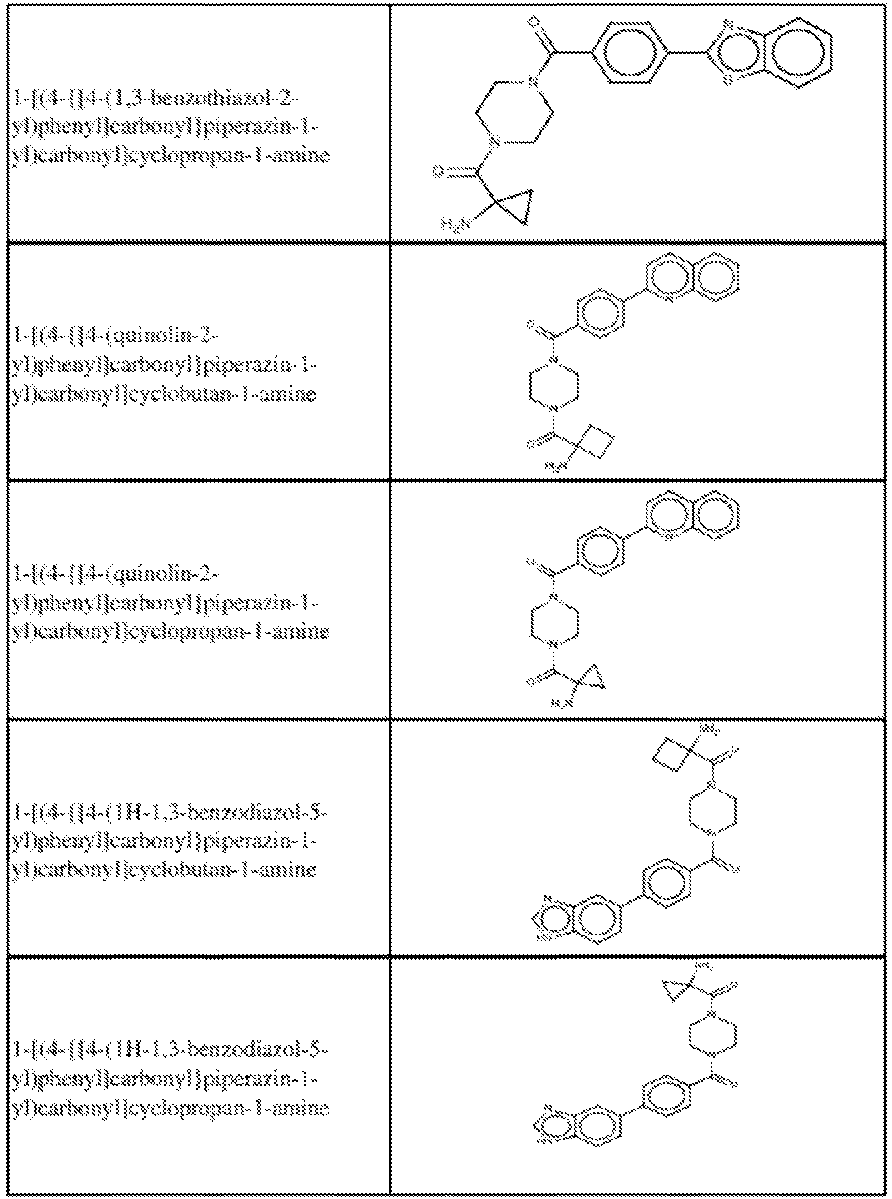

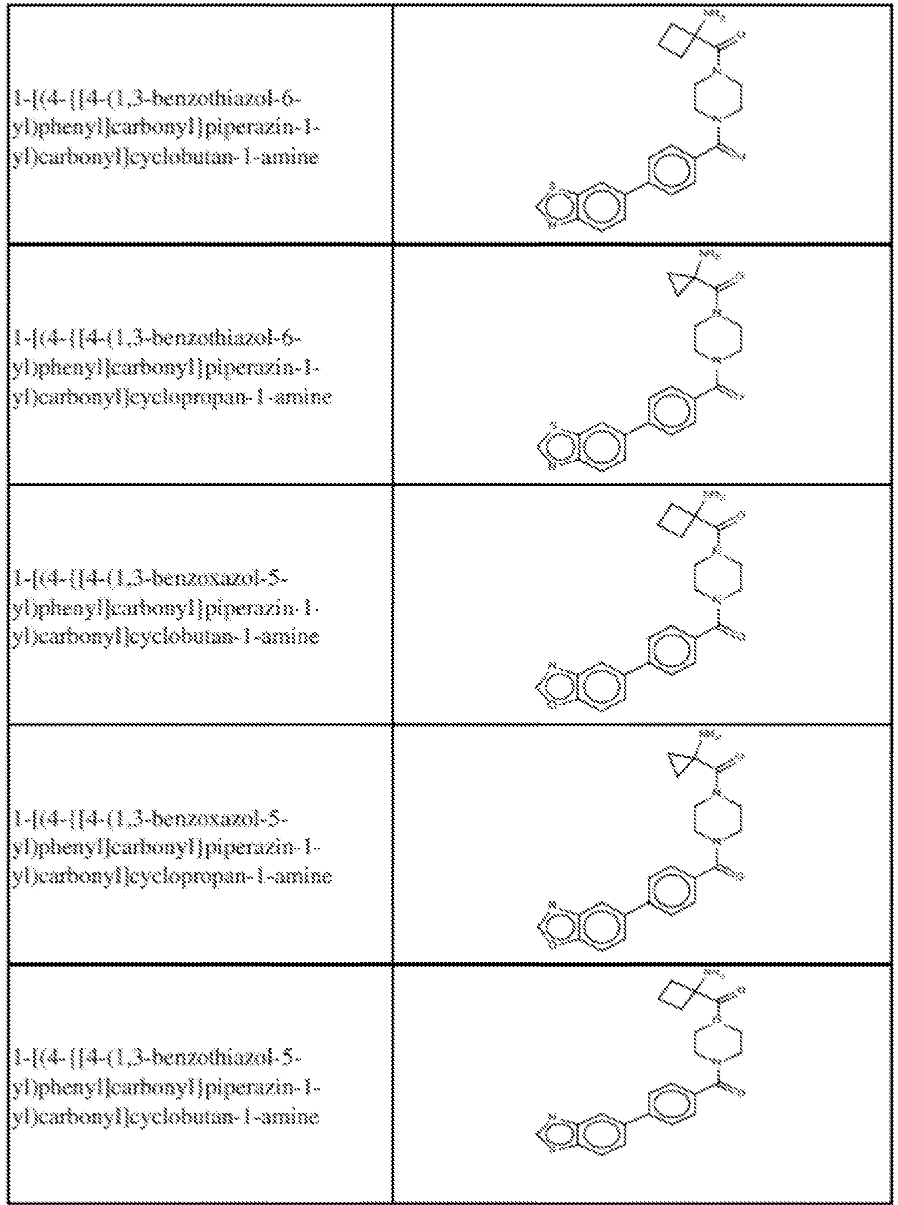

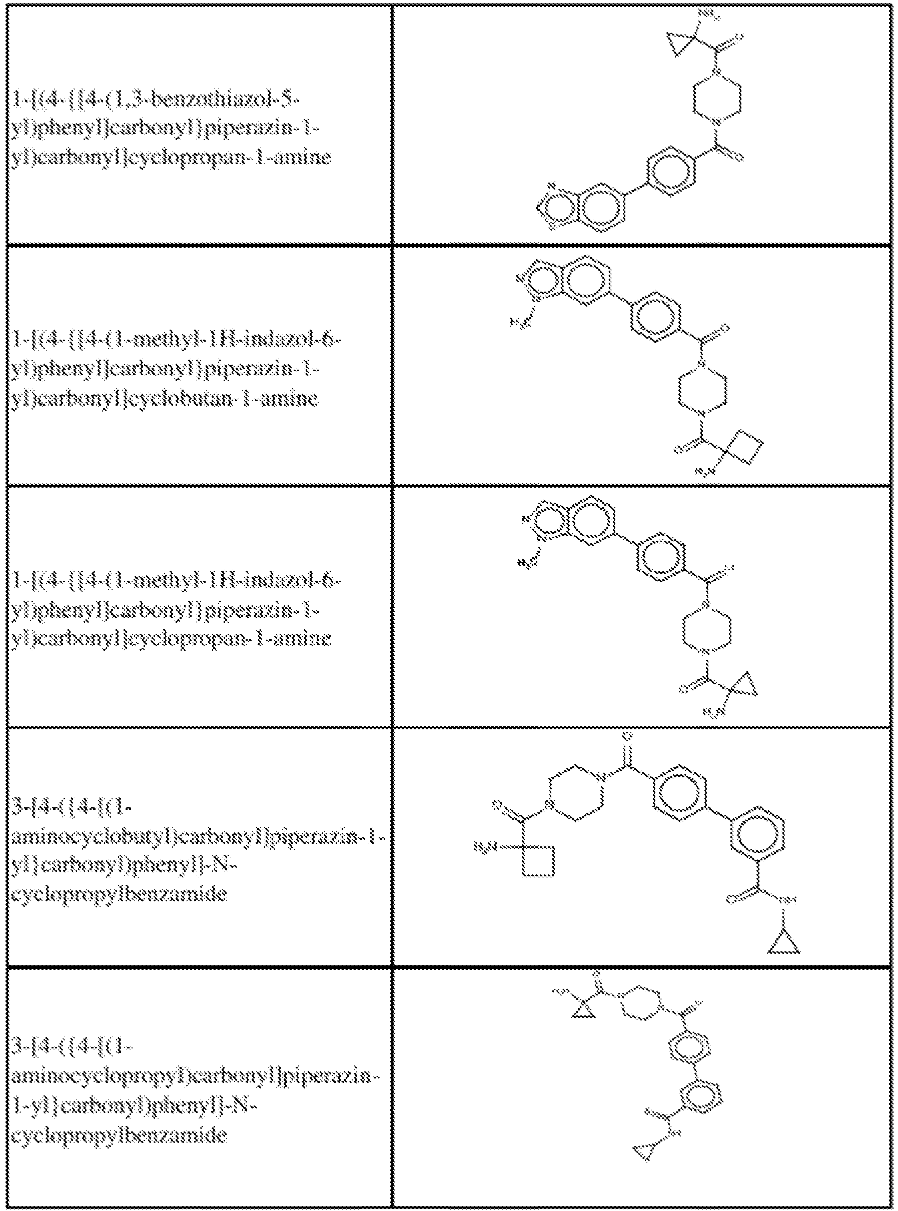

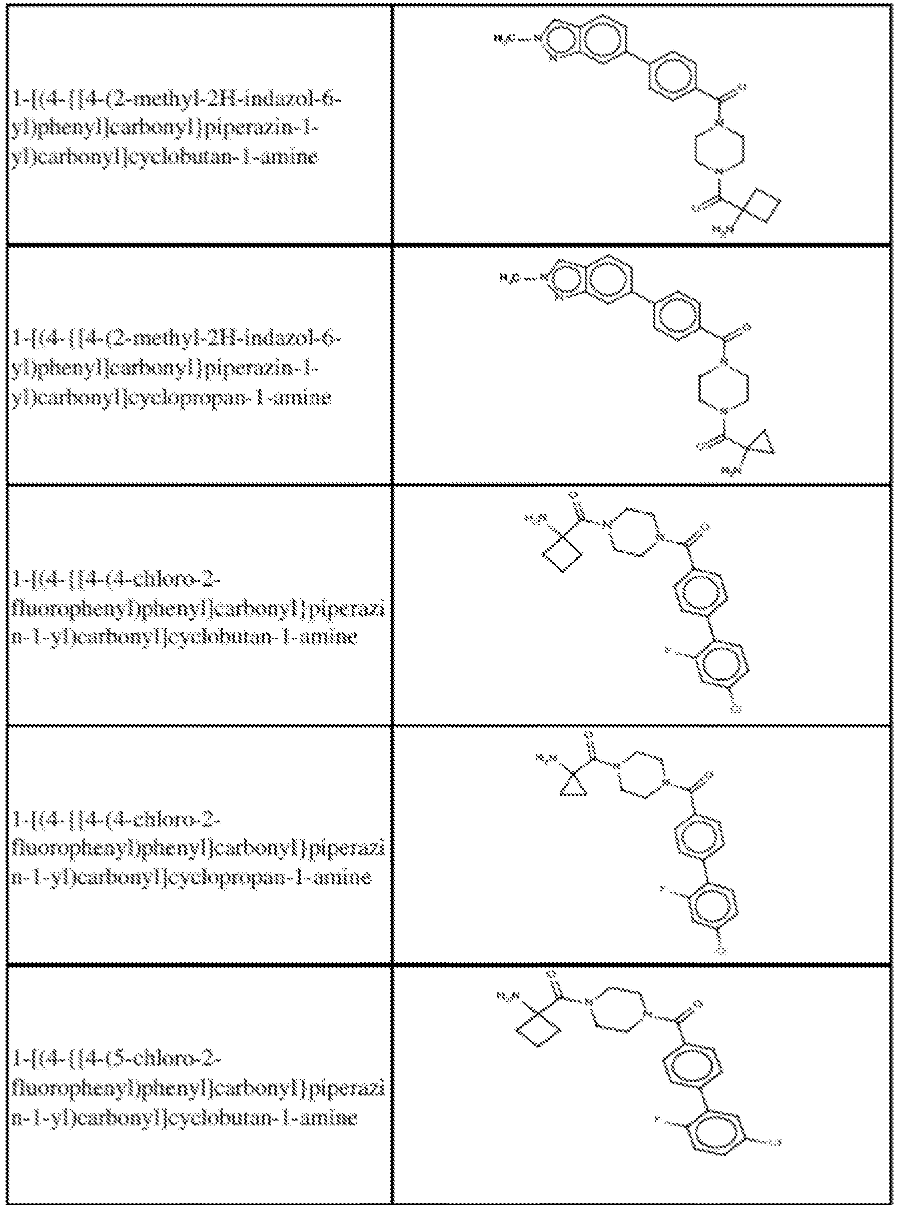

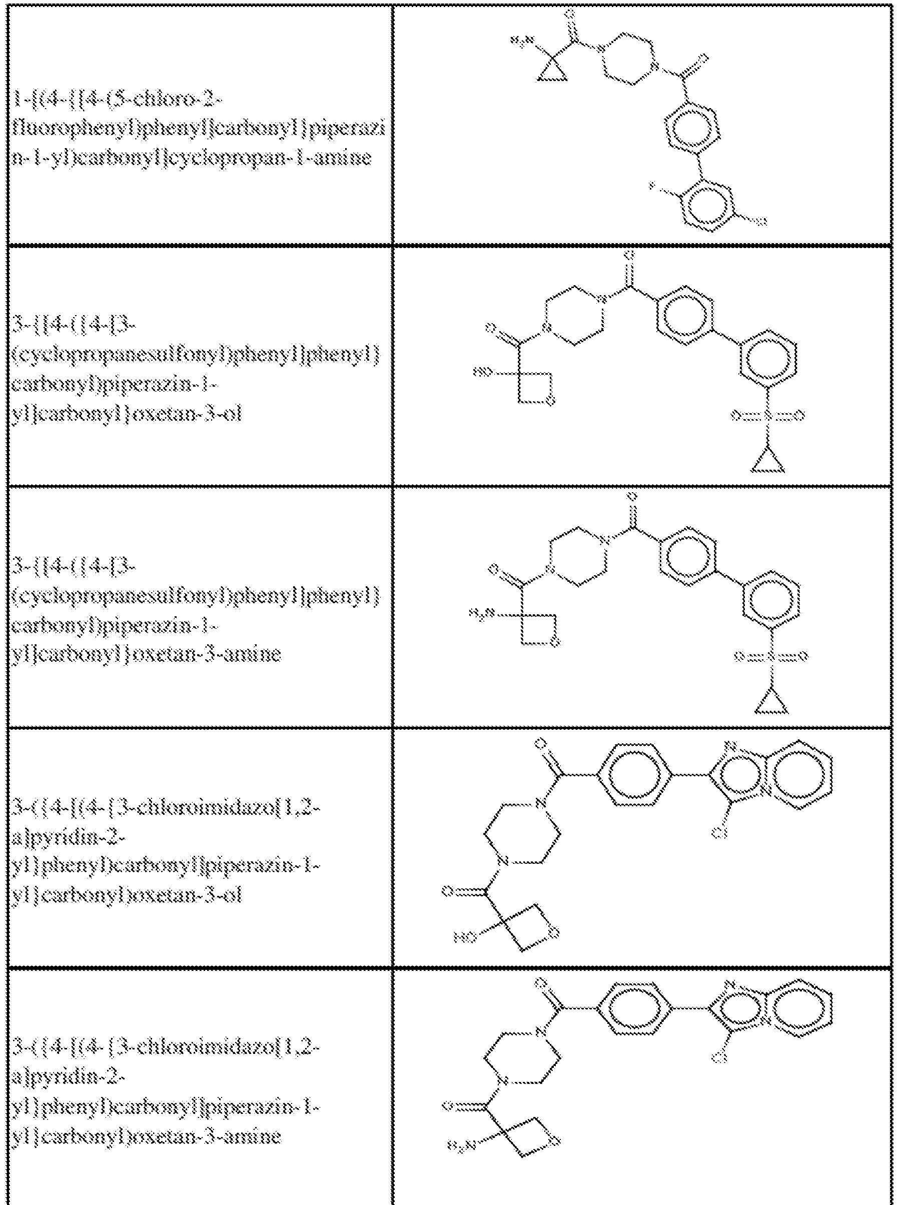

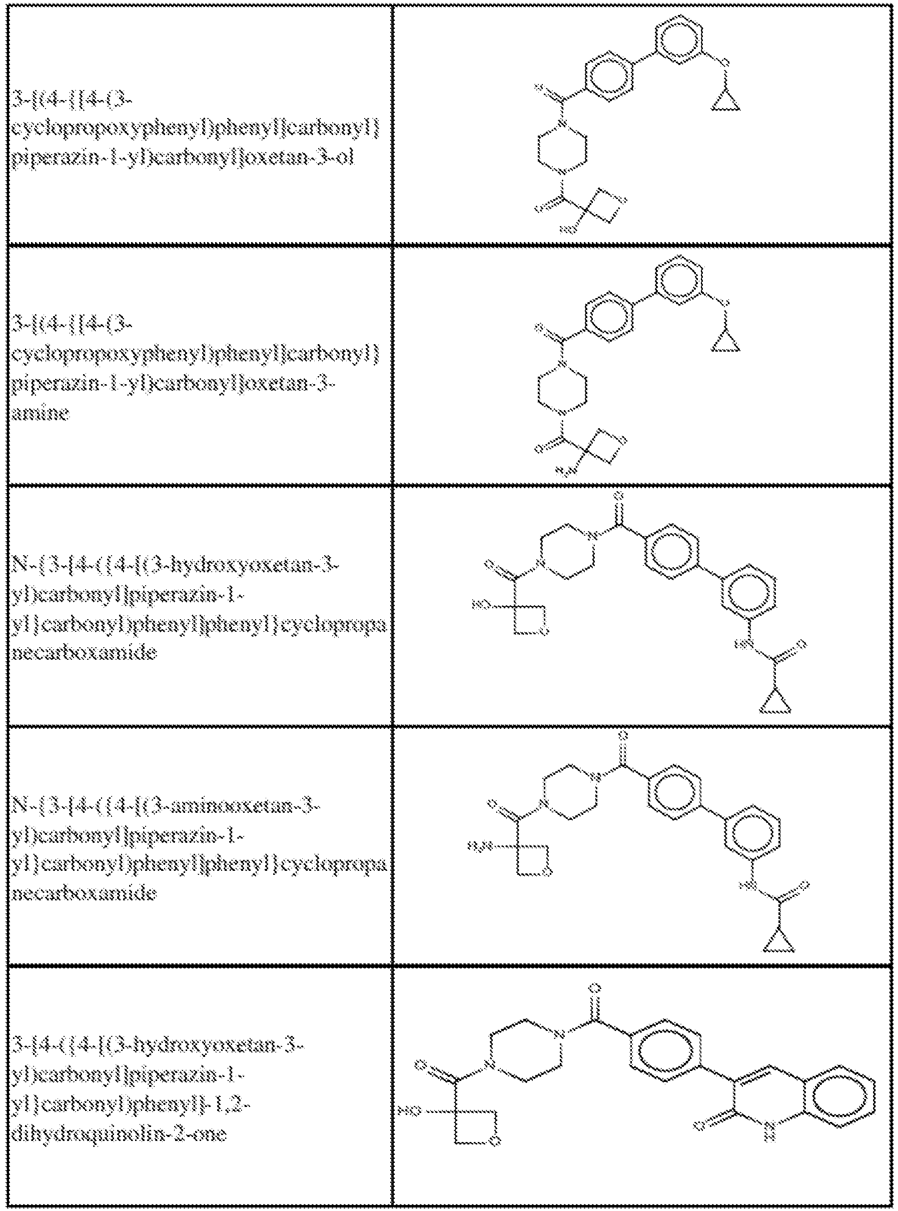

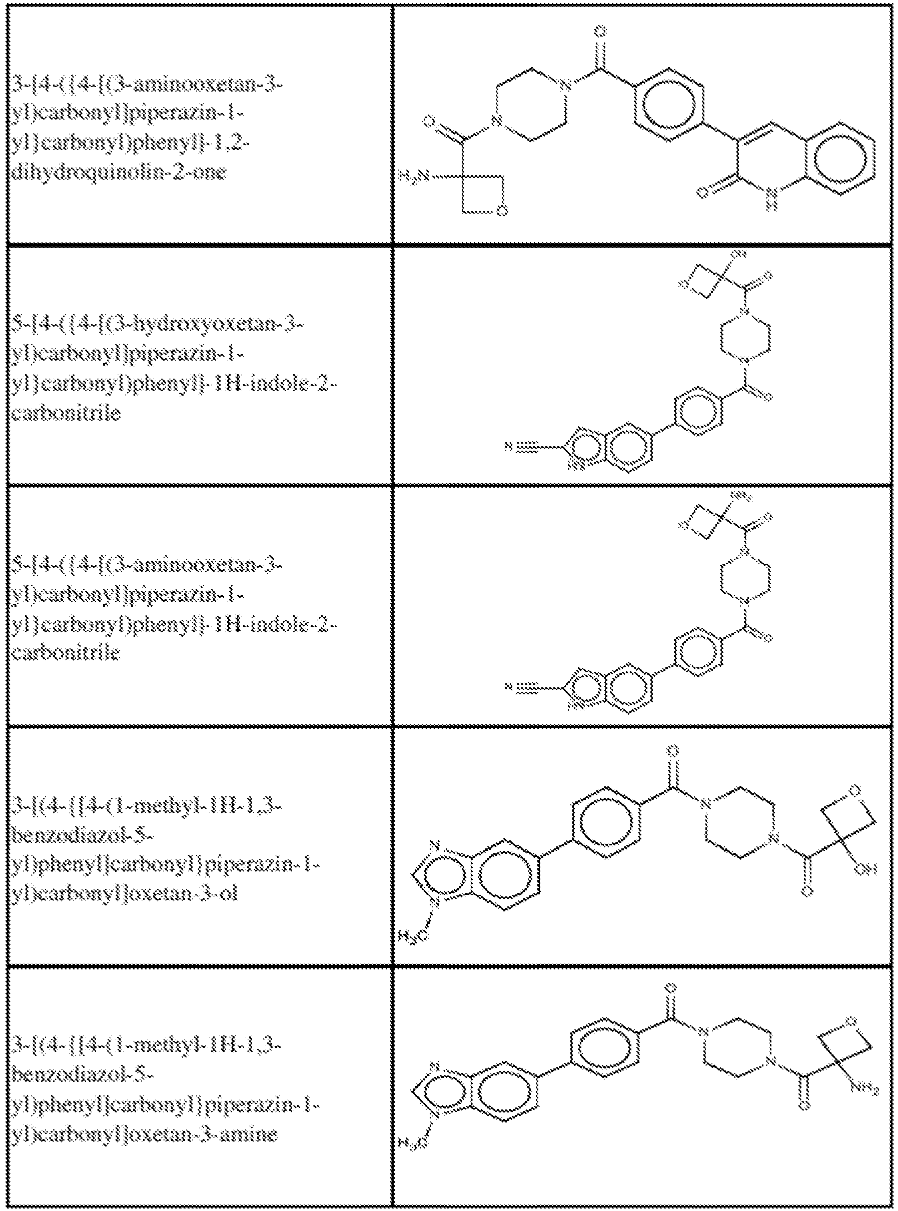

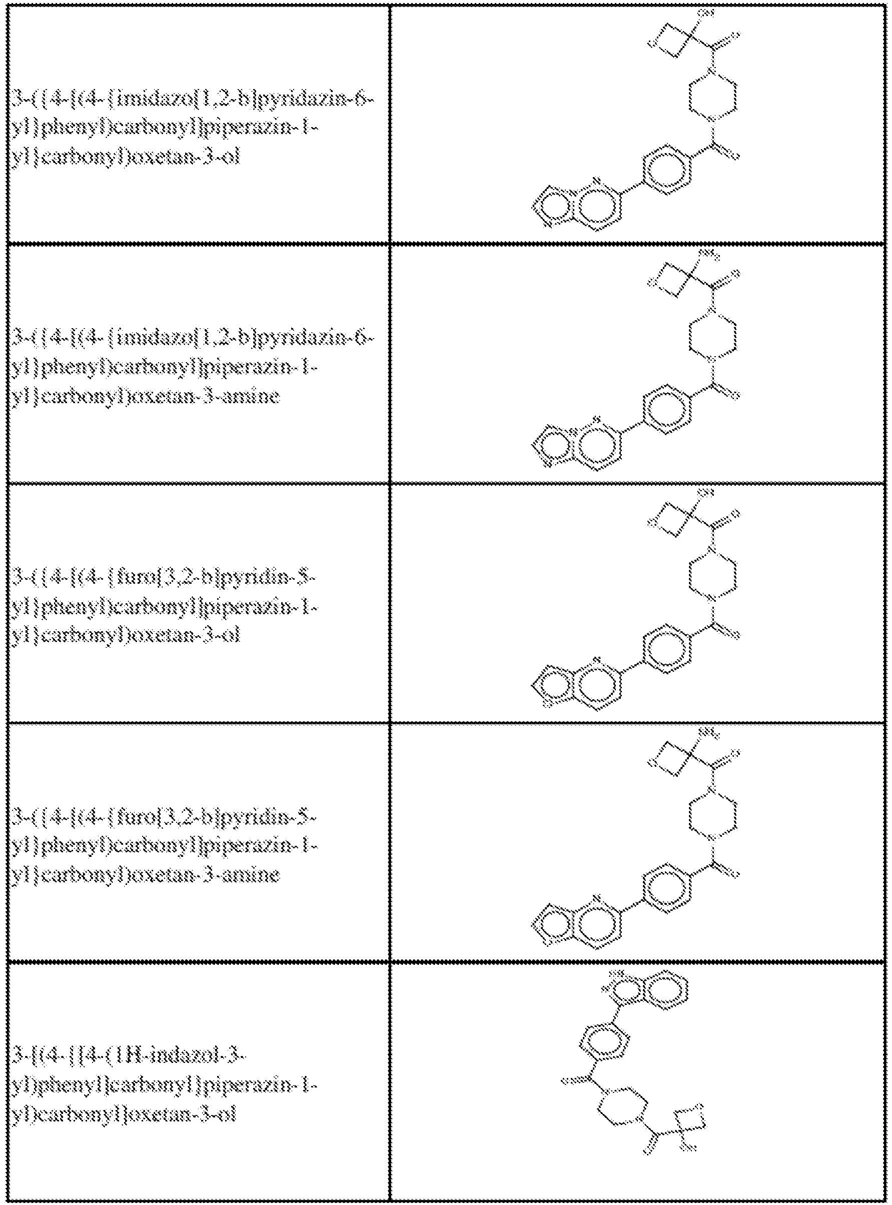

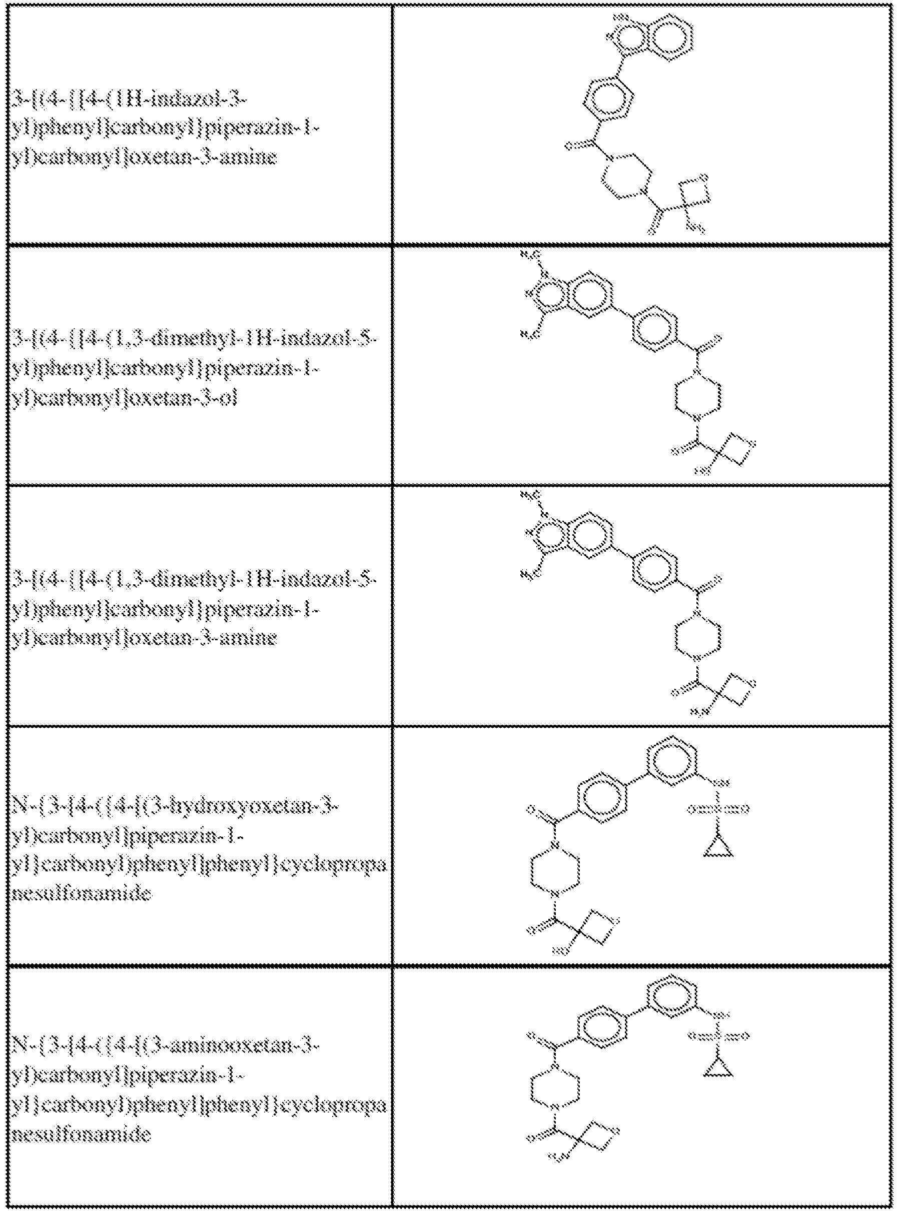

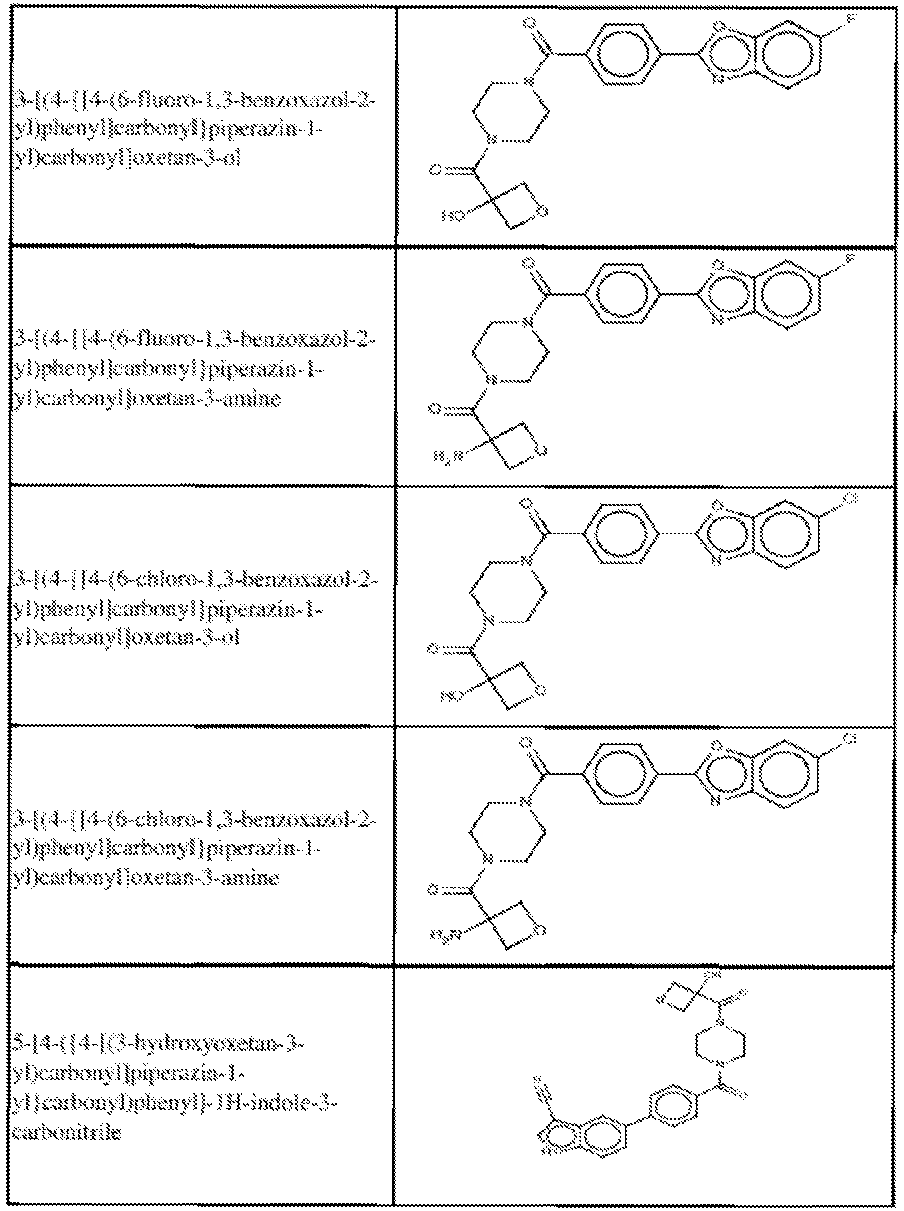

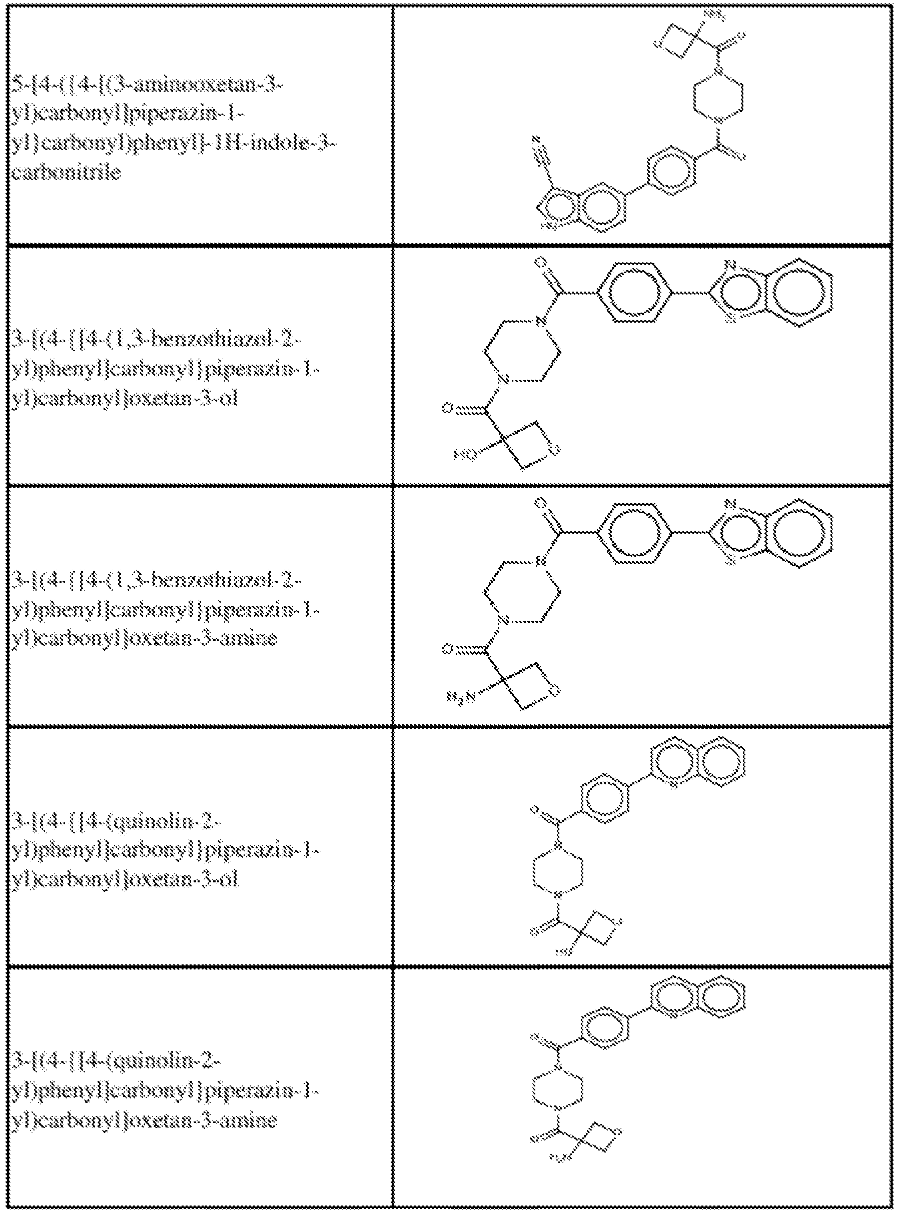

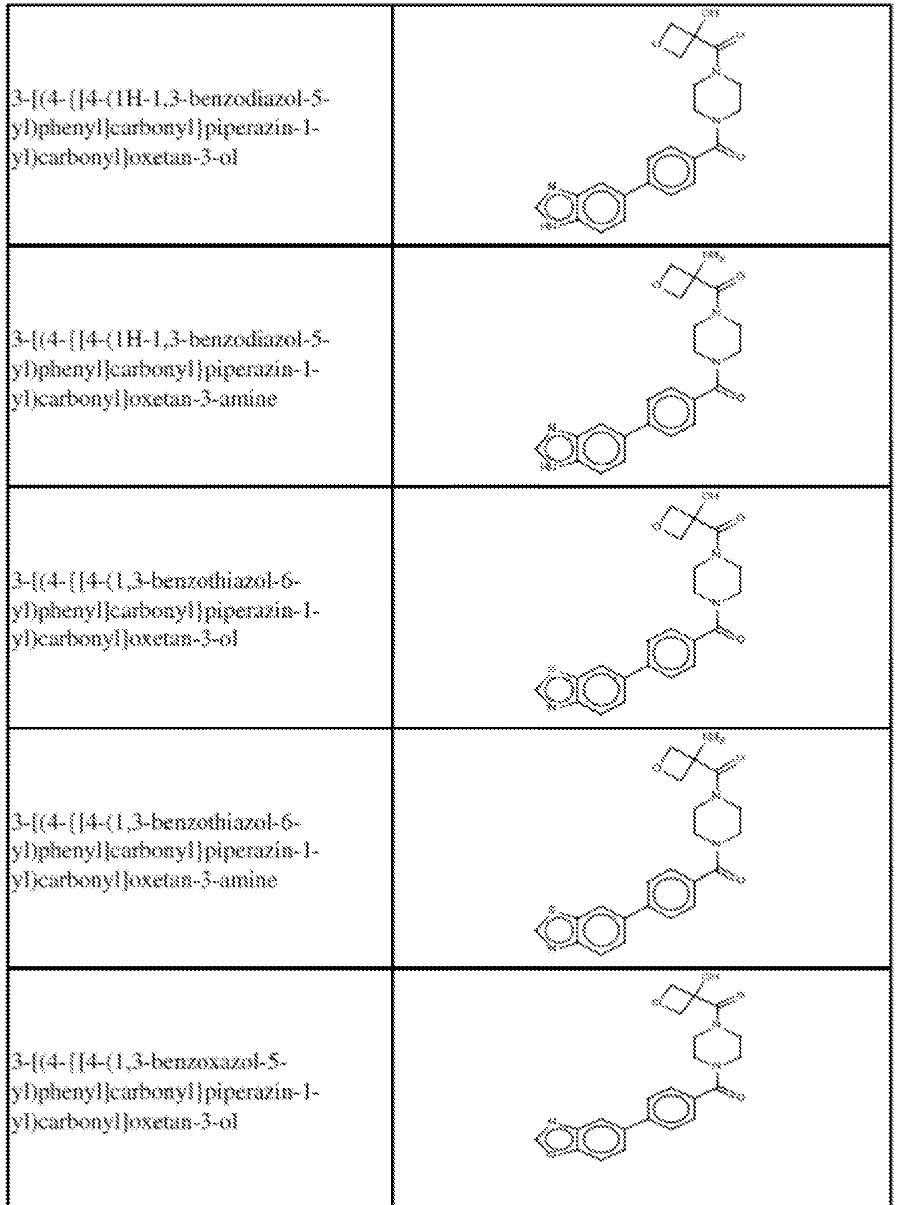

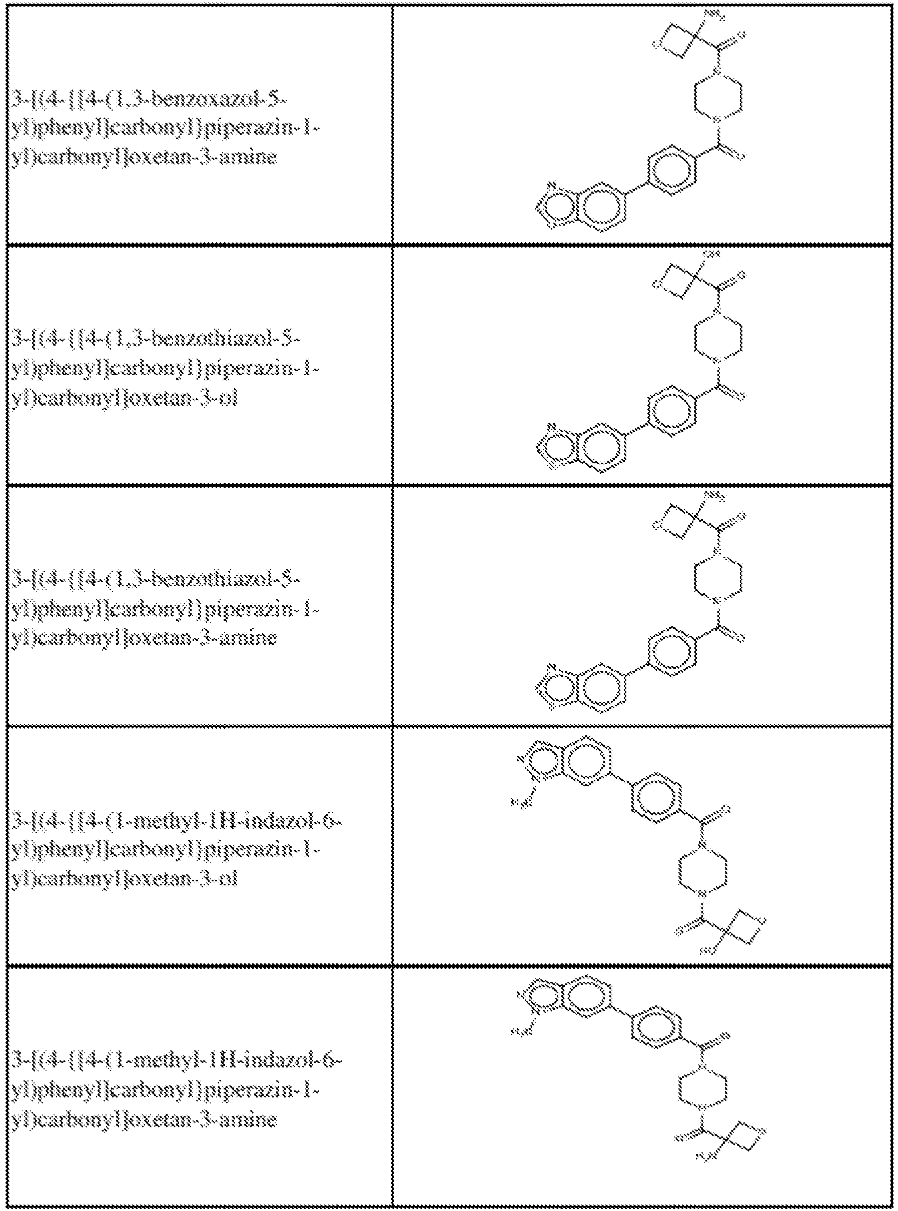

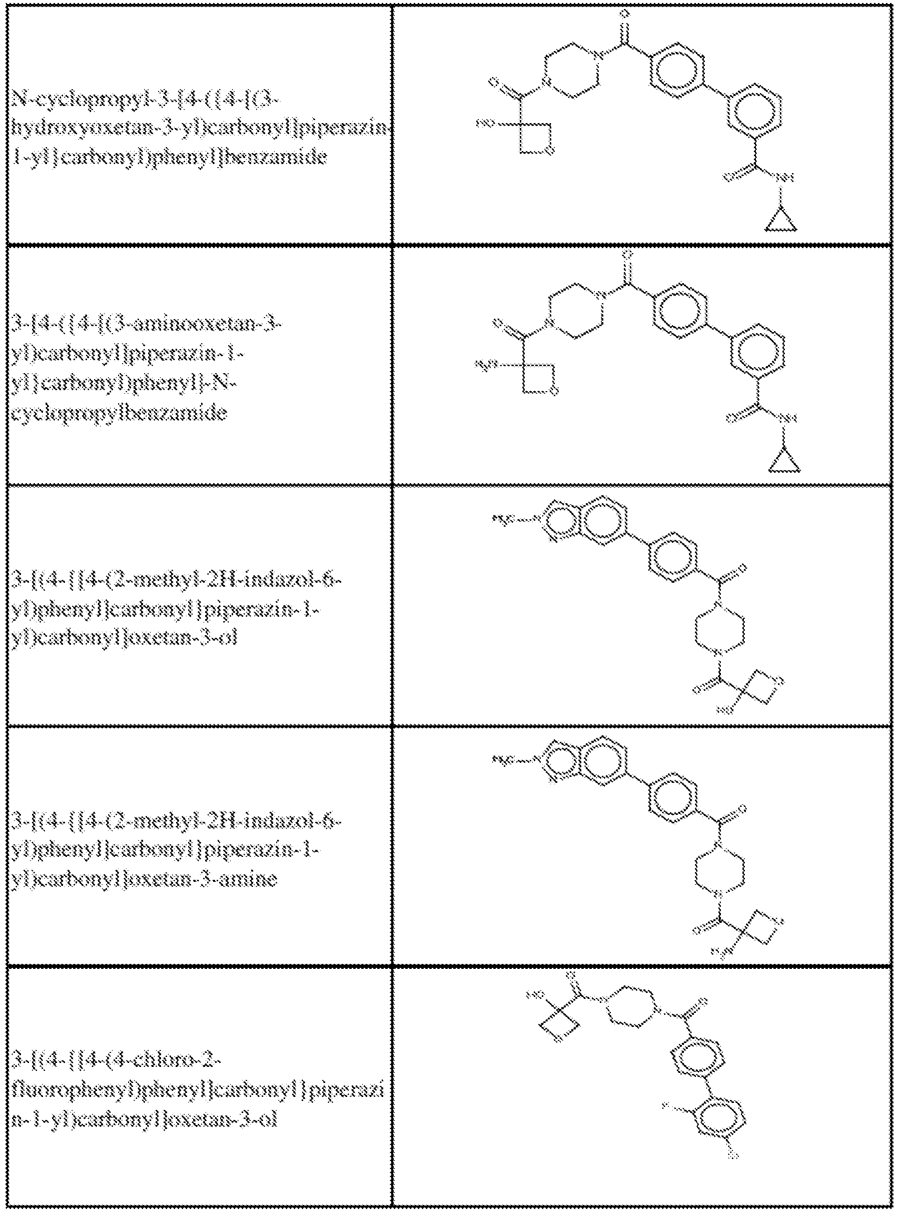

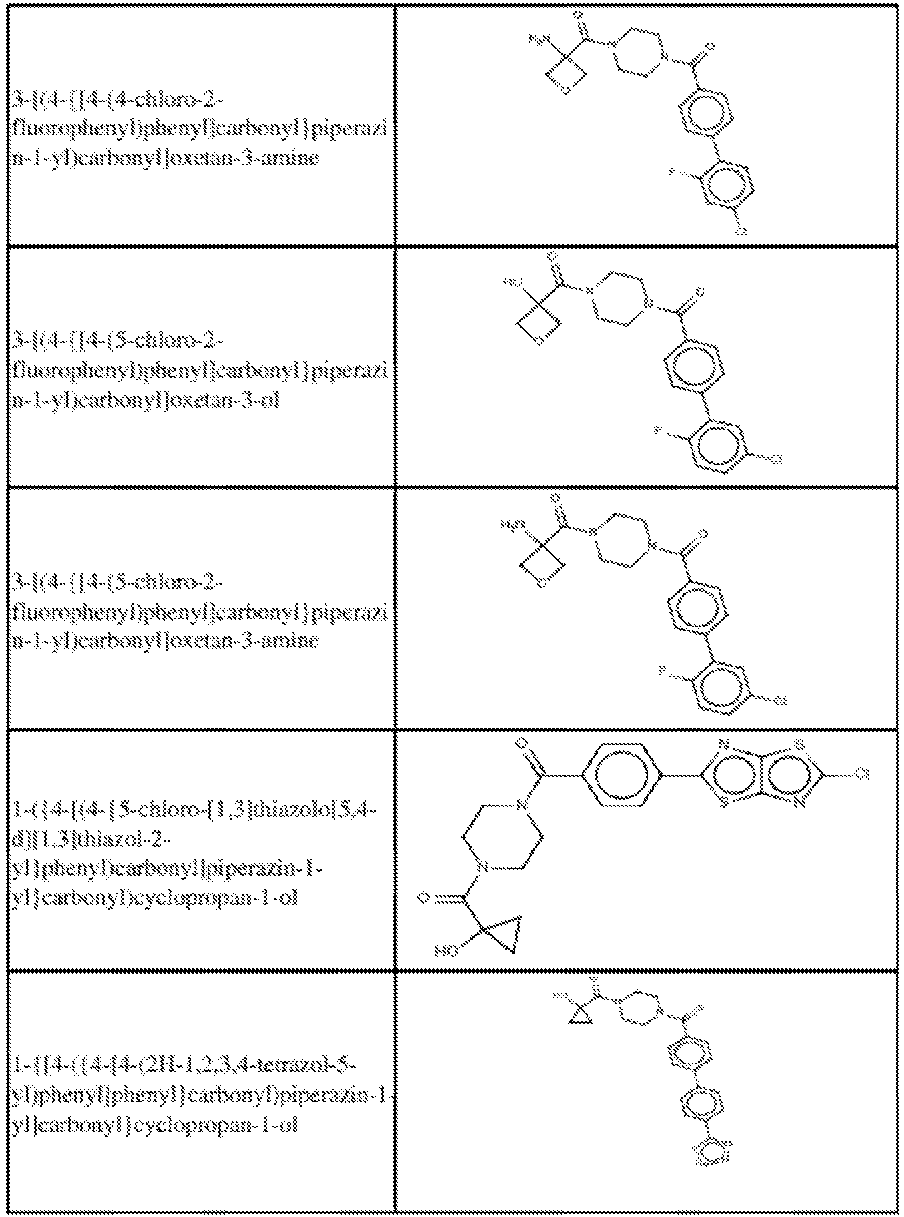

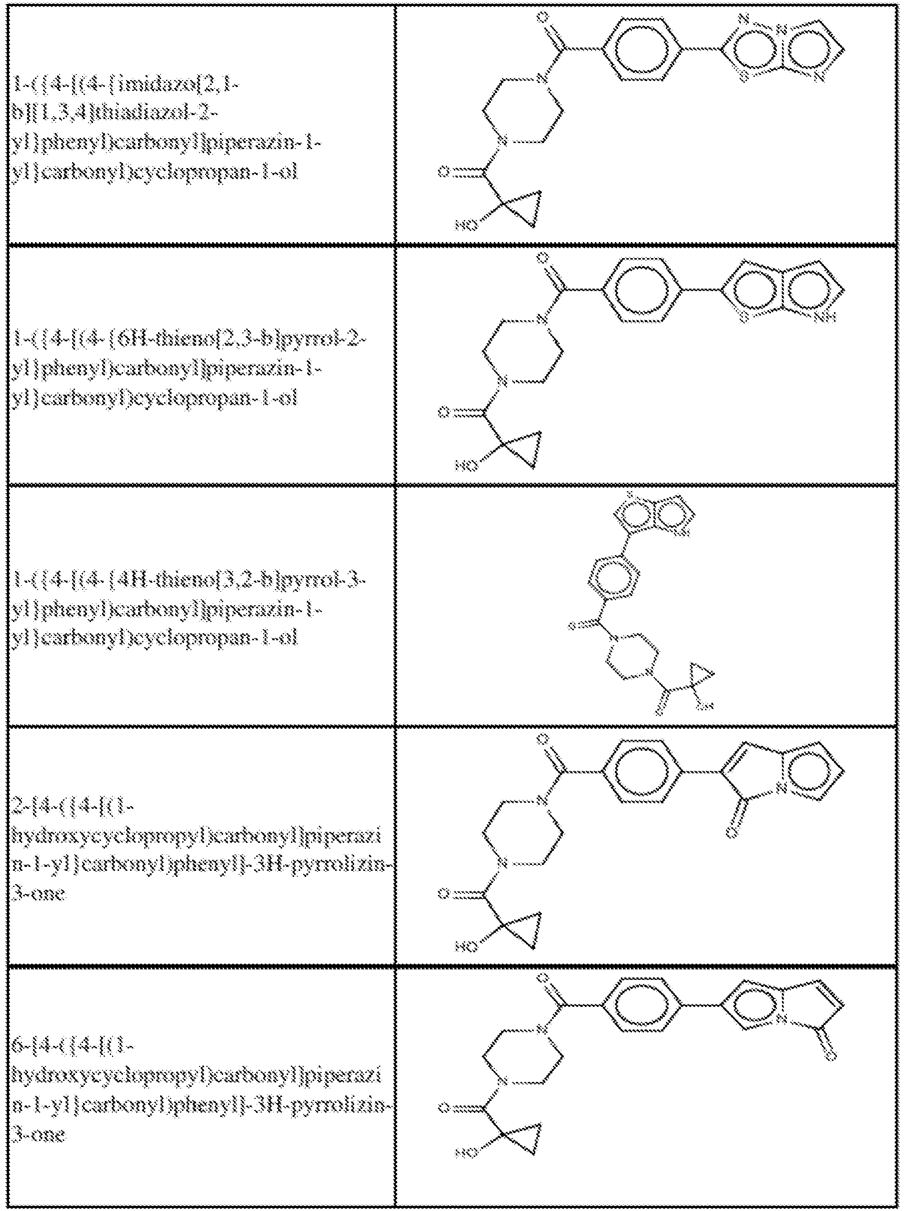

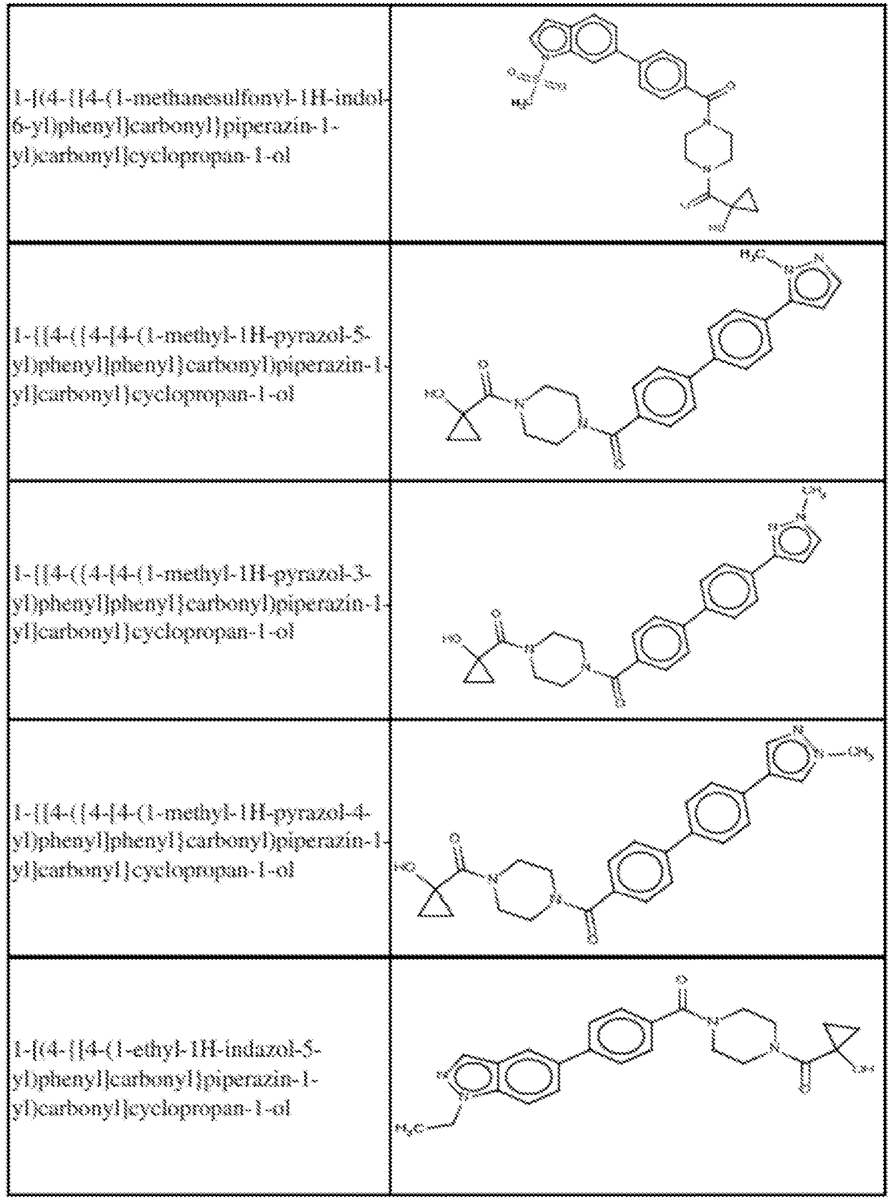

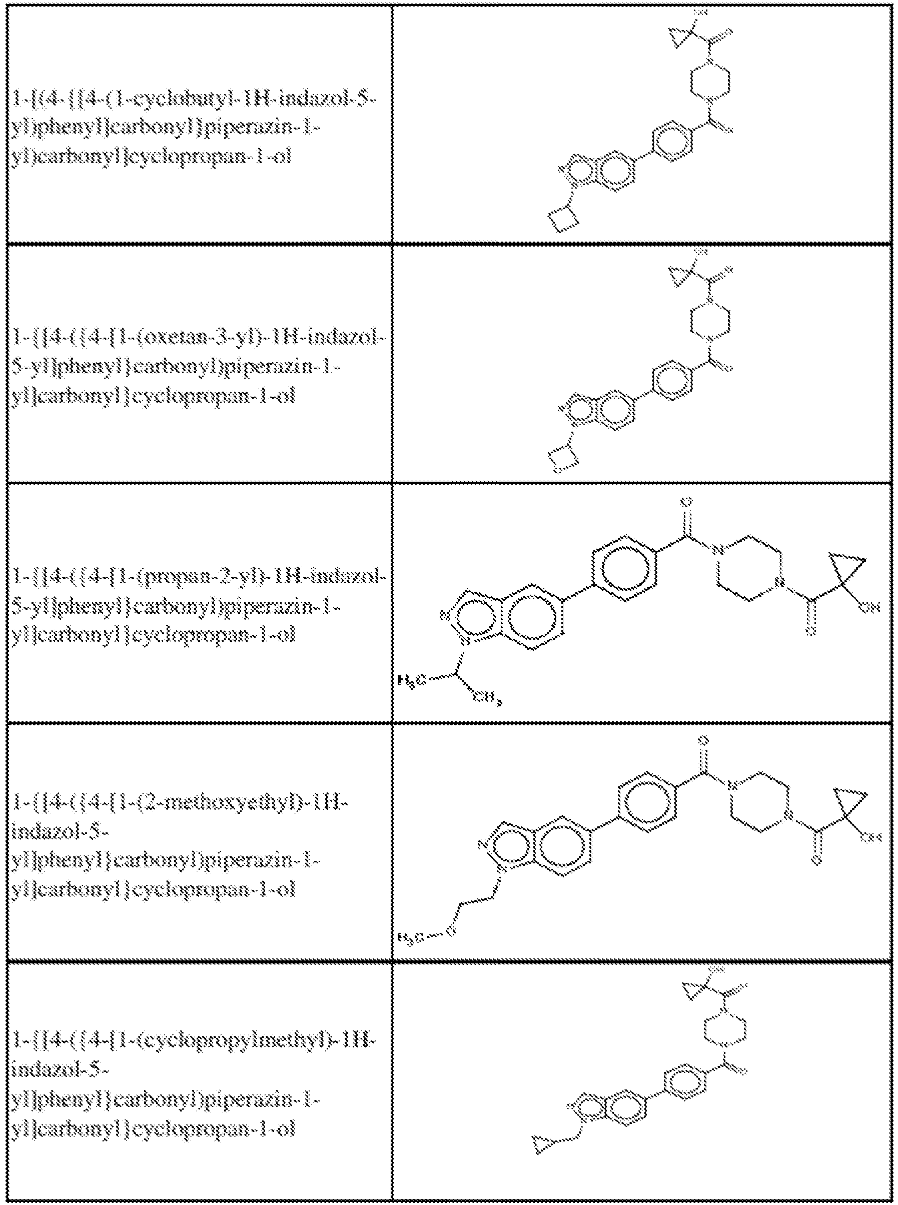

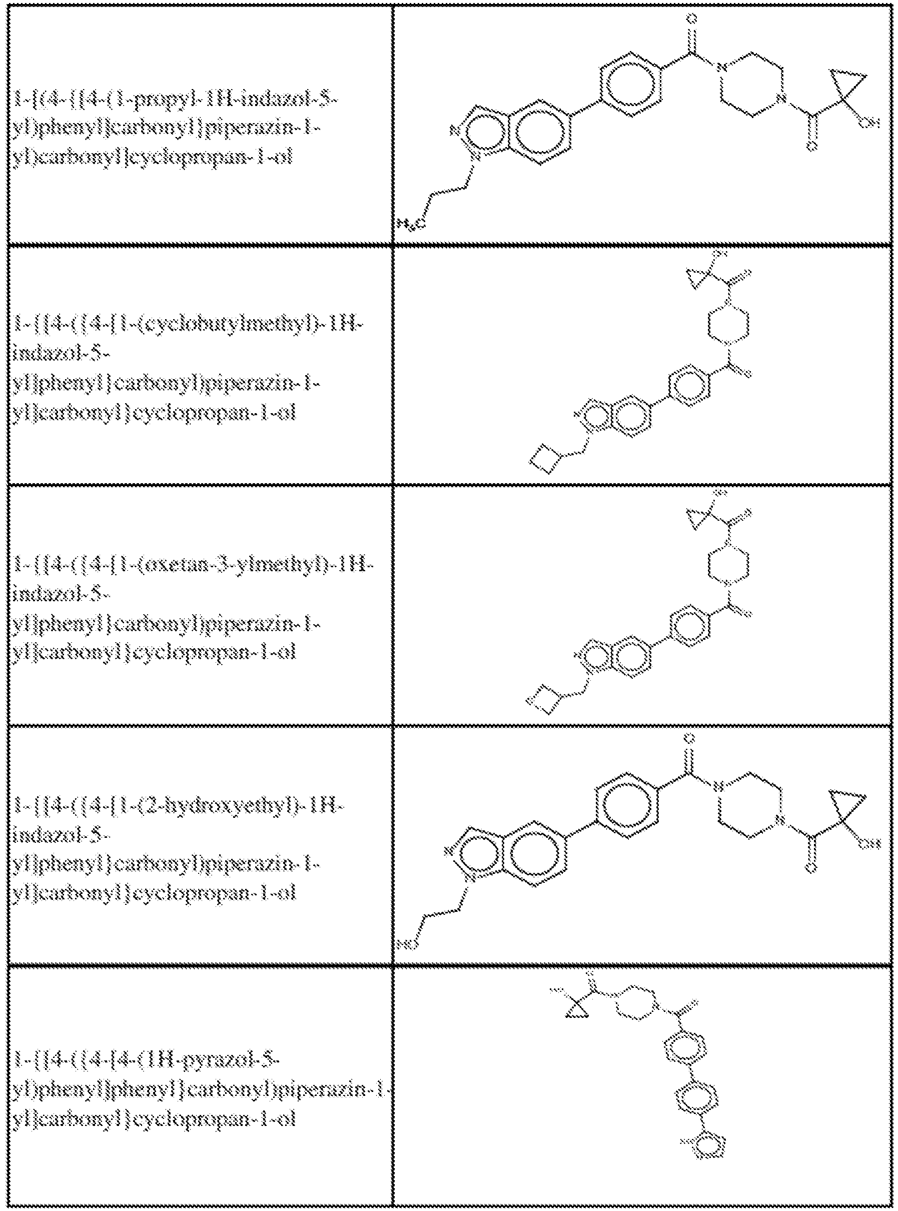

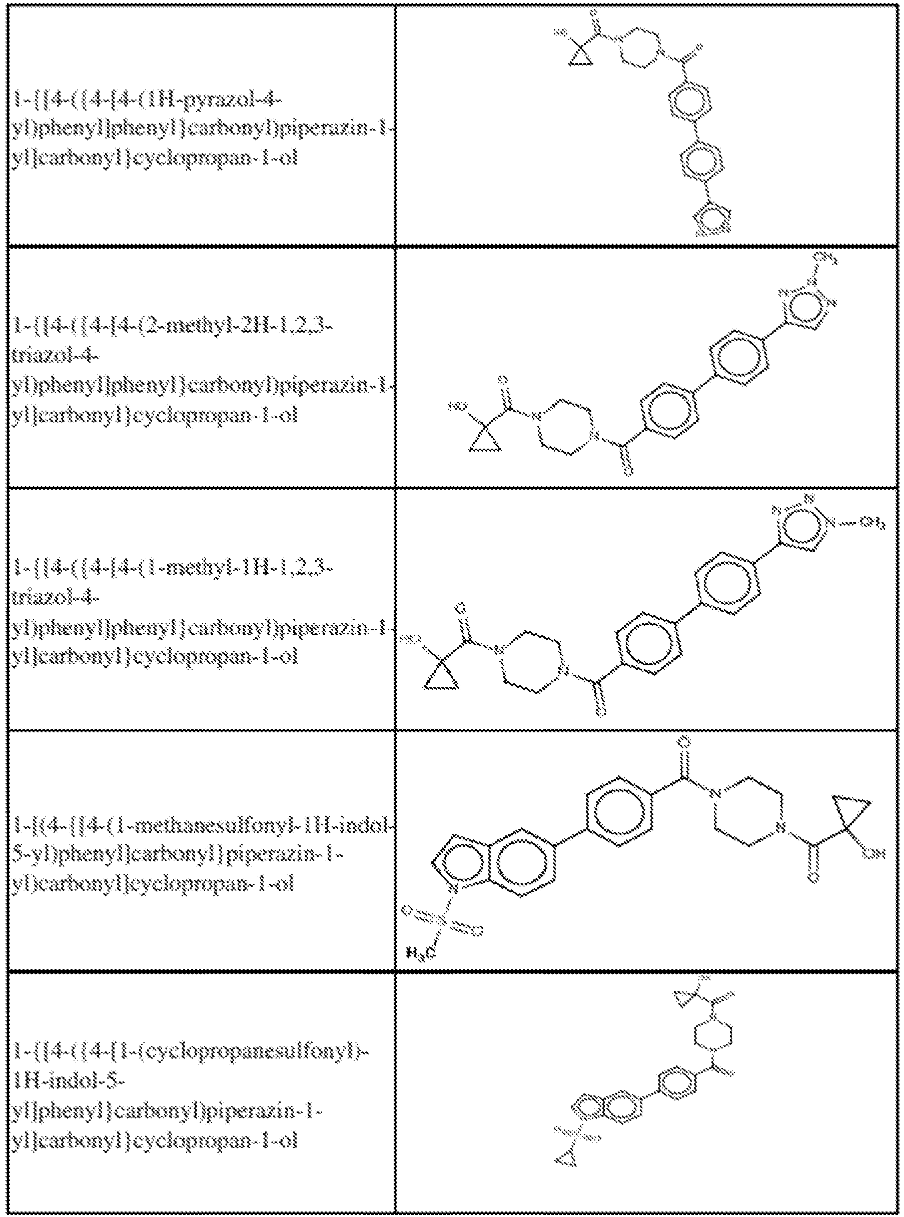

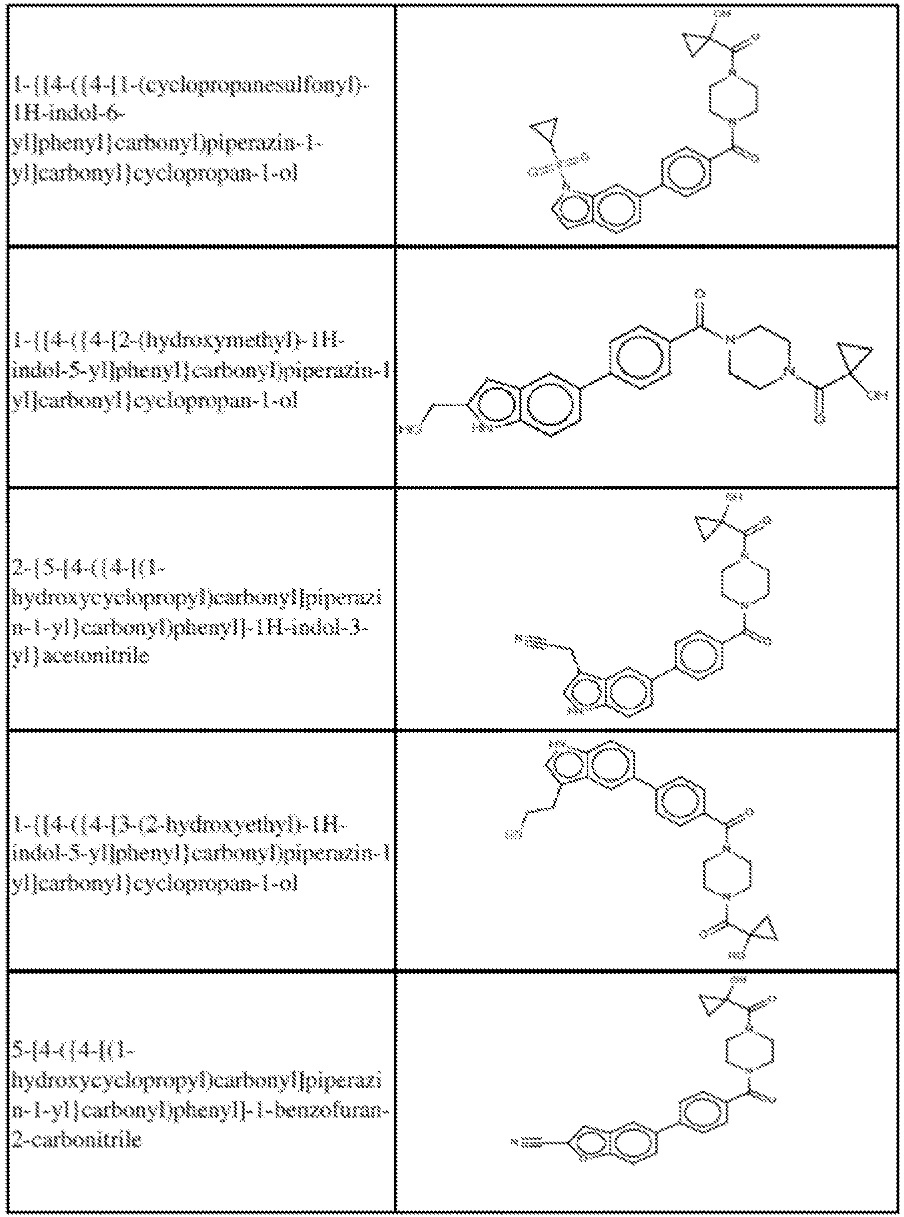

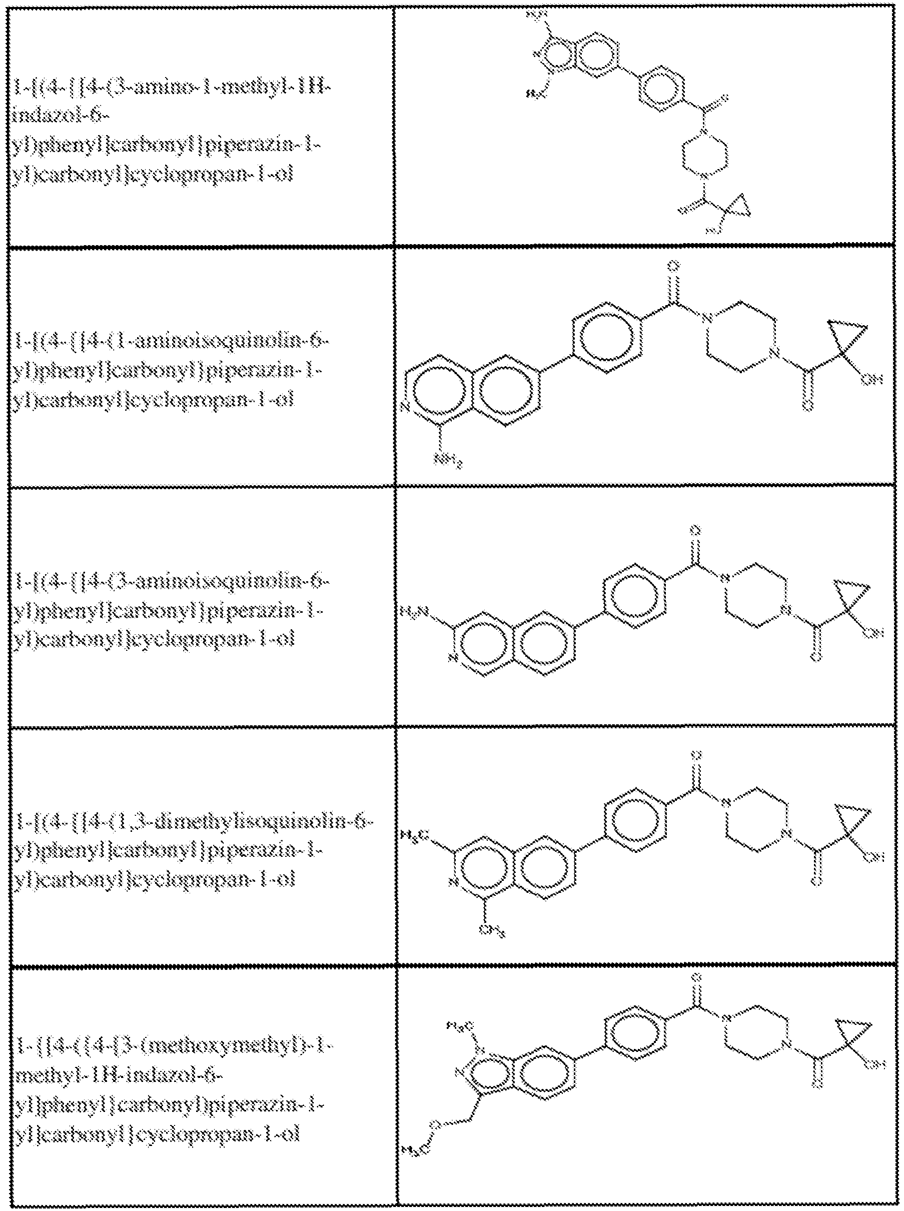

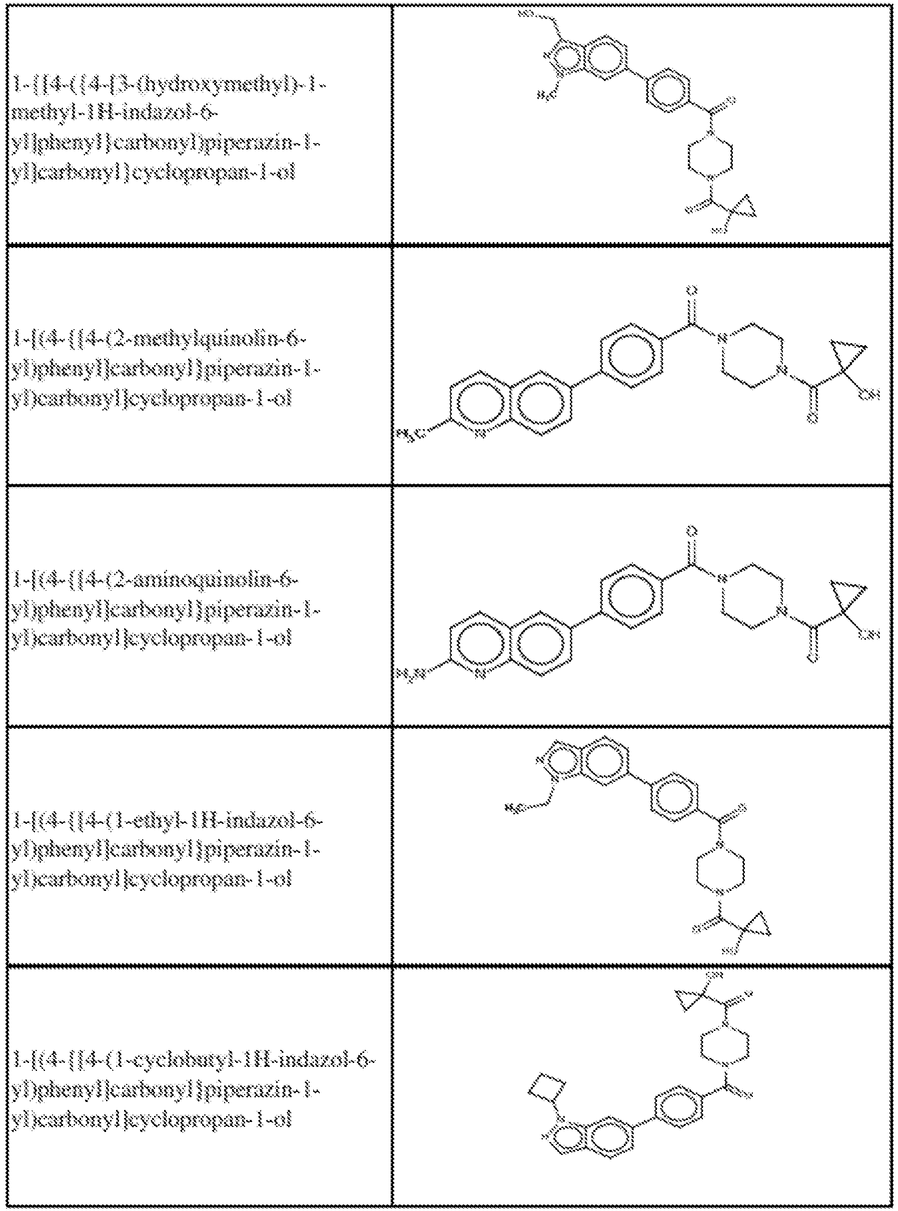

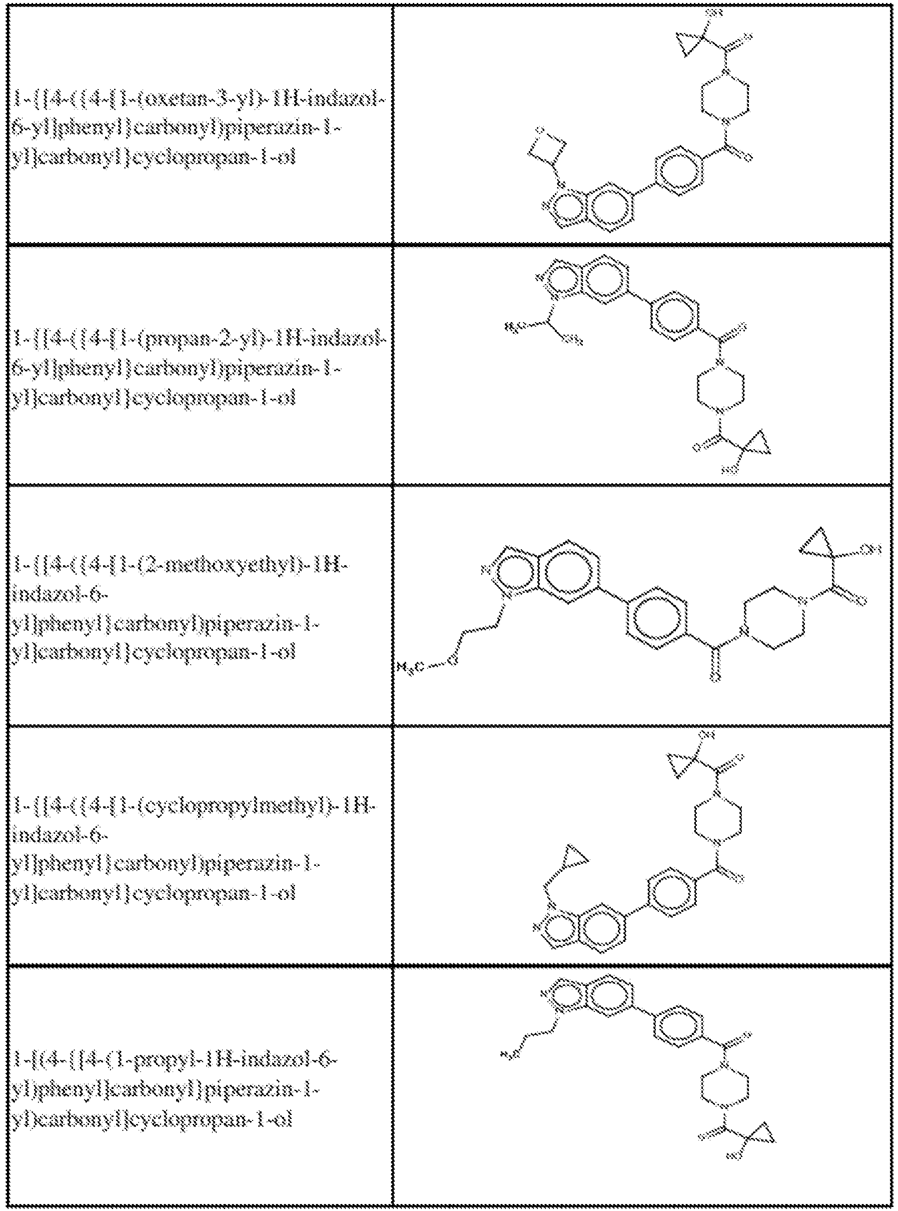

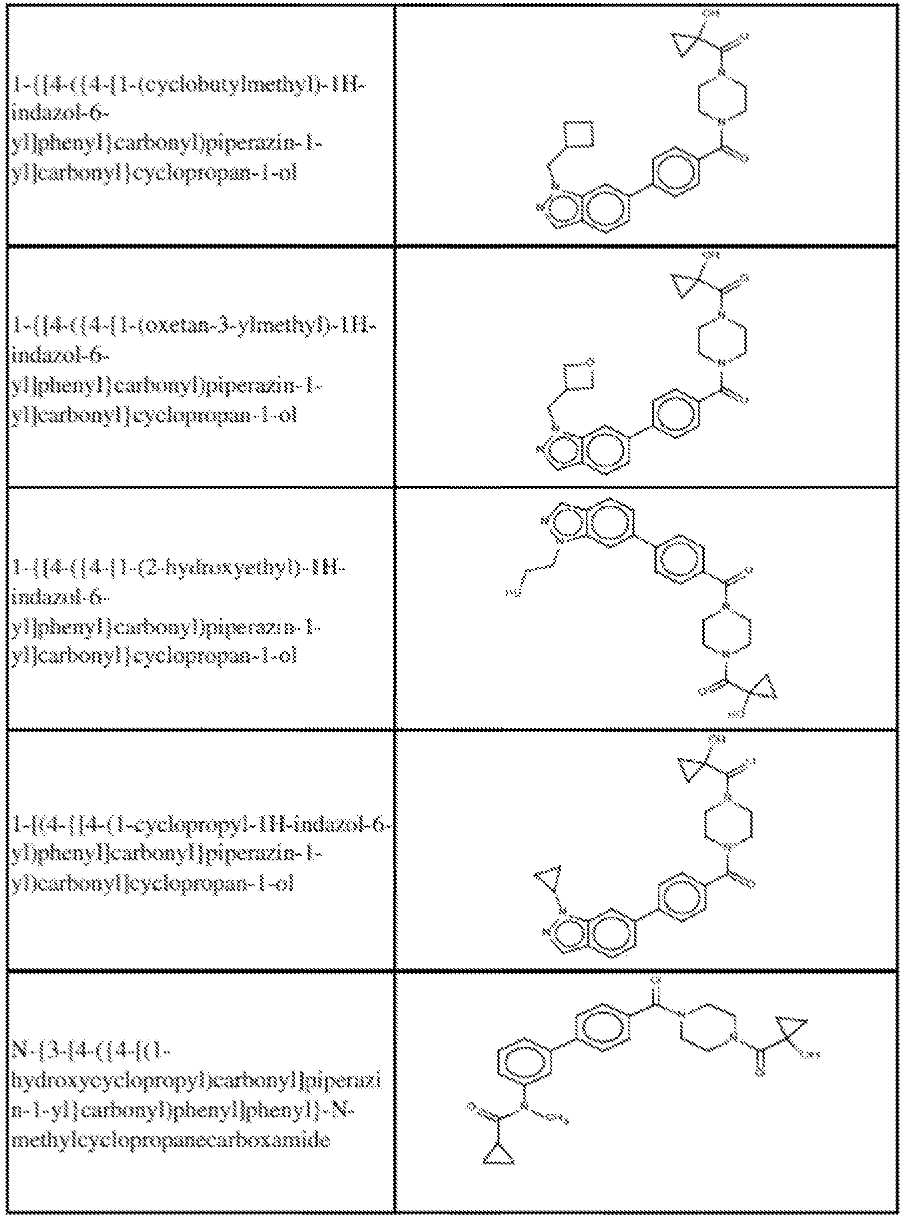

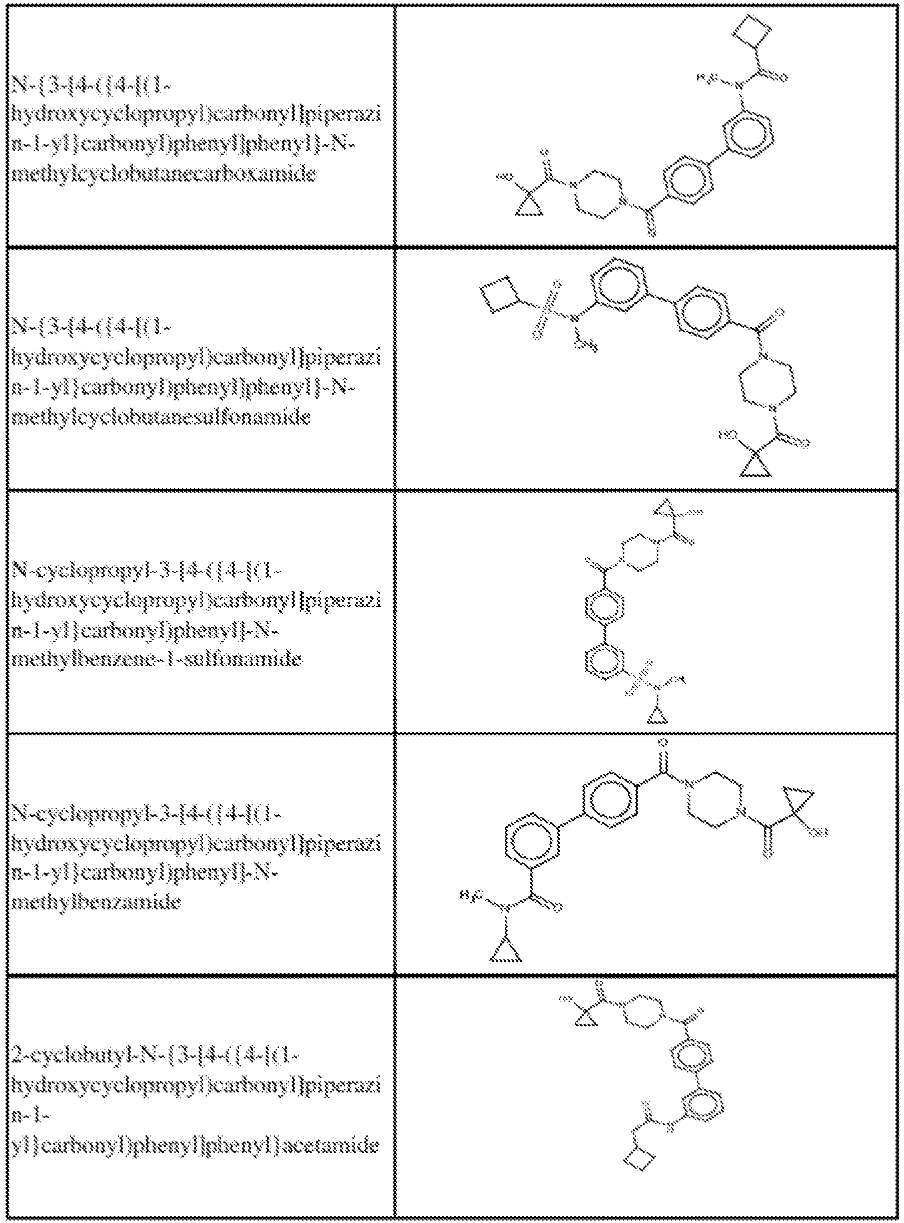

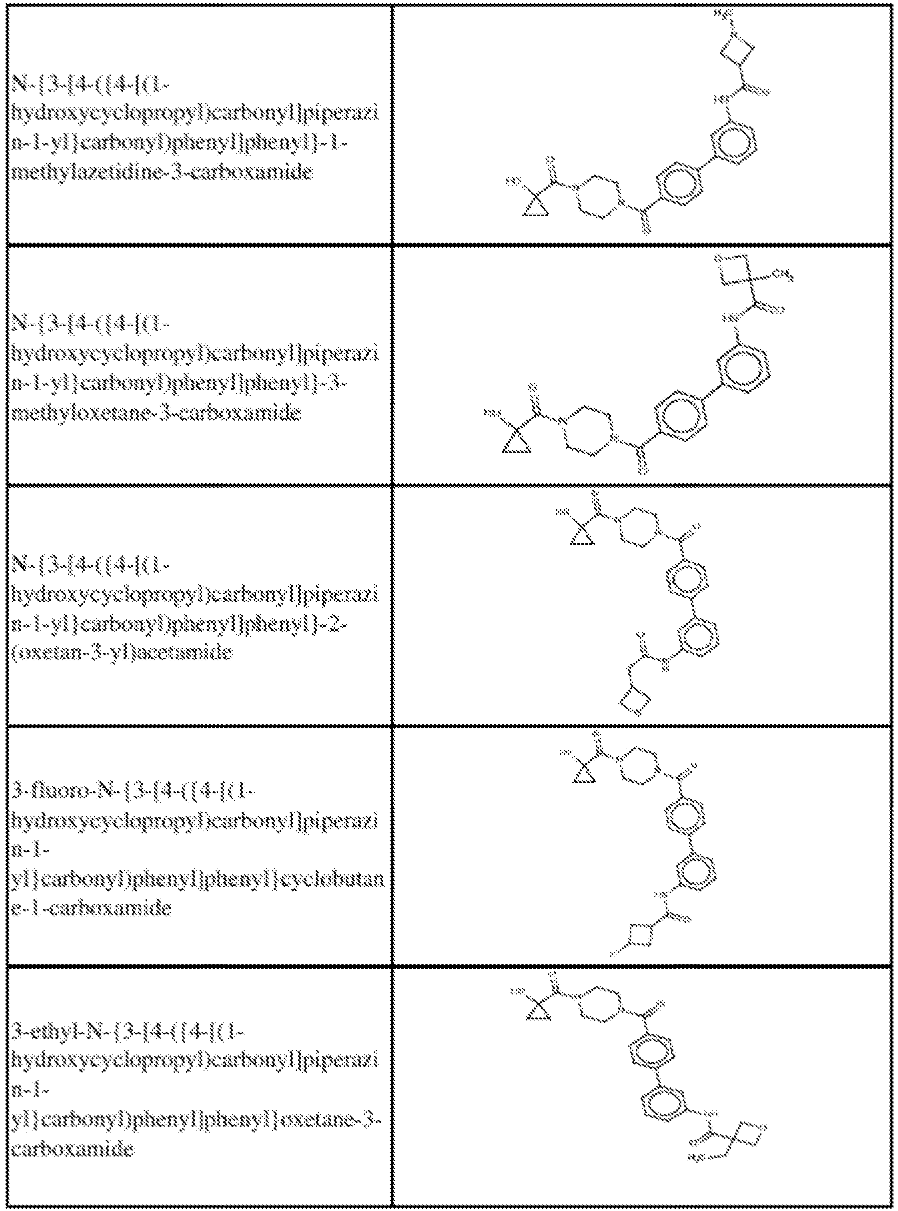

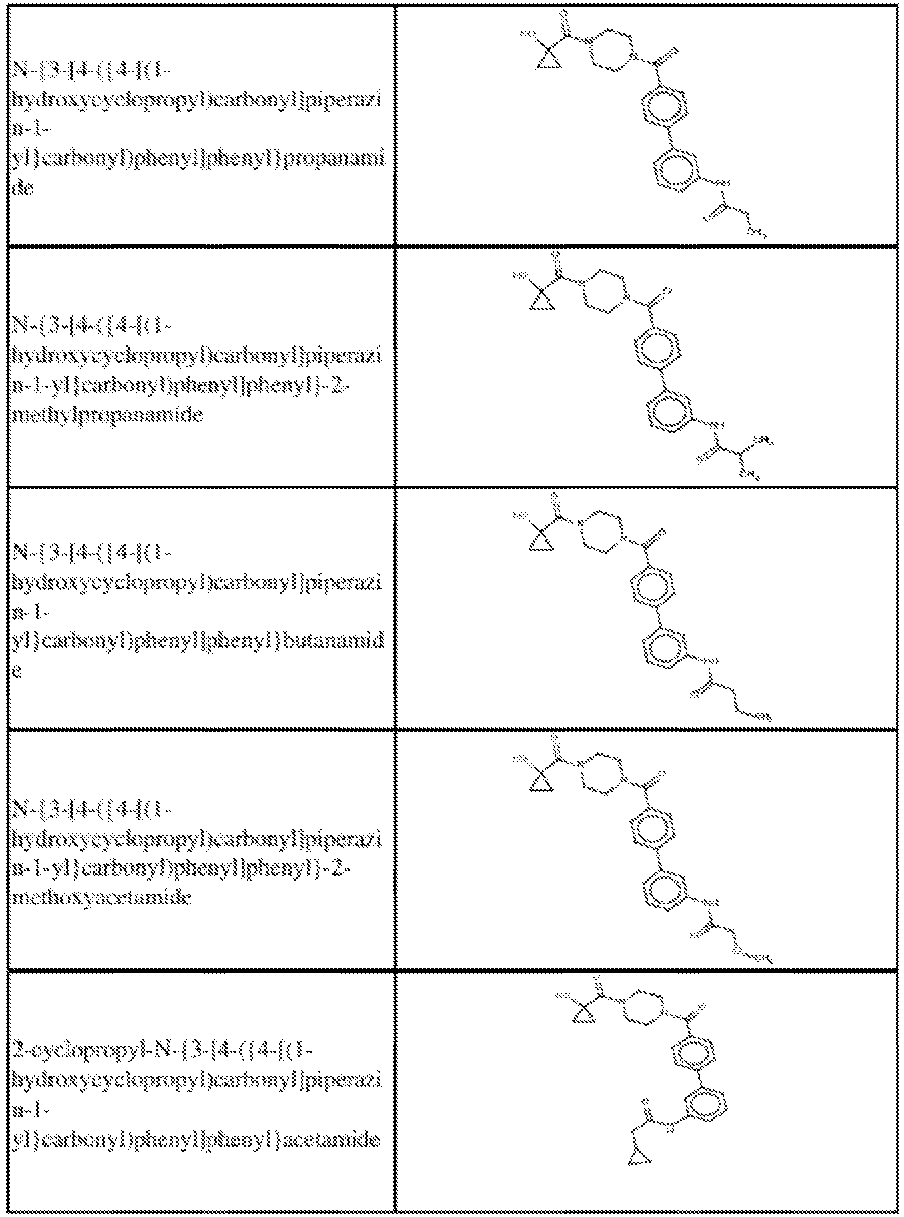

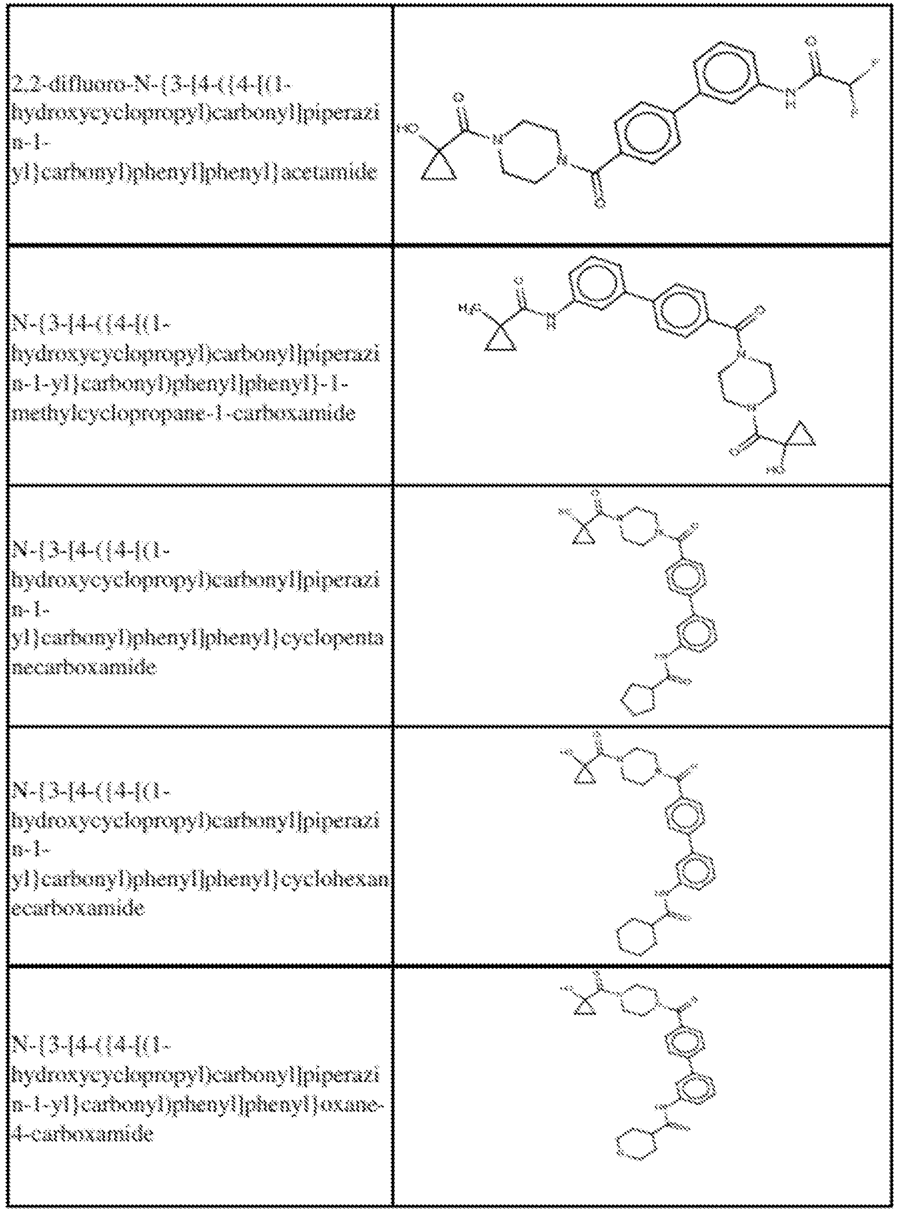

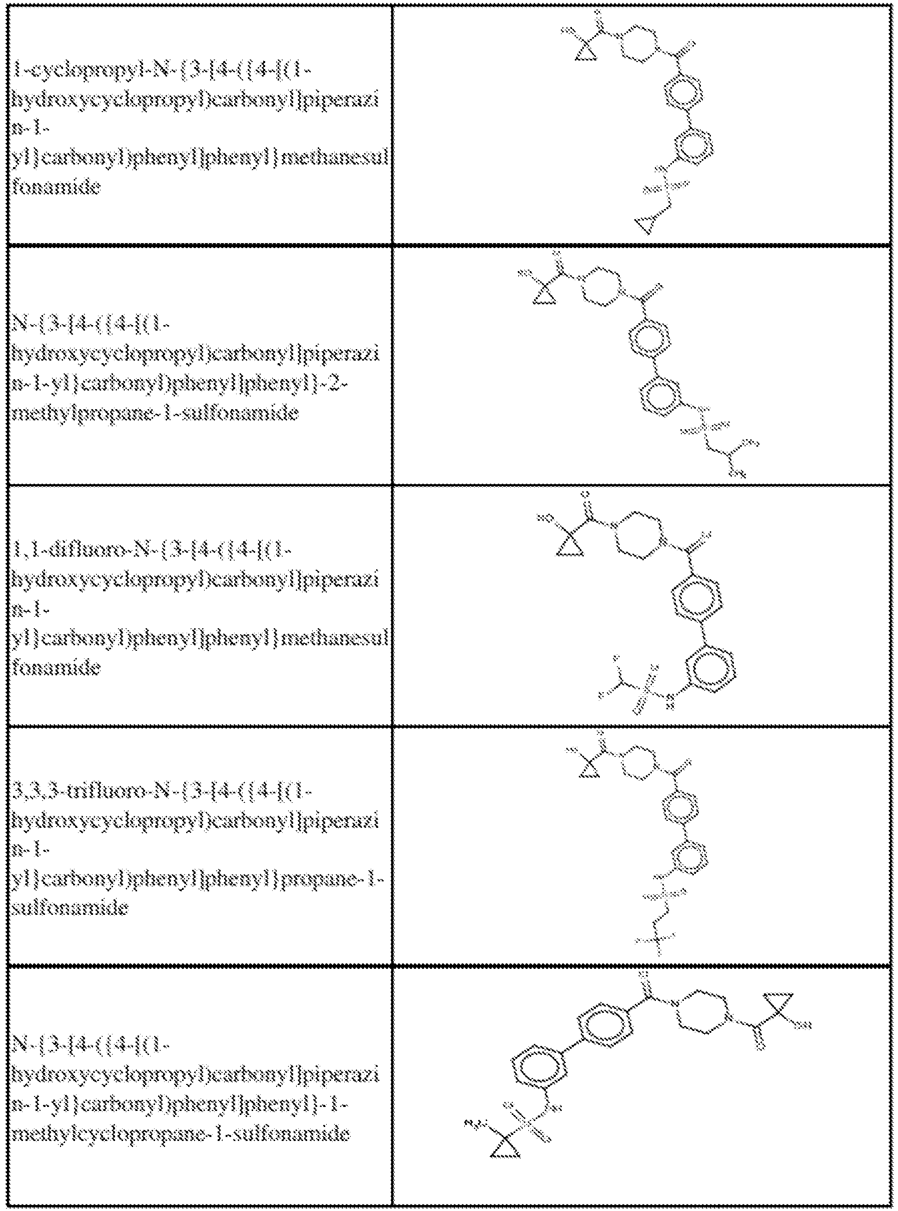

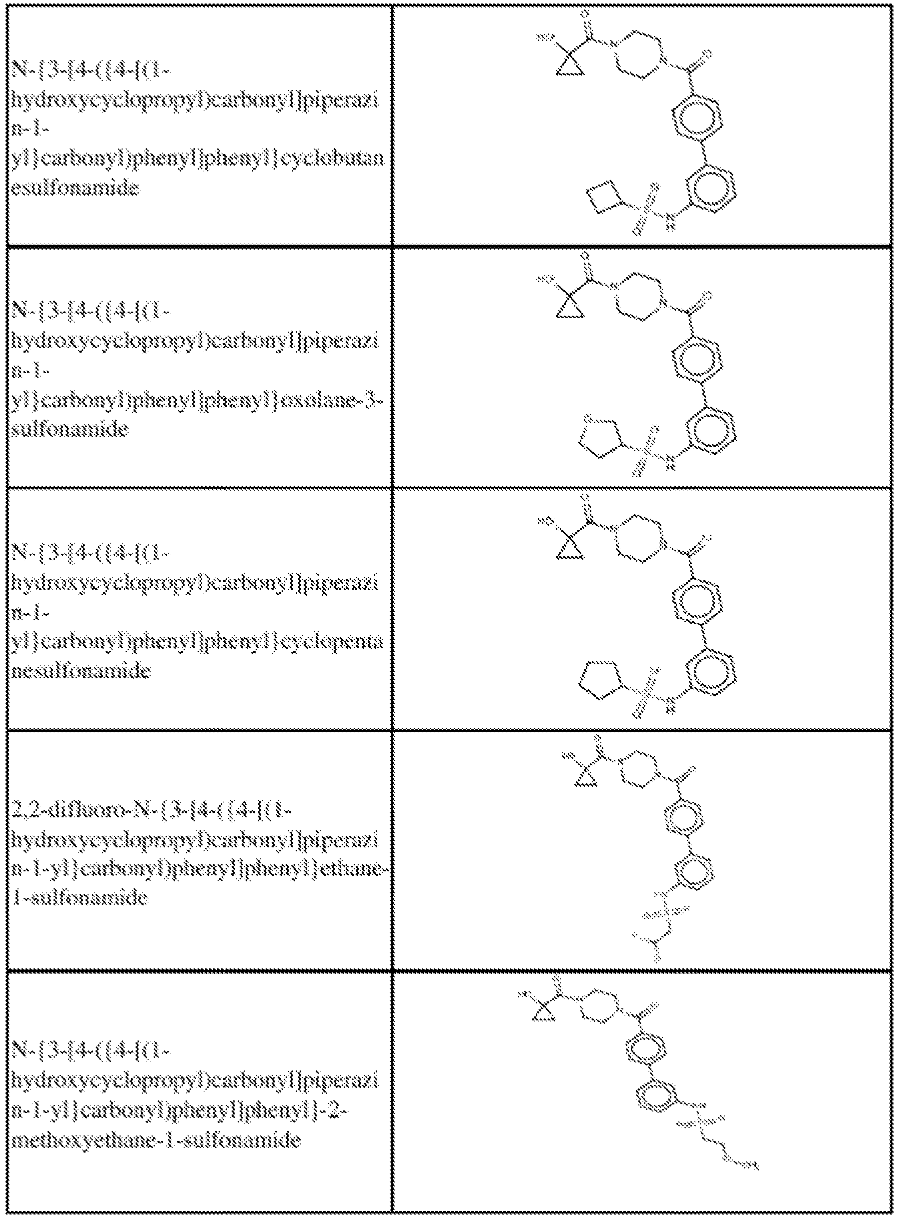

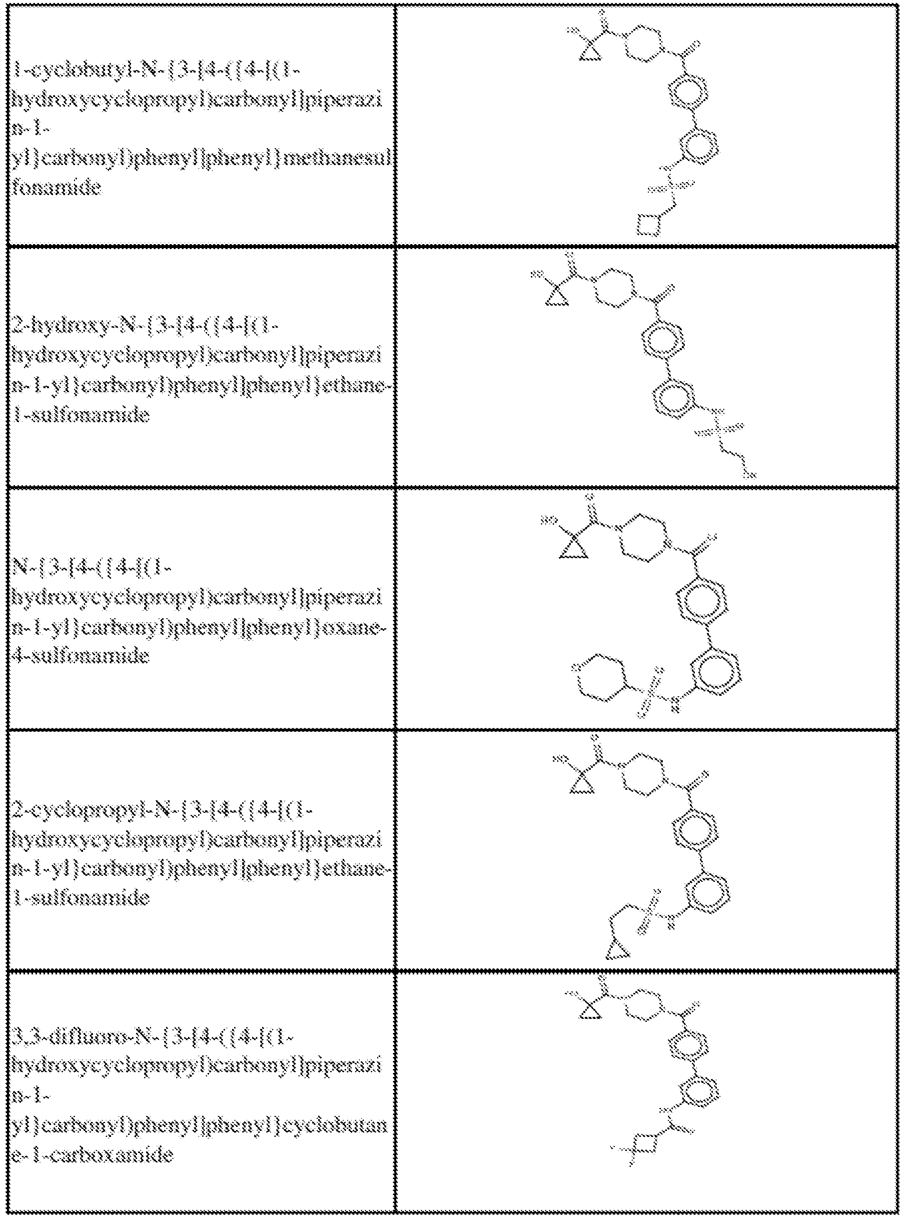

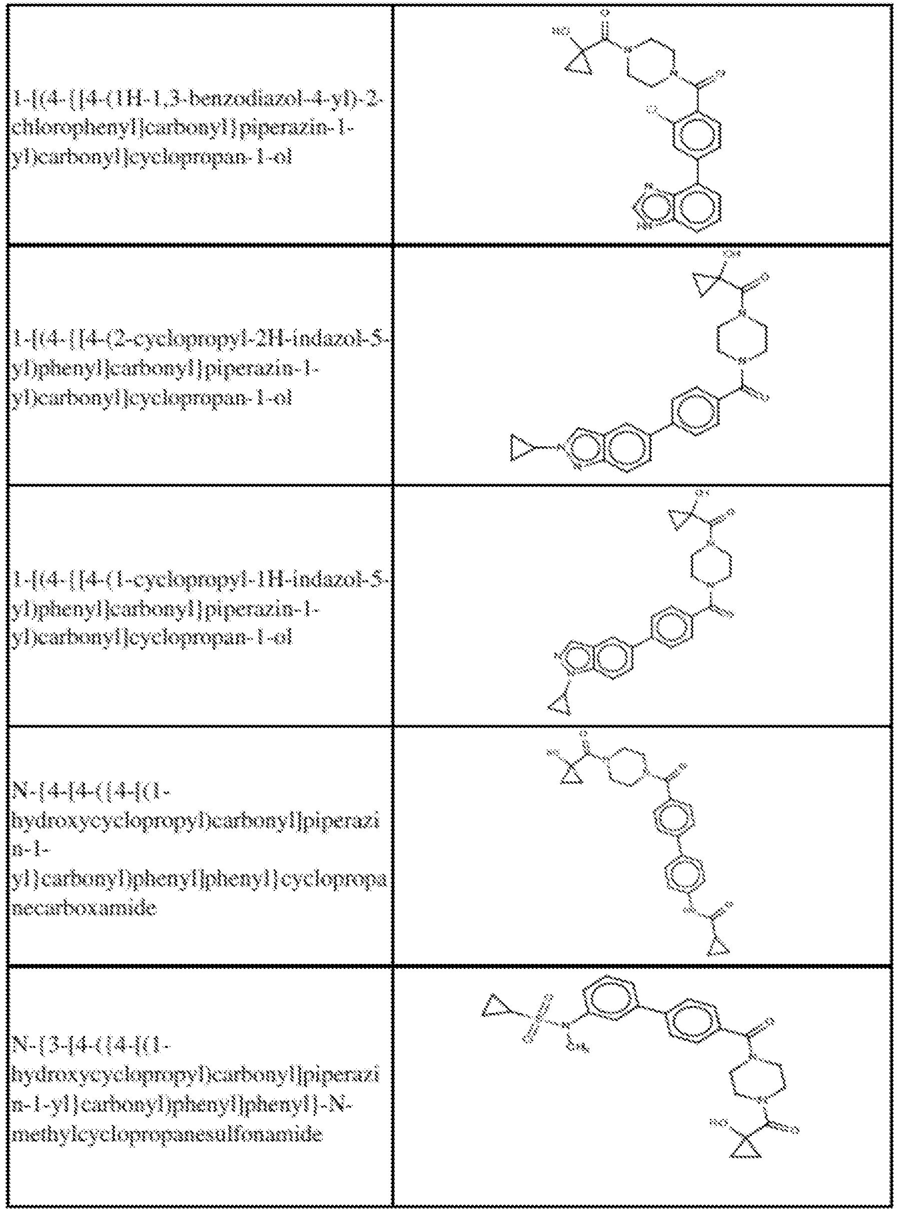

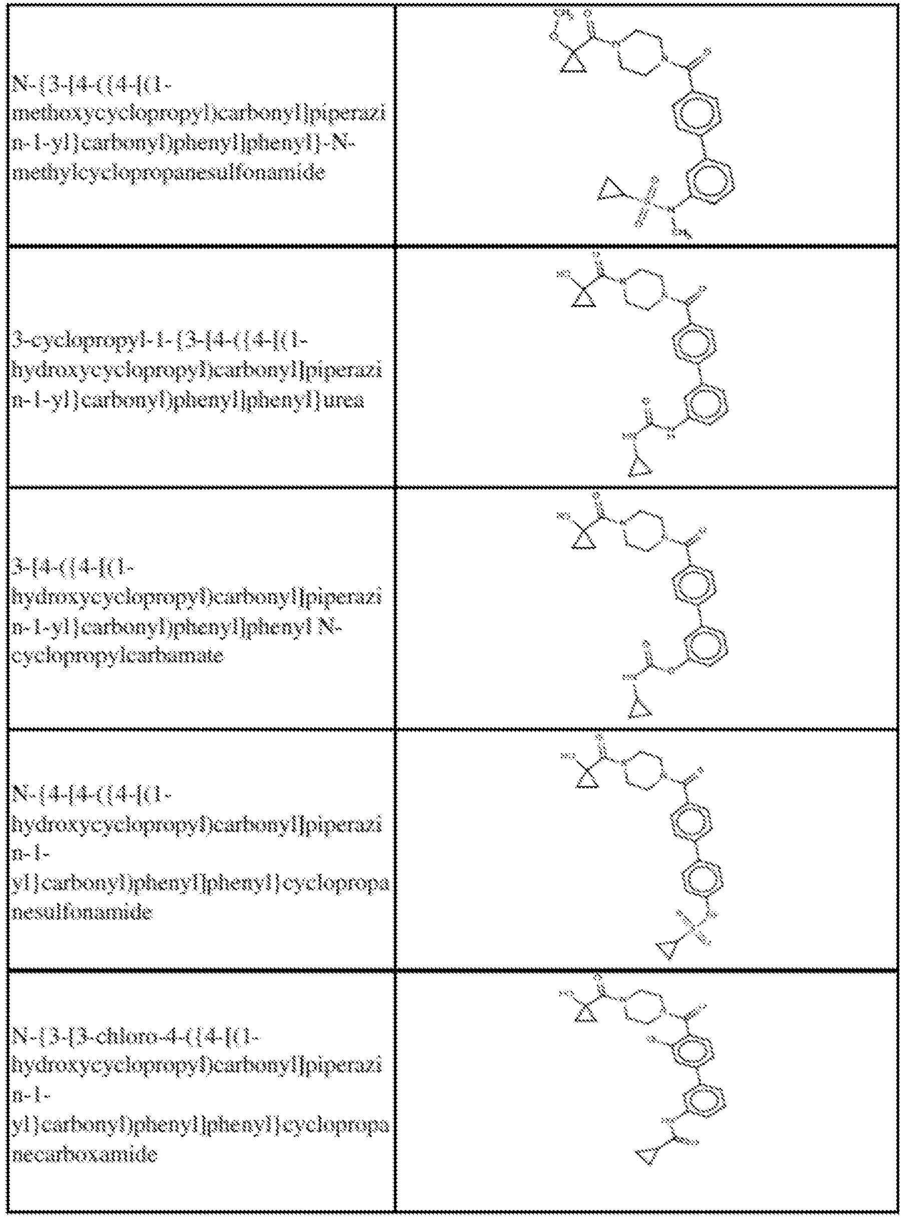

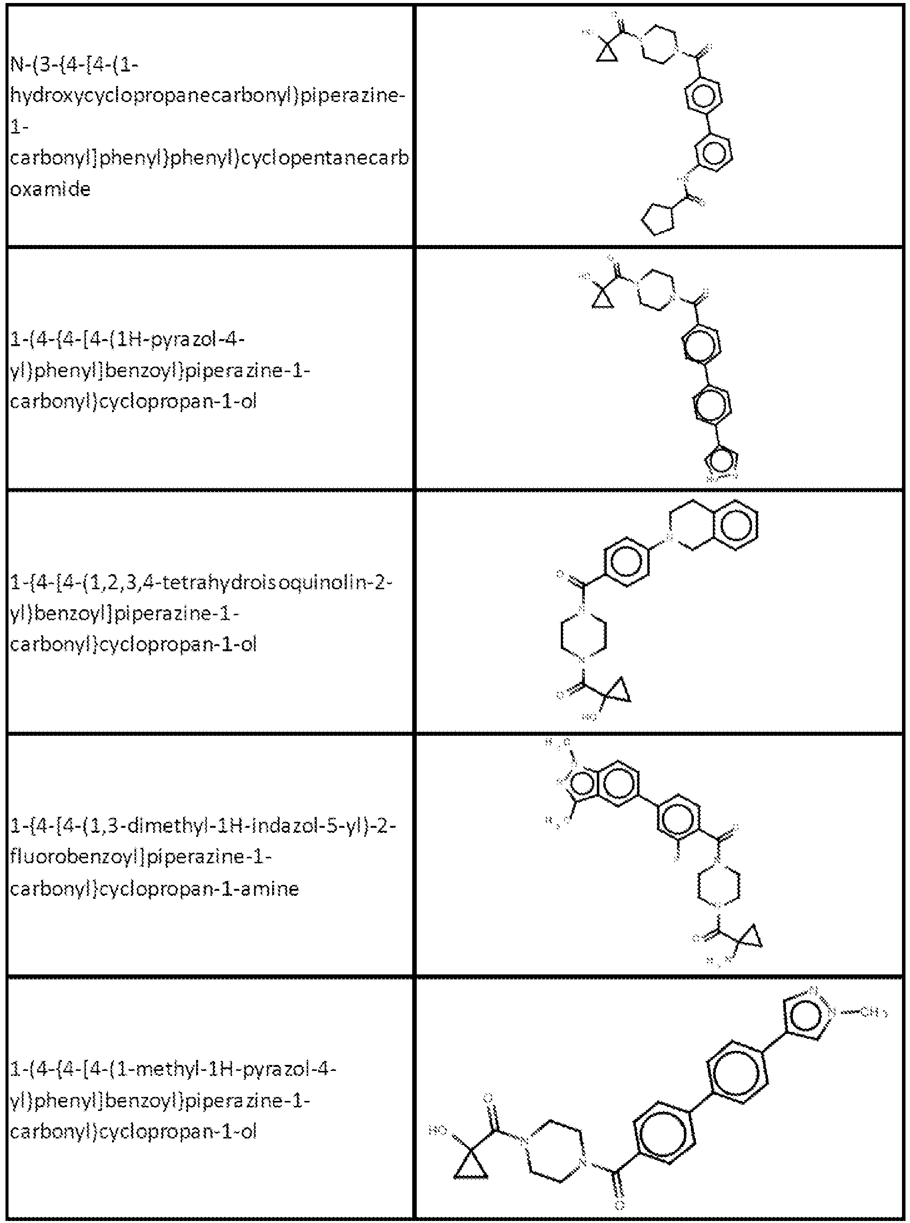

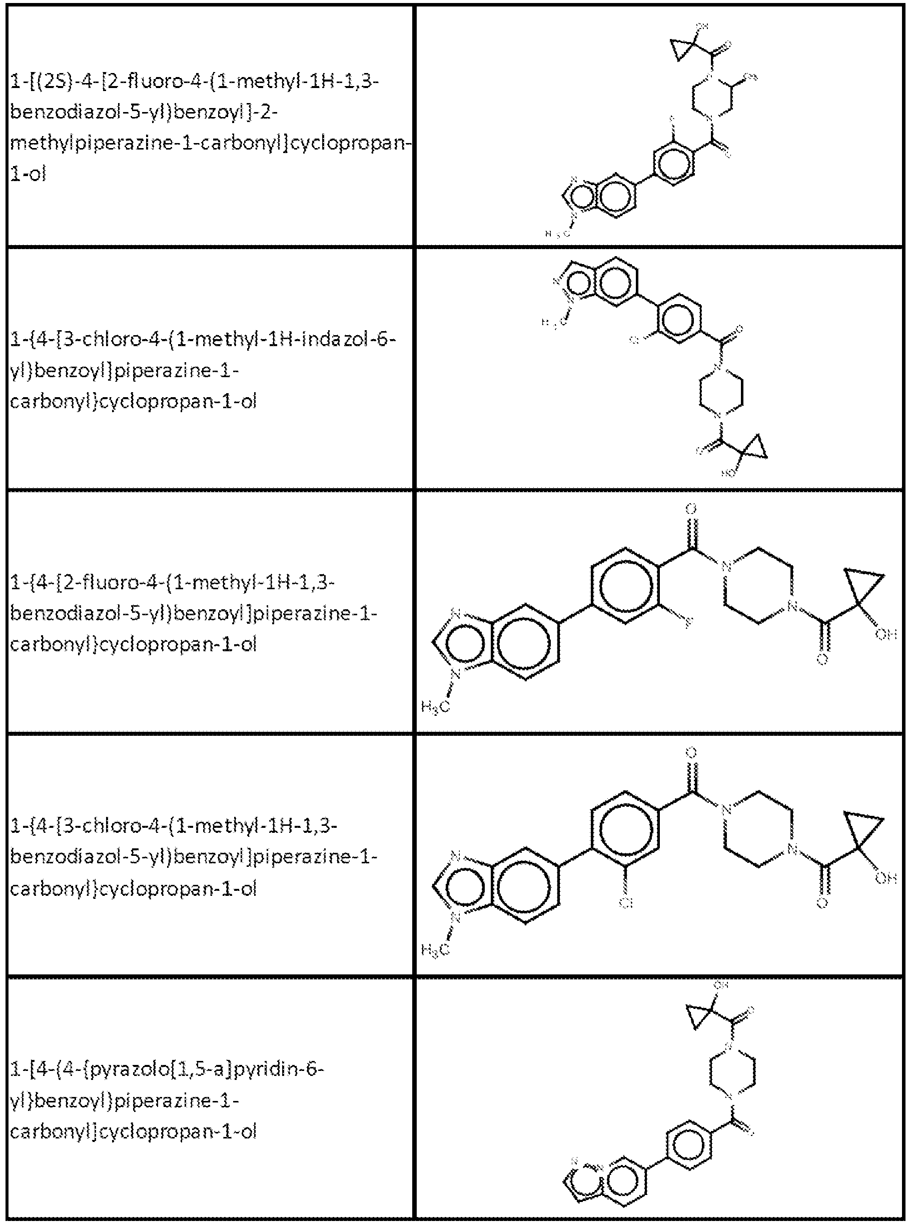

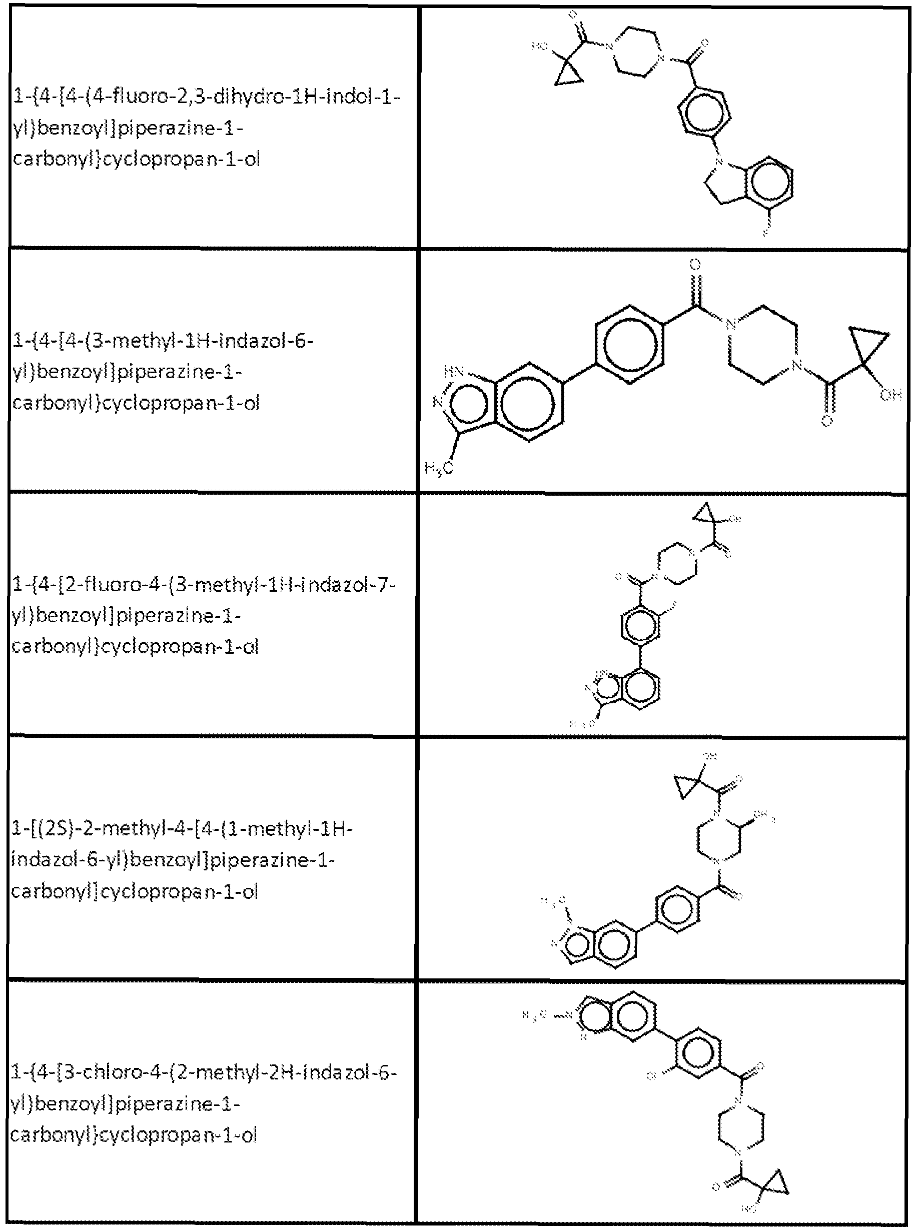

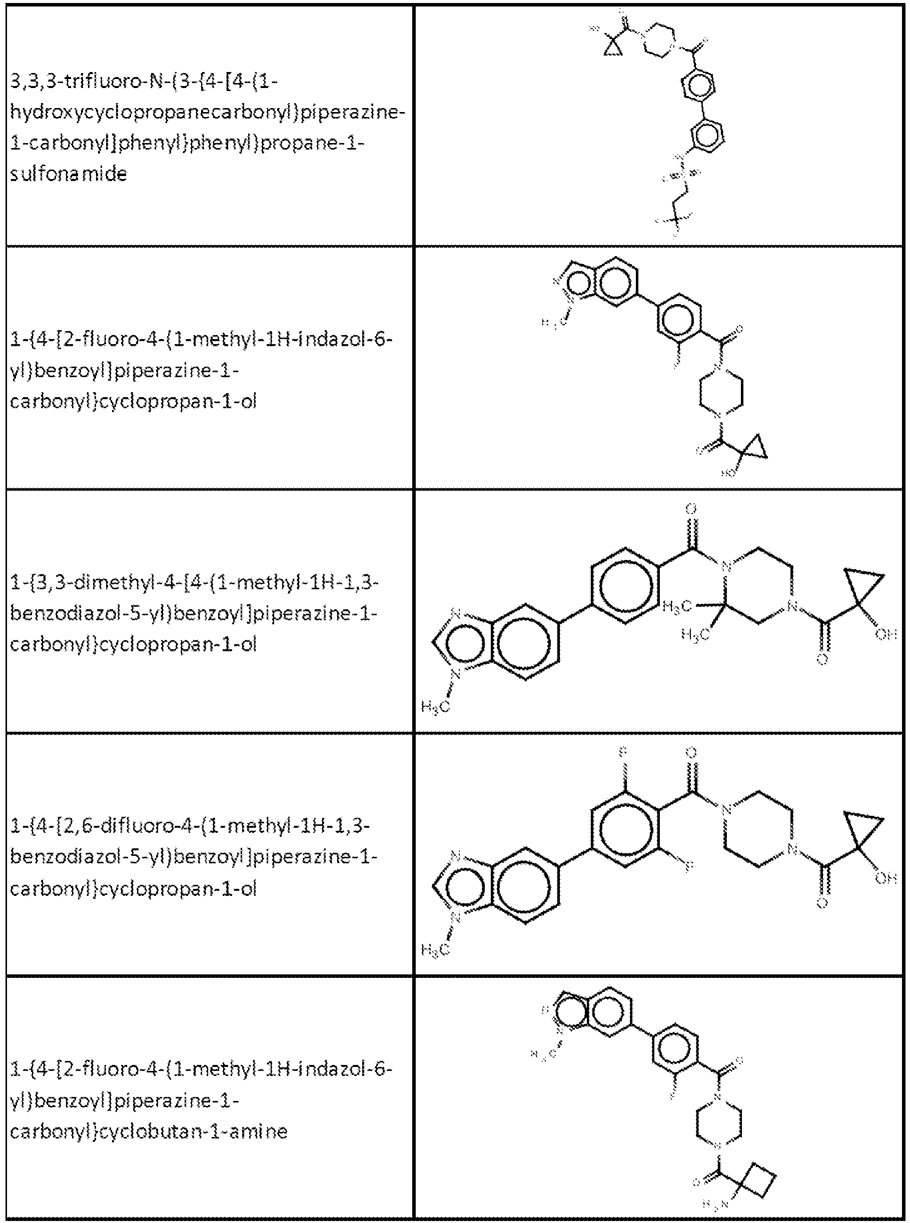

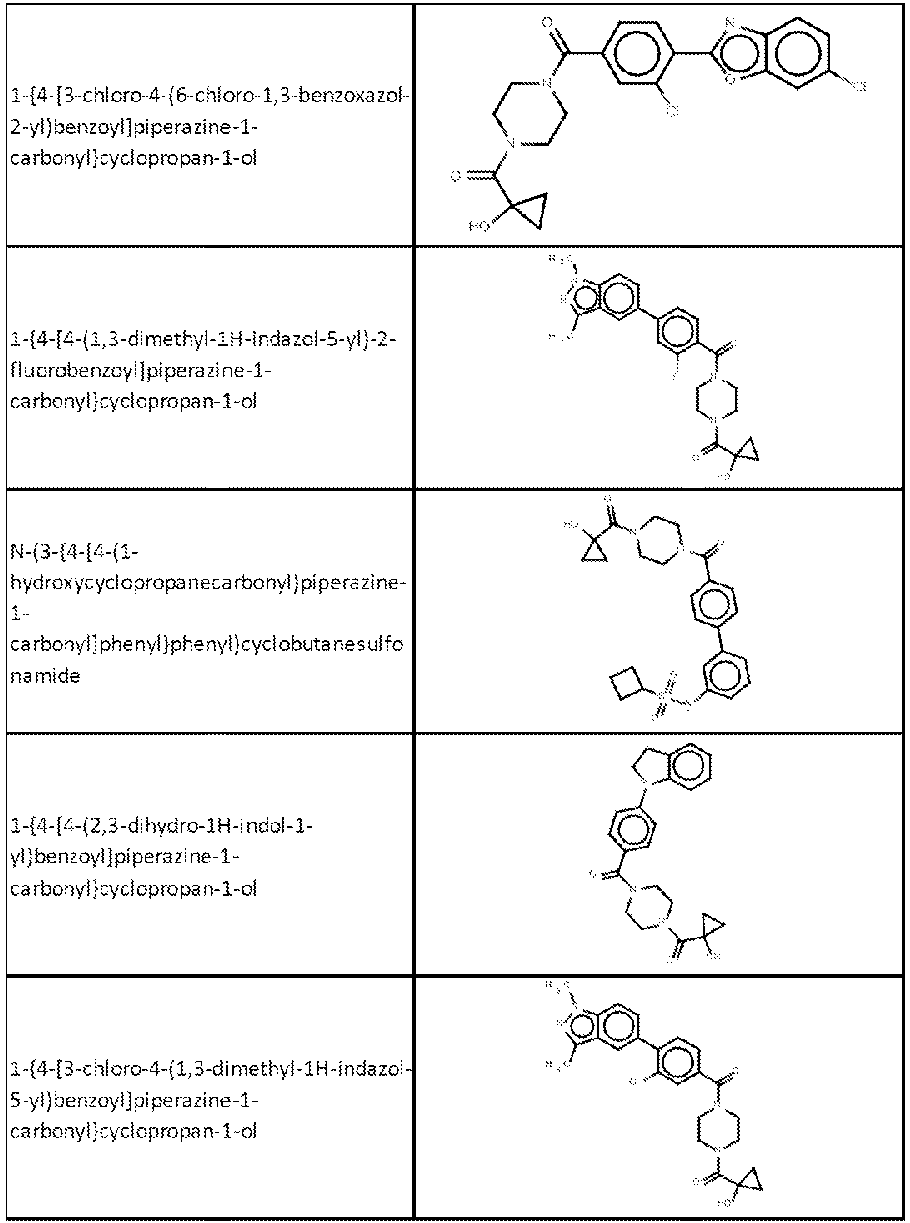

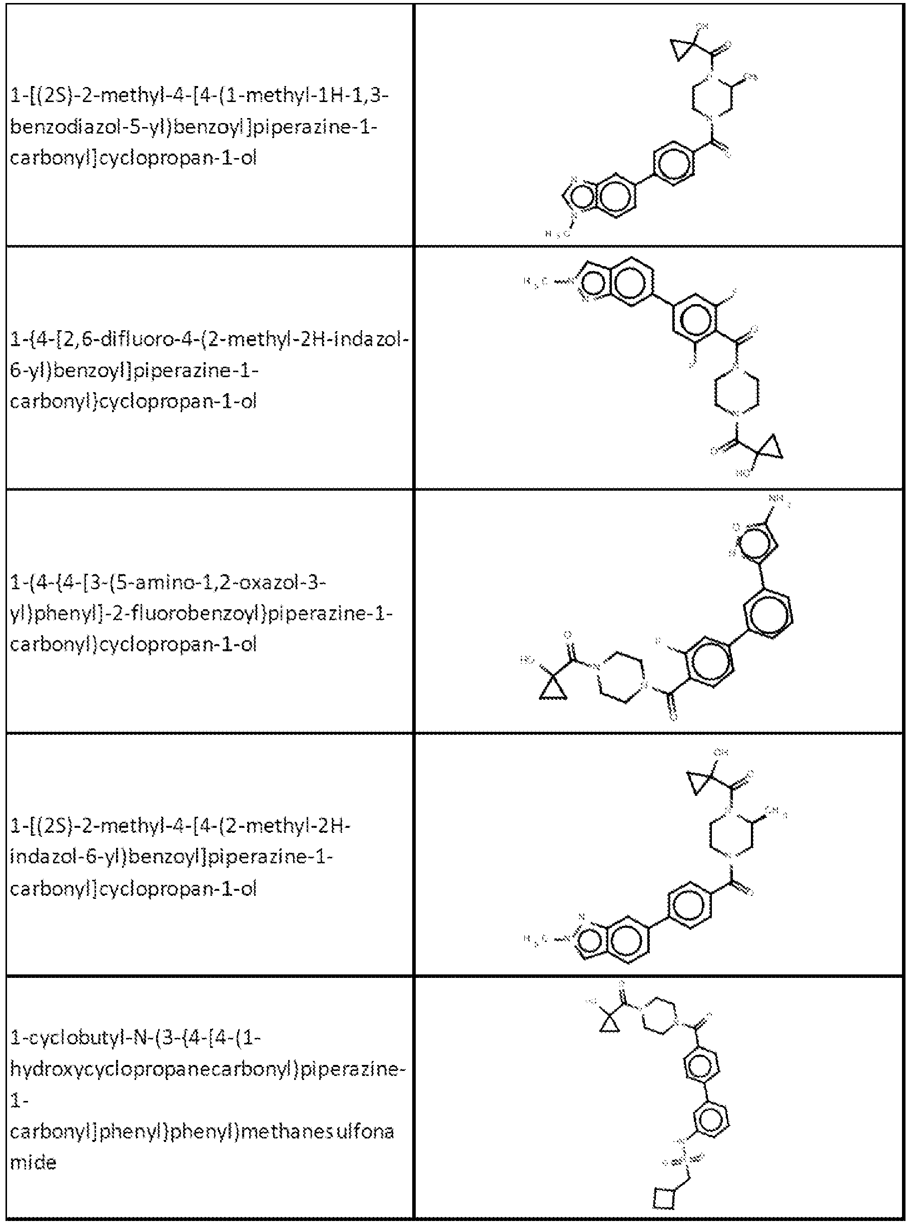

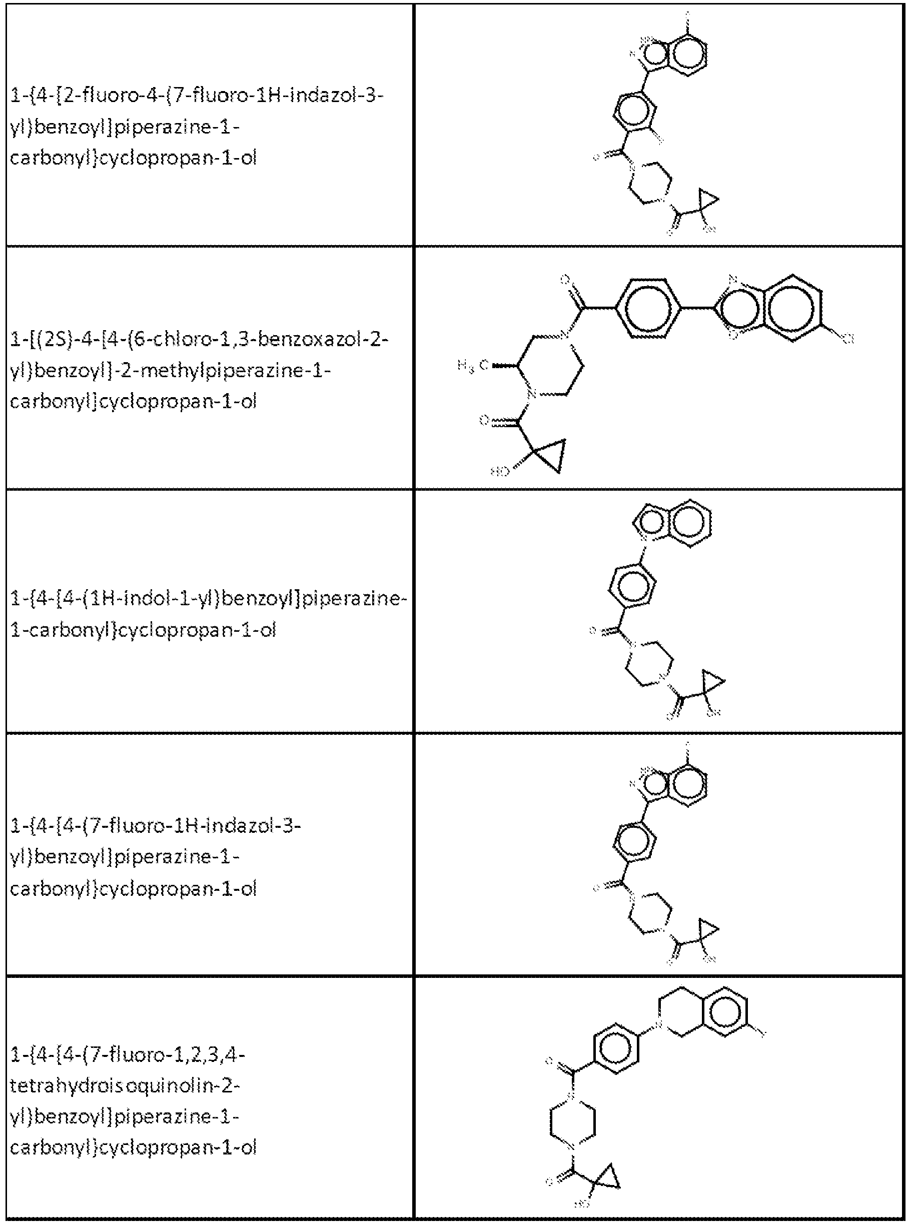

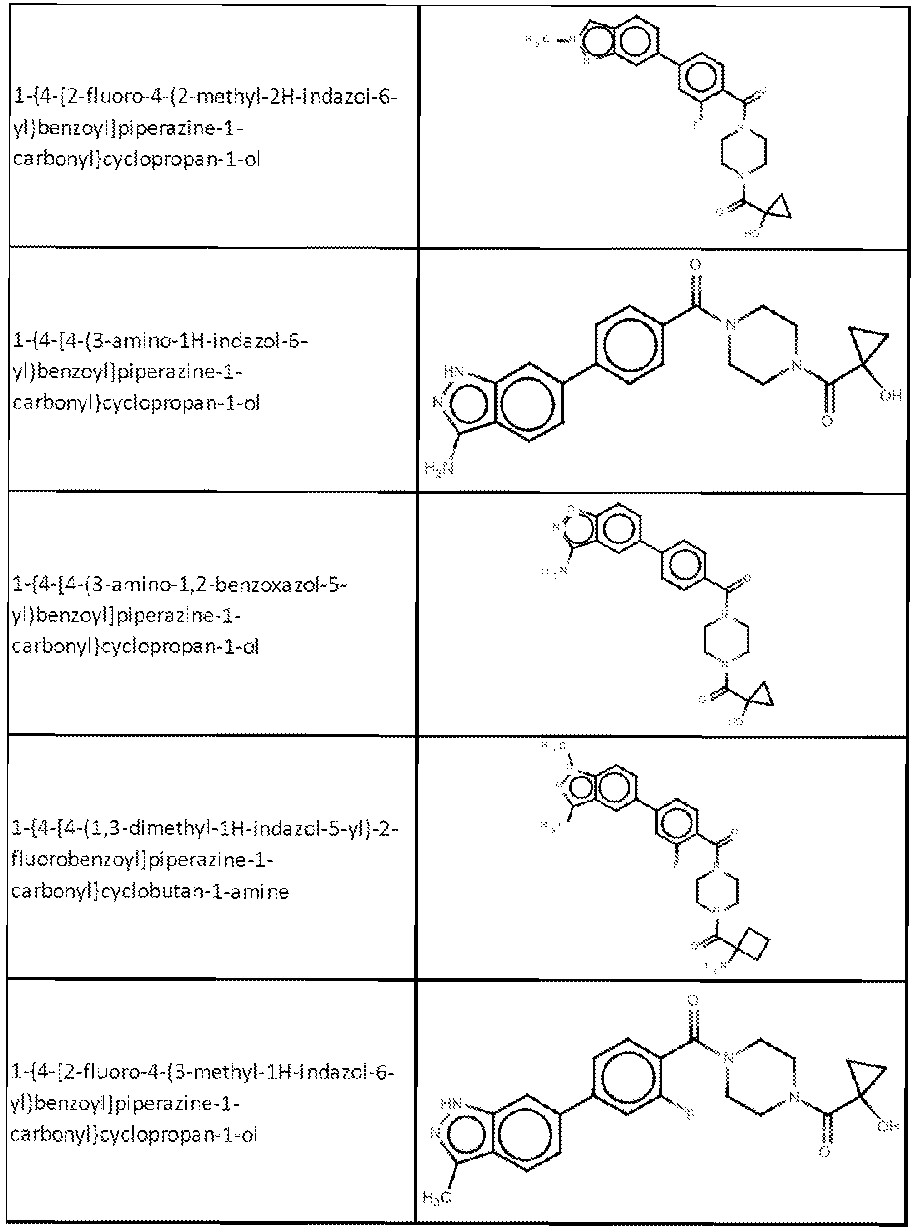

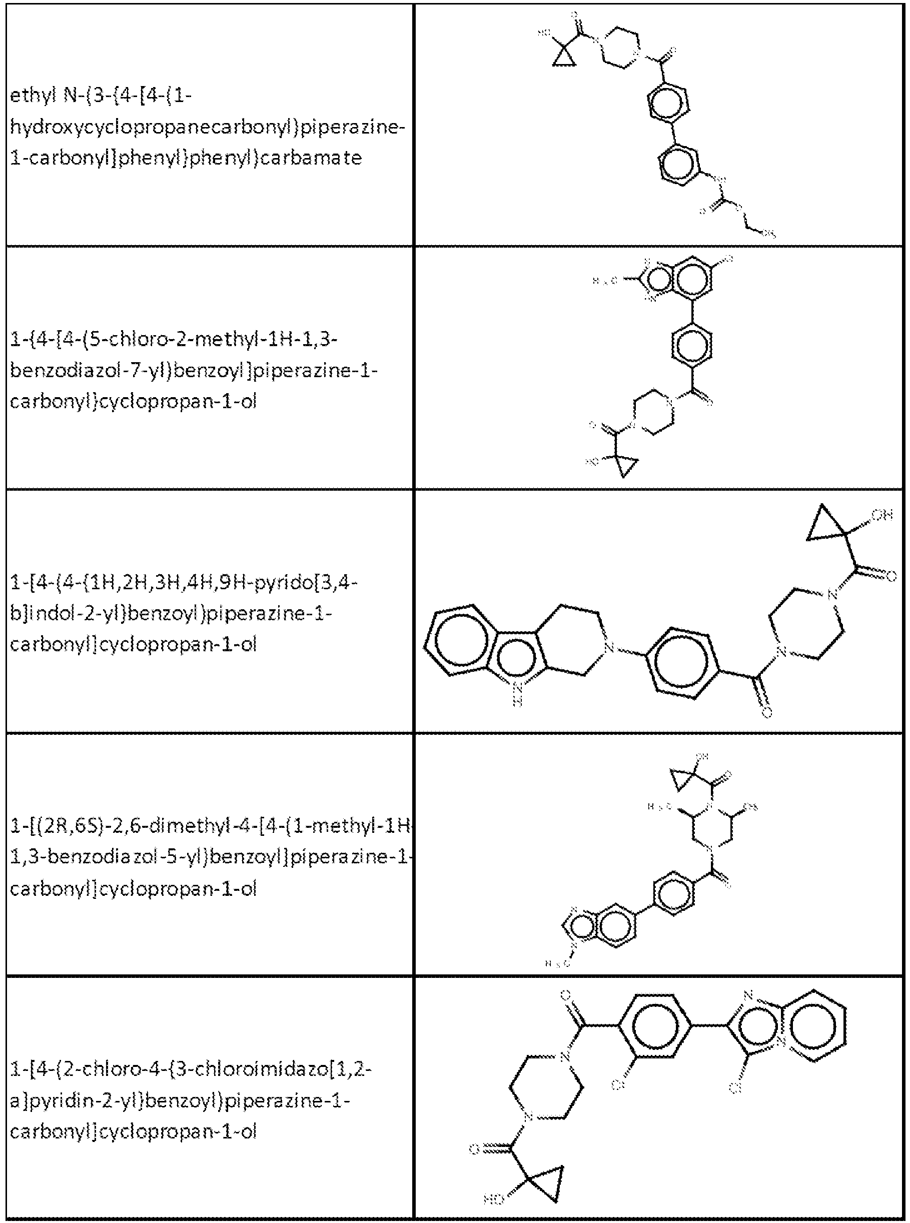

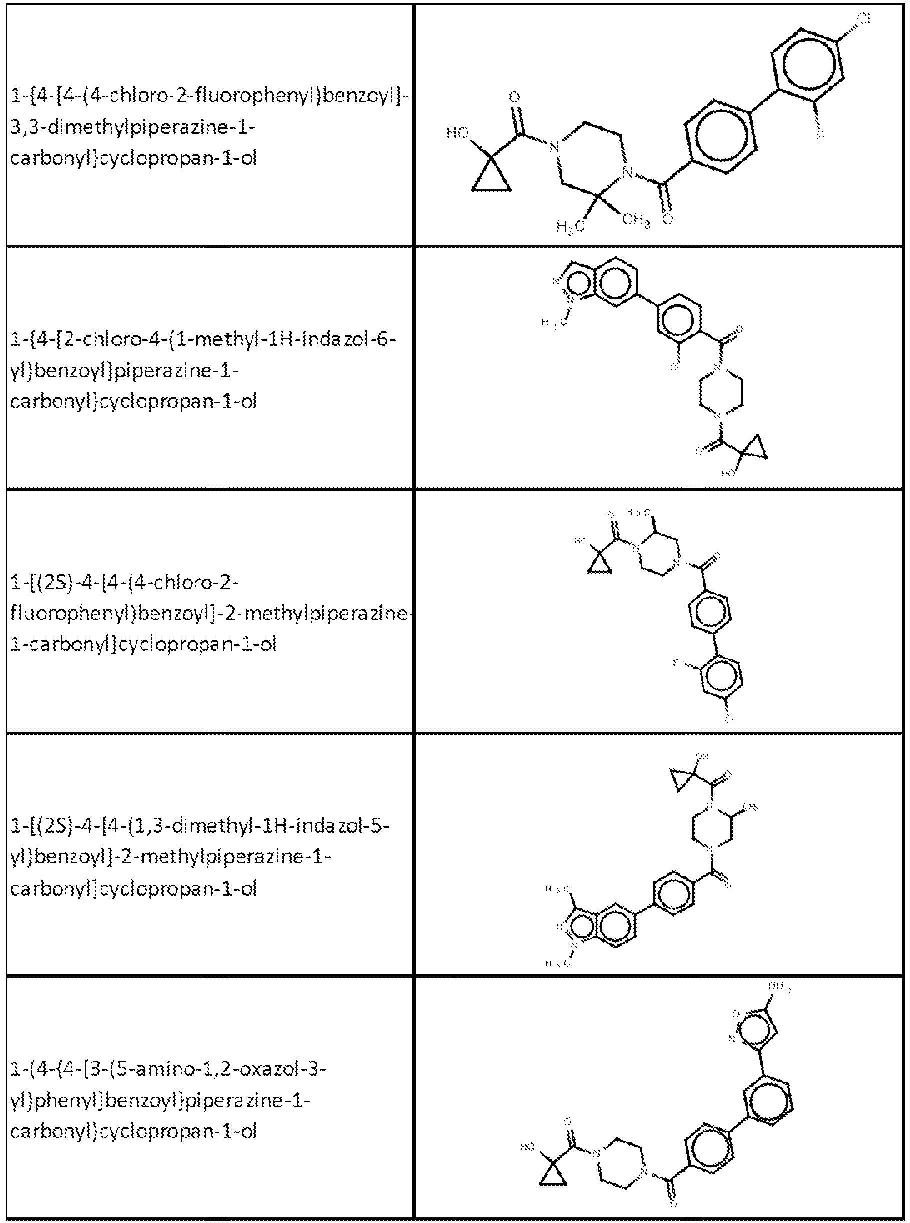

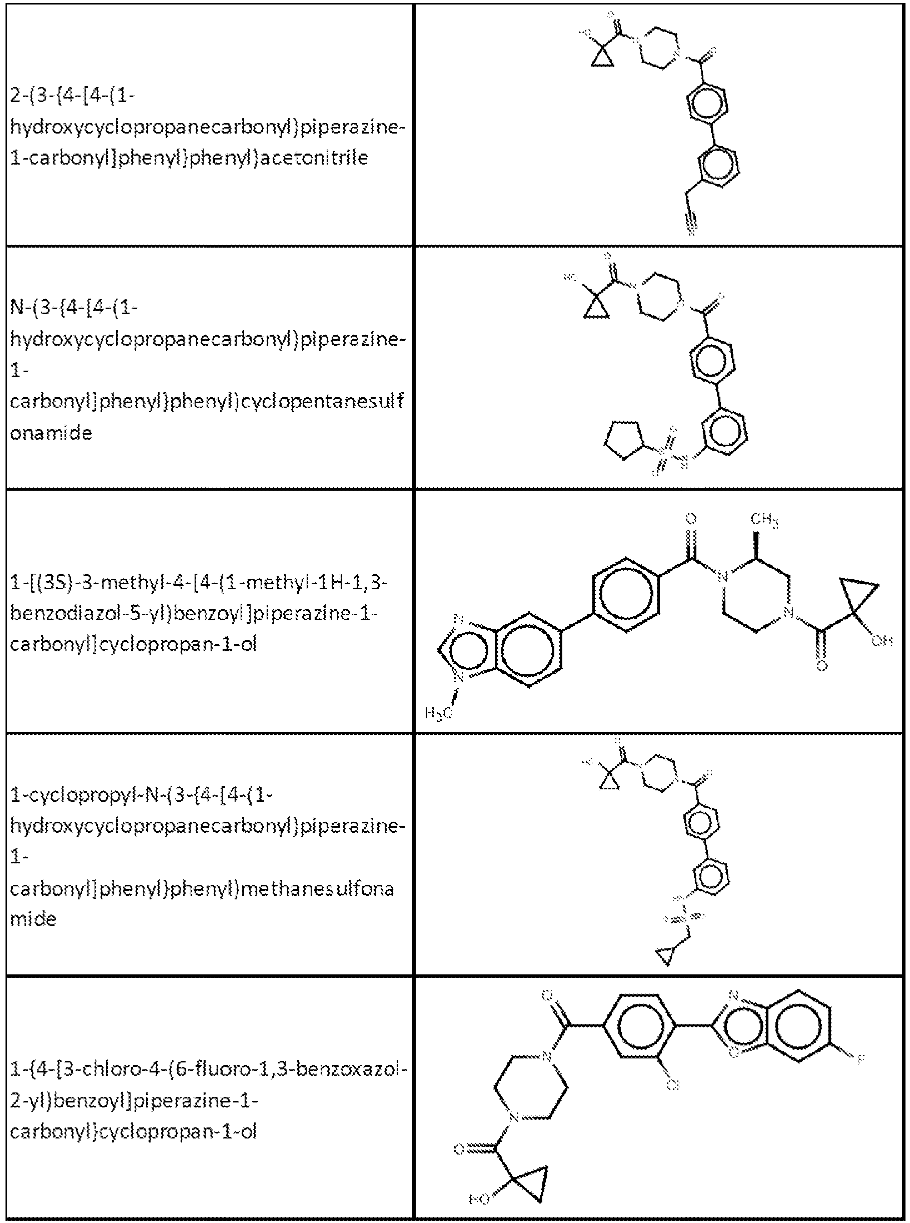

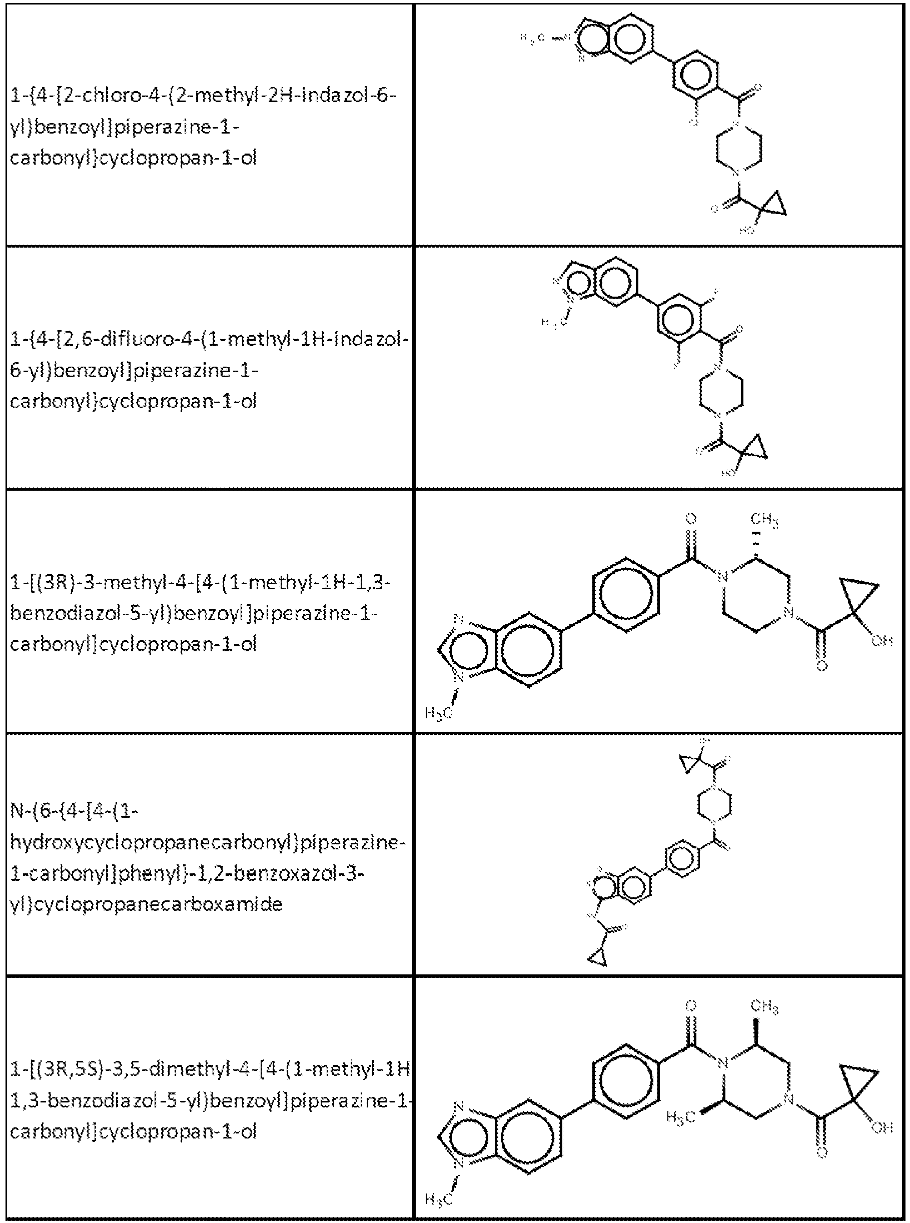

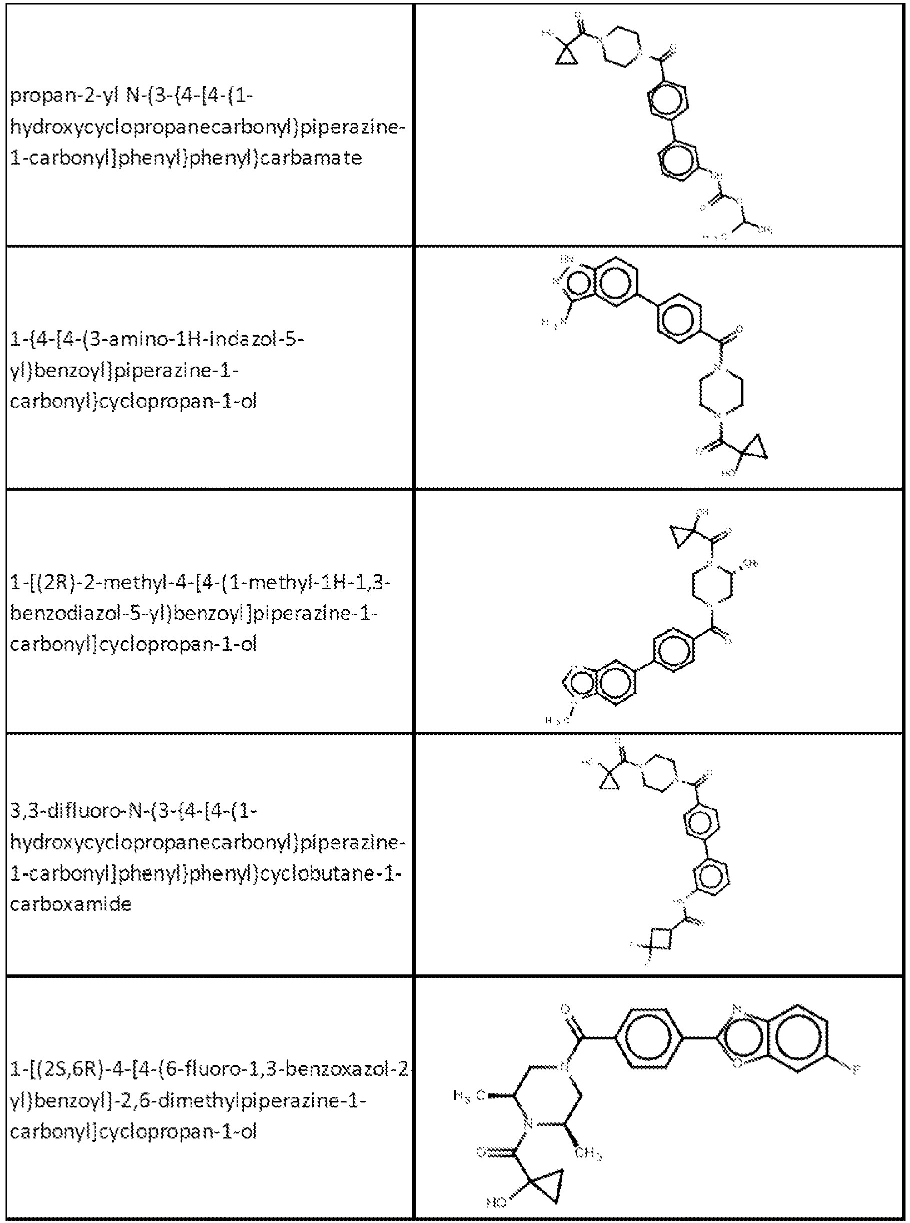

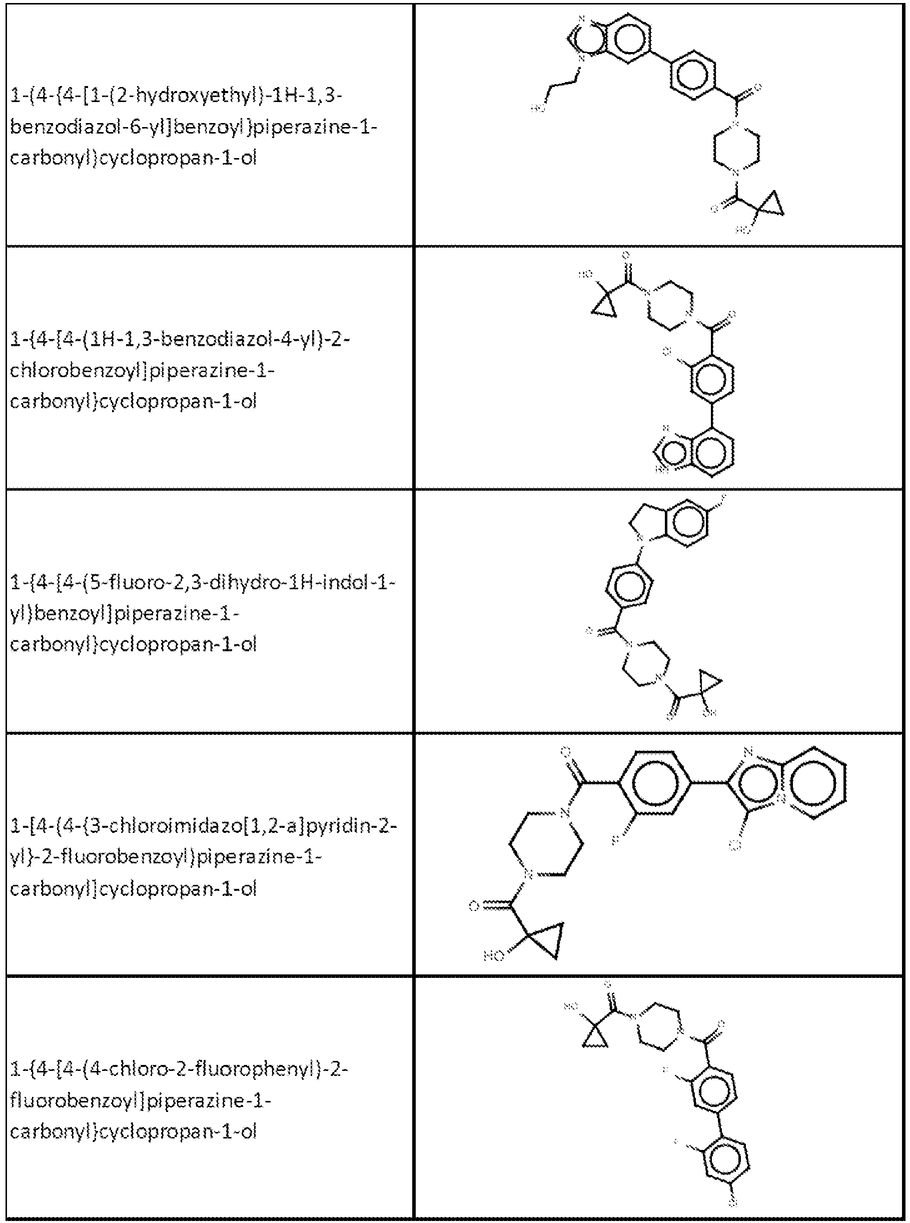

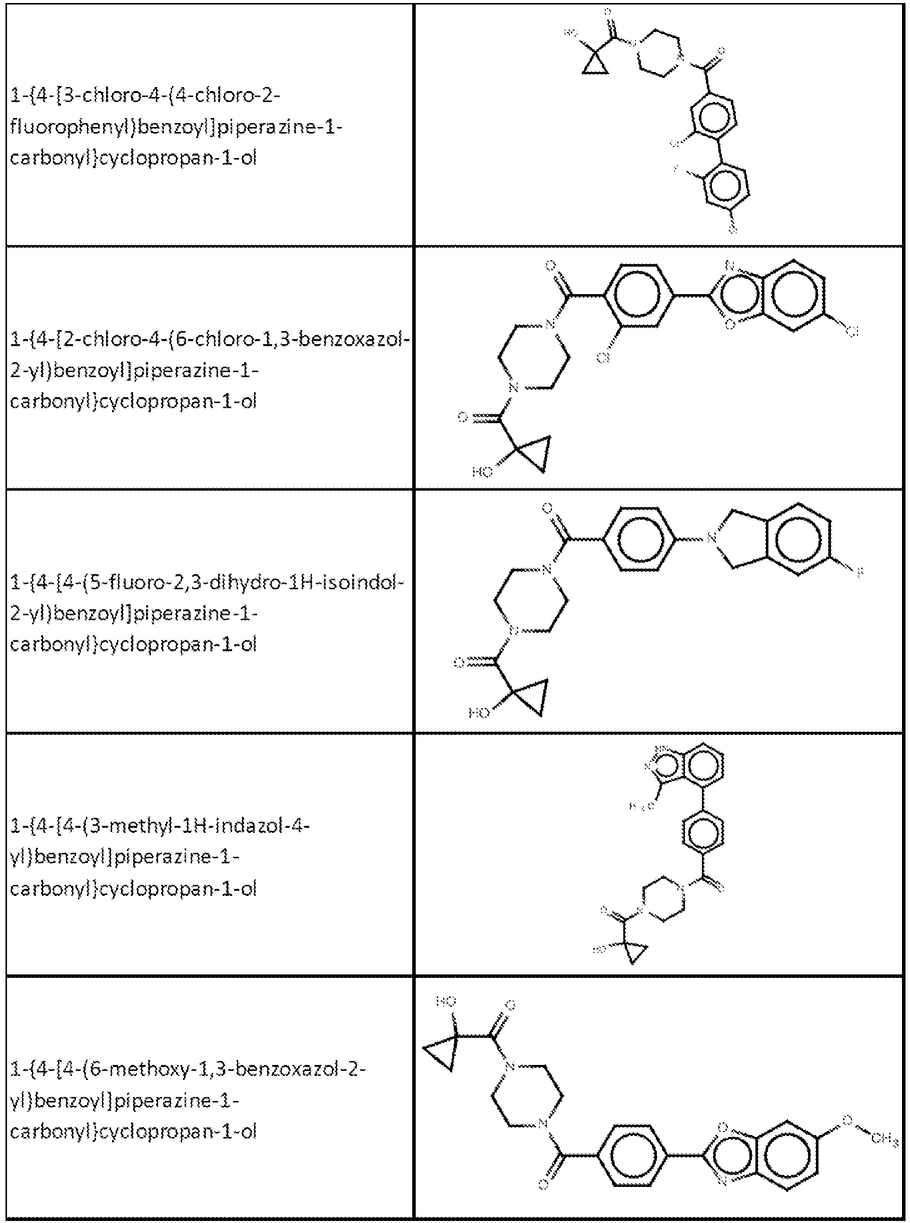

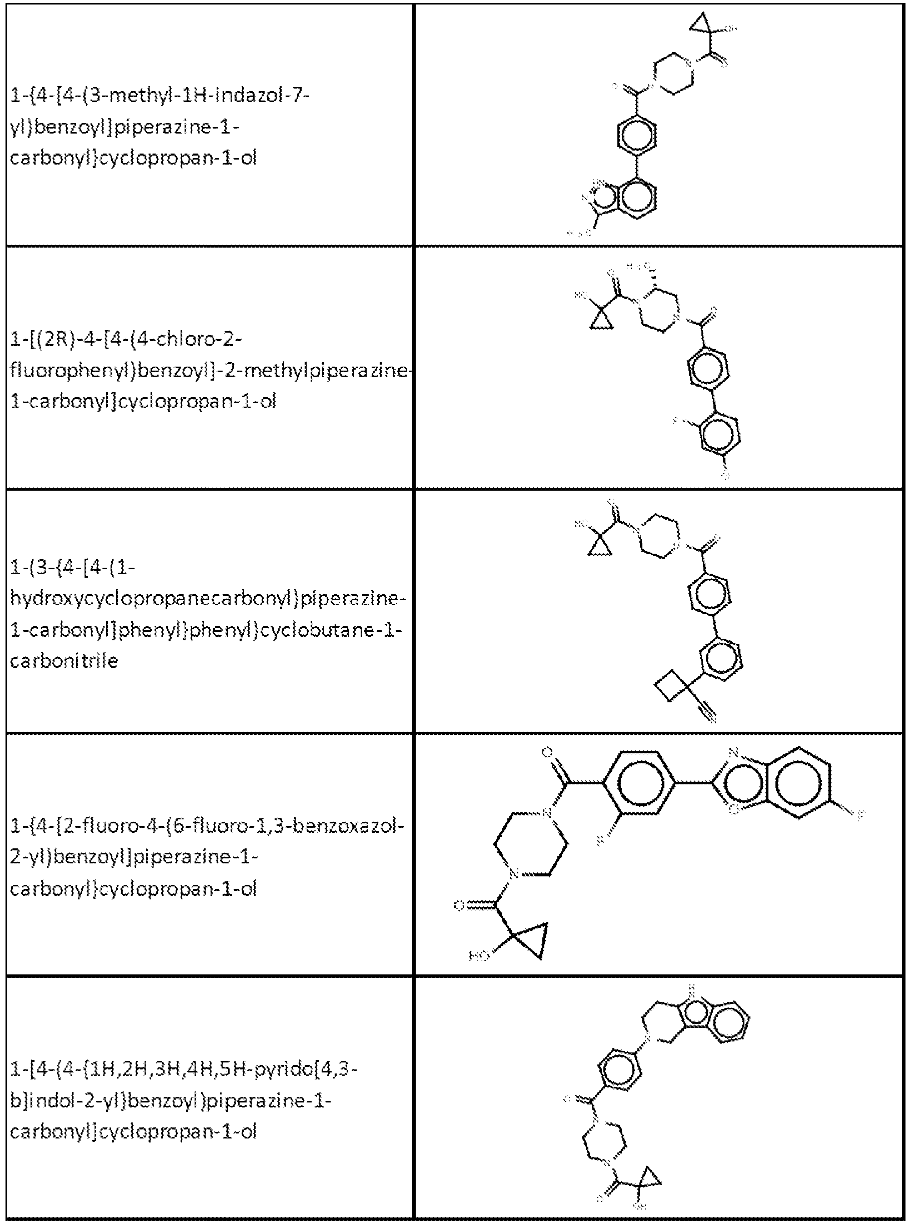

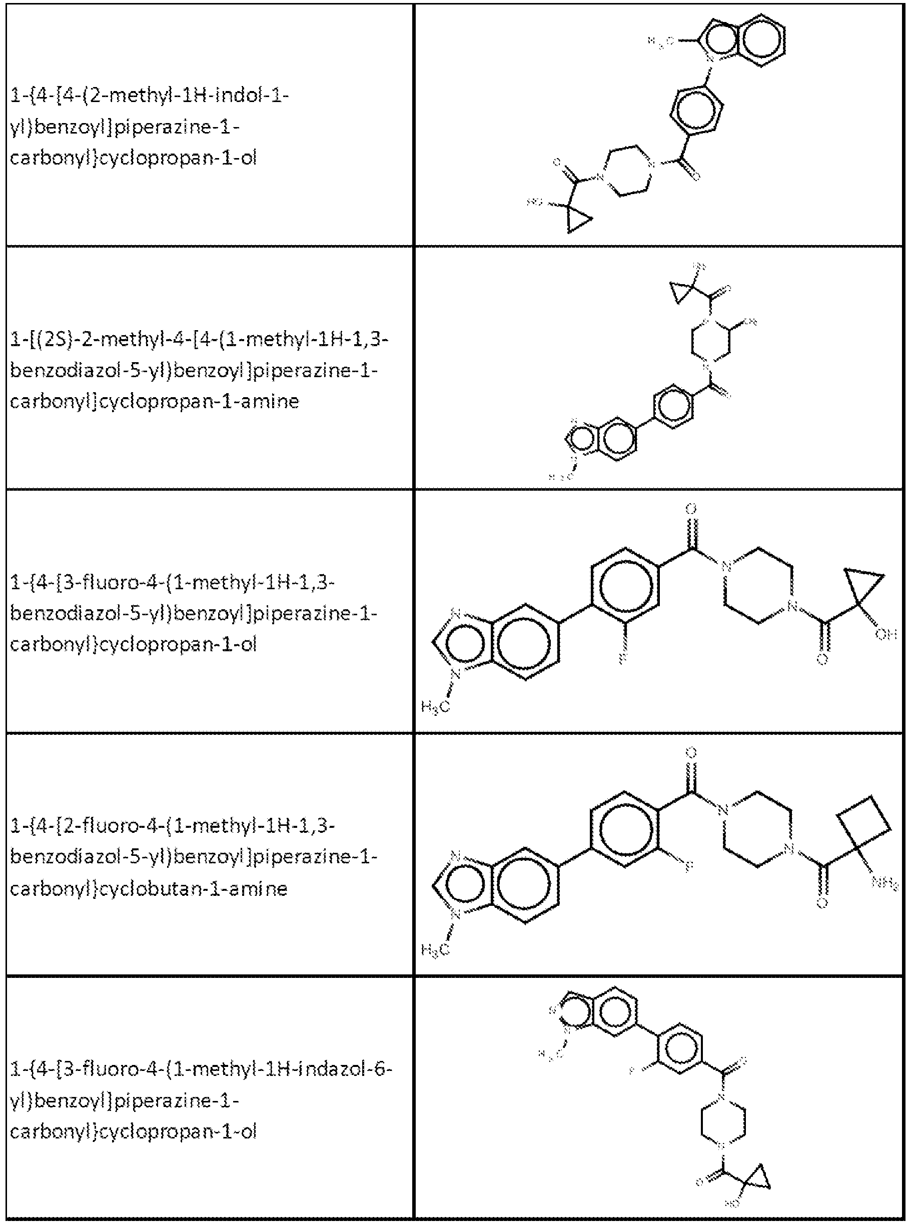

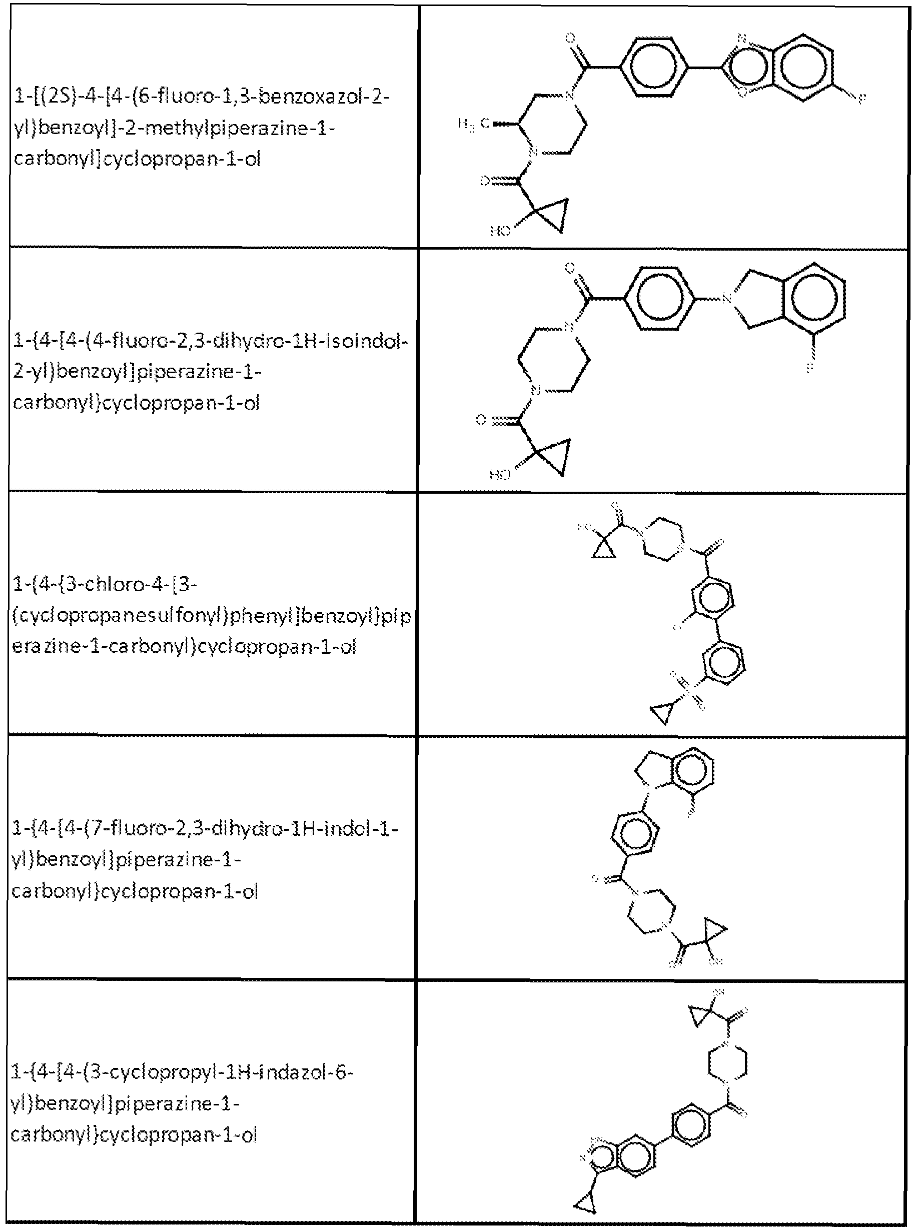

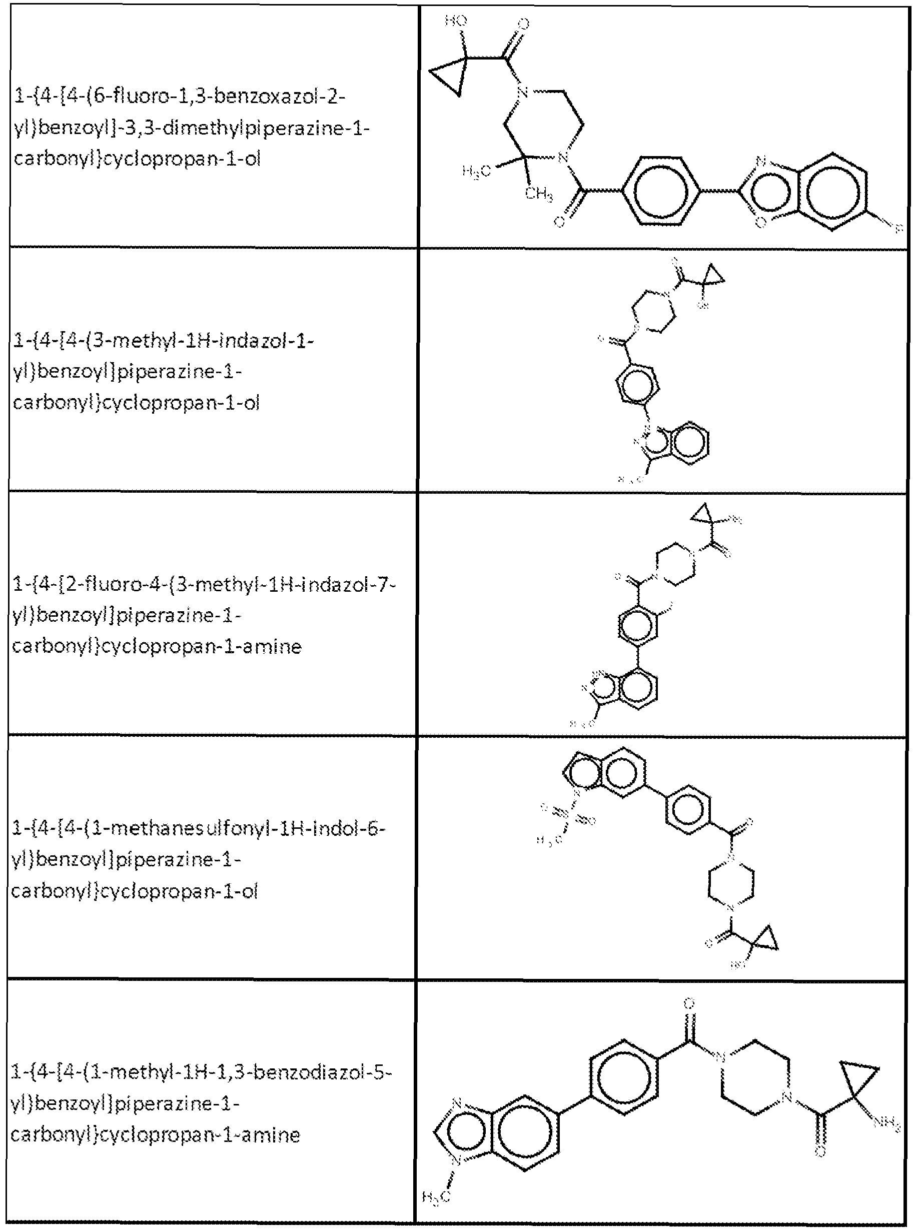

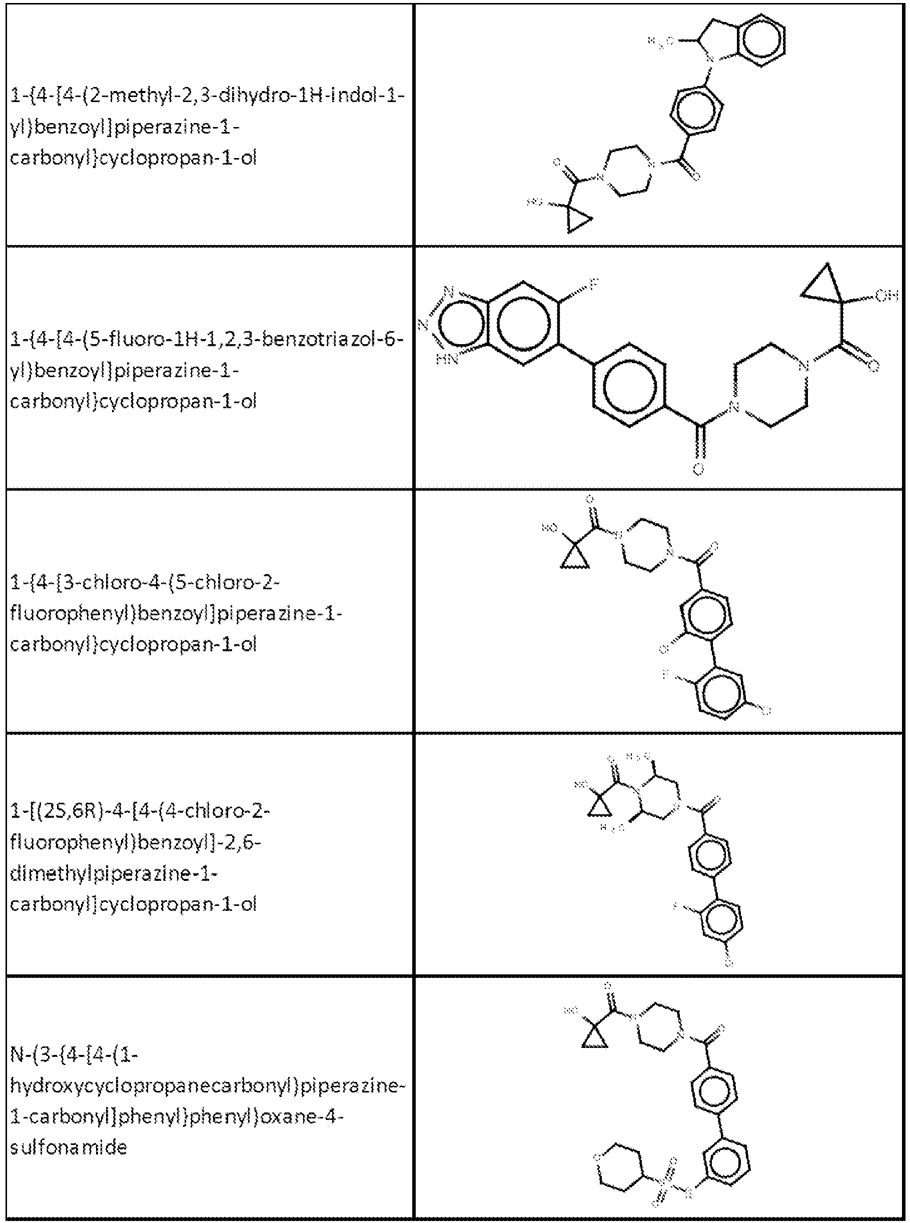

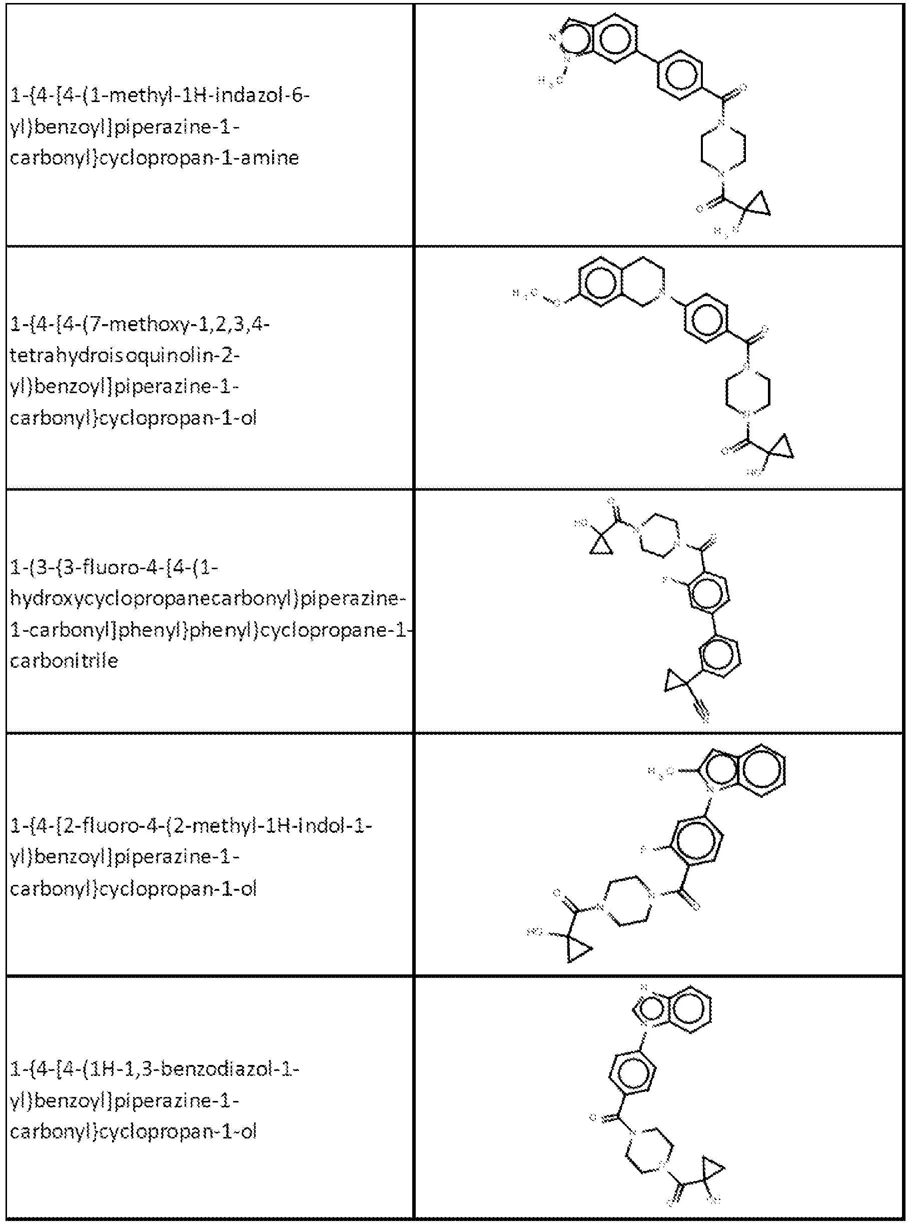

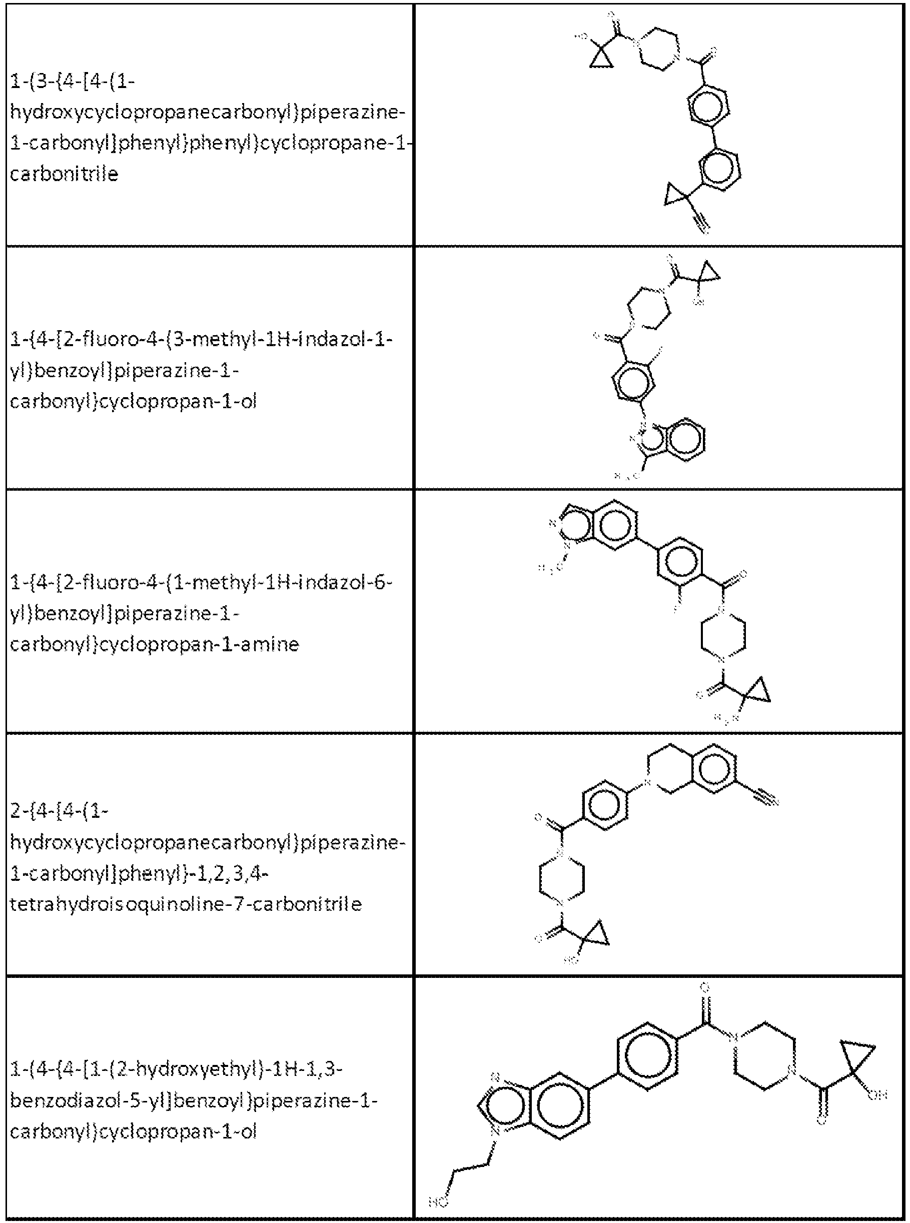

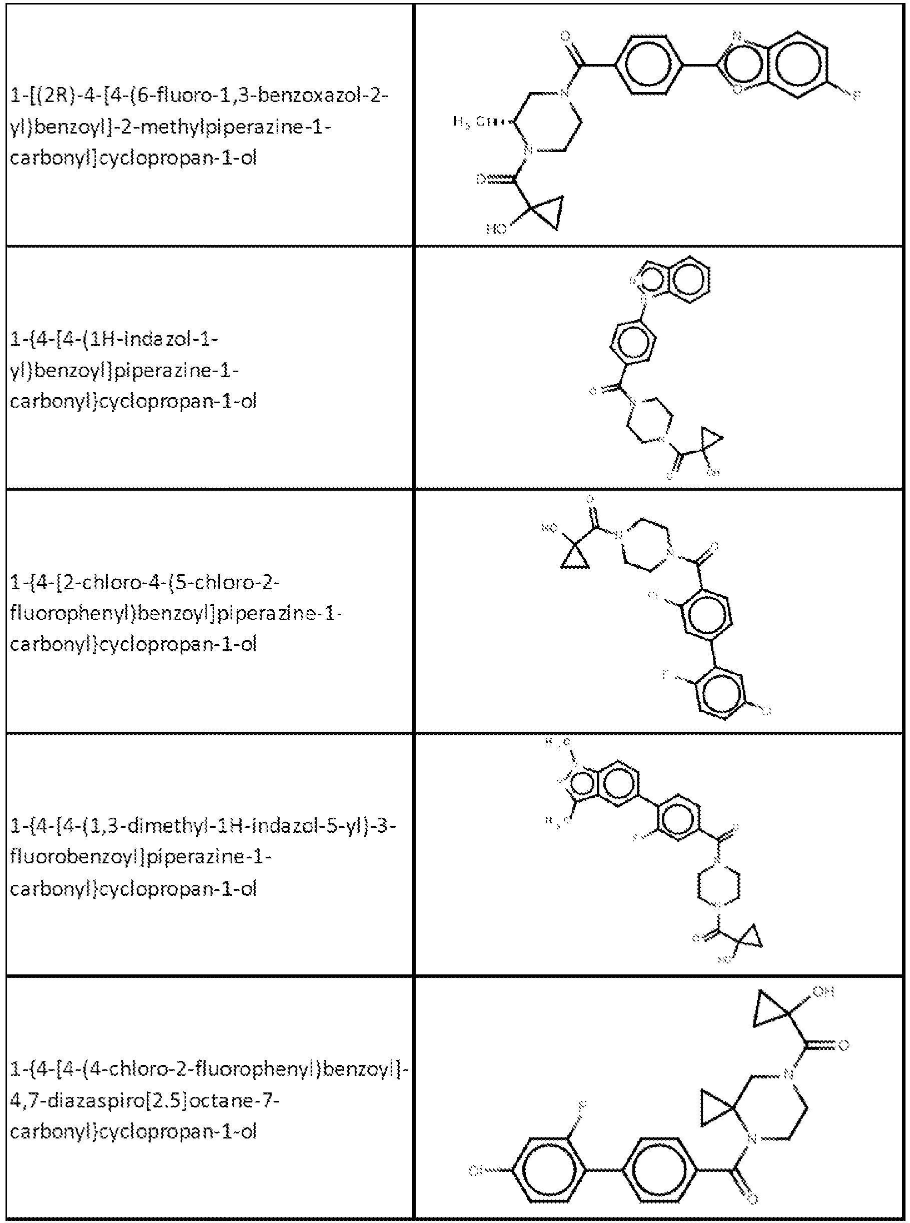

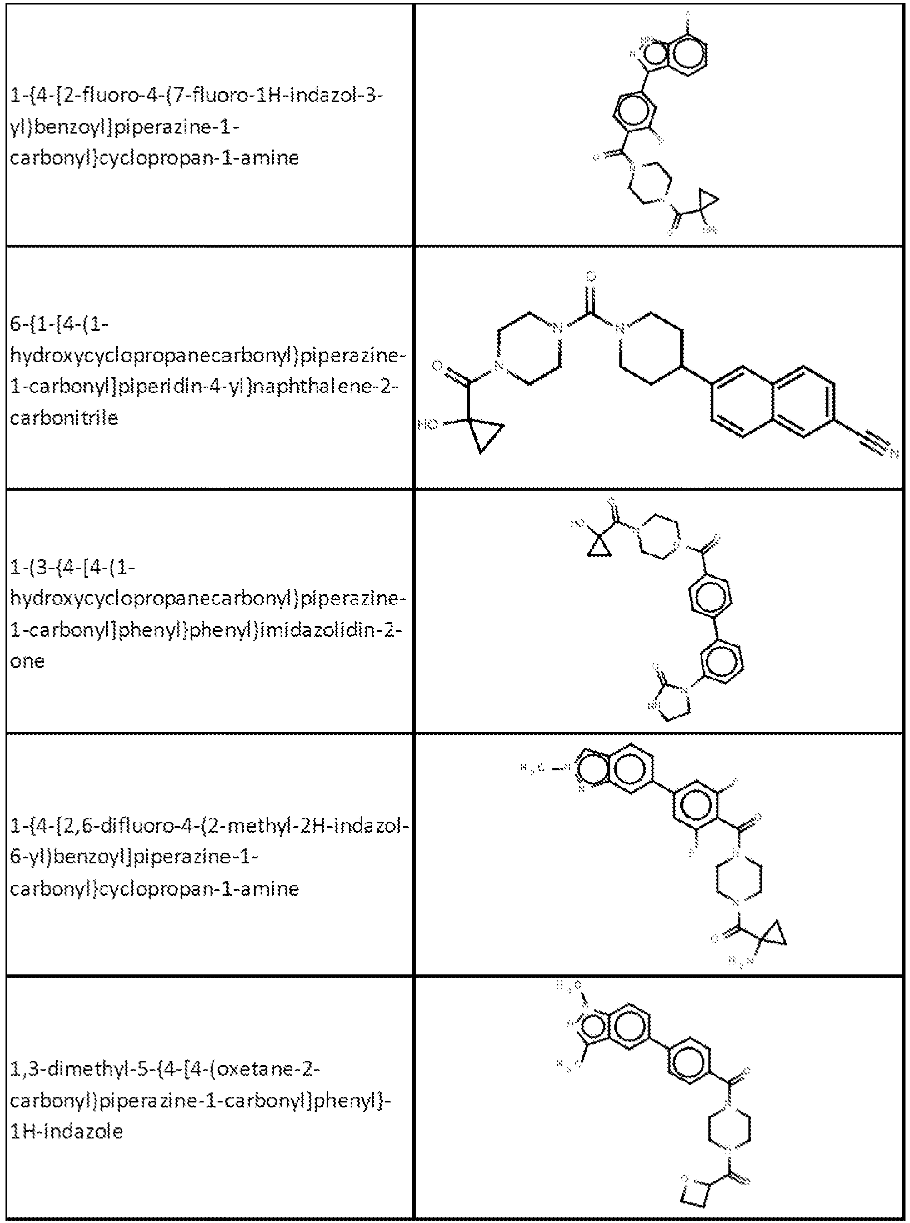

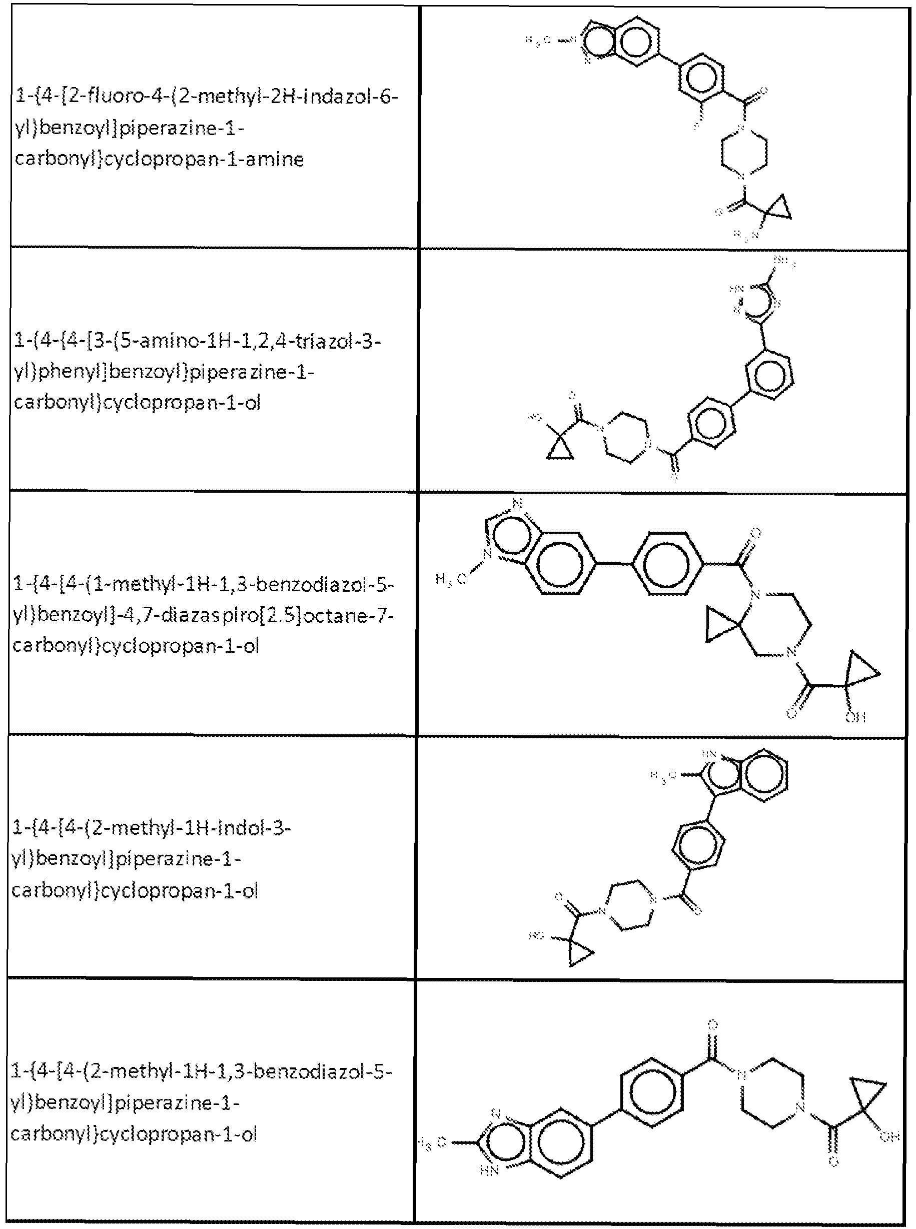

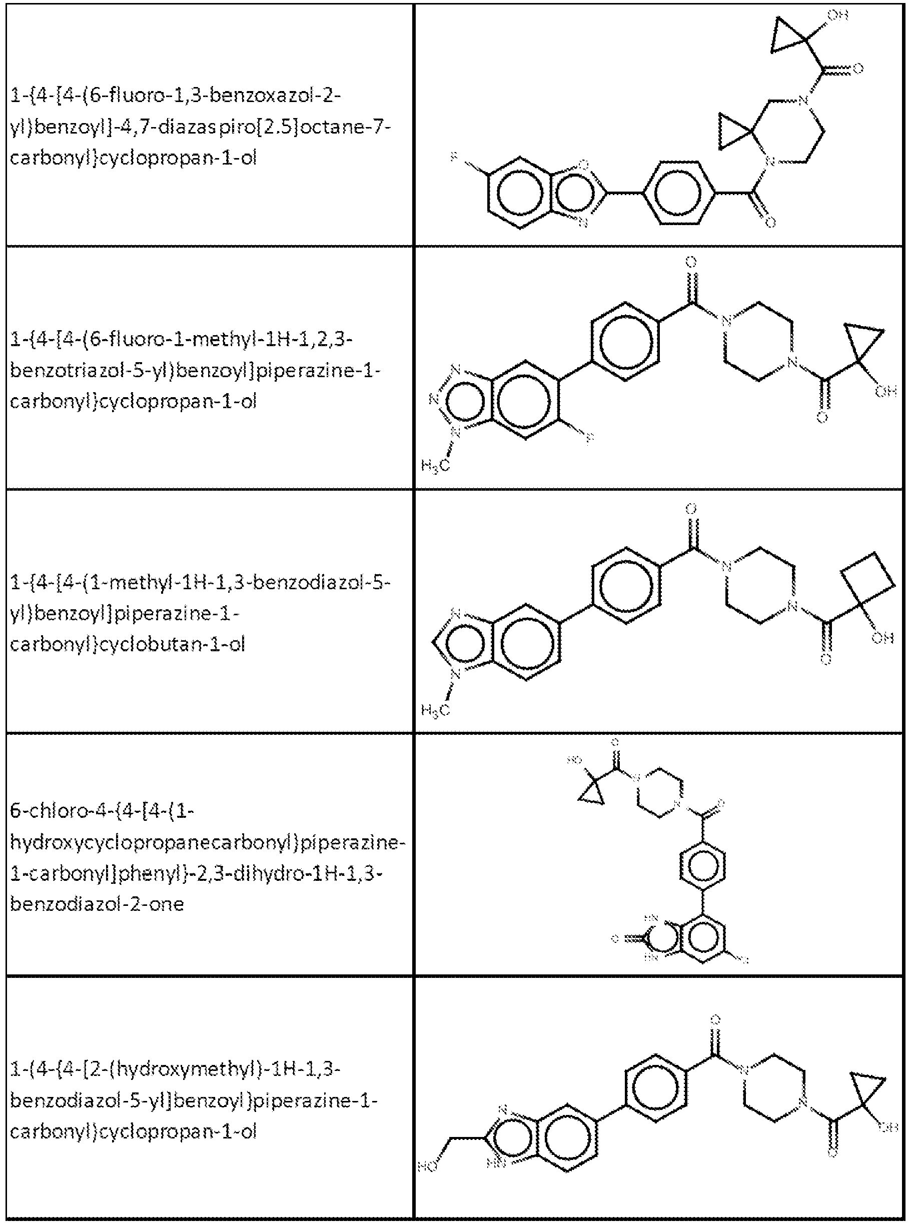

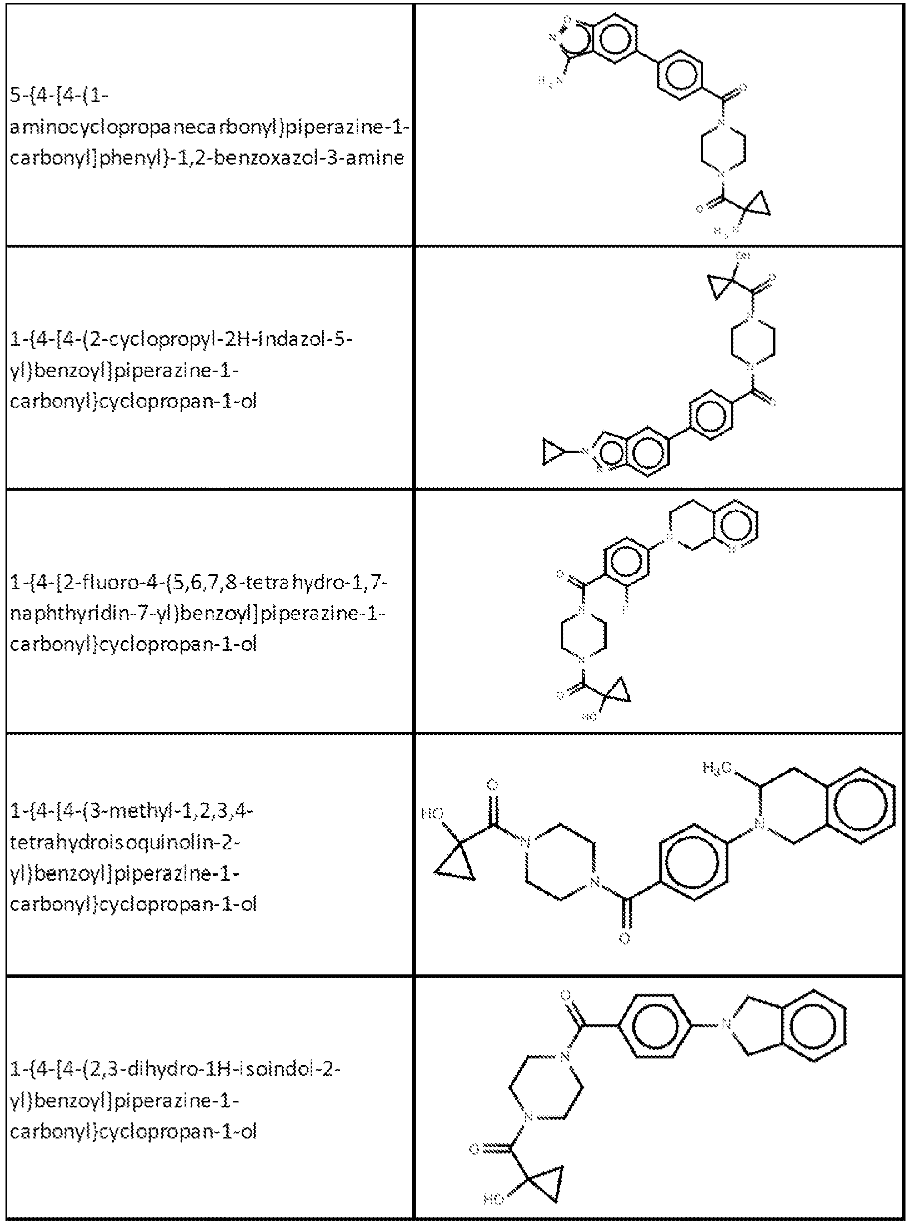

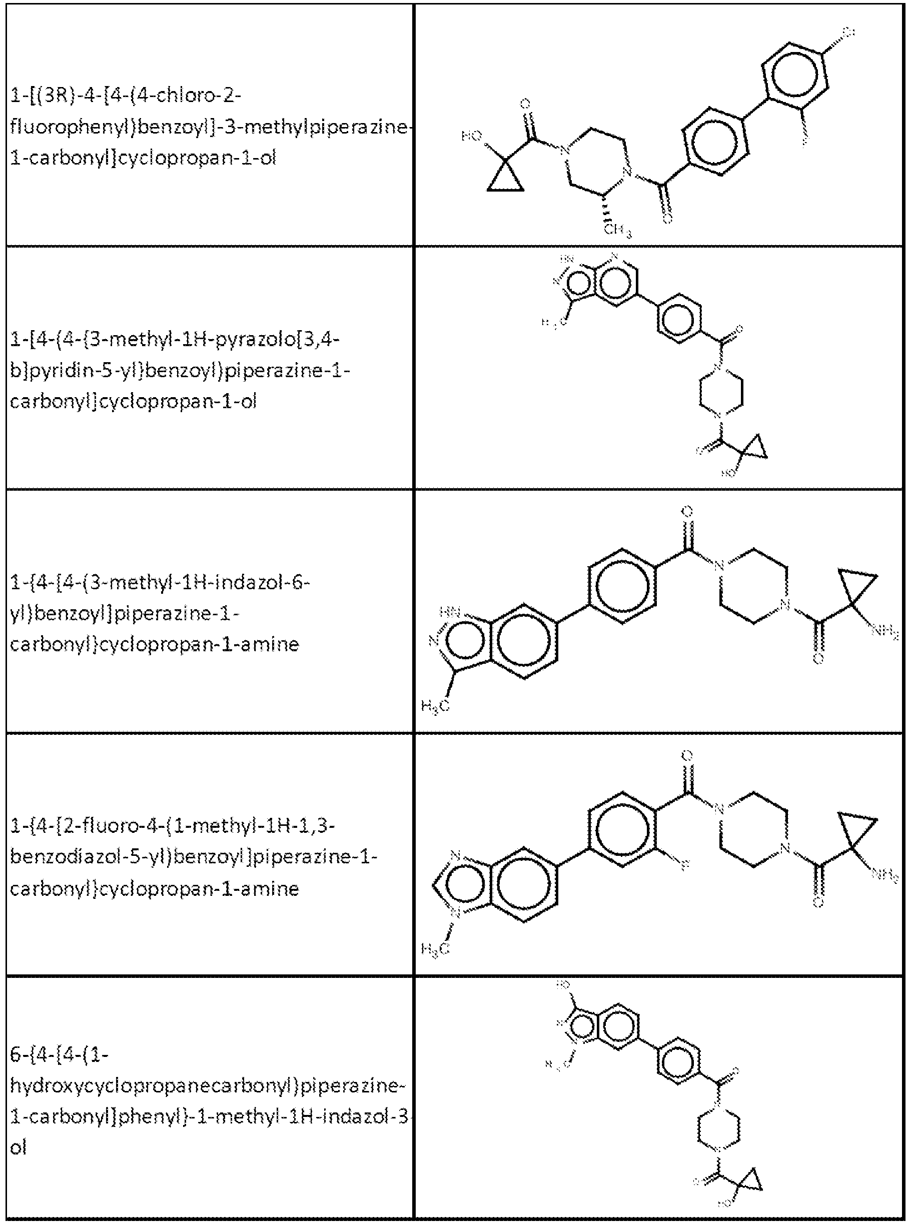

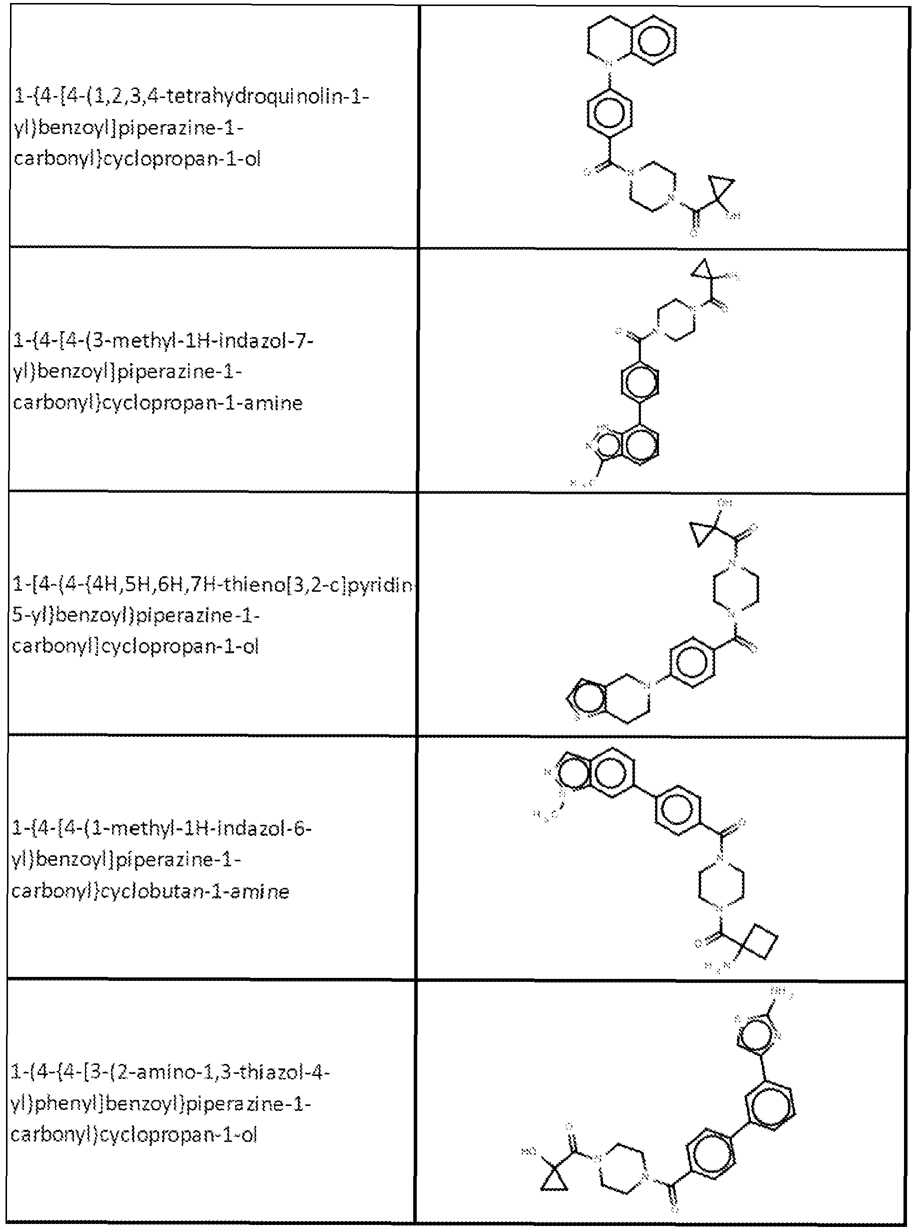

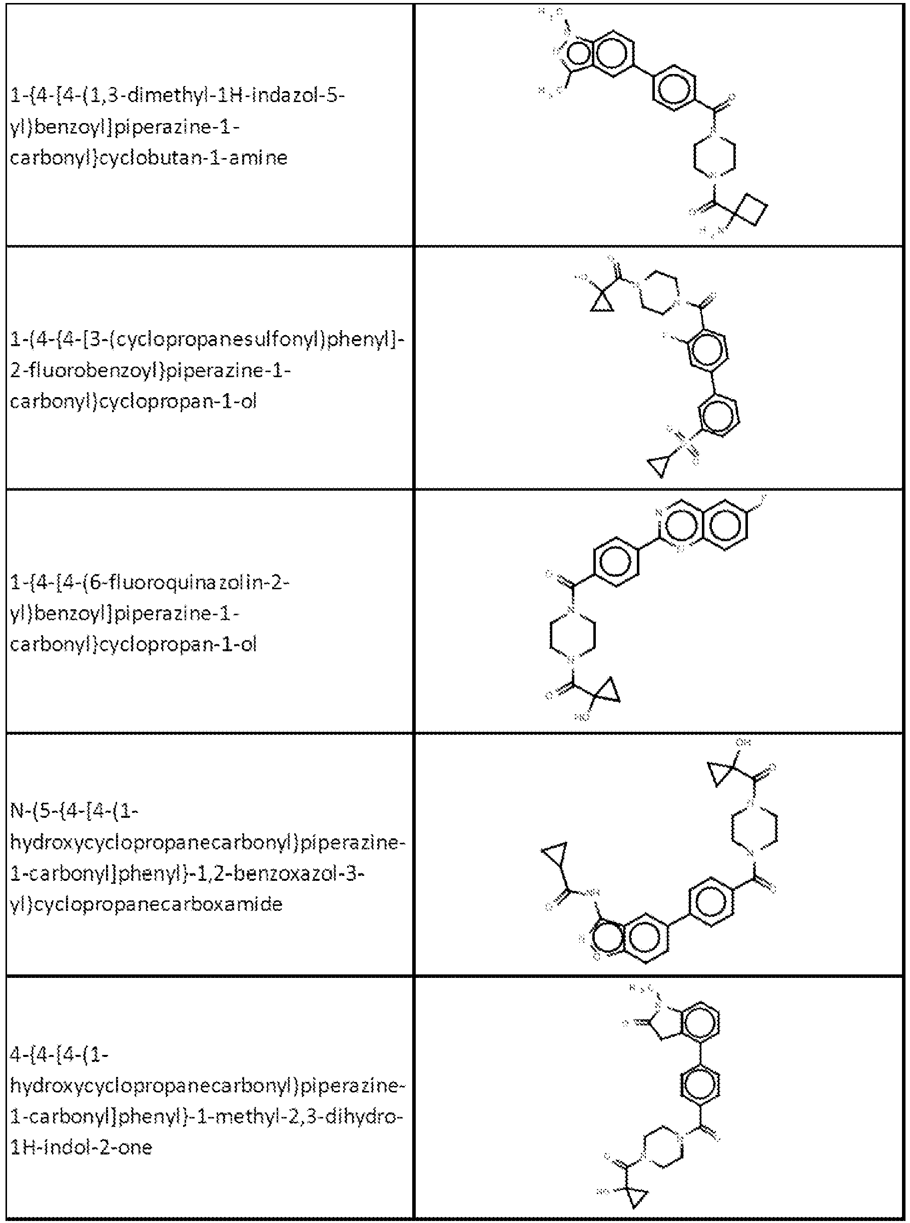

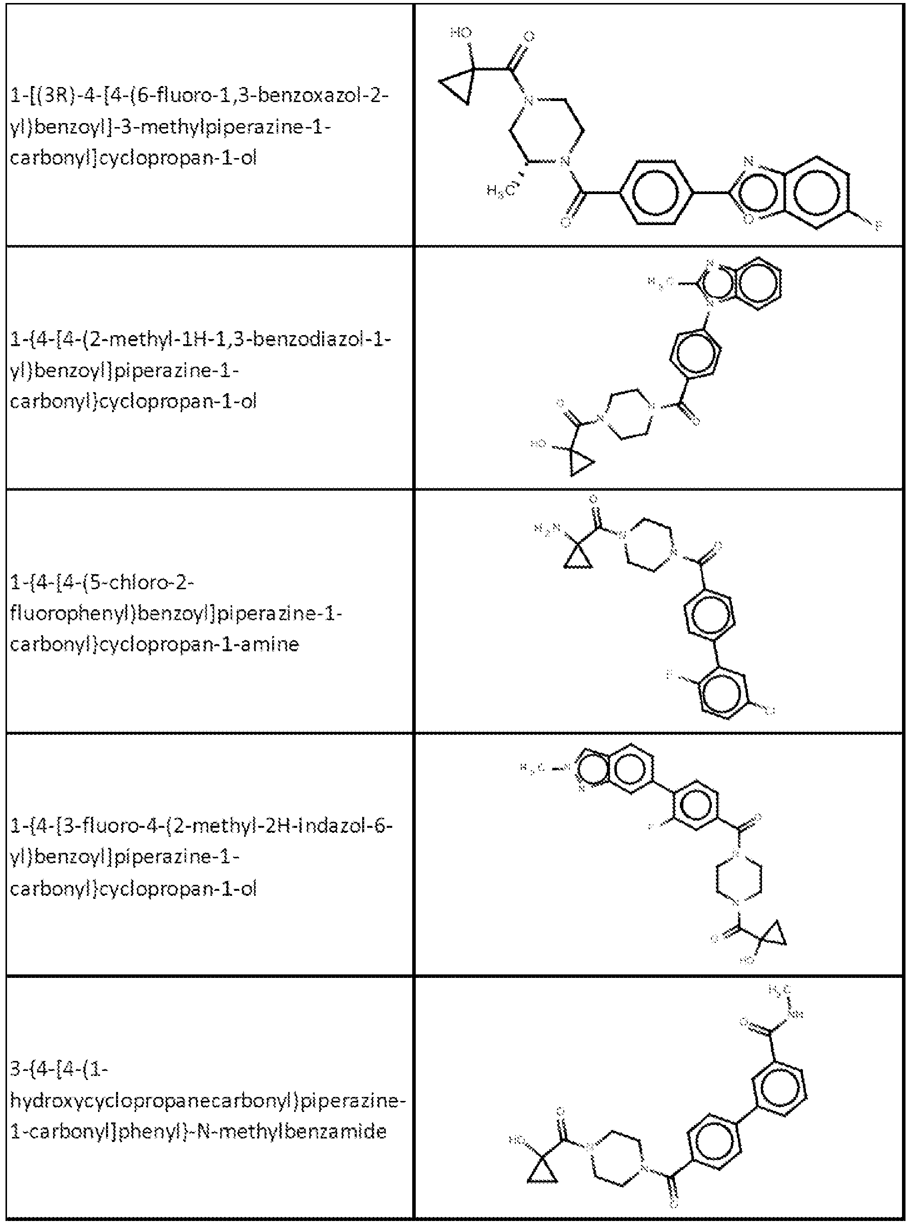

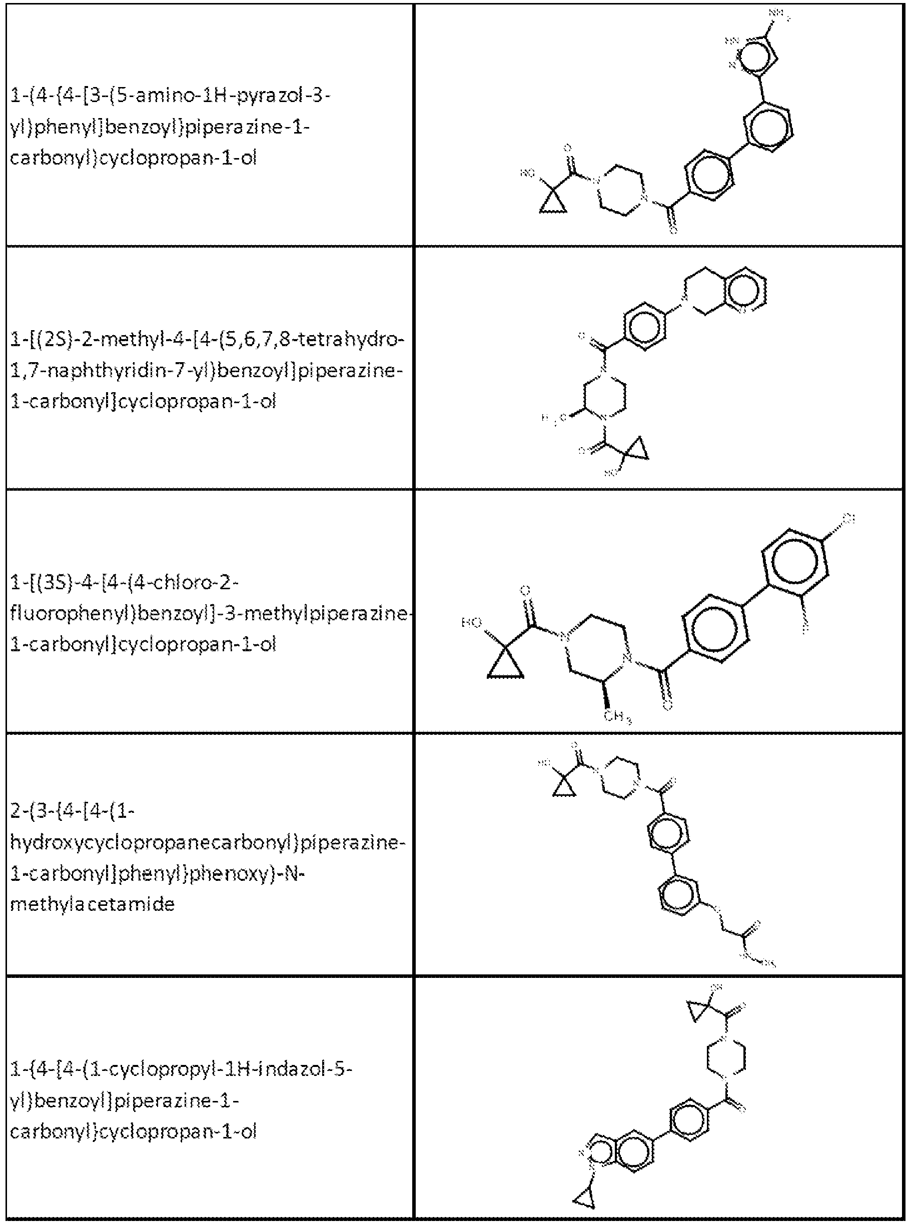

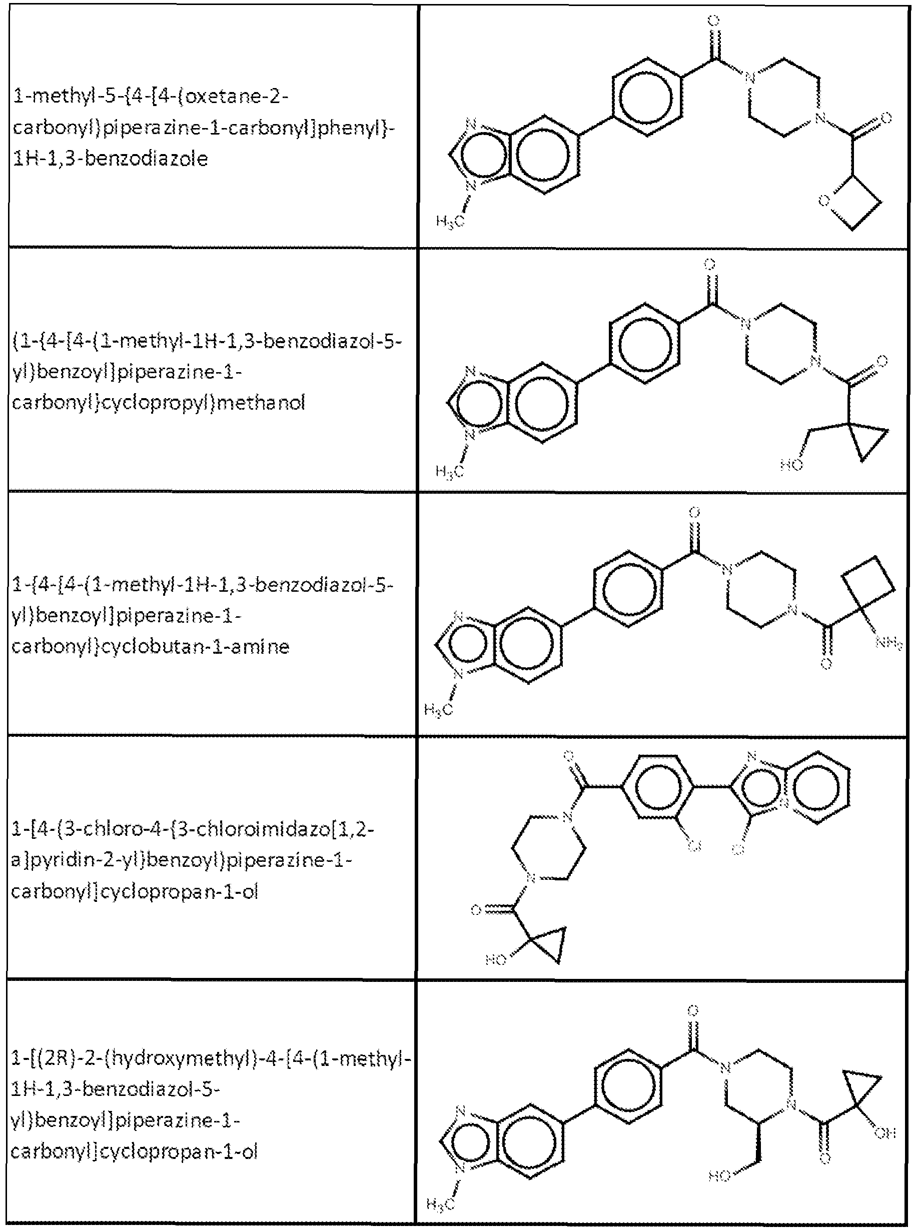

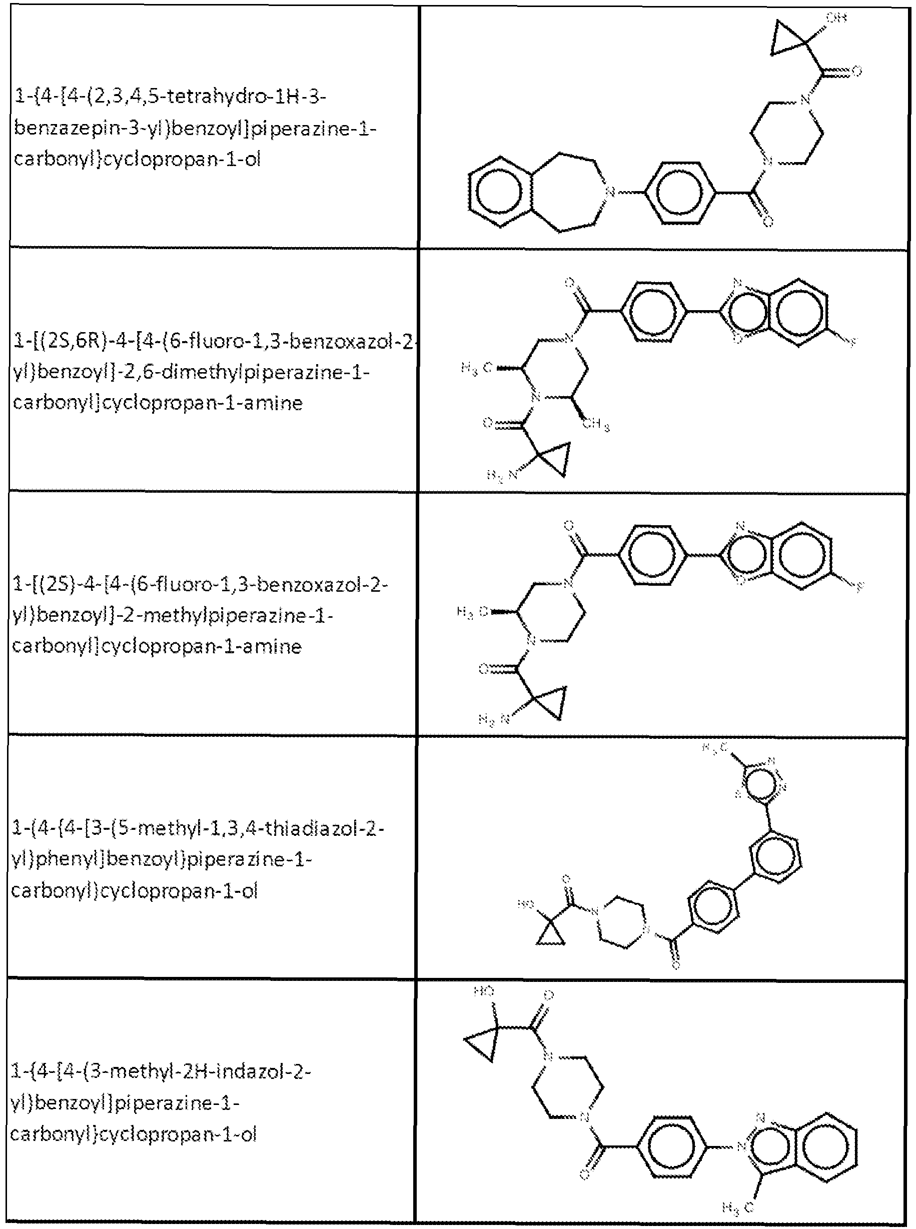

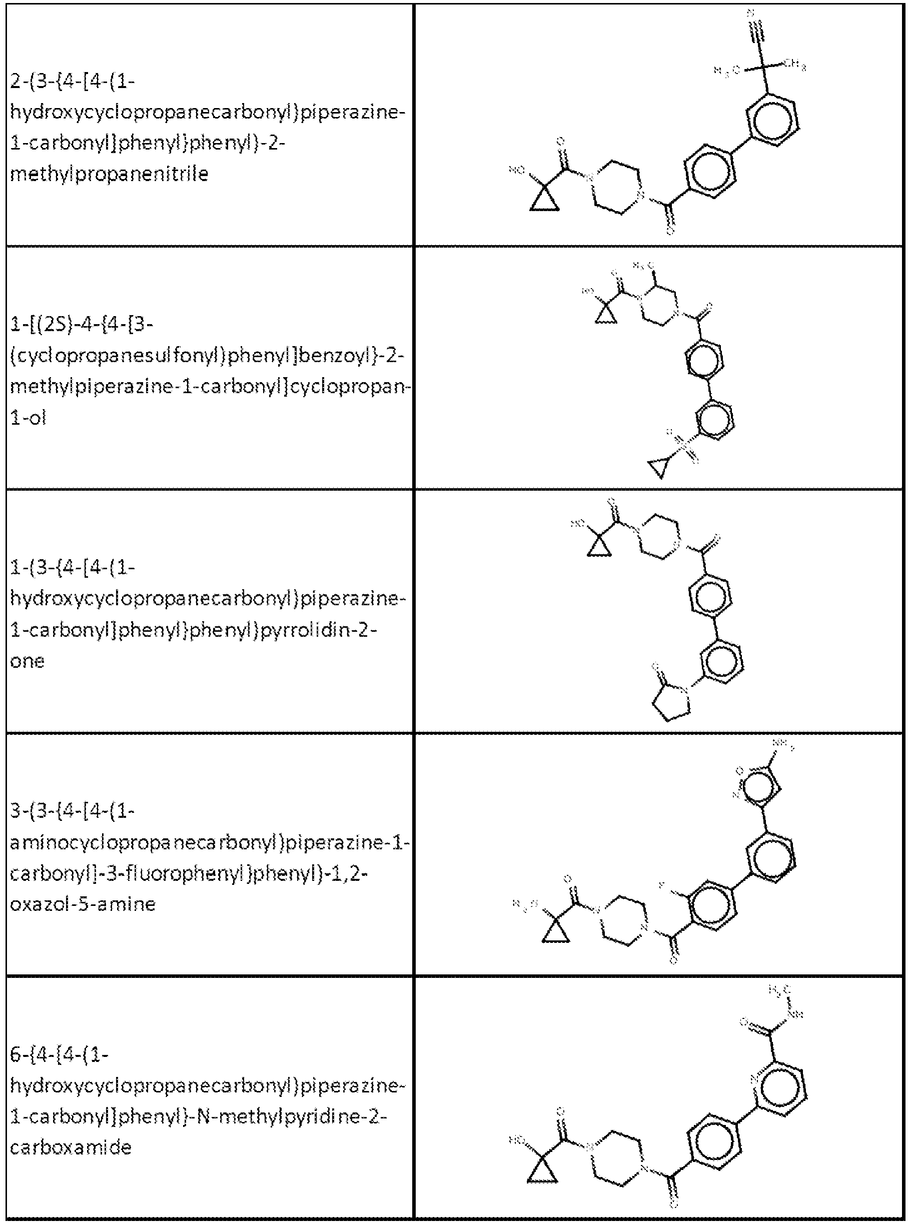

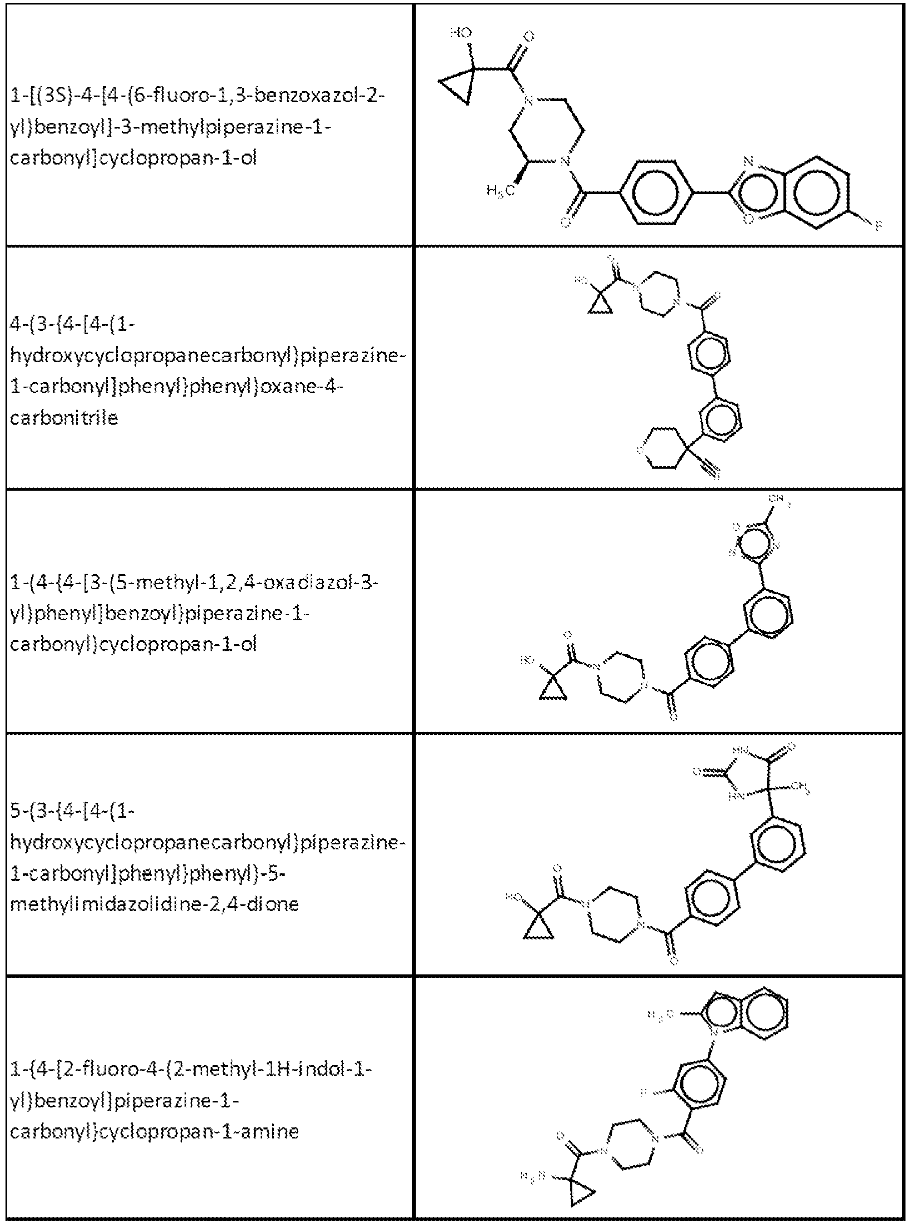

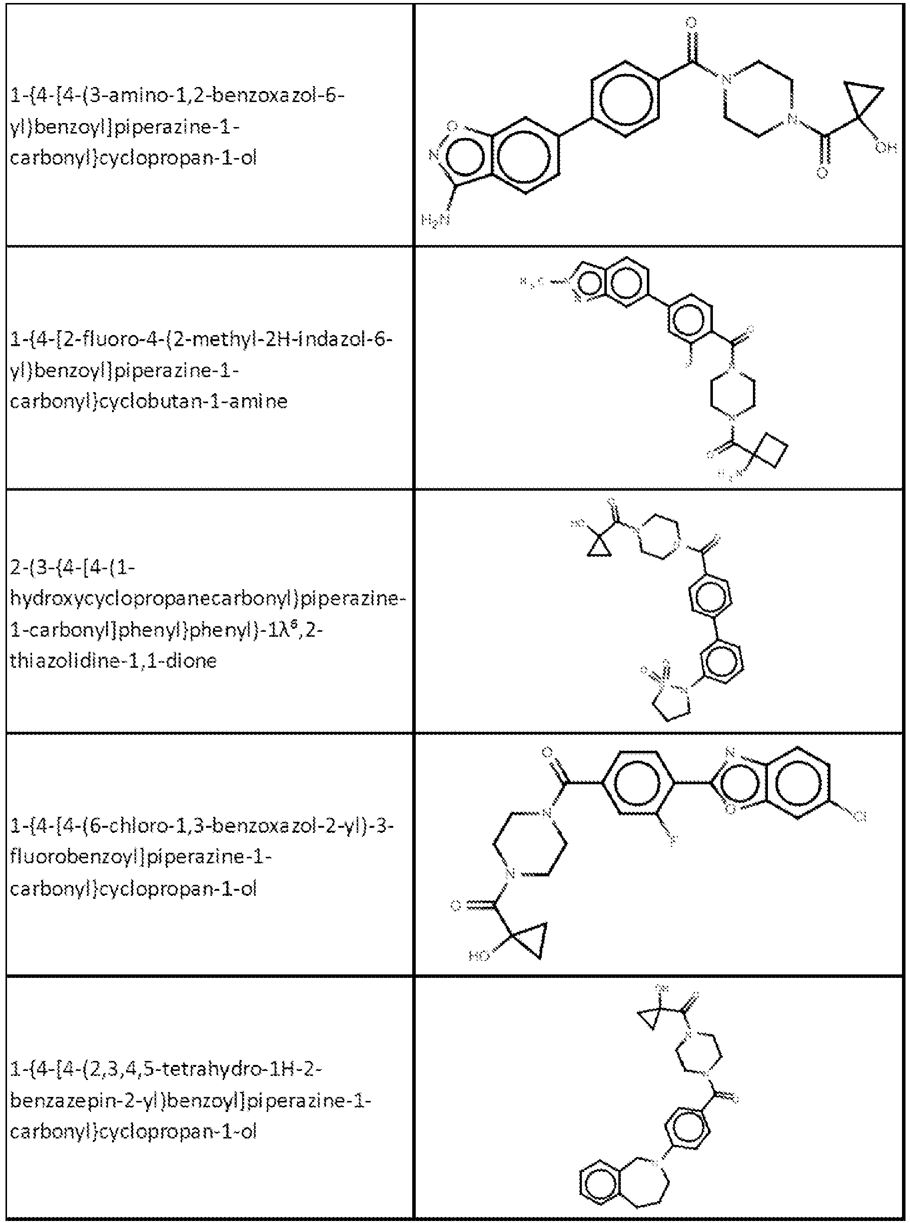

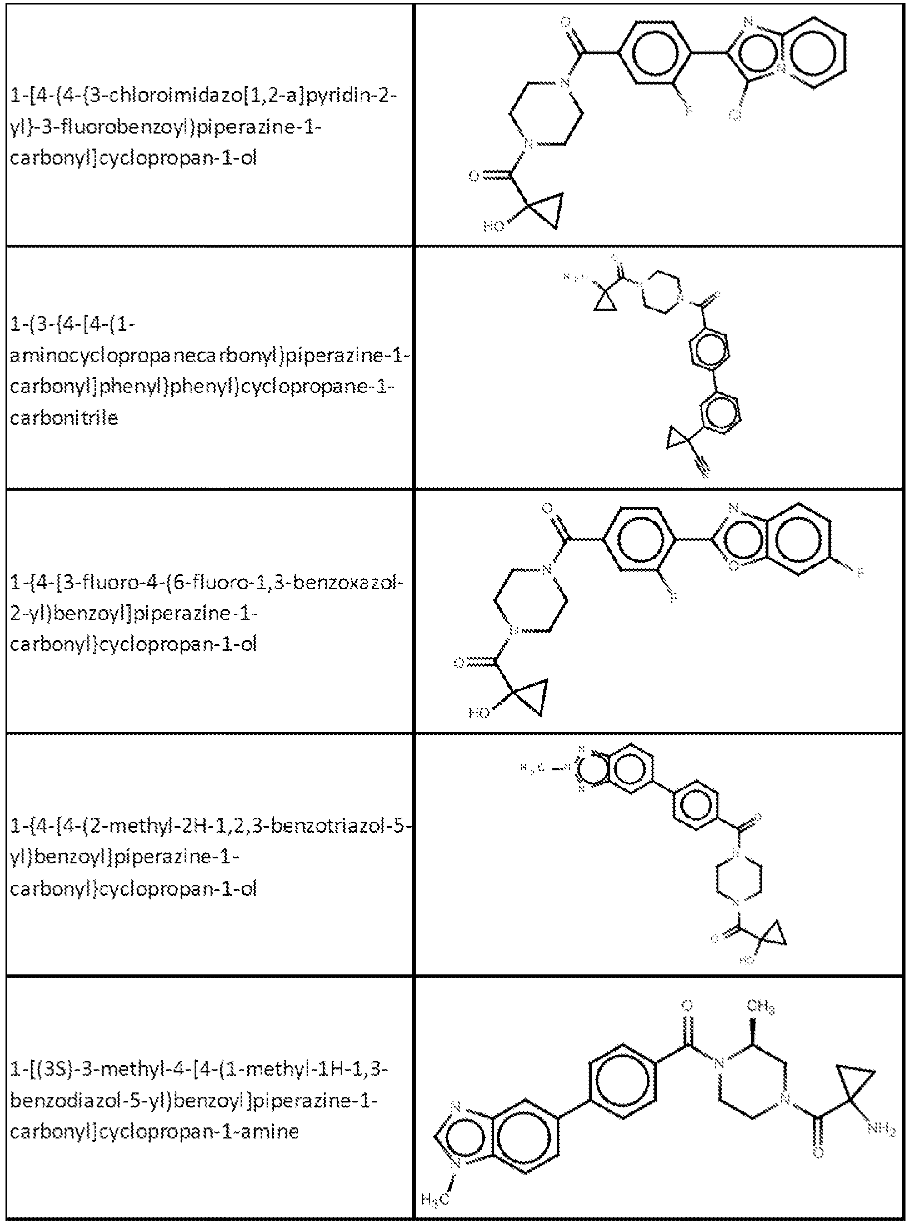

### Pharmaceutical Compositions

In another aspect, the present disclosure comprises treating conditions and diseases which may be associated IL-17 or T_{H}17, or as otherwise described herein, comprising administering a pharmaceutical composition comprising a provided compound optionally in combination with a pharmaceutically acceptable excipient (e.g. carrier).

Suitable pharmaceutical compositions include optical isomers, diastereomers, or pharmaceutically acceptable salts of the compounds disclosed herein. For example, in some embodiments, pharmaceutical compositions include a pharmaceutically acceptable salt. A compound included in the pharmaceutical composition may be covalently attached to a pharmaceutically acceptable carrier. The inventive compound included in the pharmaceutical composition is not covalently linked to a pharmaceutically acceptable carrier.

A "pharmaceutically acceptable carrier," as used herein refers to pharmaceutical excipients, for example, pharmaceutically, physiologically, acceptable organic, or inorganic carrier substances suitable for enteral or parenteral application which do not deleteriously react with the compounds used in accordance with the provided methods. Suitable pharmaceutically acceptable carriers include water, salt solutions (such as Ringer's solution), alcohols, oils, gelatins and carbohydrates such as lactose, amylose or starch, fatty acid esters, hydroxymethycellulose, and polyvinyl pyrrolidine. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances which do not deleteriously react with the compounds used in accordance with the provided methods.

Compounds can be administered alone or can be coadministered to a patient along with one or more other drugs. Coadministration is meant to include simultaneous or sequential administration of the compounds individually or in combination (more than one compound). In some embodiments, the preparations are combined with other active substances (e.g. to reduce metabolic degradation).

### Formulations

In another aspect, the present disclosure comprises treating conditions and diseases which may be associated IL-17 or T_{H}17, or as otherwise described herein, comprising administering of a formulation of a compound or composition described herein. Suitable formulations can be prepared and administered in a wide variety of oral, parenteral, and topical dosage forms. In some embodiments, provided compounds are administered by injection (e.g. intravenously, intramuscularly, intracutaneously, subcutaneously, intraduodenally, or intraperitoneally). In some embodiments, compounds described herein are administered by inhalation, for example, intranasally. In some embodiments, provided compounds are administered transdermally. It is also envisioned that multiple routes of administration (e.g., intramuscular, oral, transdermal) can be used to administer the compounds of the invention. The present invention also provides pharmaceutical compositions comprising one or more provided compounds and one or more pharmaceutically acceptable carriers or excipients.

Pharmaceutically acceptable carriers can be either solid or liquid. Solid form preparations include powders, tablets, pills, capsules, cachets, suppositories, and dispersible granules. In some embodiments, a solid carrier is one or more substances, which may also act as diluents, flavoring agents, binders, preservatives, tablet disintegrating agents, or an encapsulating material.

In some embodiments, when a composition is a powder, a carrier is a finely divided solid in a mixture with the finely divided active component. In some embodiments, when a composition is formulated for a tablet, an active component is mixed with the carrier having the necessary binding properties in suitable proportions and compacted in the shape and size desired.

In some embodiments, powders and tablets contain from 5% to 70% of the active compound. Suitable carriers include magnesium carbonate, magnesium stearate, talc, sugar, lactose, pectin, dextrin, starch, gelatin, tragacanth, methylcellulose, sodium carboxymethylcellulose, a low melting wax, and cocoa butter. In some embodiments, the composition is formulated for a cachet or lozenge. In some embodiments, tablets, powders, capsules, pills, cachets, and/or lozenges are used as solid dosage forms suitable for oral administration.

In some embodiments, for preparing suppositories, a low melting wax, such as a mixture of fatty acid glycerides or cocoa butter, is first melted and the active component is dispersed homogeneously therein. The molten homogeneous mixture is then poured into convenient sized molds, allowed to cool and solidify.

Liquid form preparations include solutions, suspensions, and emulsions, for example, water or water/propylene glycol solutions. In some embodiments, for parenteral injection, liquid preparations can be formulated in solution in aqueous polyethylene glycol solution.

When parenteral application is needed or desired, particularly suitable admixtures for the compounds of the invention are injectable, sterile solutions, preferably oily or aqueous solutions, as well as suspensions, emulsions, or implants, including suppositories. In particular, carriers for parenteral administration include aqueous solutions of dextrose, saline, pure water, ethanol, glycerol, propylene glycol, peanut oil, sesame oil, or polyoxyethylene-block polymers. Ampules are convenient unit dosages. Compounds useful in provided methods can also be incorporated into liposomes or administered via transdermal pumps or patches. Pharmaceutical admixtures suitable for use in the present invention include those described, for example, in Pharmaceutical Sciences (17th Ed., Mack Pub. Co., Easton, PA) and WO 96/05309.

Aqueous solutions suitable for oral use can be prepared by dissolving the active component in water and adding suitable colorants, flavors, stabilizers, and thickening agents as desired. Aqueous suspensions suitable for oral use can be made by dispersing the finely divided active component in water with viscous material, such as natural or synthetic gums, resins, methylcellulose, sodium carboxymethylcellulose, and other well-known suspending agents.

Also included are solid form preparations intended for conversion shortly before use to liquid form preparations for oral administration. Such liquid forms include solutions, suspensions, and emulsions. These preparations may contain, in addition to the active component, colorants, flavors, stabilizers, buffers, artificial and natural sweeteners, dispersants, thickeners, or solubilizing agents.

In some embodiments, pharmaceutical compositions are in unit dosage form. In such form the composition is subdivided into unit doses containing appropriate quantities of the active component. The unit dosage form can be a packaged preparation, the package containing discrete quantities of a pharmaceutical composition, such as packeted tablets, capsules, and powders in vials or ampoules. In some embodiments, the unit dosage form is a capsule, tablet, cachet, or lozenge itself, or it is the appropriate number of any of these in packaged form.

The quantity of active component in a unit dosage form may be varied or adjusted from 0.1 mg to 10000 mg, more typically 1.0 mg to 1000 mg, most typically 10 mg to 500 mg, according to the particular application and the potency of the active component. In some embodiments, provided compositions contain other compatible therapeutic agents.

Some compounds may have limited solubility in water and may require a surfactant or other appropriate co-solvent in the composition. Such co-solvents include: Polysorbate 20, 60 and 80, Pluronic F-68, F-84 and P-103, cyclodextrin, and polyoxyl 35 castor oil. Such co-solvents are typically employed at a level between about 0.01 % and about 2% by weight.

In some embodiments, viscosity greater than that of simple aqueous solutions may be desirable to decrease variability in dispensing the formulations, to decrease physical separation of components of a suspension or emulsion of formulation and/or otherwise to improve the formulation. Such viscosity building agents include, for example, polyvinyl alcohol, polyvinyl pyrrolidone, methyl cellulose, hydroxy propyl methylcellulose, hydroxyethyl cellulose, carboxymethyl cellulose, hydroxy propyl cellulose, chondroitin sulfate and salts thereof, hyaluronic acid and salts thereof, and combinations of the foregoing. Such agents are typically employed at a level between about 0.01% and about 2% by weight.

Compositions used in provided methods may additionally include components to provide sustained release and/or comfort. Such components include high molecular weight, anionic mucomimetic polymers, gelling polysaccharides and finely-divided drug carrier substrates. These components are discussed in greater detail in U.S. Pat. Nos. 4,911,920; 5,403,841; 5,212,162; and 4,861,760.

### Combinations

In some embodiments, a provided method further comprises administration of an additional therapeutic agent. In some aspects , the present disclosure comprises treating conditions and diseases which may be associated IL-17 or T_{H}17, or as otherwise described herein, comprising administering a FASN inhibitor in combination with additional therapeutic agents. In one embodiment, the additional therapeutic agent is a BRAF inhibitor. In another embodiment, the additional therapeutic agent is a PD1/PDL1 inhibitor.

In some embodiments, a BRAF inhibitor is GDC-0879, SB590885; Encorafenib (LGX818); RAF265 (CHIR-265); Dabrafenib (GSK2118436); TAK-632; PLX-4720; CEP-32496; Sorafenib Tosylate; Sorafenib; Vemurafenib (PLX4032, RG7204); AZ 628; or vemurafenib.

In some embodiments, a PD1/PDL1 inhibitor is selected from the group consisting of PD1 inhibitors Pembrolizumab, and Nivolumab; and PDL1 inhibitors Atezolizumab, MEDI4736, Avelumab, and PDR001.

The disclosure thus provides, in a further aspect, a combination comprising a compound described herein or a pharmaceutically acceptable salt thereof together with at least one additional therapeutic agent. In an exemplary aspect, the additional therapeutic agent is a compound of the disclosure.

When a compound described herein is used in combination with a second therapeutic agent active against the same disease state, the dose of each compound may differ from that when the compound is used alone. Appropriate doses will be readily appreciated by those skilled in the art. It will be appreciated that the amount of a compound required for use in treatment will vary with the nature of the condition being treated and the age and the condition of the patient and will be ultimately at the discretion of the attendant physician.

### Effective Dosage

Pharmaceutical compositions of the present disclosure include compositions wherein the active ingredient is contained in a therapeutically effective amount, i.e., in an amount effective to achieve its intended purpose. The actual amount effective for a particular application will depend, *inter alia,* on the condition being treated.

The dosage and frequency (single or multiple doses) of compound administered can vary depending upon a variety of factors, including route of administration; size, age, sex, health, body weight, body mass index, and diet of the recipient; nature and extent of symptoms of the disease being treated; presence of other diseases or other health-related problems; kind of concurrent treatment; and complications from any disease or treatment regimen. Other therapeutic regimens or agents can be used in conjunction with the methods and compounds of the invention.

For any compound described herein, the therapeutically effective amount can be initially determined from cell culture assays. Target concentrations will be those concentrations of active compound(s) that are capable of killing parasites and/or controlling their growth or reproduction as measured, for example, using the methods described.

Therapeutically effective amounts for use in humans may be determined from animal models. For example, a dose for humans can be formulated to achieve a concentration that has been found to be effective in animals. The dosage in humans can be adjusted by monitoring kinase inhibition and adjusting the dosage upwards or downwards, as described above. Therapeutically effective amounts for use in animals (e.g., cattle) may be determined from animal models (e.g., mouse models).

Dosages may be varied depending upon the requirements of the patient and the compound being employed. The dose administered to a patient, in the context of the present invention, should be sufficient to effect a beneficial therapeutic response in the patient over time. The size of the dose also will be determined by the existence, nature, and extent of any adverse side effects. Generally, treatment is initiated with smaller dosages, which are less than the optimum dose of the compound. Thereafter, the dosage is increased by small increments until the optimum effect under circumstances is reached. In some embodiments, the dosage range is 0.001% to 10% w/v. In some embodiments, the dosage range is 0.1% to 5% w/v.

In some embodiments, a dose administered to a human patient ranges from about 0.3 mg/kg to about 9 mg/kg. In some embodiments, a dose administered to a human patient is about 0.3, about 1, about 1.5, about 2, about 3, about 4, about 5, about 6, about 7, about 8, or about 9 mg/kg.

In some embodiments, a dose administered to a human patient is a dosage corresponding to a dose in a mouse from about 1 mg/kg to about 20 mg/kg. In some embodiments, a dose administered to a human patient is a dosage corresponding to a dose in a mouse of about 1, about 2, about 3, about 6, about 10, or about 20 mg/kg. In some embodiments, a dose administered to a human patient is a dosage corresponding to a dose in a rat from about 0.125 mg/kg to about 12 mg/kg. In some embodiments, a dose administered to a human patient is a dosage corresponding to a dose in a rat of about 0.125, about 0.2, about 0.4, about 0.6, about 1, about 1.2, about 2, about 2.4, about 6, or about 12 mg/kg.

Dosage amounts and intervals can be adjusted individually to provide levels of the administered compound effective for the particular clinical indication being treated. This will provide a therapeutic regimen that is commensurate with the severity of the individual's disease state.

In some embodiments, a dose is administered once daily, twice daily, or once every two days.

The disclosure is further illustrated by the following examples, which are not to be construed as limiting this disclosure tο the specific procedures herein described. It is to be understood that the examples are provided to illustrate certain embodiments and that no limitation to the scope of the disclosure is intended thereby.

### Examples

The following are illustrative, but non-limiting, examples of certain embodiments of the present invention. The process and results for the assays testing inhibition of IL-17 secretion in T_{H}17 cells differentiated *in-vitro* and evaluate the effect of FASN inhibitors on CSF-1-mediated M2 macrophage differentiation are described.

### Example 1. T_{H}17 Differentiation Assay:

CD4+ T cells were isolated using CD4+ T cell isolation kit (Miltenyi Biotech), activated with CD3/CD28 microbeads and cultured for 7-10 days in the presence of IL-1β, IL-6, IL-21, IL-23 and TGFβ and test compounds. Cells cultured with DMSO or without cytokines and/or microbeads were used as negative controls.

Read out: (1) At day 7-10, the supernatants were collected and tested for soluble IL-17. (2) Cells were also harvested and stained for intra-cellular IL-17A. (3) Cells were also stained separately by Annexin/PI for cell viability. (4) Pictures of cultures were taken for each condition.

**Table 1.1: Inhibition of IL-17 secretion in T_{H}17 cells differentiated in-vitro**

| **Cmpd #** | **Structure** | **IL17 secretion (% of DMSO)** | **% of inhibition at 1 µM** |
|---|---|---|---|
| 1* | 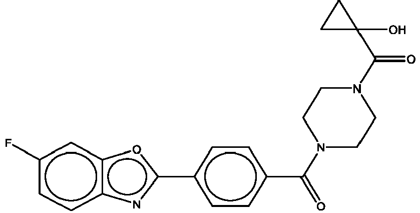 | 54 | 46 |
| 2* | 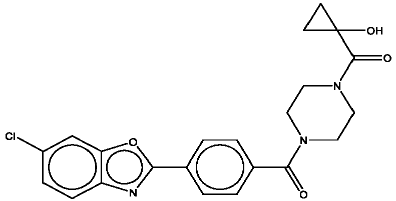 | 21 | 79 |
| 3* | 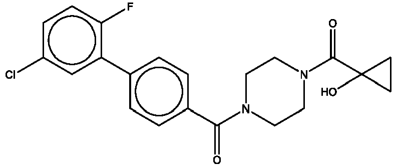 | 25 | 75 |
| 4 | 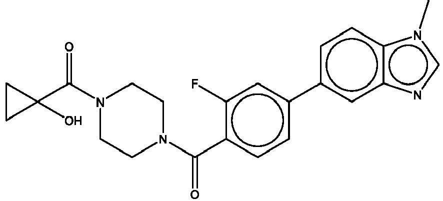 | 15 | 85 |
| 5* | 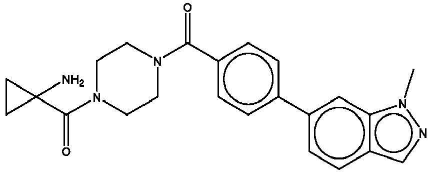 | 26 | 74 |
| 6* | 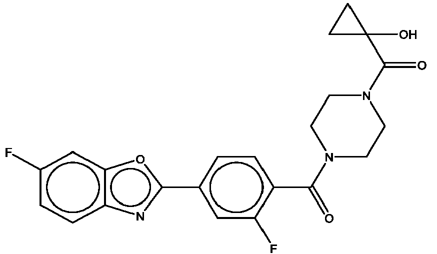 | 47 | 53 |
| 7* | 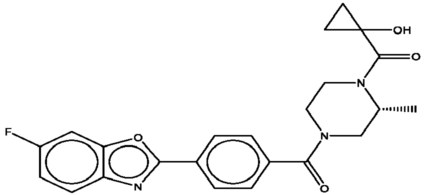 | 34 | 66 |
| 8* | 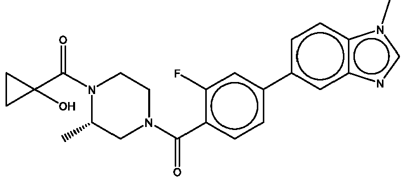 | 23 | 77 |
| 9* | 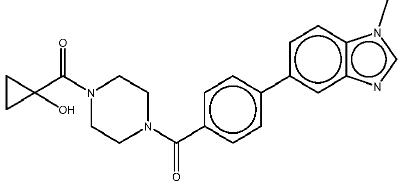 | 24 | 76 |
| 10* | 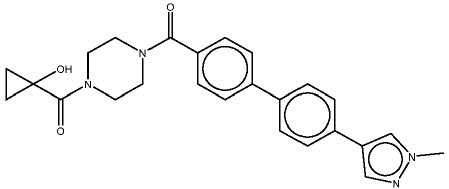 | 28 | 72 |
| 11* | 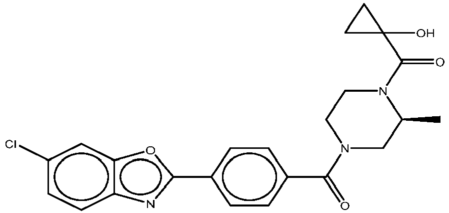 | 33 | 67 |

| | | | |
|---|---|---|---|
| *Reference compound | | | |

### Example 2. Protocol to Evaluate the Effect of FASN Inhibitors on CSF-1-Mediated M2 Macrophage Differentiation

Primary human monocytes are isolated from PBMCs by using Monocyte isolation kit (Miltenyi Biotec Inc.). Monocytes are plated in the 6-well plate at the 1.5 X 10⁶ cells/well in presence of hMCSF (50 ng/mL) in cell culture medium. The cells are treated with various concentrations of FASN inhibitor or Cerulenin (0.001 µM, 0.01 µM, 0.1 µM, 1 µM, or 10 µM) at the time of plating. The cells with 0.1% DMSO act as control. The cells are maintained for 6 days to facilitate the differentiation into macrophages. On day 6, cells are harvested for qPCR and analyzed for M1 marker (IL-1β), M2 marker (CD206) and TBP which acts as a housekeeping control.

### Example 3- HSD/HFD models and related data

### Activity in High Fat Diet (HFD) Fed Rats

In order to investigate the relationship between dose and exposure resulting in changes in clinical activity, a test compound (compound A) was evaluated in a HFD rat model. The HFD model is widely used in preclinical animal models to induce hepatic steatosis as one key pathological component of NASH (Takahashi, Y., Soejima, Y., and Fukusato, T. (2012). Animal models of nonalcoholic fatty liver disease/nonalcoholic steatohepatitis. World J. Gastroenterol. 18, 2300-2308). Steatosis is caused by the accumulation of triglycerides (TG) in the liver, and as such, decreases in TG may represent a potential marker for activity on steatosis. The obese phenotype of HFD model is dependent on the incorporation of high dietary fat and DNL-derived fat. Since these animals have large amounts of exogenous fat from the diet, this model represents a high bar for success and activity in this model may demonstrate the potential importance of FASN activity as a key mediator of steatosis. The key endpoints of this study were to evaluate the relationship between dose schedule and exposure with changes in liver steatosis score and liver TG. For these studies, male SD rats (6-8 week old) were fed a high fat diet (D124942, Research Diets Inc.) for four weeks to develop the obese phenotype and then sorted into treatment groups (N = 8) based on mean body weight. The rats were treated orally with compound A or vehicle (9:1 PEG400:EtOH) for four weeks. Rosiglitazone 5 mg/kg QD was included as a positive control. Plasma and serum samples were collected weekly throughout the study. After 28 days of treatment, animals were sacrificed and liver and plasma samples were collected. Livers were fixed in formalin, processed for H&E staining, and evaluated by a pathologist specializing in NASH.

Two HFD studies were conducted. The first HFD study was designed to demonstrate preliminary activity of compound A treatment on markers of liver steatosis. The second HFD study investigated the endpoints of steatosis and liver and plasma markers of lipogenesis (including TG and FFA).

In the first HFD study, animals were treated with vehicle, Rosiglitazone, and compound A (0.6, 2, 6 mg/kg QD and 1 mg/kg BID; N = 8 animals per treatment group) Dose levels were adjusted after the first 14 days of treatment as indicated.

A lower range of dose levels of compound A in established obese HFD rats was evaluated in HFD study 2. Rats were orally administered compound A at 0.125, 0.4 and 1.2 mg/kg QD for 4 weeks. This study also investigated dose ranging on both a BID (0.2 and 0.6 mg/kg BID) and Q2D (2.4 and 12 mg/kg every other day) schedules in order to assess the impact of schedule on the activity of compound A.

In both HFD studies, liver TG were significantly elevated compared to normal diet controls, consistent with accumulation of TG in the liver and the presence of steatosis in this disease model. The effect of compound A on steatosis was determined by evaluating liver histology post treatment. An independent pathologist evaluated liver specimens in a blinded manner. Notably, a significant decrease in the degree and severity of liver steatosis was observed for all QD treatment groups, including the 0.125 mg/kg QD dose cohort. While the greatest decrease in steatosis score was observed at 1.2 mg/kg QD, no clear dose-dependency for this endpoint was observed (Figure 1). A reduction in liver steatosis was also observed in the 0.6 mg/kg QD treatment group from HFD study 1 (data not shown). Although steatosis is primarily driven by dietary fat in the HFD model, inhibition of DNL by compound A showed clear benefit in improving steatosis and may have therapeutic benefit in NASH. In addition, the lowest dose of compound A tested (0.125 mg/kg; AUC: 3,228 hr^{∗}ng/mL) was potentially as effective as doses and exposures up to 10-fold higher. Of note, the 0.125 mg/kg AUClast is 9-fold and 12-fold less than the AUClast of the NOAEL dose determined in the Rat DRF and Rat MTD study, respectively.

No increased inhibition of steatosis by compound A was seen on BID dosing schedules as compared to equivalent daily doses (and similar exposures). By contrast, steatosis inhibition was reduced with every other day dosing (Figure 1).

Steatosis is caused by the accumulation of TG in the liver, and as such, decreases in TG may represent a potential marker for steatosis. In the HFD model, liver TG were significantly elevated in HFD controls compared to normal diet controls, consistent with accumulation of TG in the liver and the presence of steatosis in this disease model. Decreases in liver TG were demonstrated in all QD dosing groups and, consistent with the steatosis scores, no clear dose-dependent effect was observed (Table 3.1). The lack of a dose-dependent response of compound A on liver TG may be due to the variation within the model and the smaller sample size, but also reflects a significant activity of Compound A at low doses and exposures in the livers of HFD animals. By contrast, plasma TG levels were not significantly elevated in this model, when compared to normal diet controls; however, a dose-dependent decrease in plasma TG was demonstrated with daily dosing (Table 3.1).

**Table 3.1. Effects of compound A on plasma and liver TG from HFD rat model. The data are presented as Mean ± SEM.**

| Group | Plasma TG | | Liver TG | |
|---|---|---|---|---|
| | Raw Data (mmol/L) | % Decrease of treatment group (mean) vs. vehicle treated HFD group (mean) | Raw Data (µmol/g) | % Decrease of treatment group (mean) vs. vehicle treated HFD group (mean) |
| | Day 14/ Day 26 (Mean ± SEM) | Day 14 /Day 26 | Day 28 (Mean ± SEM) | Day 28 |
| HFD + vehicle | 1.01 ± 0.12 / 1.15 ± 0.09 | 0.0 / 0.0 | 57.3 ± 7.7 | 0.0 |
| HFD + 5 mg/kg QD Rosiglitazone | 0.48 ± 0.02 / 0.46 ± 0.05 * | 52.5 ± 60.0 | 17.2 ± 0.8 * | 70.0 |
| HFD + 0.125 mg/kg QD Compound A | 0.85 ± 0.16 / 0.95 ± 0.14 | 15.8 / 17.4 | 38.0 ± 4.3 * | 33.7 |
| HFD + 0.4 mg/kg QD Compound A | 0.72 ± 0.09 / 0.85 ± 0.11 | 28.7 / 26.1 | 48.2 ± 8.4 | 15.9 |
| HFD + 1.2 mg/kg QD Compound A | 0.74 ± 0.08 / 0.66 ± 0.10 * | 26.7/42.6 | 42.0 ± 5.3 | 26.7 |
| HFD + 0.2 mg/kg BID Compound A | 0.76 ± 0.13 / 0.96 ± 0.19 | 24.8 / 16.5 | 28.0 ± 4.6 * | 51.1 |
| HFD + 0.6 mg/kg BID Compound A | 0.70 ± 0.07 / 0.71 ± 0.07 * | 30.7/38.3 | 35.5 ± 3.8 * | 38.0 |
| HFD + 2.4 mg/kg Q2D Compound A | 0.93 ± 0.12 / 0.99 ± 0.11 | 7.9/13.9 | 52.0 ± 4.8 | 9.2 |
| HFD + 12 mg/kg Q2D Compound A | 0.55 ± 0.07 / 0.59 ± 0.06 * | 45.5 / 48.7 | 35.2 ± 7.2 | 38.6 |
| Normal diet + vehicle | 0.98 ± 0.15 / 1.15 ± 0.14 | | 10.4 ± 0.9 * | |

| | | | | |
|---|---|---|---|---|
| *P<0.05 relative to vehicle treated group. | | | | |

**Table 3.2. Effect of compound A on plasma leptin from HFD rat model. The data are presented as Mean ± SEM. Plasma samples were collected on Day 28.**

| Group | Plasma Leptin (ng/mL) |
|---|---|
| HFD + vehicle | 16.5 ± 4.3 |
| HFD + 5 mg/kg QD Rosiglitazone | 16.0 ± 1.2 |
| HFD + 0.125 mg/kg QD Compound A | 12.8 ± 1.3 |
| HFD + 0.4 mg/kg QD Compound A | 9.7 ± 1.4 |
| HFD + 1.2 mg/kg QD Compound A | 8.4 ± 0.9 |
| HFD + 0.2 mg/kg BID Compound A | 7.8 ± 1.7 |
| HFD + 0.6 mg/kg BID Compound A | 5.3 ± 1.1 |
| HFD + 2.4 mg/kg Q2D Compound A | 11.0 ± 1.3 |
| HFD + 12 mg/kg Q2D Compound A | 2.5 ± 0.3 |
| Normal diet + vehicle | 5.3 ± 0.4 |

### Example 4. Activity in High-Sucrose Diet (HSD) Fed Rats

A HSD represents an alternative experimental model for obesity and steatosis. In this model, 60-70% of the caloric intake is from simple carbohydrates, and thus, the primary source of increased TG is through DNL. Inhibition of FASN and, thus, DNL by compound A would be expected to have a significant impact on TG production and steatosis in this model.

The goal of the HSD study was to evaluate the in vivo potency of compound A in blocking FA synthesis to reduce fat deposition in liver as monitored by steatosis scores, as well as changes in liver and plasma TG and liver FFA. An additional single administration PK/PD study in HSD rats was also used to determine the relationship between the induction of liver and plasma malonyl-carnitine and compound A in this diseased model.

Male SD rats (6-8 week old) were fed a HSD (D10001, Research Diets Inc.) for six weeks to develop the obese phenotype and then sorted into treatment groups (N = 8 each) based on mean body weight. Rats were treated with 0.15, 0.3, 0.6, or 1.2 mg/kg Compound A or vehicle (9:1 PEG400:EtOH) orally once daily for four weeks and with a single administration of 0.3, 1.2 and 3 mg/kg compound A or vehicle.

In line with the minimal degree of steatosis in this model, the HSD vehicle treatment group had significantly lower liver TG levels as compared to HFD vehicle group at the end of the study (14.5 ± 2.0 µmol/g in HSD vs. 57.3 ± 7.7 µmol/g in HFD). Treatment with compound A with doses as low as 0.3 mg/kg resulted in reduction of liver TG to levels near those observed in normal diet animals, consistent with DNL as the primary source of excess TG in this model (Table 4.1). Similarly, plasma TG were reduced following compound A treatment (up to 30%) as compared to vehicle controls in a dose-dependent manner following 28 days of treatment. Notably, in this model, an increased reduction of plasma TG were observed with 28 days of treatment as compared to 14 days.

**Table 4.1. Effect of compound A on plasma and liver triglycerides from HSD rat model. The data are presented as Mean ± SEM.**

| Group | Plasma TG | | Liver TG | |
|---|---|---|---|---|
| | Raw Data (mmol/L) | % Decrease of treatment group (mean) vs. vehicle treated HSD group (mean) | Raw Data (µmol/g) | % Decrease of treatment group (mean) vs. vehicle treated HSD group (mean) |
| | Day 14 / Day 26 (Mean ± SEM) | Day 14 / Day 26 | Day 28 (Mean ± SEM) | Day 28 |
| HSD + vehicle | 2.75 ± 0.35 /2.62 ± 0.27 | 0.0 / 0.0 | 14.5 ± 2.0 | 0.0 |
| HSD + 5 mg/kg QD Rosiglitazone | 1.07 ± 0.10 / 1.24 ± 0.17 | 60.6 ± 62.3 | 8.5 ± 1.5 | 41.2 * |
| HSD + 0.15 mg/kg QD Compound A | 2.29 ± 0.45 / 2.17 ± 0.49 | 15.3 / 16.4 | 13.5 ± 1.7 | 7.2 |
| HSD + 0.3 mg/kg QD Compound A | 2.22 ± 0.29 / 2.16 ± 0.26 | 17.8 / 16.8 | 9.5 ± 1.1 | 34.2 * |
| HSD + 0.6 mg/kg QD Compound A | 2.30 ± 0.23 / 1.99 ± 0.33 | 14.9 / 23.4 | 9.7 ± 1.0 | 33.4 * |
| HSD + 1.2 mg/kg QD Compound A | 1.94 ± 0.25 / 1.85 ± 0.32 | 28.3 / 28.9 * | 9.5 ± 1.3 | 34.7 * |
| Normal diet + vehicle | NA / 1.39 ± 0.13 | | 8.4 ± 0.6 | |

| | | | | |
|---|---|---|---|---|
| *P<0.05 relative to vehicle treated group | | | | |

Similar to the HFD model, compound A reduced plasma leptin levels to levels at or near those observed in normal diet control animals (Table 4.2).

**Table 4.2. Effect of compound A on plasma leptin from HSD rat model. Plasma samples were taken at Day 28. The data were presented as Mean ± SEM.**

| Group | Plasma Leptin (ng/mL) |
|---|---|
| HSD + vehicle | 10.4 ± 1.0 |
| HSD + 5 mg/kg QD Rosiglitazone | 12.6 ± 2.5 |
| HSD + 0.15 mg/kg QD Compound A | 8.1 ± 1.9 |
| HSD + 0.3 mg/kg QD Compound A | 8.6 ± 0.9 |
| HSD + 0.6 mg/kg QD Compound A | 8.2 ± 1.3 |
| HSD + 1.2 mg/kg QD Compound A | 8.9 ± 0.9 |
| Normal diet + vehicle | 8.8 ± 0.8 |

### Example 5. Rat stellate cell

In addition to IL-17-mediated fibrosis, palmitate-induced lipid accumulation in hepatocytes can release factors that promote the activation and proliferation of hepatic stellate cells (HSC). HSC are central mediators of liver fibrosis. When liver damage occurs, HSC change from a quiescent to activated state. Activated HSC secretes extracellular matrix and fibrogenic cytokines, which lead to fibrosis and cirrhosis. To begin to assess the role of DNL as a potential pathophysiological link between hepatic steatosis and fibrosis, we evaluated the effect of compound A on proliferation of activated rat HSC in primary cell culture. Following 72-hour treatment, compound A inhibited proliferation of rat HSC in a concentration-dependent manner with IC50 of 0.42 µM. These data support the potential for compounds of the invention to reduce liver fibrosis by blocking the proliferation of activated HSC, in addition to mitigating TH17-mediated fibrosis.

### Example 6. EAE data

To demonstrate compound A has anti-inflammatory properties in vivo and investigate the relationship between inhibition of FASN and TH17-mediated T cell responses, the myelin oligodendrocyte glycoprotein (MOG)-induced mouse model of experimental autoimmune encephalomyelitis (EAE) was evaluated. In this model, animals are immunized with MOG (35-55) peptide in combination with pertussis toxin, which results in the breakdown of the blood-brain barrier and promotes infiltration of T cells into the CNS. Autoreactive IL-17-producing TH17 cells are highly potent at inducing CNS immune pathology. Importantly, inhibition of the DNL pathway, either by using T cell-specific deletion of the ACC1 gene or pharmacological inhibitor of ACC1/2, led to significant reduction in TH17 biomarkers and disease pathology in this model. Therefore, the EAE model is a validated and mechanistic relevant animal model to assess the anti-inflammatory potential of compound A.

In the mouse EAE model, C57BL6 mice (8-10 weeks old female) were immunized subcutaneously with MOG (35-55) on Day 1, followed by intravenous (IV) injection of pertussis toxin on Days 1 and 3. Starting on Day 4 until Day 29, mice were treated with vehicle, prednisolone (10 mg/kg QD), or compound A (N = 12 per group) at the indicated doses and schedule. Clinical symptoms of EAE were scored in a blinded manner daily and recorded as the mean daily clinical score. On Day 29, animals were euthanized, and tissues were collected for evaluation for evidence of inflammatory cell infiltration into the tissues of CNS. To confirm target engagement, liver samples were collected for measurement of malonyl-carnitine levels. In addition, to demonstrate PD effects pertinent to TH17, splenic mRNAs were measured for expression of mRORc, which encodes key transcription factor RAR-related orphan receptor gamma, thymus-specific isoform (RORγT) essential for murine TH17 cell differentiation.

Two mouse EAE studies were conducted. The first study was designed to assess the anti-inflammatory effects of compound A in terms of reducing EAE clinical scores and pathology at the conclusion of the study on Day 29. In addition, compound A exposure, PD and clinical activity relationships were assessed. To better demonstrate PD effects of compound A on TH17 biomarkers in the relevant tissues, a second EAE study was conducted in which lymph nodes and CNS tissues were analyzed for the presence of IL-17+ CD4+ cells at the peak of the disease (based on clinical scores) on Day 15.

In EAE Study 1, the impacts of compound A on EAE clinical score and pathology were assessed at doses of 2, 6, and 20 mg/kg QD and at 1, 3 and 10 mg/kg. BID.

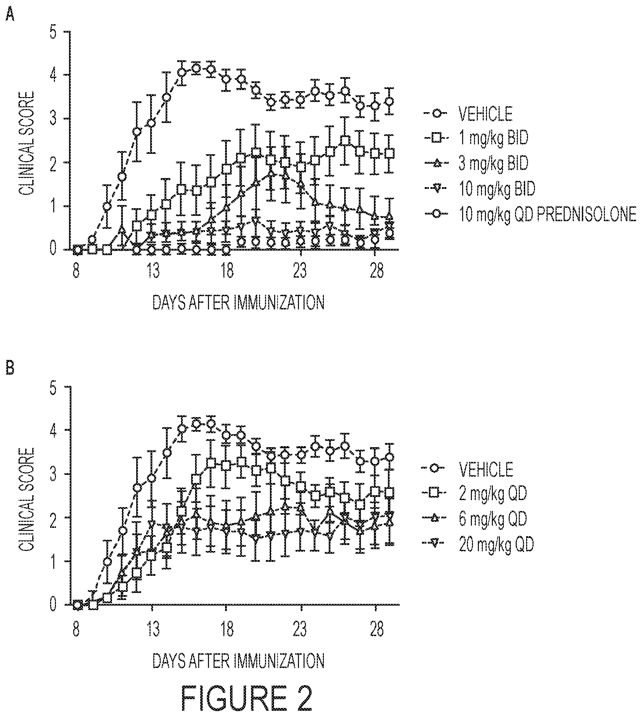

Compound A had a significant and dose-dependent impact on clinical scores as measured by both the onset and severity of the disease compared to the vehicle group. Notably, BID regimens were significant in improving clinical scores as compared to the vehicle group (p<0.05; 2-way RM-ANOVA) (Figure 2) while QD regimens lowered scores but did not reach statistical significance.

**Table 6.1. Clinical scoring criteria for EAE model.**

| | |
|---|---|
| 0 | No weakness whatsoever; mouse has full tail strength and will fully resist turning onto back |
| 0.5 | Distal limp or spastic, curling tail: When holding mouse 2/3 of the way down the tail towards the tip, lower half of the mouse flips completely onto back and front paws stay in place. Animal rights himself easily. Partial tail limpness/weakness. |
| 1 | Complete tail limpness/paralysis. |
| 1.5 | Mouse is easy to turn but also rights himself easily and quickly. When applying minimal pressure to the tail, the mouse will make a complete rotation, not stopping on his back. Complete tail paralysis. |
| 2 | Mouse easily flips onto back and struggles to turn back onto feet, but there is no hind limb weakness. Lands ready to run. |
| 2.5 | Mouse easily flips onto back and struggles to turn back onto feet with some hind limb weakness (Mild hind limb weakness). The animal will slip a little when dropped from above the bedding, and the mouse may stumble slightly when walking around cage. |
| 3 | Mouse easily flips onto back and is only able to right himself with great effort. Moderate hind limb weakness will be obvious; mouse will hobble around the cage with a wobbly gait. His feet will remain tucked close under the body, and the hind end of the mouse will be very close to the ground. |
| 3.5 | Severe hind limb weakness. Mouse is still able to bring feet fully forward with the hind footpads not resting flat on the bedding. The animal will walk around the cage on toes with a very wobbly gait. |
| 4 | The mouse is still able to bring feet fully forward with the hind footpads resting flat on the bedding. Feet will drag and pickup. |
| 4.5 | Partial hind limb paralysis. The mouse will drag one or both feet behind his body, but will still be able to move the feet. |
| 5 | Complete hind limb paralysis. Mouse can only move around the cage with his forelimbs; the hind legs and back of body will drag on the bedding. There is no |
| | movement in the hind feet and toes whatsoever. |
| 5.5 | Complete hind limb paralysis, mild front limb weakness. The mouse will most likely be found on his side, unable to right himself and unable to move about the cage. |
| 6 | Dead. |

The impact of treatment on the disease states in the EAE model is characterized by degree of inflammatory cell infiltration into tissues of the CNS. To determine whether the reduced clinical scores in compound A-treated groups were associated with a reduction of CNS inflammation, demyelination in the spinal cords and CNS inflammation of treated animals were by evaluated. The spinal cords of compound A-treated animals showed significantly reduced visible signs of demyelinating lesions or inflammation as judged cellular infiltrates by Luxol fast blue staining and H&E staining (Table 6.2). Reductions in brain and spinal cord inflammation corresponded with the clinical scores, and were observed with compound A at doses as low as 1 mg/kg BID, and achieved statistical significance at 3 and 10 mg/kg BID.

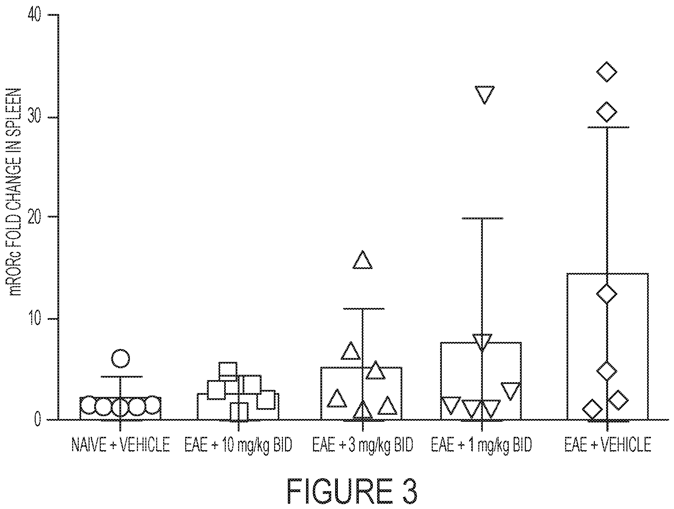

Finally, the effects of compound A on TH17 cells, which are important for the development of EAE, were examined by measuring splenic mRORc mRNA levels as a biomarker for TH17, by quantitative TaqMan RT-PCR analysis. Compound A markedly reduced the gene expression level of splenic mRORc in a dose-dependent manner. In summary, in the EAE study 1 compound A was effective in reducing EAE clinical scores and CNS inflammation, which corroborated with reduced splenic TH17, supporting the notion that targeting FASN will impact TH17-mediated T cell responses in vivo (Figure 3).

**Table 6.2. Effect of compound A on spinal cord demyelination, spinal cord inflammation and brain inflammation in EAE model. Data were presented as Mean ±SD. *P<0.05 relative to MOG-vehicle group. The grading scales for each measurement are listed in Table 6.3.**

| Group | Spinal Cord Demyelination | Spinal Cord Inflammation | Brain Inflammation |
|---|---|---|---|
| Naive Vehicle | 0.0 ± 0.0 * | 0.0 ± 0.0 * | 0.0 ± 0.0 * |
| MOG-Prednisolone | 0.2 ± 0.5 * | 0.4 ± 0.5 * | 0.7 ± 0.9 * |
| MOG-20 mg/kg QD Compound A | 1.0 ± 1.0 | 1.1 ± 0.7 | 1.0 ± 0.9 |
| MOG-6 mg/kg QD Compound A | 1.2 ± 0.8 | 1.1 ± 0.6 | 2.2 ± 1.0 |
| MOG-2 mg/kg QD Compound A | 1.4 ± 0.7 | 1.4 ± 0.6 | 2.3 ± 0.8 |
| MOG-10 mg/kg BID Compound A | 0.5 ± 0.6 * | 0.7 ± 0.7 * | 1.7 ± 1.0 * |
| MOG-3 mg/kg BID Compound A | 0.6 ± 0.7 * | 0.8 ± 0.6 * | 1.3 ± 1.1 * |
| MOG-1 mg/kg BID Compound A | 1.1 ± 0.8 | 1.2 ± 0.6 | 2.1 ± 0.6 |
| MOG-Vehicle | 1.6 ± 0.7 | 1.4 ± 0.5 | 2.7 ± 0.7 |

**Table 6.3. Evaluation of Spinal cord and brain inflammation for EAE model.**

| **Grading Scale for EAE Model Spinal Cord Demyelination (Luxol Fast Blue)** | |
|---|---|
| 0 | (NVL) No demyelination observed |
| 1 | (Minimal) Demyelination affects < 10% of myelinated cross sectional area |
| 2 | (Slight) Demyelination affects ∼10-30% of myelinated cross sectional area |
| 3 | (Moderate) Demyelination affects ∼30-60% of myelinated cross sectional area |
| 4 | (Marked) Demyelination affects ∼60-90% of myelinated cross sectional area |
| 5 | (Severe) Demyelination affects >90% of myelinated cross sectional area |

| **Grading Scale for EAE Model Spinal Cord Inflammation (H&E)** | |
|---|---|
| 0 | (NVL) No inflammation observed1 |
| 1 | (Minimal) Inflammation affects < 10% of cross sectional area |
| 2 | (Slight) Inflammation affects ∼10-30% of cross sectional area |
| 3 | (Moderate) Inflammation affects ∼30-60% of cross sectional area |
| 4 | (Marked) Inflammation affects ∼60-90% of cross sectional area |
| 5 | (Severe) Inflammation affects >90% of cross sectional area |

| **Grading Scale for EAE Model Brain Inflammation (H&E)** | |
|---|---|
| 1 | Inflammation confined to the meninges |
| 2 | Minimal focal to multifocal infiltration of brain tissue |
| 3 | Slight infiltration of brain tissue |
| 4 | Moderate infiltration of brain tissue |
| 5 | Marked infiltration of brain tissue |

In the second study, to better assess the impact of compound A treatment on TH17 responses and in relevant tissues, lymph nodes and spinal cords were collected for flow cytometry analysis of IL-17+ CD4+ cells at the peak of the EAE disease. The EAE disease state was induced in animals as described above and animals were sacrificed on Day 15. Mice were treated on a BID schedule with vehicle control or compound A at 0.3, 1, 3, and 10 mg/kg from Day 4 to Day 15.

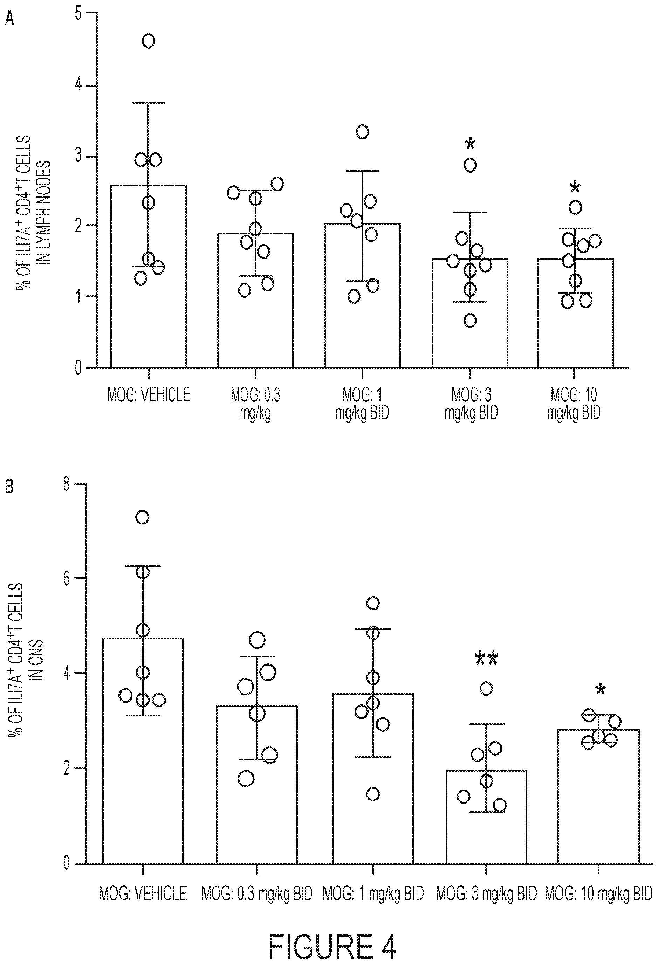

Compound A reduced the population of IL-17+ CD4+ T cells in lymph nodes and CNS at doses as low as 0.3 mg/kg BID and achieved statistical significance at 3 and 10 mg/kg BID (Figure 4). These data are consistent with the role for FASN in TH17 cell development and indicate pharmacological inhibition of FASN could be an effective means to reduce the TH17-driven inflammatory responses, such as those reported in NASH patients.

In summary, compound A reduced disease progression and severity in the mouse EAE model, with evidence of decreased CNS inflammation as well as systemic PD effects on TH17 relevant biomarkers. Evidence for this came through reduced gene expression of mRORc in spleen, decreased IL-17+ and CD4+ T cells in lymph nodes and CNS tissues. Given the role of inflammatory responses in NASH pathogenesis, these data lend further support for the therapeutic utility of compound A, at least by dampening TH17-driven inflammation and potentially the resultant fibrosis.

## Claims

1. A FASN inhibitor for use in a method of treating non-alcoholic steatohepatitis (NASH), whereby the FASN inhibitor reduces steatosis, TH17-dependent inflammation, and fibrosis, wherein said method comprises administering said FASN inhibitor to said patient, and wherein the FASN inhibitor is: 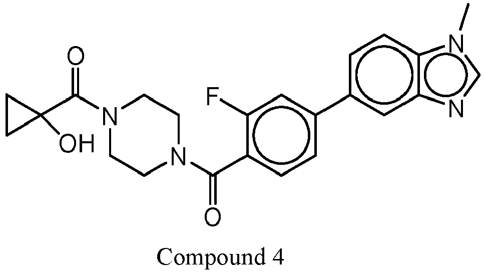 or a pharmaceutically acceptable salt thereof.

## Patentansprüche

1. FASN-Inhibitor zur Verwendung in einem Verfahren zur Behandlung von nichtalkoholischer Steatohepatitis (NASH), wobei der FASN-Inhibitor Steatose, TH17-abhängige Entzündung und Fibrose reduziert, wobei das Verfahren die Verabreichung des FASN-Inhibitors an den Patienten umfasst und wobei es sich bei dem FASN-Inhibitor um: 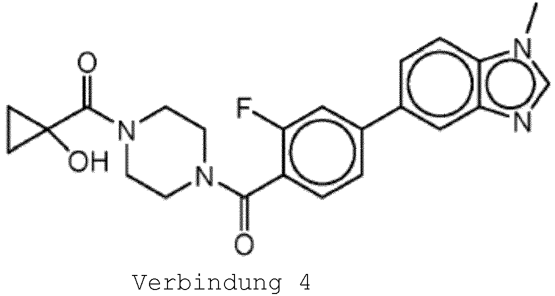 oder ein pharmazeutisch unbedenkliches Salz davon handelt.

## Revendications

1. Inhibiteur de FASN destiné à être utilisé dans un procédé de traitement de la stéato-hépatite non alcoolique (NASH), moyennant quoi l'inhibiteur de FASN réduit la stéatose, l'inflammation dépendante des TH17 et la fibrose, dans lequel ledit procédé comprend une administration dudit inhibiteur de FASN au dit patient, et l'inhibiteur de FASN étant : 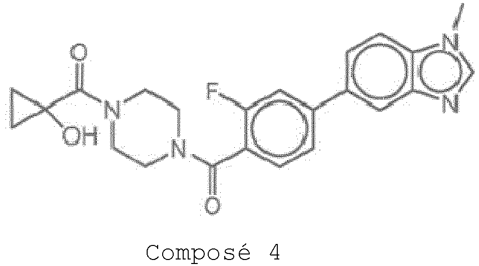 ou un sel de celui-ci acceptable d'un point de vue pharmaceutique.
